# EUROPEAN PATENT APPLICATION

(11) **EP 4 686 757 A1**
(43) Date of publication of application: **04.02.2026**
(21) Application number: 24192162.6
(22) Date of filing: 31.07.2024
(51) Int. Cl.: C12N 15/113, A61K 31/713

(54) **INHIBITORS OF EXPRESSION AND/OR FUNCTION**

(71) Applicant: e-therapeutics PLC, London W2 6BD (GB)
(72) Inventor: The designation of the inventor has not yet been filed
(74) Representative: CMS Cameron McKenna Nabarro Olswang LLP

(57) **Abstract**

The present invention relates to inhibitors, and compositions containing inhibitors, and uses of the same in the treatment or prevention of a metabolic disease or disorder, such as a metabolic disease or disorder associated with non-alcoholic fatty liver disease (NAFLD) and/or obesity and/or a disease or disorder associated with adipogenesis.

## Description

### FIELD

The present invention provides inhibitors, such as nucleic acid compounds, such as siRNAs, suitable for therapeutic use. Additionally, the present invention provides methods of making these compounds, as well as methods of using such compounds for the treatment of various diseases and conditions.

### BACKGROUND OF THE INVENTION

Inhibitors, such as oligonucleoside / oligonucleotide compounds which are inhibitors of gene expression and/or expression or function of other targets such as LNCRNAs, can have important therapeutic applications in medicine. Oligonucleotides/ oligonucleosides can be used to silence genes that are responsible for a particular disease. Gene-silencing prevents formation of a protein (or other gene product such as a regulatory non-coding RNA) by inhibiting translation. Importantly, gene-silencing agents are a promising alternative to traditional small, organic compounds that inhibit the function of the protein (or gene product) linked to the disease. siRNA, antisense RNA, and micro-RNA are oligonucleoside /oligonucleotides that prevent the formation of proteins by gene-silencing.

A number of modified siRNA compounds in particular have been developed in the last two decades for diagnostic and therapeutic purposes, including siRNA / RNAi therapeutic agents for the treatment of various diseases including central-nervous-system diseases, inflammatory diseases, metabolic disorders, oncology, infectious diseases, and ocular diseases.

The present invention relates to inhibitors, such oligomers e.g. nucleic acids, e.g. oligonucleoside /oligonucleotide compounds, and their use in the treatment and / or prevention of disease.

The *SLC25A5* gene belongs to the ANT gene family, which itself belongs to the superfamily that includes genes encoding brown fat mitochondrial uncoupling proteins and mitochondrial phosphate carrier proteins. This gene is a member of the mitochondrial carrier subfamily of solute carrier protein genes. The product of this gene, adenine nucleotide translocator 2 (ANT2), functions as a major constituent of the mitochondrial permeability-transition pore complex and catalyses the exchange of mitochondrial ATP with cytosolic ADP. As a result of its antiporter function, ANT2 maintains mitochondrial membrane potential by regulating ADP/ATP ratios in oxidative phosphorylation. ANT2 facilitates uncoupling of the mitochondrial membrane when acylated by SIRT4. Though uncoupling the membrane potential typically leads to apoptosis, ANT2 was found to be antiapoptotic. As a result, it is postulated to mediate the TFIIH-dependent response to DNA damage as a component of the MMS 19-XPD.

### STATEMENTS OF INVENTION

The invention is defined as in the claims and relates to, *inter alia*:
In one aspect, the invention relates to an inhibitor of expression and / or function of SLC25A5/ANT2, wherein said inhibitor is conjugated to one or more ligand moieties.

In a further aspect, the invention relates to an inhibitor according to the invention, wherein said inhibitor is an siRNA oligomer.

In another aspect, the invention relates to an inhibitor of expression and / or function of SLC25A5/ANT2, wherein said inhibitor is an siRNA oligomer.

In a further aspect, the invention relates to an inhibitor according to the invention, wherein said inhibitor comprises an siRNA oligomer conjugated to one or more ligand moieties.

In a further aspect, the invention relates to an inhibitor according to the invention, for use in prevention and/or treatment of metabolic disease or disorder, such as a metabolic disease or disorder associated with non-alcoholic fatty liver disease (NAFLD) and/or obesity and/or a disease or disorder associated with adipogenesis and/or for use in reducing adipogenesis.

In a further aspect, the invention relates to an inhibitor for use according to the invention, wherein the inhibitor is to be used in combination with a GLP-1 agonist and/or a THR-beta agonist.

In a further aspect, the invention relates to an inhibitor for use according to the invention, wherein the inhibitor is to be used in combination with a GLP-1 agonist and/or an FGF-21 analogue.

In a further aspect, the invention relates to an inhibitor for use according to the invention, wherein the GLP-1 agonist is a GLP-1/GIP dual agonist, a GLP-1/FGF21 dual agonist, a GLP-1/GCGR dual agonist, or a GLP-1/GIP/GCGR triple agonist.

In a further aspect, the invention relates to an inhibitor for use according to the invention, wherein the GLP-1 agonist is semaglutide.

In a further aspect, the invention relates to an inhibitor for use according to the invention, wherein the THR-beta agonist is resmetirom.

In a further aspect, the invention relates to an inhibitor for use according to the invention, wherein the GLP-1/GIP dual agonist is tirzepatide.

In a further aspect, the invention relates to an inhibitor for use according to the invention, wherein the FGF-21 analogue is efruxifermin.

In a further aspect, the invention relates to an inhibitor for use according to the invention, wherein the inhibitor is to be used in combination with one or more of: an amylin receptor agonist (such as pramlintide), and/or a dual amylin + calcitonin receptor agonist, and/or a glucagon receptor agonist, and/or an FXR receptor agonist (such as cilofexor or obeticholic acid), and/or an FGF-21 analogue or FGF-21 receptor agonist (such as efruxifermin), and/or an FGF-19 analogue or FGF-19 receptor agonist (such as aldafermin), and/or a galectin 3 inhibitor (such as belapectin), and/or a PPARα agonist (such as elafibrinor), and/or a PPARγ agonist (such as pioglitazone or rosiglitazone), and/or a mixed PPARα and/or δ and/or γ agonist, and/or a pan PPAKαγδ agonist (such as lanafibranor), and/or an acetyl CoA desaturase activator, and/or an ASK1 inhibitor (such as selonsertib), and/or an LOXL2 inhibitor (such as simtuzumab), and/or a dual CCR2/5 inhibitor (such as cenicriviroc), and/or an inhibitor of an enzyme in the de novo lipogenesis (DNL) pathway including citrate/isocitrate carrier (CIC), ATP-citrate lyase (ACLY), acetyl-CoA carboxylase (ACC) and fatty acid synthase (FAS), and/or an inhibitor of an enzyme in the cholesterol biosynthesis pathway (such as an HMGCoA reductase inhibitor, such as atorvastatin).

In a further aspect, the invention relates to an inhibitor according to the invention, wherein said one or more ligand moieties comprise one or more GalNAc ligands or comprise one or more GalNAc ligand derivatives.

In a further aspect, the invention relates to an inhibitor for use according to the invention, wherein said one or more ligand moieties comprise one or more GalNAc ligand derivatives.

In a further aspect, the invention relates to an inhibitor for use according to the invention, wherein the target of the inhibitor is SLC25A5/ANT2.

In a further aspect, the invention relates to an inhibitor or an inhibitor for use according to the invention, which is an siRNA oligomer having a first and a second strand wherein:
i) the first strand of the siRNA has a length in the range of 15 to 30 nucleosides, preferably 19 to 25 nucleosides, more preferably 23 or 25; even more preferably 23; and / or
ii) the second strand of the siRNA has a length in the range of 15 to 30 nucleosides, preferably 19 to 25 nucleosides, more preferably 21 nucleosides.

In a further aspect, the invention relates to an inhibitor or an inhibitor for use according to the invention, wherein the second sense strand further comprises one or more abasic nucleosides in a terminal region of the second strand, and wherein said abasic nucleoside(s) is / are connected to an adjacent nucleoside through a reversed internucleoside linkage.

In a further aspect, the invention relates to an inhibitor or an inhibitor for use according to the invention, wherein the second strand comprises:
i) 2, or more than 2, abasic nucleosides in a terminal region of the second strand; and / or
ii) 2, or more than 2, abasic nucleosides in either the 5' or 3' terminal region of the second strand; and / or
iii) 2, or more than 2, abasic nucleosides in either the 5' or 3' terminal region of the second strand, wherein the abasic nucleosides are present in an overhang as herein described; and / or
iv) 2, or more than 2, consecutive abasic nucleosides in a terminal region of the second strand, wherein preferably one such abasic nucleoside is a terminal nucleoside; and / or
v) 2, or more than 2, consecutive abasic nucleosides in either the 5' or 3' terminal region of the second strand, wherein preferably one such abasic nucleoside is a terminal nucleoside in either the 5' or 3' terminal region of the second strand; and / or
vi) a reversed internucleoside linkage connects at least one abasic nucleoside to an adjacent basic nucleoside in a terminal region of the second strand; and / or
vii) a reversed internucleoside linkage connects at least one abasic nucleoside to an adjacent basic nucleoside in either the 5' or 3' terminal region of the second strand; and / or
viii) an abasic nucleoside as the penultimate nucleoside which is connected via the reversed linkage to the nucleoside which is not the terminal nucleoside (called the antepenultimate nucleoside herein); and / or
ix) abasic nucleosides as the 2 terminal nucleosides connected via a 5'-3' linkage when reading the strand in the direction towards that terminus;
x) abasic nucleosides as the 2 terminal nucleosides connected via a 3'-5' linkage when reading the strand in the direction towards the terminus comprising the terminal nucleosides;
xi) abasic nucleosides as the terminal 2 positions, wherein the penultimate nucleoside is connected via the reversed linkage to the antepenultimate nucleoside, and wherein the reversed linkage is a 5-5' reversed linkage or a 3'-3' reversed linkage;
xii) abasic nucleosides as the terminal 2 positions, wherein the penultimate nucleoside is connected via the reversed linkage to the antepenultimate nucleoside, and wherein either
   (1) the reversed linkage is a 5-5' reversed linkage and the linkage between the terminal and penultimate abasic nucleosides is 3'5' when reading towards the terminus comprising the terminal and penultimate abasic nucleosides; or
   (2) the reversed linkage is a 3-3' reversed linkage and the linkage between the terminal and penultimate abasic nucleosides is 5'3' when reading towards the terminus comprising the terminal and penultimate abasic nucleosides.

In a further aspect, the invention relates to an inhibitor or an inhibitor for use according to the invention, wherein the reversed internucleoside linkage is at a terminal region which is distal to the 5' terminal region of the second strand, or at a terminal region which is distal to the 3' terminal region of the second strand.

In a further aspect, the invention relates to an inhibitor or an inhibitor for use according to the invention, wherein the reversed internucleoside linkage is a 3'3 reversed linkage.

In a further aspect, the invention relates to an inhibitor or an inhibitor for use according to the invention, wherein the reversed internucleoside linkage is a 5'5 reversed linkage.

In a further aspect, the invention relates to an inhibitor or an inhibitor for use according to the invention, wherein one or more nucleosides on the first strand and / or the second strand is / are modified, to form modified nucleosides.

In a further aspect, the invention relates to an inhibitor or an inhibitor for use according to the invention, wherein the modification is a modification at the 2'-OH group of the ribose sugar, optionally selected from 2'-Me or 2'-F modifications.

In a further aspect, the invention relates to an inhibitor or an inhibitor for use according to the invention, wherein the first strand comprises a 2'-F at any of position 14, position 2, position 6, or any combination thereof, counting from position 1 of said first strand.

In a further aspect, the invention relates to an inhibitor or an inhibitor for use according to the invention, wherein the second strand comprises a 2'-F modification at position 7 and / or 9, and / or 11 and / or 13, counting from position 1 of said second strand.

In a further aspect, the invention relates to an inhibitor or an inhibitor for use according to the invention, wherein the first and second strand each comprise 2'-Me and 2'-F modifications.

In a further aspect, the invention relates to an inhibitor or an inhibitor for use according to the invention, which is an siRNA, wherein the siRNA comprises at least one thermally destabilizing modification, suitably at one or more of positions 1 to 9 of the first strand counting from position 1 of the first strand, and / or at one or more of positions on the second strand aligned with positions 1 to 9 of the first strand, wherein the destabilizing modification is selected from a modified unlocked nucleic acid (UNA) and a glycol nucleic acid (GNA), preferably a glycol nucleic acid.

In a further aspect, the invention relates to an inhibitor or an inhibitor for use according to the invention, wherein the siRNA comprises at least one thermally destabilizing modification at position 7 of the first strand, counting from position 1 of the first strand.

In a further aspect, the invention relates to an inhibitor or an inhibitor for use according to the invention, which is an siRNA, wherein the siRNA comprises 3 or more 2'-F modifications at positions 7 to 13 of the second strand, such as 4, 5, 6 or 7 2'-F modifications at positions 7 to 13 of the second strand, counting from position 1 of said second strand

In a further aspect, the invention relates to an inhibitor or an inhibitor for use according to the invention, which is an siRNA, wherein said second strand comprises at least 3, such as 4, 5 or 6, 2'-Me modifications at positions 1 to 6 of the second strand, counting from position 1 of said second strand.

In a further aspect, the invention relates to an inhibitor or an inhibitor for use according to the invention, which is an siRNA, wherein said first strand comprises at least 5 2'-Me consecutive modifications at the 3' terminal region, preferably including the terminal nucleoside at the 3' terminal region, or at least within 1 or 2 nucleosides from the terminal nucleoside at the 3' terminal region.

In a further aspect, the invention relates to an inhibitor or an inhibitor for use according to the invention, which is an siRNA wherein said first strand comprises 7 2'-Me consecutive modifications at the 3' terminal region, preferably including the terminal nucleoside at the 3' terminal region.

In a further aspect, the invention relates to an inhibitor or an inhibitor for use according to the invention, wherein the siRNA oligomer further comprises one or more phosphorothioate internucleoside linkages.

In a further aspect, the invention relates to an inhibitor or an inhibitor for use according to the invention, wherein said one or more phosphorothioate internucleoside linkages are respectively between at least three consecutive positions in a 5' or 3' near terminal region of the second strand, whereby said near terminal region is preferably adjacent said terminal region wherein said one or more abasic nucleosides of said second strand is / are located as defined herein.

In a further aspect, the invention relates to an inhibitor or an inhibitor for use according to the invention, wherein said one or more phosphorothioate internucleoside linkages are respectively between at least three consecutive positions in a 5' and / or 3' terminal region of the first strand, whereby preferably a terminal position at the 5' and / or 3' terminal region of said first strand is attached to its adjacent position by a phosphorothioate internucleoside linkage.

In a further aspect, the invention relates to an inhibitor or an inhibitor for use according to the invention, wherein the oligomer is an siRNA and the second strand of the siRNA is conjugated directly or indirectly to one or more ligand moiety(s), wherein said ligand moiety is typically present at a terminal region of the second strand, preferably at the 3' terminal region thereof.

In a further aspect, the invention relates to an inhibitor or an inhibitor for use according to the invention, wherein the ligand moiety comprises
i) one or more GalNAc ligands; and / or
ii) one or more GalNAc ligand derivatives; and / or
iii) one or more GalNAc ligands and / or GalNAc ligand derivatives conjugated to said SiRNA through a linker.

In a further aspect, the invention relates to an inhibitor or an inhibitor for use according to the invention, wherein said one or more GalNAc ligands and / or GalNAc ligand derivatives are conjugated directly or indirectly to the 5' or 3' terminal region of the second strand of the siRNA oligomer, preferably at the 3' terminal region thereof.

In a further aspect, the invention relates to an inhibitor or an inhibitor for use according to the invention, wherein the ligand moiety comprises

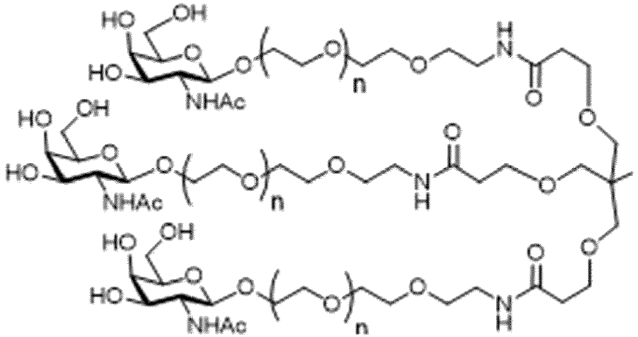

In a further aspect, the invention relates to an inhibitor or an inhibitor for use according to the invention, having the structure: 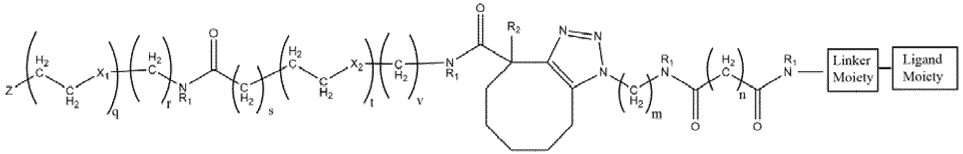 wherein:
R₁ at each occurrence is independently selected from the group consisting of hydrogen, methyl and ethyl;
R₂ is selected from the group consisting of hydrogen, hydroxy, -OC₁₋₃alkyl, -C(=O)OC₁₋₃alkyl, halo and nitro;
X₁ and X₂ at each occurrence are independently selected from the group consisting of methylene, oxygen and sulfur;
m is an integer of from 1 to 6;
n is an integer of from 1 to 10;
q, r, s, t, v are independently integers from 0 to 4, with the proviso that:
   (i) q and r cannot both be 0 at the same time; and
   (ii) s, t and v cannot all be 0 at the same time;
Z is an oligomer

In a further aspect, the invention relates to an inhibitor or an inhibitor for use according to the invention, having the structure 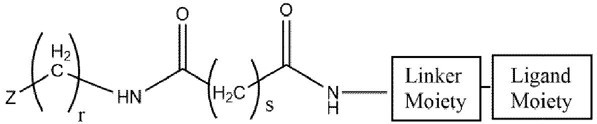 wherein:
r and s are independently an integer selected from 1 to 16; and
Z is an oligomer.

In a further aspect, the invention relates to an inhibitor or an inhibitor for use according to the invention, formulated as a pharmaceutical composition with an excipient and / or carrier.

In another aspect, the invention relates to a pharmaceutical composition comprising an inhibitor according to the invention, in combination with a pharmaceutically acceptable excipient or carrier.

In a further aspect, the invention relates to a pharmaceutical composition comprising an inhibitor according to the invention, in combination with a pharmaceutically acceptable excipient or carrier, for use in the prevention and/or treatment of metabolic disease or disorder, such as a metabolic disease or disorder associated with non-alcoholic fatty liver disease (NAFLD) and/or obesity and/or a disease or disorder associated with adipogenesis and/or for use in reducing adipogenesis.

In a further aspect, the invention relates to a pharmaceutical composition for use according to the invention, wherein the pharmaceutical composition is to be used in combination with a GLP-1 agonist and/or a THR-beta agonist.

In a further aspect, the invention relates to a pharmaceutical composition for use according to the invention, wherein the pharmaceutical composition is to be used in combination with a GLP-1 agonist and/or an FGF-21 analogue.

In a further aspect, the invention relates to a pharmaceutical composition for use according to the invention, wherein the GLP-1 agonist is a GLP-1/GIP dual agonist, a GLP-1/FGF21 dual agonist, a GLP-1/GCGR dual agonist, or a GLP-1/GIP/GCGR triple agonist.

In a further aspect, the invention relates to a pharmaceutical composition for use according to the invention, wherein the GLP-1 agonist is semaglutide.

In a further aspect, the invention relates to a pharmaceutical composition for use according to the invention, wherein the GLP-1/GIP dual agonist is tirzepatide.

In a further aspect, the invention relates to a pharmaceutical composition for use according to the invention, wherein the THR-beta agonist is resmetirom.

In a further aspect, the invention relates to a pharmaceutical composition for use according to the invention, wherein the FGF-21 analogue is efruxifermin.

In a further aspect, the invention relates to a pharmaceutical composition for use according to the invention, wherein the composition is to be used in combination with one or more of: an amylin receptor agonist (such as pramlintide), and/or a dual amylin + calcitonin receptor agonist, and/or a glucagon receptor agonist, and/or an FXR receptor agonist (such as cilofexor or obeticholic acid), and/or an FGF-21 analogue or FGF-21 receptor agonist (such as efruxifermin), and/or an FGF-19 analogue or FGF-19 receptor agonist (such as aldafermin), and/or a galectin 3 inhibitor (such as belapectin), and/or a PPARα agonist (such as elafibrinor), and/or a PPARγ agonist (such as pioglitazone or rosiglitazone), and/or a mixed PPARα and/or δ and/or γ agonist, and/or a pan PPARαγδ agonist (such as lanafibranor), and/or an acetyl CoA desaturase activator, and/or an ASK1 inhibitor (such as selonsertib), and/or an LOXL2 inhibitor (such as simtuzumab), and/or a dual CCR2/5 inhibitor (such as cenicriviroc), and/or an inhibitor of an enzyme in the de novo lipogenesis (DNL) pathway including citrate/isocitrate carrier (CIC), ATP-citrate lyase (ACLY), acetyl-CoA carboxylase (ACC) and fatty acid synthase (FAS), and/or an inhibitor of an enzyme in the cholesterol biosynthesis pathway (such as an HMGCoA reductase inhibitor, such as atorvastatin).

In another aspect, the invention relates to the use of SLC25A5/ANT2 as a target for identifying one or more therapeutic agents for the treatment or prevention of a metabolic disease or disorder, such as a metabolic disease or disorder associated with non-alcoholic fatty liver disease (NAFLD) and/or obesity and/or a disease or disorder associated with adipogenesis and/or for use in reducing adipogenesis.

In another aspect, the invention relates to a method of treating or preventing metabolic disease or disorder, such as a metabolic disease or disorder associated with non-alcoholic fatty liver disease (NAFLD) and/or obesity and/or a disease or disorder associated with adipogenesis and/or for use in reducing adipogenesis, which comprises administering to a patient an inhibitor of expression and/or function of SLC25A5/ANT2, such as an inhibitor according to the invention.

In a further aspect, the invention relates to a method according to the invention, wherein the inhibitor of SLC25A5/ANT2 is administered together with a GLP-1 agonist and/or a THR-beta agoni st.

In a further aspect, the invention relates to a method according to the invention, wherein the inhibitor of SLC25A5/ANT2 is administered together with a GLP-1 agonist and/or an FGF-21 analogue.

In a further aspect, the invention relates to a method according to the invention, wherein the GLP-1 agonist is a GLP-1/GIP dual agonist, a GLP-1/FGF21 dual agonist, a GLP-1/GCGR dual agonist, or a GLP-1/GIP/GCGR triple agonist.

In a further aspect, the invention relates to a method according to the invention, wherein the GLP-1 agonist is semaglutide.

In a further aspect, the invention relates to a method according to the invention, wherein the GLP-1/GIP dual agonist is tirzepatide.

In a further aspect, the invention relates to a method according to the invention, wherein the THR-beta agonist is resmetirom.

In a further aspect, the invention relates to a method according to the invention, wherein the FGF-21 analogue is efruxifermin.

In a further aspect, the invention relates to a method according to the invention, wherein the inhibitor is to be used in combination with one or more of: an amylin receptor agonist (such as pramlintide), and/or a dual amylin + calcitonin receptor agonist, and/or a glucagon receptor agonist, and/or an FXR receptor agonist (such as cilofexor or obeticholic acid), and/or an FGF-21 analogue or FGF-21 receptor agonist (such as efruxifermin), and/or an FGF-19 analogue or FGF-19 receptor agonist (such as aldafermin), and/or a galectin 3 inhibitor (such as belapectin), and/or a PPARα agonist (such as elafibrinor), and/or a PPARγ agonist (such as pioglitazone or rosiglitazone), and/or a mixed PPARα and/or δ and/or γ agonist, and/or a pan PPARαγδ agonist (such as lanafibranor), and/or an acetyl CoA desaturase activator, and/or an ASK1 inhibitor (such as selonsertib), and/or an LOXL2 inhibitor (such as simtuzumab), and/or a dual CCR2/5 inhibitor (such as cenicriviroc), and/or an inhibitor of an enzyme in the de novo lipogenesis (DNL) pathway including citrate/isocitrate carrier (CIC), ATP-citrate lyase (ACLY), acetyl-CoA carboxylase (ACC) and fatty acid synthase (FAS), and/or an inhibitor of an enzyme in the cholesterol biosynthesis pathway (such as an HMGCoA reductase inhibitor, such as atorvastatin).

In another aspect, the invention relates to SLC25A5/ANT2 for use as a biomarker of a metabolic disease or disorder, such as a metabolic disease or disorder associated with non-alcoholic fatty liver disease (NAFLD) and/or obesity and/or a disease or disorder associated with adipogenesis.

In another aspect, the invention relates to SLC25A5/ANT2 for use in an *in vivo* method of predicting susceptibility to prevention and/or treatment of metabolic disease or disorder, such as a metabolic disease or disorder associated with non-alcoholic fatty liver disease (NAFLD) and/or obesity and/or a disease or disorder associated with adipogenesis and/or for reducing adipogenesis, typically by monitoring the sequence and/ or level of expression and / or function of SLC25A5/ANT2 in a sample obtained from a patient.

In another aspect, the invention relates to a method of predicting susceptibility to a metabolic disease or disorder, such as a metabolic disease or disorder associated with non-alcoholic fatty liver disease (NAFLD) and/or obesity and/or a disease or disorder associated with adipogenesis, in a patient, said method comprising:
(a) obtaining a sample from the patient,
(b) detecting the sequence and / or expression and / or function of SLC25A5/ANT2 in said sample obtained from the patient,
(c) predicting susceptibility to a disease related to a metabolic disease or disorder, such as a metabolic disease or disorder associated with non-alcoholic fatty liver disease (NAFLD) and/or obesity and/or a disease or disorder associated with adipogenesis, based on the sequence and / or expression and / or function of SLC25A5/ANT2 in said sample obtained from the patient,
(d) preferably administering to the diagnosed patient an effective amount of an inhibitor of SLC25A5/ANT2.

In another aspect, the invention relates to an inhibitor or composition according to the invention, in the preparation of a medicament for use in the treatment or prevention of a metabolic disease or disorder, such as a metabolic disease or disorder associated with non-alcoholic fatty liver disease (NAFLD) and/or obesity and/or a disease or disorder associated with adipogenesis.

### FIGURES

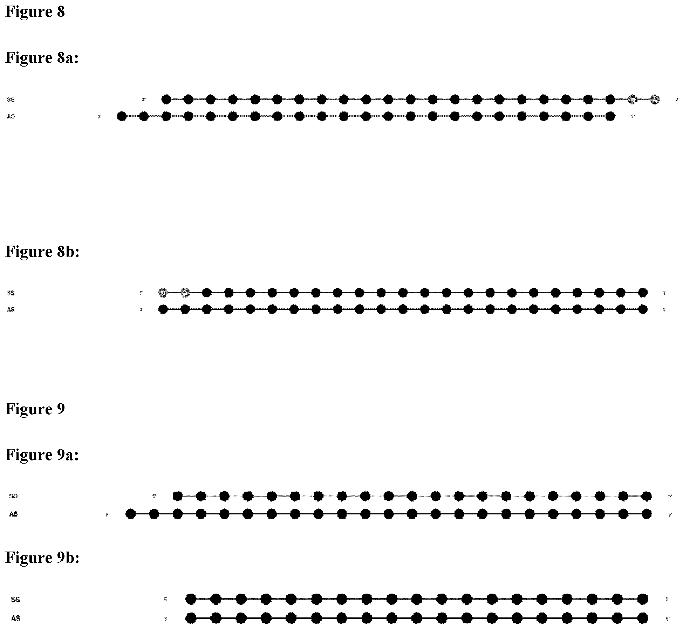

**Figure 1a** shows an exemplary linear configuration for a conjugate.
**Figure 1b** shows an exemplary branched configuration for a conjugate.

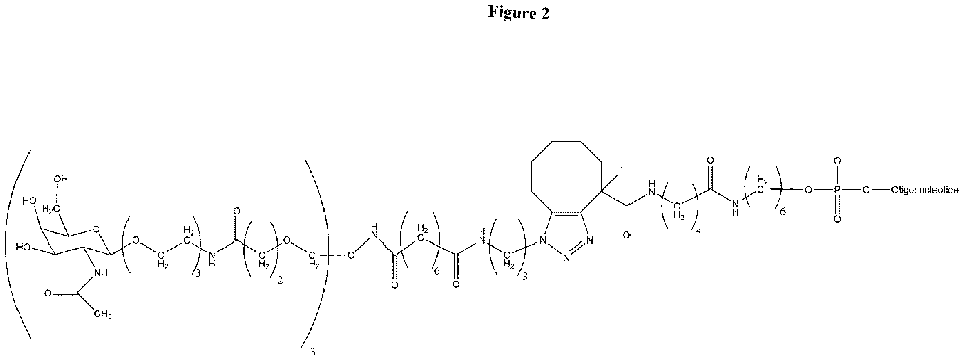

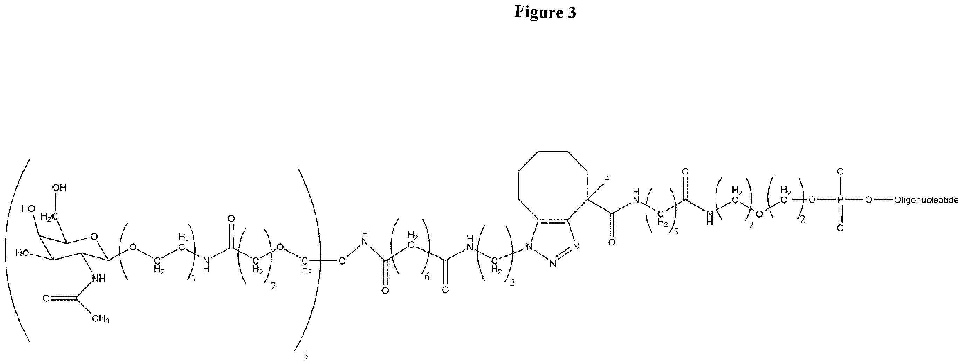

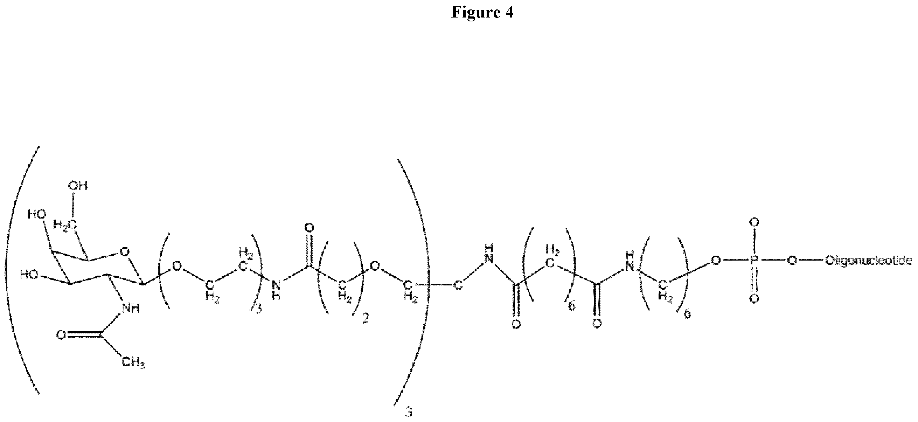

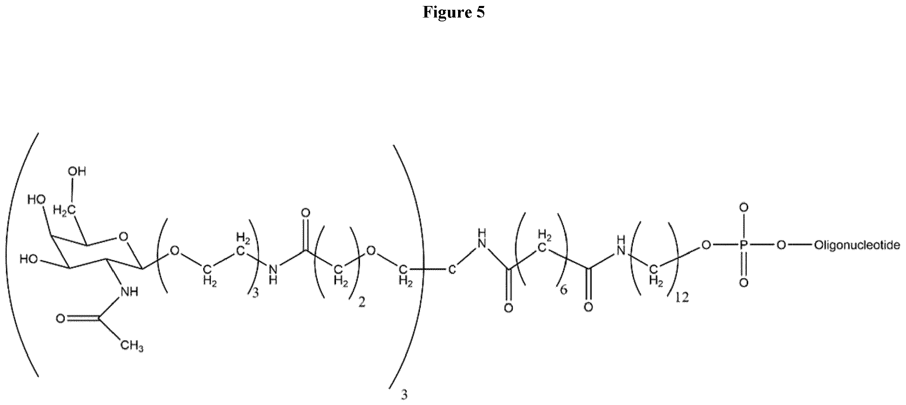

**Figures 2-5** show preferred oligomer - linker - ligand constructs of the invention.
**Figure 6** shows the detail of the formulae described in Sentences 1-101 disclosed herein.
**Figure 7** shows the detail of formulae described in Clauses 1-56 disclosed herein.
**Figures 8a and 8b****:** Inverted abasic constructs that can be used with nucleic acid sequences according to the present invention as described herein. For Figure 8a, a GalNAc linker is attached to the 5' end region of the sense strand in use (not depicted in Figure 8a). For Figure 8b, a GalNAc linker is attached to the 3' end region of the sense strand in use (not depicted in Figure 8b).
   iaia as shown at the 3' end region of the sense strand in Figure 8a represents (i) two abasic nucleosides provided as the penultimate and terminal nucleosides at the 3' end region of the sense strand, (ii) wherein a 3'-3' reversed linkage is provided between the antepenultimate nucleoside (namely at position 21 of the sense strand, wherein position 1 is the terminal 5' nucleoside of the sense strand) and the adjacent penultimate abasic residue of the sense strand, and (iii) the linkage between the terminal and penultimate abasic nucleosides is 5'-3' when reading towards the 3' end region comprising the terminal and penultimate abasic nucleosides.
   iaia as shown at the 5' end region of the sense strand in Figure 8b represents (i) two abasic nucleosides provided as the penultimate and terminal nucleosides at the 5' end region of the sense strand, (ii) wherein a 5'-5' reversed linkage is provided between the antepenultimate nucleoside (namely at position 1 of the sense strand, not including the iaia motif at the 5' end region of the sense strand in the nucleoside position numbering on the sense strand) and the adjacent penultimate abasic residue of the sense strand, and (iii) the linkage between the terminal and penultimate abasic nucleosides is 3'-5' when reading towards the 5' end region comprising the terminal and penultimate abasic nucleosides.
**Figures 9a and 9b****:** Duplex constructs according to Table 5.

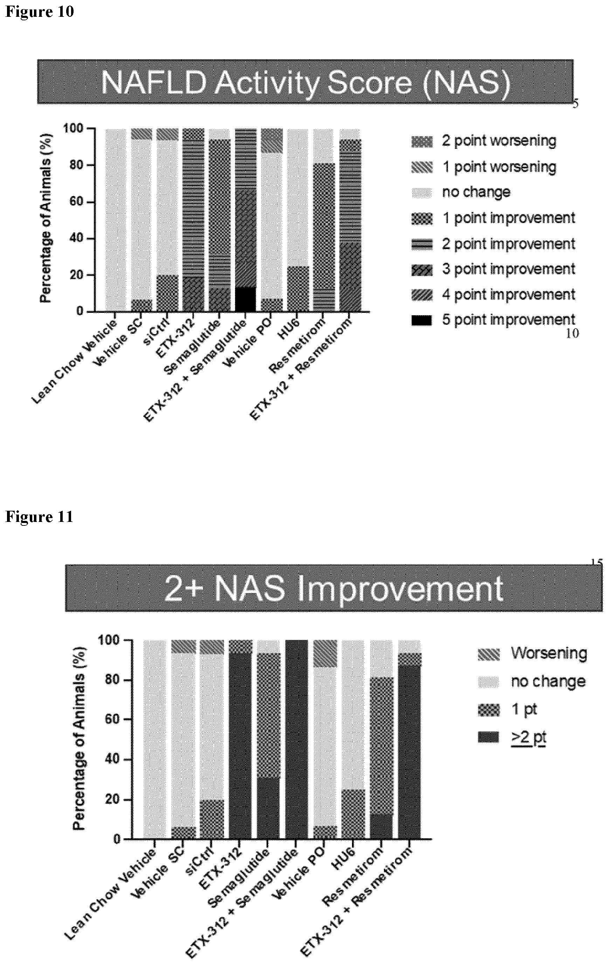

**Figures 10 and 11** show the NAFLD Activity Scores (NAS) of mice with diet-induced NAFLD in response to different treatments. Liver samples stained with H&E were given a score for NAFLD Activity Score (NAS) using the clinical criteria outlined by Kleiner et al. (2005). Total NAS represents the sum of scores for steatosis, inflammation, and ballooning, and ranges from 0-8. NAS score was determined by Gubra Histopathological Objective Scoring Technology (GHOST) deep learning app developed by Gubra using the VIS software (Visiopharm, Denmark) for a more accurate and objective method for staging disease in DIO-NASH mouse models. Results are presented as change in NAS score (improvement or worsening) at study termination compared to the pre-dose biopsy. Also shown is the percentage of animals with at least a 1- or 2-point improvement in NAS.

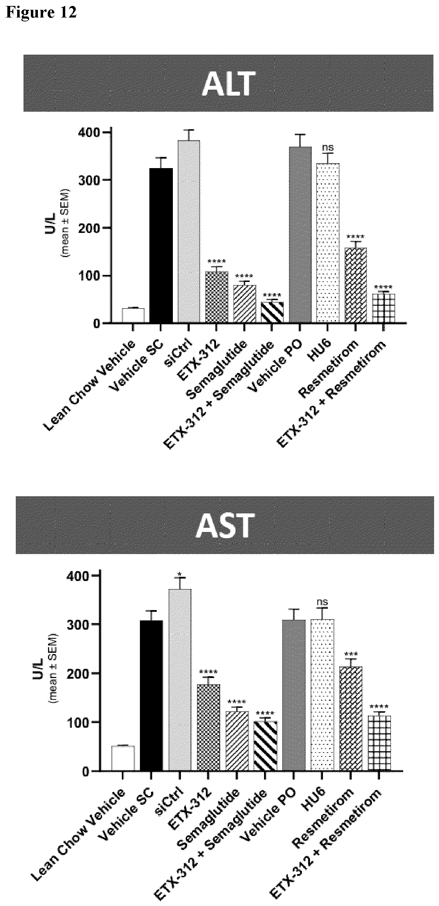

**Figure 12****:** ALT and AST were measured in plasma samples after 12 weeks ETX-312 (ETX-M00001378) treatment using commercial kits (Roche Diagnostics), on the cobas c501 autoanalyzer. ALT and AST levels were increased in DIO-NASH mice (vehicle sc, siCtrl, vehicle PO). Treatment with ETX-312 (ETX-M00001378) alone or in combination with semaglutide or resmetirom significantly reduced both ALT and AST levels. Combination therapies demonstrated a significantly enhanced effect compared to semaglutide and resmetirom treatments administered as monotherapies. Results are presented as absolute levels as Mean ± SEM from n=16 experiment. * p < 0.05; *** p < 0.001; **** p < 0.0001; ns non-significant.

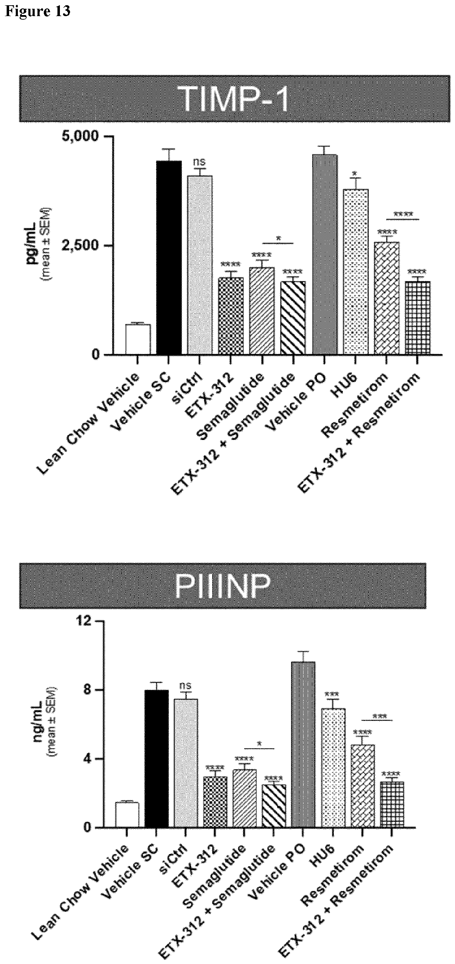

**Figure 13****:** TIMP-1 and PIIINP are non-invasive blood biomarkers for NAFLD/NASH that predict hepatic fibrosis. TIMP-1 was measured in plasma collected in EDTA tubes using a commercial ELISA kit (R&D Systems). PIIINP was measured in plasma collected in EDTA tubes using a commercial ELISA kit (Cusabio). TIMP-1 and PIIINP levels were increased in DIO-NASH mice (vehicle sc, siCtrl, vehicle PO). Treatment with ETX-312 (ETX-M00001378) alone or in combination with semaglutide or resmetirom significantly reduced both TIMP-1 and PIIINP levels. Combination therapies led to a further reduction in TIMP-1 and PIIINP levels when compared to semaglutide and resmetirom treatments alone. Results are presented as absolute levels as Mean ± SEM from n=16 experiment. An outlier analysis was conducted by comparing the studentised residuals of the linear model fitted to the subcutaneous treatment subset of the data to the critical Bonferroni alpha level (0.05/88 = -0.00057). One animal in the semaglutide group was identified as an outlier which was verified by an influence analysis. This animal was concluded as both highly outlying and influential and was excluded from all TIMP-1 and PIIINP analysis. * p < 0.05; *** p < 0.001; **** p < 0.0001; ns non-significant.

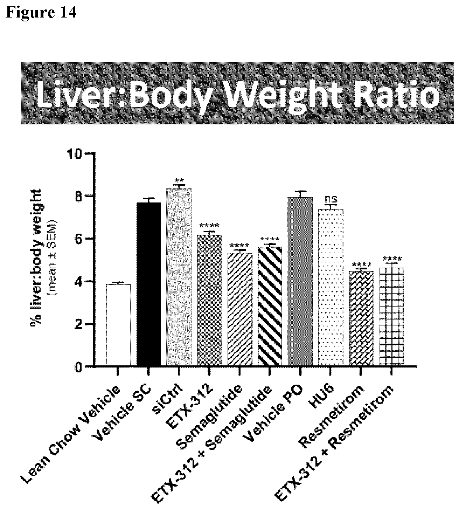

**Figure 14****:** Terminal liver weight to body weight ratio demonstrates hepatomegaly in DIO-NASH mice. Treatment with ETX-312 (ETX-M00001378) alone or in combination with semaglutide or resmetirom significantly reduced liver to body weight ratio. No difference was observed between the combination therapies and the monotherapies of semaglutide and resmetirom. Results are presented as percent liver:body weight as Mean ± SEM from n=16 experiment. ** p < 0.01; **** p < 0.0001; ns non-significant.

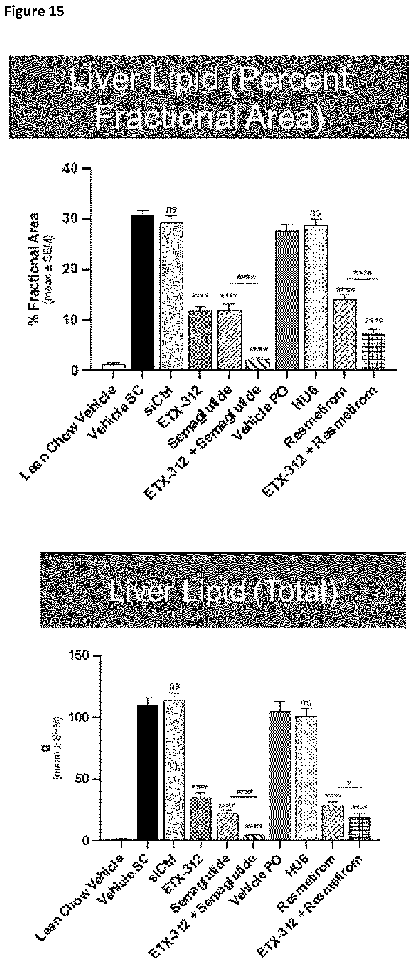

**Figure 15****:** Liver lipid content, or steatosis, is quantified on H&E stained slides by image analysis using the VIS software (Visiopharm, Denmark). Treatment with ETX-312 (ETX-M00001378) alone or in combination with semaglutide or resmetirom significantly reduced liver steatosis. Combination therapies resulted in a significantly greater effect than semaglutide and resmetirom used as monotherapies. Results are presented as a percent area fraction (on the left) and the total level (percent area fraction multiplied by liver weight, on the right), as Mean ± SEM from n=16 experiment. * p < 0.05; **** p < 0.0001; ns non-significant.

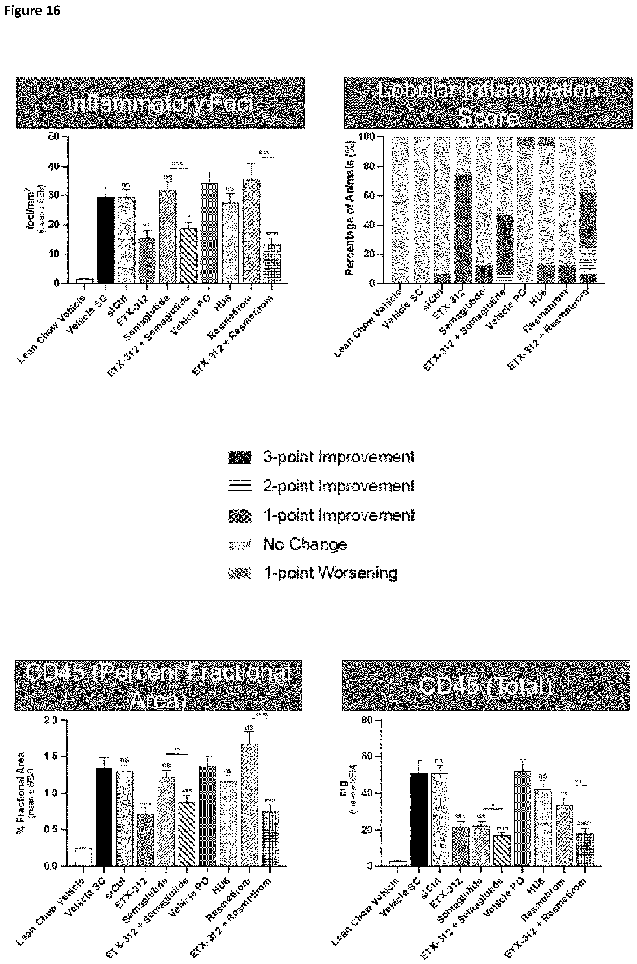

**Figure 16****:** Treatment with ETX-312 (ETX-M00001378) alone or in combination with semaglutide or resmetirom significantly reduced inflammation in liver, a key factor differentiating NASH from NAFLD, as measured by inflammatory foci density, lobular inflammation score, and CD45 staining. Combination therapies exhibited a notably improved effect over semaglutide and resmetirom monotherapies. Inflammatory foci density and lobular inflammation score were assessed by Gubra Histopathological Objective Scoring Technology (GHOST) deep learning app developed by Gubra, by image analysis of H&E stained slides using the VIS software (Visiopharm, Denmark). Inflammatory foci density is the number of inflammatory foci per surface area, where foci are clusters of more than 3 inflammatory cells. The lobular inflammation score was determined using the clinical criteria outlined by Kleiner et al. (2005). CD45, a surface marker of immune cells, was assessed via immunohistochemistry using a rabbit anti-CD45 primary antibody (AbCam, Cat. Ab10558). CD45-positive staining was quantified using the VIS software (Visiopharm, Denmark), reported as a percent area fraction (bottom left) and the total level (percent area fraction multiplied by liver weight, bottom right). Lobular inflammation scores show changes between pre-dose and post-dose liver biopsies (improvement or worsening), while results for inflammatory foci and CD45 staining are presented as Mean ± SEM from n=16 experiment. * p < 0.05; ** p < 0.01; *** p < 0.001; **** p < 0.0001; ns non-significant.

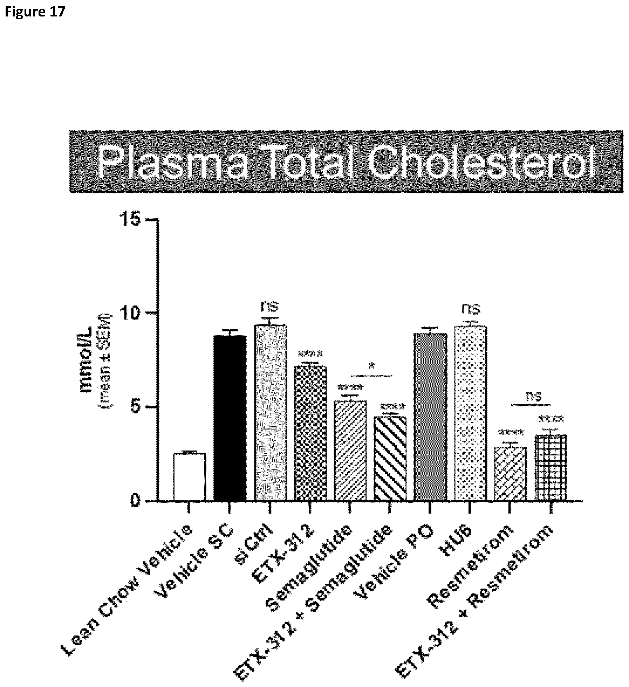

**Figure 17****:** Total cholesterol (TC) levels were measured in plasma samples after 12 weeks ETX-312 (ETX-M00001378) treatment using commercial kits (Roche Diagnostics), on the cobas c501 autoanalyzer. GAN diet elevated plasma TC in DIO NASH animals (Vehicle SC, siCtrl, Vehicle PO). Treatment with ETX-312 (ETX-M00001378) alone or in combination with semaglutide or resmetirom significantly reduced plasma TC levels. The combination of semaglutide with ETX-312 further reduced TC compared to semaglutide alone. Results are presented as absolute levels as Mean ± SEM from n=16 experiment. * p < 0.05; **** p < 0.0001; ns non-significant.

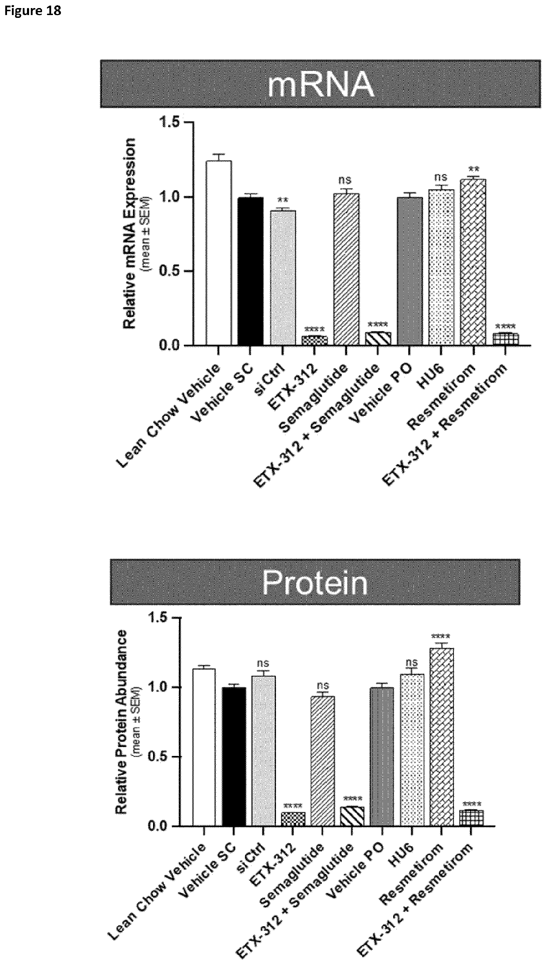

**Figure 18****:** Weekly treatment of ETX-312 (ETX-M00001378) for 12 weeks greatly reduced target expression in liver, at both mRNA and protein levels. mRNA or protein was extracted from 50 mg of mouse liver per animal and homogenized in lysis buffer. mRNA expression was analyzed by qPCR in technical triplicates, with TaqMan gene expression assays for the target and the house keeping genes (Mm00846873_g1, Mm99999915_g1, Thermo Fisher Scientific). Relative mRNA expression for each sample was calculated with the ddCt method, normalized to vehicle treated samples. Relative protein abundance was calculated based on quantification of two peptides per liver lysate per animal (QIFLGGVDK - SEQ ID NO: 1390, EQGVLSFWR - SEQ ID NO: 1391). Results are presented as Mean ± SEM from n=16 experiment. ** p < 0.01; **** p < 0.0001; ns non-significant.

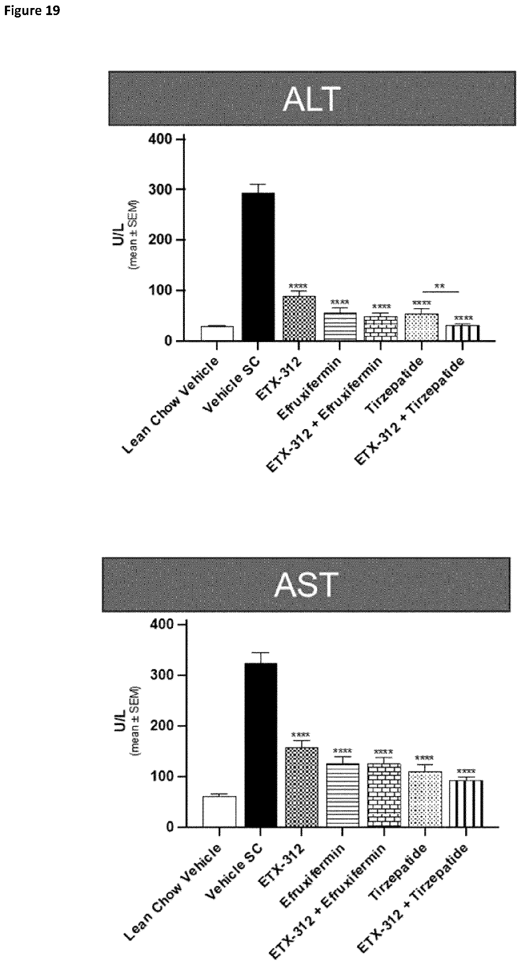

**Figure 19****:** ALT and AST were measured in plasma samples after 16 weeks ETX-312 (ETX-M00001378) treatment using commercial kits (Roche Diagnostics), on the cobas c501 autoanalyzer. ALT and AST levels were increased in DIO-NASH mice (vehicle sc, siCtrl, vehicle PO). Treatment with ETX-312 (ETX-M00001378) alone or in combination with efruxifermin or tirzepatide significantly reduced both ALT and AST levels. Combination of ETX-312 and Tirzepatide further reduced plasma ALT as compared to tirzepatide alone. Results are presented as absolute levels as Mean ± SEM from n=18 experiment. ** p < 0.01; **** p < 0.0001.

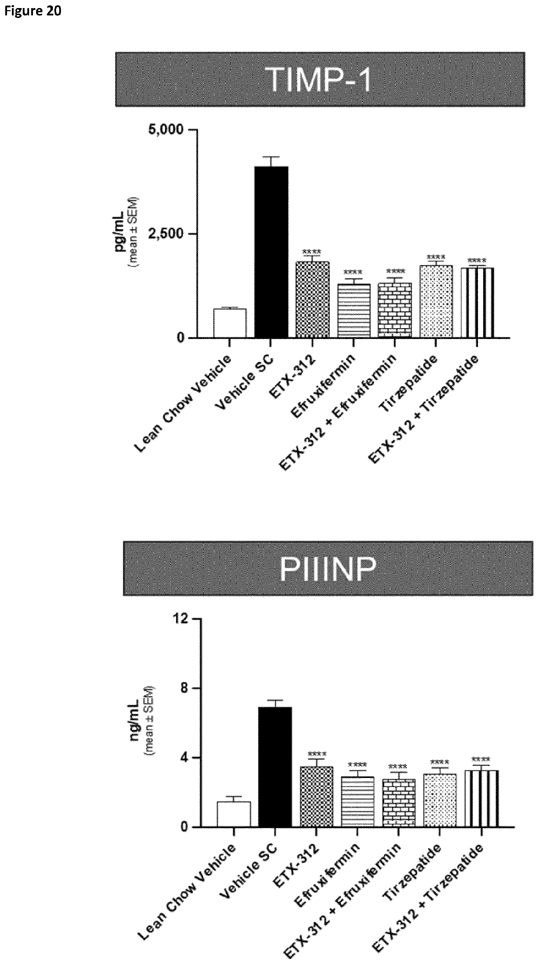

**Figure 20****:** TIMP-1 and PIIINP are non-invasive blood biomarkers for NAFLD/NASH that predict hepatic fibrosis. TIMP-1 was measured in plasma collected in EDTA tubes using a commercial ELISA kit (R&D Systems). PIIINP was measured in plasma collected in EDTA tubes using a commercial ELISA kit (Cusabio). TIMP-1 and PIIINP levels were increased in DIO-NASH mice (vehicle sc, siCtrl, vehicle PO). Treatment with ETX-312 (ETX-M00001378) alone or in combination with efruxifermin or tirzepatide significantly reduced both TIMP-1 and PIIINP levels. Results are presented as absolute levels as Mean ± SEM from n = 18 experiments for TIMP-1; and n=2 (lean chow vehicle) , n=17 (vehicle sc), n=15 (ETX-312), n=11 (efruxifermin), n=12 (ETX-312 + efruxifermin), n=15 (tirzepatide), n=16 (ETX-312 + tirzepatide) experiment for PIIINP. **** p < 0.0001.

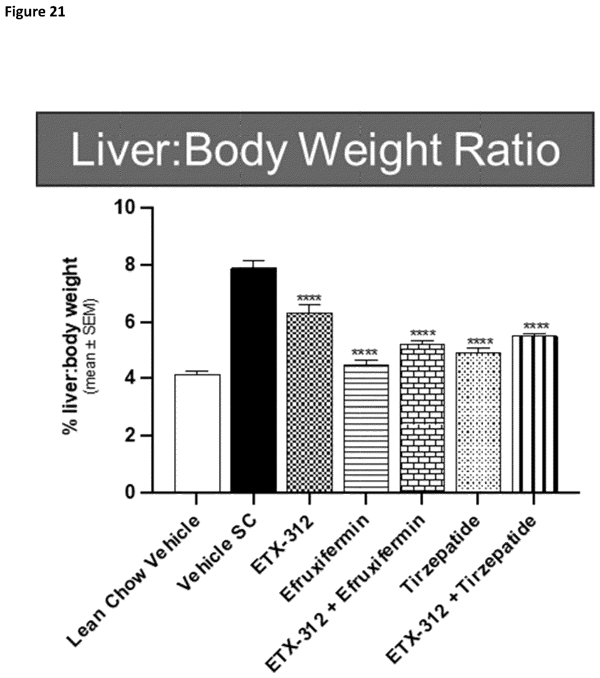

**Figure 21****:** Terminal liver weight to body weight ratio demonstrates hepatomegaly in DIO-NASH mice. Treatment with ETX-312 (ETX-M00001378) alone or in combination with efruxifermin or tirzepatide significantly reduced liver to body weight ratio. Results are presented as percent liver:body weight as Mean ± SEM from n=16 experiment. **** p < 0.0001.

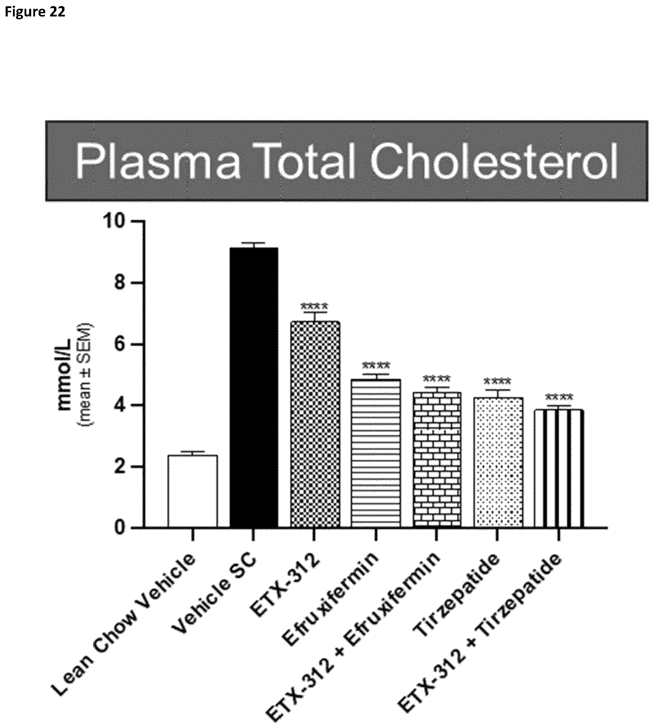

**Figure 22****:** Total cholesterol (TC) levels were measured in plasma samples after 16 weeks ETX-312 (ETX-M00001378) treatment using commercial kits (Roche Diagnostics), on the cobas c501 autoanalyzer. GAN diet elevated plasma TC in DIO NASH animals (Vehicle SC, siCtrl, Vehicle PO). Treatment with ETX-312 (ETX-M00001378) alone or in combination with efruxifermin or tirzepatide significantly reduced plasma TC levels. Results are presented as absolute levels as Mean ± SEM from n=16 experiment. **** p < 0.0001.

### DETAILED DESCRIPTION

The present invention, inter alia, provides inhibitors, for example oligomers such as nucleic acids, such as inhibitory RNA molecules (which may be referred to as iRNA or siRNA), and compositions containing the same which can affect expression of a target, for example by binding to mRNA transcribed from a gene. The target may be within a cell, e.g. a cell within a subject, such as a human. The inhibitors can be used to prevent and/or treat medical conditions associated with the e.g. the expression of a target gene.

In particular the present invention identifies inhibitors of expression and/or function of SLC25A5/ANT2 as useful in the prevention and/or treatment of a metabolic disease or disorder, such as a metabolic disease or disorder associated with non-alcoholic fatty liver disease (NAFLD) and/or obesity and/or a disease or disorder associated with adipogenesis.

ADP/ATP translocase 2 (ANT2) is a protein that in humans is encoded by the *SLC25A5* gene on the X chromosome. This protein functions as an antiporter for ADP/ATP exchange between the mitochondrial matrix and cytoplasm.

The present invention relates to an inhibitor of expression and/or function of SLC25A5/ANT2. Accordingly, in certain embodiments, the invention relates to an inhibitor of expression of the *SLC25A5* gene, such as an siRNA that targets an mRNA transcribed from the *SLC25A5* gene. In certain embodiments, the invention relates to an inhibitor of function of the gene product ANT2. Both options are encompassed when it referred herein to an inhibitor of SLC25A5/ANT2 or an inhibitor of the invention.

In humans, ANT2 is encoded by the *SLC25A5* gene (SEQ ID NO: 1381).

SEQ ID NO: 1381 (SLC25A5)

ATCGGCCATTTGCTTCGCTCCGCCCCGCAGCGCCGGAGTCAAAGCCGGTTCCCGGCCCAGTCCCGTCCT
GCAGCAGTCTGCCTCCTCTTTCAACATGACAGATGCCGCTGTGTCCTTCGCCAAGGACTTCCTGGCAGG
TGGAGTGGCCGCAGCCATCTCCAAGACGGCGGTAGCGCCCATCGAGCGGGTCAAGCTGCTGCTGCAG
GTACGTCCTGGGATCCAGGAGCCCAACCAGGAAGTGGGGGGAAGGGTCGCACAGAAGGCGGGCGCCC
GAGGGGTGGCGGGGAGCGAACTCTAAAGACATGGCCAGGGAAGCGGCTTAGGAGAGGCCAGAGCGG
GGCGCAGAGGCAGAACAGAAGTCAAACTGGTGGGAGGCGCCTTTAGTGACCTGAAGTAGTGAGTCTA
GGAAGGGGCCGGGGGCAGAGGGCAGGACCAGGCTCTCGGCATCTCCGAGGCGGCGGACTCGGATGG
AGCAGTTTCTGAGTGACGGCCTCCCCGGGCCTGGGCGTCAAGGGCGAAGGCCGAAAGCCGGCGTTAG
AAAGAGGAACGCCAGTTCTTACCGAAGACCTCAAGGTCGCGGCAAGGAGATAACTGCCCGGGGGAGG
CCATGCGCCCGGGTCCAGCGGCCTCCCAGCCCGCGGACGCGCTCAAACCTCGCCGGGCCGGAAGCCG
GCGCCGGGAAGCGCGTGTGCCTTTTACGTCCGCCCCCGCGCAGCCGCGGCCGCTGCCGCCGCGTCTCC
GCCTGCCTCCCTGCGCCGCGCGCTCTCCAGTGCCGGCTCTAGAGGGCGCTCCTGGGCTAGCGTGTAGG
GCTGGCGGCGGCGGCGCTCGGGTCACCTCTGGGAGCGGAGTGGGGGCGGAGCGAGACGGAAGCAGCT
CAGGAGACTTGAGGCGTAGGCTGCGGTCCCCAAGGTGACCGCGCCCTATGTGGGACTCGCCCTAATGC
CTCTGAACCTGGGTTTGAGGTAATGACCTTTCTCCTAGGTCTGAAGGTCACGGGTCCGCTGGAGGATG

CCCCCTCTCCACTCAGAGGGGTGGAGGCTTAATGCTACTGGTGCAGATCACCTCTTCCCCTGTGACAGC
CTCAGAGGGTTGGGAGGGTCCAGCCAGTATGATATACGAAGACTAGATTTGAGAGAGGGGAGCCTAC
CTTAAAGGGCATTGATCGAGATGGCATAAGCTCTTCTCTTTCCCTTCCCCATGGTTATAACTGTCCCTG
TTGGCTTCCTTCCTGTCTGTTAGGTGCAGCATGCCAGCAAGCAGATCACTGCAGATAAGCAATACAAA
GGCATTATAGACTGCGTGGTCCGTATTCCCAAGGAGCAGGGAGTTCTGTCCTTCTGGCGCGGTAACCT
GGCCAATGTCATCAGATACTTCCCCACCCAGGCTCTTAACTTCGCCTTCAAAGATAAATACAAGCAGA
TCTTCCTGGGTGGTGTGGACAAGAGAACCCAGTTTTGGCTCTACTTTGCAGGGAATCTGGCATCGGGT
GGTGCCGCAGGGGCCACATCCCTGTGTTTTGTGTACCCTCTTGATTTTGCCCGTACCCGTCTAGCAGCT
GATGTGGGTAAAGCTGGAGCTGAAAGGGAATTCCGAGGCCTCGGTGACTGCCTGGTTAAGATCTACA
AATCTGATGGGATTAAGGGCCTGTACCAAGGCTTTAACGTGTCTGTGCAGGGTATTATCATCTACCGA
GCCGCCTACTTCGGTATCTATGACACTGCAAAGGGTAAGTTTGCTGTGGGCTTTAACGTTGTGTTCTTA
GGAGACAGTTTAAAAGAGCATTGTACCAACCTAACAGTCCAAGAGCTAAAGAGTTGTTTTTTTAATTG
CTAAAGGAAGCCAAGATCATCCAATGCAACCCTTGTGTACAGATGACGTGTTTAGGGGATGTGGGGA
AAGGAAGTCAGTAAAACTCTGCTTTTTGGTAAAAGCATCTCTTTCCTATTCCCAGGAATGCTTCCGGAT
CCCAAGAACACTCACATCGTCATCAGCTGGATGATCGCACAGACTGTCACTGCTGTTGCCGGGTTGAC
TTCCTATCCATTTGACACTGTTCGCCGCCGCATGATGATGCAGTCAGGGCGCAAAGGAAGTAAGTTCC
ACTTGAGCAGAAGATAAAGTTGTAGTCGTGGGGCAATCTGCTGCCACAAACTGGTGATACATACCTTT
AAAATGGCTGTCTGTCCAAGTCAAGGGATGGGGTTGATAGCATCTGTGTCTGTTCCACAACTGCCTTTG
AGCTGGCCCTTCAGATGCCTATGAATGAGGGTGCTTAAATGGTGTTAGAGGTTAAGACCAATGGGTAG
TCTGTATTCCTGTGGTCATAGCATTAATATTTCAGTGTTGCCCATGCTAATGTGTGAATGTTGGATTTA
AAGCTGACGTTCTTAGAGGTGGGGCTCTGCTTTATTTAGCCTAGTGAATCTTAGGATTTTTCATCGGCC
TTCAGTCACTAACTCCATGTCTTTATTCTTTGCAGCTGACATCATGTACACAGGCACGCTTGACTGCTG
GCGGAAGATTGCTCGTGATGAAGGAGGCAAAGCTTTTTTCAAGGGTGCATGGTCCAATGTTCTCAGAG
GCATGGGTGGTGCTTTTGTGCTTGTCTTGTATGATGAAATCAAGAAGTACACATAAGTTATTTCCTAGG
ATTTTTCCCCCTGTGAACAGGCATGTTGTATTATATAACATATCTTGAGCATTCTTGACAGACTCCTGG
CTGTCAGTTTCTCAGTGGCAACTATTTACTGGTTGAAAATGGGAAGCAATAATATTCATCTGACCAGTT
TTCTCTTAAAGCCATTTCCATGATGATGATGATGGGACTCAATTGTATTTTTTATTTCAGTCACTCCTGA
TAAATAACAAATTTGGAGAAATAAAAATATCTAAAATAAATTTTGTCTGCAGTATATTTTCATATAAA
AATGCATATTTGAGTGCTACATTCGAATAAATACTACCTTTTTAGTGAA

The inventors employed network analysis that allows them to allocate multiple genes or proteins to a smaller number of driver processes; and to mine these processes for impactful drug targets. The approach takes advantage of information that is usually ignored in standard gene set analyses - the known and predicted interactions between genes (and proteins) and the inclusion of other genes in the same or related pathways. In particular, the inventors analysed Genome Wide Association Studies (GWAS) metanalyses for Non-Alcoholic Fatty Liver Disease (NAFLD) using network models which highlighted SLC25A5/ANT2 as a preferred target for NAFLD among other known targets associated with NAFLD.

The inhibition disclosed herein may be of the gene or protein resulting from expression of the SLC25A5 gene and reference to SLC25A5/ANT2 hereby explicitly incorporates a reference to inhibition of the expression or function of the gene and, separately, of the protein product.

### DEFINITIONS

The "first strand", also called the antisense strand or guide strand herein and which can be used interchangeably herein, refers to the nucleic acid strand, e.g. the strand of an siRNA, e.g. a dsiRNA, which includes a region that is substantially complementary to a target sequence, e.g. to an mRNA. As used herein, the term "region of complementarity" refers to the region on the antisense strand that is substantially complementary to a sequence, for example a target sequence. Where the region of complementarity is not fully complementary to the target sequence, the mismatches can be in the internal or terminal regions of the molecule. In some embodiments, a double stranded nucleic acid e.g. an siRNA agent of the invention includes a nucleotide mismatch in the antisense strand.

The "second strand" (also called the sense strand or passenger strand herein, and which can be used interchangeably herein), refers to the strand of a nucleic acid e.g. siRNA that includes a region that is substantially complementary to a region of the antisense strand as that term is defined herein.

In the context of molecule comprising a nucleic acid provided with a ligand moiety, optionally also with a linker moiety, the nucleic acid of the invention may be referred to as an oligonucleotide moiety or oligonucleoside moiety

Oligonucleotides are short nucleic acid polymers. Whilst oligonucleotides contain phosphodiester bonds between the nucleoside component thereof (base plus sugar), the present invention is not limited to oligonucleotides always joined by such a phosphodiester bond between adjacent nucleosides, and other oligomers of nucleosides joined by bonds which are bonds other than a phosphate bond are contemplated. For example, a bond between nucleotides may be a phosphorothioate bond. Therefore, the term "oligonucleoside" herein covers both oligonucleotides and other oligomers of nucleosides. An oligonucleoside which is a nucleic acid having at least a portion which is an oligonucleotide is preferred according to the present invention. An oligonucleoside having one or more, or a majority of, phosphodiester backbone bonds between nucleosides is also preferred according to the present invention. An oligonucleoside having one or more, or a majority of, phosphodiester backbone bonds between nucleosides, and also having one or more phosphorothioate backbone bonds between nucleosides (typically in a terminal region of the first and / or second strands) is also preferred according to the present invention.

In some embodiments, a double stranded nucleic acid e.g. siRNA agent of the invention includes a nucleoside mismatch in the sense strand. In some embodiments, the nucleoside mismatch is, for example, within 5, 4, 3, 2, or 1 nucleosides from the 3 '-end of the nucleic acid e.g. siRNA.

In another embodiment, the nucleoside mismatch is, for example, in the 3'- terminal nucleoside of the nucleic acid e.g. siRNA.

A "target sequence" (which may be called a target RNA or a target mRNA) refers to a contiguous portion of the nucleoside sequence of an mRNA molecule formed during the transcription of a gene, including mRNA that is a product of RNA processing of a primary transcription product, or can be a contiguous portion of the nucleotide sequence of any RNA molecule such as a LNCRNA which it is desired to inhibit.

The target sequence may be from about 10-35 nucleosides in length, e.g., about 15-30 nucleosides in length. For example, the target sequence can be from about 15-30 nucleosides, 15-29, 15-28, 15-27, 15-26, 15-25, 15-24, 15-23, 15-22, 15-21, 15-20, 15-19, 15-18, 15-17, 18-30, 18-29, 18-28, 18-27, 18-26, 18-25, 18-24, 18-23, 18-22, 18-21, 18-20, 19-30, 19-29, 19-28, 19-27, 19-26, 19-25, 19-24, 19-23, 19-22, 19-21, 19-20, 20-30, 20-29, 20-28, 20-27, 20-26, 20-25, 20-24, 20-23, 20-22, 20- 21, 21-30, 21-29, 21-28, 21-27, 21-26, 21-25, 21-24, 21-23, or 21-22 nucleosides in length. Ranges and lengths intermediate to the above recited ranges and lengths are also contemplated to be part of the invention.

The term "ribonucleoside" or "nucleoside" can also refer to a modified nucleoside as further detailed below.

A nucleic acid can be a DNA or an RNA, and can comprise modified nucleosides. RNA is a preferred nucleic acid.

The terms "iRNA", "siRNA", "RNAi agent," and "iRNA agent," "RNA interference agent" as used interchangeably herein, refer to an agent that contains RNA, and which mediates the targeted cleavage of an RNA transcript via an RNA-induced silencing complex (RISC) pathway. siRNA directs the sequence-specific degradation of mRNA through RNA interference (RNAi).

A double stranded RNA is referred to herein as a "double stranded siRNA (dsiRNA) agent", "double stranded siRNA (dsiRNA) molecule", "double stranded RNA (dsRNA) agent", "double stranded RNA (dsRNA) molecule", "dsiRNA agent", "dsiRNA molecule", or "dsiRNA", which refers to a complex of ribonucleic acid molecules, having a duplex structure comprising two antiparallel and substantially complementary nucleic acid strands, referred to as having "sense" and "antisense" orientations with respect to a target RNA. The majority of nucleosides of each strand of the nucleic acid, e.g. a dsRNA molecule, are preferably ribonucleosides, but in that case each or both strands can also include one or more non-ribonucleosides, e.g., a deoxyribonucleoside or a modified ribonucleoside. In addition, as used in this specification, an "siRNA" may include ribonucleosides with chemical modifications.

The term "modified nucleoside" refers to a nucleoside having, independently, a modified sugar moiety, a modified internucleoside linkage, or modified nucleobase, or any combination thereof. Thus, the term modified nucleoside encompasses substitutions, additions or removal of, e.g., a functional group or atom, to internucleoside linkages, sugar moieties, or nucleobases. Any such modifications, as used in a siRNA type molecule, are encompassed by "iRNA" or "RNAi agent" or "siRNA" or "siRNA agent" for the purposes of this specification and claims.

The duplex region of a nucleic acid of the invention e.g. a dsRNA may range from about 9 to 40 base pairs in length such as 9 to 36 base pairs in length, e.g., about 15- 30 base pairs in length, for example, about 9, 10, 11, 12, 13, 14, 15, 16, 17, 18, 19, 20, 21, 22, 23, 24, 25, 26, 27, 28, 29, 30, 31, 32, 33, 34, 35, or 36 base pairs in length, such as about 15-30, 15-29, 15-28, 15-27, 15-26, 15-25, 15-24, 15-23, 15-22, 15-21, 15-20, 15-19, 15-18, 15-17, 18-30, 18-29, 18-28, 18- 27, 18-26, 18-25, 18-24, 18-23, 18-22, 18-21, 18-20, 19-30, 19-29, 19-28, 19-27, 19-26, 19-25, 19-24, 19-23, 19-22, 19-21, 19-20, 20-30, 20-29, 20-28, 20-27, 20-26, 20-25, 20-24,20-23, 20-22, 20-21, 21-30, 21-29, 21-28, 21-27, 21-26, 21-25, 21-24, 21-23, or 21-22 base pairs in length.

The two strands forming the duplex structure may be different portions of one larger molecule, or they may be separate molecules e.g. RNA molecules.

The term "nucleoside overhang" refers to at least one unpaired nucleoside that extends from the duplex structure of a double stranded nucleic acid. A ds nucleic acid can comprise an overhang of at least one nucleoside; alternatively the overhang can comprise at least two nucleosides, at least three nucleosides, at least four nucleosides, at least five nucleosides, or more. A nucleoside overhang can comprise or consist of a nucleoside analog, including a deoxynucleoside. The overhang(s) can be on the sense strand, the antisense strand, or any combination thereof. Furthermore, the /nucleoside(s) of an overhang can be present on the 5'-end, 3'-end, or both ends of either an antisense or sense strand.

In certain embodiments, the antisense strand has a 1-10 nucleoside, e.g., 0-3, 1-3, 2-4, 2-5, 4-10, 5-10, 1, 2, 3, 4, 5, 6, 7, 8, 9, or 10 nucleoside overhang at the 3'-end or the 5'-end.

"Blunt" or "blunt end" means that there are no unpaired nucleoside at that end of the double stranded nucleic acid, i.e., no nucleoside overhang. The nucleic acids of the invention include those with no nucleoside overhang at one end or with no nucleoside overhangs at either end.

Unless otherwise indicated, the term "complementary," when used to describe a first nucleoside sequence in relation to a second nucleoside sequence, refers to the ability of an oligonucleoside comprising the first nucleoside sequence to hybridize and form a duplex structure under certain conditions with an oligonucleoside or polynucleoside comprising the second nucleoside sequence, as will be understood by the skilled person. Such conditions can, for example, be stringent conditions, where stringent conditions can include: 400 mM NaCl, 40 mM PIPES pH 6.4, 1 mM EDTA, 50°C or 70°C for 12-16 hours followed by washing (see, e.g., "Molecular Cloning: A Laboratory Manual, Sambrook, et al. (1989) Cold Spring Harbor Laboratory Press).

Complementary sequences within nucleic acid e.g. a dsiRNA, as described herein, include base-pairing of the oligonucleoside or polynucleoside comprising a first nucleoside sequence to an oligonucleoside or polynucleoside comprising a second nucleoside sequence over the entire length of one or both nucleoside sequences. Such sequences can be referred to as "fully complementary" with respect to each other herein. However, where a first sequence is referred to as "substantially complementary" or "partially complementary" with respect to a second sequence herein, the two sequences can be fully complementary, or they can form one or more mismatched base pairs, such as 2, 4, or 5 mismatched base pairs, but preferably not more than 5, while retaining the ability to hybridize under the conditions most relevant to their ultimate application, e.g., inhibition of gene expression via a RISC pathway. Overhangs shall not be regarded as mismatches with regard to the determination of complementarity. For example, a nucleic acid e.g. dsRNA comprising one oligonucleoside 17 nucleosides in length and another oligonucleoside 19 nucleosides in length, wherein the longer oligonucleoside comprises a sequence of 17 nucleosides that is fully complementary to the shorter oligonucleoside, can yet be referred to as "fully complementary".

"Complementary" sequences, as used herein, can also include, or be formed entirely from, non-Watson-Crick base pairs or base pairs formed from non-natural and modified nucleosides, in so far as the above requirements with respect to their ability to hybridize are fulfilled. Such non-Watson-Crick base pairs include, but are not limited to, G:U Wobble or Hoogstein base pairing.

The terms "complementary," "fully complementary" and "substantially/partially complementary" herein can be used with respect to the base matching between the sense strand and the antisense strand of a nucleic acid e.g. dsiRNA, or between the antisense strand of a double stranded nucleic acid e.g. siRNA agent and a target sequence.

Within the present invention, the second strand of the nucleic acid according to the invention, in particular a dsiRNA for inhibiting SLC25A5/ANT2, is at least partially complementary to the first strand of said nucleic acid. In certain embodiments, a first and second strand of a nucleic acid according to the invention are partially complementary if they form a duplex region having a length of at least 17 base pairs and comprising not more than 1, 2, 3, 4, or 5 mismatched base pairs.

In certain embodiments, a first and second strand of the nucleic acid according to the invention are partially complementary if they form a duplex region having a length of 19 base pairs and comprising not more than 1, 2, 3, 4, or 5 mismatched base pairs. In certain embodiments, a first and second strand of the nucleic acid according to the invention are partially complementary if they form a duplex region having a length of 21 base pairs comprising not more than 1, 2, 3, 4, or 5 mismatched base pairs.

Alternatively, a first and second strand of the nucleic acid according to the invention are partially complementary if they form a duplex region having a length of at least 17 base pairs, wherein at least 14, 15, 16 or 17 of said base pairs are complementary base pairs, in particular Watson-Crick base pairs.

In certain embodiments, a first and second strand of the nucleic acid according to the invention are partially complementary if they form a duplex region having a length of 19 base pairs, wherein at least 14, 15, 16, 17, 18 or all 19 base pairs are complementary base pairs, in particular Watson-Crick base pairs. In certain embodiments, a first and second strand of the nucleic acid according to the invention are partially complementary if they form a duplex region having a length of 21 base pairs, wherein at least 16, 17, 18, 19, 20 or all 21 base pairs are complementary base pairs, in particular Watson-Crick base pairs. As used herein, a nucleic acid that is "substantially complementary" or "partially complementary" to at least part of a messenger RNA (mRNA) refers to a polynucleoside that is substantially or partially complementary to a contiguous portion of the mRNA of interest (e.g., an mRNA encoding a gene). In certain embodiments, the contiguous portion of the mRNA is a sequence as listed in Table 1, i.e., any one of SEQ ID NOs: 1-276. For example, a polynucleoside is complementary to at least a part of an mRNA of a gene of interest if the sequence is substantially or partially complementary to a non-interrupted portion of an mRNA encoding that gene.

Accordingly, in some preferred embodiments, the antisense oligonucleosides as disclosed herein are fully complementary to the target gene sequence.

In other embodiments, the antisense oligonucleosides disclosed herein are substantially or partially complementary to a target RNA sequence and comprise a contiguous nucleoside sequence which is at least about 80% complementary over its entire length to the equivalent region of the target RNA sequence, such as at least about 85%, 86%, 87%, 88%, 89%, about 90%, 91 %, 92%, 93%, 94%, 95%, 96%, 97%, 98%, or 99% complementary or 100% complementary.

In certain embodiments, the first (antisense) strand of a nucleic acid according to the invention is partially or fully complementary to a contiguous portion of RNA transcribed from the *SLC25A5* gene. In certain embodiments, the first strand of the nucleic acid according to the invention is partially or fully complementary to a contiguous portion of at least 17 nucleosides of the *SLC25A5* mRNA. In certain embodiments, the first strand of the nucleic acid according to the invention is partially or fully complementary to a contiguous portion of 17, 18, 19, 20, 21, 22 or 23 nucleosides of the *SLC25A5* mRNA. In certain embodiments, the first strand of the nucleic acid according to the invention is partially or fully complementary to a contiguous portion of 17, 18, 19, 20, 21, 22 or 23 nucleosides of any one of the sequences as listed in Table 1, i.e., any one of SEQ ID NOs: 1-276.

In certain embodiments, the first (antisense) strand of the nucleic acid according to the invention is partially complementary to a contiguous portion of the *SLC25A5* mRNA if it comprises a contiguous nucleoside sequence of at least 17 nucleosides, wherein at least 14, 15, 16 or 17 nucleosides of said contiguous nucleoside sequence are complementary to a contiguous portion of the *SLC25A5* mRNA. In certain embodiments, the first strand of the nucleic acid according to the invention comprises a contiguous nucleoside sequence of at least 17 nucleosides, wherein at least 14, 15, 16 or 17 nucleosides of said contiguous nucleoside sequence are complementary to a contiguous portion of any one of the sequences listed in Table 1, i.e., any one of SEQ ID NOs: 1-276. In certain embodiments, the first strand of the nucleic acid according to the invention comprises a contiguous nucleoside sequence of 19 nucleosides, wherein at least 14, 15, 16, 17, 18 or all 19 nucleosides of said contiguous nucleoside sequence are complementary to a contiguous portion of any one of the sequences listed in Table 1, i.e., any one of SEQ ID NOs: 1-276. In certain embodiments, the first strand of the nucleic acid according to the invention comprises a contiguous nucleoside sequence of 23 nucleosides, wherein at least 18, 19, 20, 21, 22 or all 23 nucleosides of said contiguous nucleoside sequence are complementary to a contiguous portion of any one of the sequences listed in Table 1, i.e., any one of SEQ ID NOs: 1-276.

In some embodiments, a nucleic acid e.g. an siRNA of the invention includes a sense strand that is substantially or partially complementary to an antisense polynucleoside which, in turn, is complementary to a target gene sequence and comprises a contiguous nucleoside sequence which is at least about 80% complementary over its entire length to the equivalent region of the nucleoside sequence of the antisense strand, such as about 85%, 86%, 87%, 88%, 89%, 90%, 91 %, 92%, 93%, 94%, 95%, 96%, 97%, 98%, or 99% complementary, or 100% complementary.

In some embodiments, a nucleic acid e.g. an siRNA of the invention includes an antisense strand that is substantially or partially complementary to the target sequence and comprises a contiguous nucleoside sequence which is at least 80% complementary over its entire length to the target sequence such as about 85%, 86%, 87%, 88%, 89%, 90%, 91 %, 92%, 93%, 94%, 95%, 96%, 97%, 98%, or 99% complementary, or 100% complementary.

As used herein, a "subject" is an animal, such as a mammal, including a primate (such as a human, a non-human primate, e.g., a monkey, and a chimpanzee), or a non-primate or a bird that expresses the target gene, either endogenously or heterologously, when the target gene sequence has sufficient complementarity to the nucleic acid e.g. iRNA agent to promote target knockdown. In certain preferred embodiments, the subject is a human.

The terms "treating" or "treatment" refer to a beneficial or desired result including, but not limited to, alleviation or amelioration of one or more symptoms associated with gene expression. "Treatment" can also mean prolonging survival as compared to expected survival in the absence of treatment. Treatment can include prevention of development of co-morbidities, e.g.reduced liver damage in a subject with a hepatic infection.

"Therapeutically effective amount," as used herein, is intended to include the amount of a nucleic acid e.g. an iRNA that, when administered to a patient for treating a subject having disease, is sufficient to effect treatment of the disease (e.g., by diminishing, ameliorating or maintaining the existing disease or one or more symptoms of disease or its related comorbidities).

The phrase "pharmaceutically acceptable" is employed herein to refer to compounds, materials, compositions, or dosage forms which are suitable for use in contact with the tissues of human subjects and animal subjects without excessive toxicity, irritation, allergic response, or other problem or complication, commensurate with a reasonable benefit/risk ratio.

The phrase "pharmaceutically-acceptable carrier" as used herein means a pharmaceutically-acceptable material, composition, or vehicle, such as a liquid or solid filler, diluent, excipient, manufacturing aid or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the subject being treated.

Where a value or range of values of a parameter are recited, it is intended that values and ranges intermediate to the recited values are also intended to be part of this invention.

The articles "a" and "an" are used herein to refer to one or to more than one (i.e. to at least one) of the grammatical object of the article.

The term "including" is used herein to mean, and is used interchangeably with, the phrase "including but not limited to".

The term "or" is used herein to mean, and is used interchangeably with, the term "and/or," unless context clearly indicates otherwise. For example, "sense strand or antisense strand" is understood as "sense strand or antisense strand or sense strand and antisense strand."

The term "about" is used herein to mean within the typical ranges of tolerances in the art. For example, "about" can be understood as about 2 standard deviations from the mean. In certain embodiments, about means +10%. In certain embodiments, about means +5%. When about is present before a series of numbers or a range, it is understood that "about" can modify each of the numbers in the series or range.

The term "at least" prior to a number or series of numbers is understood to include the number adjacent to the term "at least", and all subsequent numbers or integers that could logically be included, as clear from context. For example, the number of nucleotides in a nucleic acid molecule must be an integer. For example, "at least 18 nucleosides of a 21 nucleoside nucleic acid molecule" means that 18, 19, 20, or 21 nucleosides have the indicated property. When at least is present before a series of numbers or a range, it is understood that "at least" can modify each of the numbers in the series or range.

As used herein, "no more than" or "less than" is understood as the value adjacent to the phrase and logical lower values or integers, as logical from context, to zero. For example, a duplex with an overhang of "no more than 2 nucleosides" has a 2, 1, or 0 nucleoside overhang. When "no more than" is present before a series of numbers or a range, it is understood that "no more than" can modify each of the numbers in the series or range.

The terminal region of a strand is the last 5 nucleotides from the 5' or the 3' end.

A nucleobase sequence is the sequence of the bases of the nucleic acid in an oligomer.

Various embodiments of the invention can be combined as determined appropriate by one of skill in the art.

### TARGET

A target for inhibition disclosed herein may be, without limitation, an mRNA, polypeptide, protein, or gene.

These targets are a target the inhibition of which helps in the prevention and/or treatment of a metabolic disease or disorder associated with non-alcoholic fatty liver disease (NAFLD) and/or obesity and/or a disease or disorder associated with adipogenesis and/or for use in reducing adipogenesis.

The target for inhibition is the gene SLC25A5 or a gene product thereof, such as an mRNA transcribed from the SLC25A5 gene or the ANT2 protein, and inhibition may be effected by inhibition of expression or function of the SLC25A5/ANT2 gene or protein or both.

In a preferred embodiment, the target in an mRNA expressed from the SLC25A5 gene. Exemplary target sequences on the SLC25A5 mRNA are listed below in Table 1.

**Table 1**

| SEQ ID NO | Oligonucleotide mRNA target sequence 5' → 3' | Starting position on ENST00000317881.9 |
|---|---|---|
| SEQ ID NO: 1 | ACUAUUUACUGGUUGAAAAUGGG | 1072 |
| SEQ ID NO: 2 | UACAAAGGCAUUAUAGACUGCGU | 223 |
| SEQ ID NO: 3 | UAUGAUGAAAUCAAGAAGUACAC | 943 |
| SEQ ID NO: 4 | AUGAUGAUGGGACUCAAUUGUAU | 1146 |
| SEQ ID NO: 5 | AGGCAUUAUAGACUGCGUGGUCC | 228 |
| SEQ ID NO: 6 | CAGUCACUCCUGAUAAAUAACAA | 1178 |
| SEQ ID NO: 7 | CAAUACAAAGGCAUUAUAGACUG | 220 |
| SEQ ID NO: 8 | GAGCCGCCUACUUCGGUAUCUAU | 635 |
| SEQ ID NO: 9 | CGAGCCGCCUACUUCGGUAUCUA | 634 |
| SEQ ID NO: 10 | AUAGACUGCGUGGUCCGUAUUCC | 235 |
| SEQ ID NO: 11 | AACACUCACAUCGUCAUCAGCUG | 691 |
| SEQ ID NO: 12 | UAACUUCGCCUUCAAAGAUAAAU | 333 |
| SEQ ID NO: 13 | GCCUACUUCGGUAUCUAUGACAC | 640 |
| SEQ ID NO: 14 | UAUUUACUGGUUGAAAAUGGGAA | 1074 |
| SEQ ID NO: 15 | UUUGCCCGUACCCGUCUAGCAGC | 478 |
| SEQ ID NO: 16 | GCAGUCAGGGCGCAAAGGAACUG | 792 |
| SEQ ID NO: 17 | AUUUACUGGUUGAAAAUGGGAAG | 1075 |
| SEQ ID NO: 18 | CCGAGCCGCCUACUUCGGUAUCU | 633 |
| SEQ ID NO: 19 | GGCCUCGGUGACUGCCUGGUUAA | 538 |
| SEQ ID NO: 20 | CACAUCGUCAUCAGCUGGAUGAU | 697 |
| SEQ ID NO: 21 | UAUUUCAGUCACUCCUGAUAAAU | 1173 |
| SEQ ID NO: 22 | GCUGGAUGAUCGCACAGACUGUC | 710 |
| SEQ ID NO: 23 | AAGCAAUACAAAGGCAUUAUAGA | 217 |
| SEQ ID NO: 24 | AGGCCUCGGUGACUGCCUGGUUA | 537 |
| SEQ ID NO: 25 | GGCUUUAACGUGUCUGUGCAGGG | 598 |
| SEQ ID NO: 26 | ACAAAGGCAUUAUAGACUGCGUG | 224 |
| SEQ ID NO: 27 | CUGUGAACAGGCAUGUUGUAUUA | 993 |
| SEQ ID NO: 28 | UCAUCUGACCAGUUUUCUCUUAA | 1107 |
| SEQ ID NO: 29 | CAAGGCUUUAACGUGUCUGUGCA | 595 |
| SEQ ID NO: 30 | AUGCAGUCAGGGCGCAAAGGAAC | 790 |
| SEQ ID NO: 31 | UGGAUGAUCGCACAGACUGUCAC | 712 |
| SEQ ID NO: 32 | AAAGGCAUUAUAGACUGCGUGGU | 226 |
| SEQ ID NO: 33 | GAUGAUCGCACAGACUGUCACUG | 714 |
| SEQ ID NO: 34 | GACUGCGUGGUCCGUAUUCCCAA | 238 |
| SEQ ID NO: 35 | AGCAAUACAAAGGCAUUAUAGAC | 218 |
| SEQ ID NO: 36 | GUCAUCAGCUGGAUGAUCGCACA | 703 |
| SEQ ID NO: 37 | GCAUUAUAGACUGCGUGGUCCGU | 230 |
| SEQ ID NO: 38 | AUUUUGCCCGUACCCGUCUAGCA | 476 |
| SEQ ID NO: 39 | UCACAUCGUCAUCAGCUGGAUGA | 696 |
| SEQ ID NO: 40 | CCUCUUGAUUUUGCCCGUACCCG | 469 |
| SEQ ID NO: 41 | UUAUAGACUGCGUGGUCCGUAUU | 233 |
| SEQ ID NO: 42 | GCCGCCUACUUCGGUAUCUAUGA | 637 |
| SEQ ID NO: 43 | UAGACUGCGUGGUCCGUAUUCCC | 236 |
| SEQ ID NO: 44 | CGUCAUCAGCUGGAUGAUCGCAC | 702 |
| SEQ ID NO: 45 | CAUUAUAGACUGCGUGGUCCGUA | 231 |
| SEQ ID NO: 46 | CGCCUACUUCGGUAUCUAUGACA | 639 |
| SEQ ID NO: 47 | UUUUAUUUCAGUCACUCCUGAUA | 1170 |
| SEQ ID NO: 48 | UCGUCAUCAGCUGGAUGAUCGCA | 701 |
| SEQ ID NO: 49 | UGAUUUUGCCCGUACCCGUCUAG | 474 |
| SEQ ID NO: 50 | UGCAAAGGGAAUGCUUCCGGAUC | 663 |
| SEQ ID NO: 51 | CCAAGGCUUUAACGUGUCUGUGC | 594 |
| SEQ ID NO: 52 | CAAAGGCAUUAUAGACUGCGUGG | 225 |
| SEQ ID NO: 53 | UCUUGAUUUUGCCCGUACCCGUC | 471 |
| SEQ ID NO: 54 | ACCGAGCCGCCUACUUCGGUAUC | 632 |
| SEQ ID NO: 55 | UUAUCAUCUACCGAGCCGCCUAC | 623 |
| SEQ ID NO: 56 | CUUGAUUUUGCCCGUACCCGUCU | 472 |
| SEQ ID NO: 57 | AUUAUCAUCUACCGAGCCGCCUA | 622 |
| SEQ ID NO: 58 | AUCAUCUACCGAGCCGCCUACUU | 625 |
| SEQ ID NO: 59 | GGCUCUUAACUUCGCCUUCAAAG | 327 |
| SEQ ID NO: 60 | CCCCACCCAGGCUCUUAACUUCG | 318 |
| SEQ ID NO: 61 | GUCCGUAUUCCCAAGGAGCAGGG | 247 |
| SEQ ID NO: 62 | AGGAGCAGGGAGUUCUGUCCUUC | 260 |
| SEQ ID NO: 63 | AUUCCCAAGGAGCAGGGAGUUCU | 253 |
| SEQ ID NO: 64 | GGUCCGUAUUCCCAAGGAGCAGG | 246 |
| SEQ ID NO: 65 | GAGCAGGGAGUUCUGUCCUUCUG | 262 |
| SEQ ID NO: 66 | UGGUCCGUAUUCCCAAGGAGCAG | 245 |
| SEQ ID NO: 67 | GGAGCAGGGAGUUCUGUCCUUCU | 261 |
| SEQ ID NO: 68 | AAGGAGCAGGGAGUUCUGUCCUU | 259 |
| SEQ ID NO: 69 | UAUUCCCAAGGAGCAGGGAGUUC | 252 |
| SEQ ID NO: 70 | CAAGGAGCAGGGAGUUCUGUCCU | 258 |
| SEQ ID NO: 71 | UCCCCACCCAGGCUCUUAACUUC | 317 |
| SEQ ID NO: 72 | GCUCUUAACUUCGCCUUCAAAGA | 328 |
| SEQ ID NO: 73 | AUUUCAGUCACUCCUGAUAAAUA | 1174 |
| SEQ ID NO: 74 | UUCAGUCACUCCUGAUAAAUAAC | 1176 |
| SEQ ID NO: 75 | UUUCAGUCACUCCUGAUAAAUAA | 1175 |
| SEQ ID NO: 76 | GAUGAUGGGACUCAAUUGUAUUU | 1148 |
| SEQ ID NO: 77 | AUGAUGGGACUCAAUUGUAUUUU | 1149 |
| SEQ ID NO: 78 | GUCACUCCUGAUAAAUAACAAAU | 1180 |
| SEQ ID NO: 79 | AUAUUCAUCUGACCAGUUUUCUC | 1103 |
| SEQ ID NO: 80 | UGAACAGGCAUGUUGUAUUAUAU | 996 |
| SEQ ID NO: 81 | AAAGGGAAUGCUUCCGGAUCCCA | 666 |
| SEQ ID NO: 82 | AAGGGAAUGCUUCCGGAUCCCAA | 667 |
| SEQ ID NO: 83 | UCCGAGGCCUCGGUGACUGCCUG | 533 |
| SEQ ID NO: 84 | GAACAGGCAUGUUGUAUUAUAUA | 997 |
| SEQ ID NO: 85 | GCAAUACAAAGGCAUUAUAGACU | 219 |
| SEQ ID NO: 86 | UUAUUUCAGUCACUCCUGAUAAA | 1172 |
| SEQ ID NO: 87 | UGAUGAUGGGACUCAAUUGUAUU | 1147 |
| SEQ ID NO: 88 | AUCUGACCAGUUUUCUCUUAAAG | 1109 |
| SEQ ID NO: 89 | UCUGACCAGUUUUCUCUUAAAGC | 1110 |
| SEQ ID NO: 90 | CAGAUAAGCAAUACAAAGGCAUU | 212 |
| SEQ ID NO: 91 | AGAUAAGCAAUACAAAGGCAUUA | 213 |
| SEQ ID NO: 92 | CUUUAACGUGUCUGUGCAGGGUA | 600 |
| SEQ ID NO: 93 | UUUAACGUGUCUGUGCAGGGUAU | 601 |
| SEQ ID NO: 94 | ACUCACAUCGUCAUCAGCUGGAU | 694 |
| SEQ ID NO: 95 | GAUGAUGAUGGGACUCAAUUGUA | 1145 |
| SEQ ID NO: 96 | AGACUGCGUGGUCCGUAUUCCCA | 237 |
| SEQ ID NO: 97 | GUGAACAGGCAUGUUGUAUUAUA | 995 |
| SEQ ID NO: 98 | UUUAUUUCAGUCACUCCUGAUAA | 1171 |
| SEQ ID NO: 99 | GAUAAGCAAUACAAAGGCAUUAU | 214 |
| SEQ ID NO: 100 | ACACUCACAUCGUCAUCAGCUGG | 692 |
| SEQ ID NO: 101 | AGCCGCCUACUUCGGUAUCUAUG | 636 |
| SEQ ID NO: 102 | CUCACAUCGUCAUCAGCUGGAUG | 695 |
| SEQ ID NO: 103 | CCGCCUACUUCGGUAUCUAUGAC | 638 |
| SEQ ID NO: 104 | GCUUUAACGUGUCUGUGCAGGGU | 599 |
| SEQ ID NO: 105 | GUUCGCCGCCGCAUGAUGAUGCA | 772 |
| SEQ ID NO: 106 | GGCAUUAUAGACUGCGUGGUCCG | 229 |
| SEQ ID NO: 107 | ACUGCGUGGUCCGUAUUCCCAAG | 239 |
| SEQ ID NO: 108 | UCAUCUACCGAGCCGCCUACUUC | 626 |
| SEQ ID NO: 109 | UUUUGCCCGUACCCGUCUAGCAG | 477 |
| SEQ ID NO: 110 | CACUCACAUCGUCAUCAGCUGGA | 693 |
| SEQ ID NO: 111 | AUUAUAGACUGCGUGGUCCGUAU | 232 |
| SEQ ID NO: 112 | GAGGCCUCGGUGACUGCCUGGUU | 536 |
| SEQ ID NO: 113 | CUGGAUGAUCGCACAGACUGUCA | 711 |
| SEQ ID NO: 114 | UUGAUUUUGCCCGUACCCGUCUA | 473 |
| SEQ ID NO: 115 | AUGAUCGCACAGACUGUCACUGC | 715 |
| SEQ ID NO: 116 | GGAUGAUCGCACAGACUGUCACU | 713 |
| SEQ ID NO: 117 | GAUUUUGCCCGUACCCGUCUAGC | 475 |
| SEQ ID NO: 118 | UAUCAUCUACCGAGCCGCCUACU | 624 |
| SEQ ID NO: 119 | UAUAGACUGCGUGGUCCGUAUUC | 234 |
| SEQ ID NO: 120 | CACUGCAGAUAAGCAAUACAAAG | 207 |
| SEQ ID NO: 121 | UCACUGCAGAUAAGCAAUACAAA | 206 |
| SEQ ID NO: 122 | AUCACUGCAGAUAAGCAAUACAA | 205 |
| SEQ ID NO: 123 | CAGCAAGCAGAUCACUGCAGAUA | 195 |
| SEQ ID NO: 124 | AGAUCACUGCAGAUAAGCAAUAC | 203 |
| SEQ ID NO: 125 | GAUCACUGCAGAUAAGCAAUACA | 204 |
| SEQ ID NO: 126 | AAGCAGAUCACUGCAGAUAAGCA | 199 |
| SEQ ID NO: 127 | AGCAGAUCACUGCAGAUAAGCAA | 200 |
| SEQ ID NO: 128 | CCAGCAAGCAGAUCACUGCAGAU | 194 |
| SEQ ID NO: 129 | CAGAUCACUGCAGAUAAGCAAUA | 202 |
| SEQ ID NO: 130 | UGAUAAAUAACAAAUUUGGAGAA | 1188 |
| SEQ ID NO: 131 | ACUCCUGAUAAAUAACAAAUUUG | 1183 |
| SEQ ID NO: 132 | CUGAAAGGGAAUUCCGAGGCCUC | 521 |
| SEQ ID NO: 133 | AUGUUGUAUUAUAUAACAUAUCU | 1005 |
| SEQ ID NO: 134 | AAUUCCGAGGCCUCGGUGACUGC | 530 |
| SEQ ID NO: 135 | UGAAAGGGAAUUCCGAGGCCUCG | 522 |
| SEQ ID NO: 136 | GUUGUAUUAUAUAACAUAUCUUG | 1007 |
| SEQ ID NO: 137 | GGAAUUCCGAGGCCUCGGUGACU | 528 |
| SEQ ID NO: 138 | AAGGGAAUUCCGAGGCCUCGGUG | 525 |
| SEQ ID NO: 139 | GGAGCUGAAAGGGAAUUCCGAGG | 517 |
| SEQ ID NO: 140 | CUGGAGCUGAAAGGGAAUUCCGA | 515 |
| SEQ ID NO: 141 | AGCUGAAAGGGAAUUCCGAGGCC | 519 |
| SEQ ID NO: 142 | UGGAGCUGAAAGGGAAUUCCGAG | 516 |
| SEQ ID NO: 143 | GAGCUGAAAGGGAAUUCCGAGGC | 518 |
| SEQ ID NO: 144 | AGGCAUGUUGUAUUAUAUAACAU | 1001 |
| SEQ ID NO: 145 | UGUUGUAUUAUAUAACAUAUCUU | 1006 |
| SEQ ID NO: 146 | UUGUAUUAUAUAACAUAUCUUGA | 1008 |
| SEQ ID NO: 147 | CAGGCAUGUUGUAUUAUAUAACA | 1000 |
| SEQ ID NO: 148 | GAAAGGGAAUUCCGAGGCCUCGG | 523 |
| SEQ ID NO: 149 | GGGAAUUCCGAGGCCUCGGUGAC | 527 |
| SEQ ID NO: 150 | AAAGGGAAUUCCGAGGCCUCGGU | 524 |
| SEQ ID NO: 151 | AGGGAAUUCCGAGGCCUCGGUGA | 526 |
| SEQ ID NO: 152 | GUGCUUGUCUUGUAUGAUGAAAU | 931 |
| SEQ ID NO: 153 | CUUCGCCUUCAAAGAUAAAUACA | 336 |
| SEQ ID NO: 154 | CUGCCUGGUUAAGAUCUACAAAU | 549 |
| SEQ ID NO: 155 | UAUCUAUGACACUGCAAAGGGAA | 651 |
| SEQ ID NO: 156 | CUUUUGUGCUUGUCUUGUAUGAU | 926 |
| SEQ ID NO: 157 | GGUGACUGCCUGGUUAAGAUCUA | 544 |
| SEQ ID NO: 158 | AUGACACUGCAAAGGGAAUGCUU | 656 |
| SEQ ID NO: 159 | UUCGGUAUCUAUGACACUGCAAA | 646 |
| SEQ ID NO: 160 | UAUUCAUCUGACCAGUUUUCUCU | 1104 |
| SEQ ID NO: 161 | ACUUCGGUAUCUAUGACACUGCA | 644 |
| SEQ ID NO: 162 | CUGGUUAAGAUCUACAAAUCUGA | 553 |
| SEQ ID NO: 163 | GUAUCUAUGACACUGCAAAGGGA | 650 |
| SEQ ID NO: 164 | CAAGGGUGCAUGGUCCAAUGUUC | 885 |
| SEQ ID NO: 165 | AGAUCUACAAAUCUGAUGGGAUU | 560 |
| SEQ ID NO: 166 | UACUUCGGUAUCUAUGACACUGC | 643 |
| SEQ ID NO: 167 | CAUGGGUGGUGCUUUUGUGCUUG | 915 |
| SEQ ID NO: 168 | GGUAUUAUCAUCUACCGAGCCGC | 619 |
| SEQ ID NO: 169 | GCGUGGUCCGUAUUCCCAAGGAG | 242 |
| SEQ ID NO: 170 | AUCUAUGACACUGCAAAGGGAAU | 652 |
| SEQ ID NO: 171 | UGCUUUUGUGCUUGUCUUGUAUG | 924 |
| SEQ ID NO: 172 | UGUACCAAGGCUUUAACGUGUCU | 590 |
| SEQ ID NO: 173 | GGGUGCAUGGUCCAAUGUUCUCA | 888 |
| SEQ ID NO: 174 | CGCCUUCAAAGAUAAAUACAAGC | 339 |
| SEQ ID NO: 175 | CAGGGUAUUAUCAUCUACCGAGC | 616 |
| SEQ ID NO: 176 | CCUACUUCGGUAUCUAUGACACU | 641 |
| SEQ ID NO: 177 | UGGUUAAGAUCUACAAAUCUGAU | 554 |
| SEQ ID NO: 178 | UGUGCAGGGUAUUAUCAUCUACC | 612 |
| SEQ ID NO: 179 | AAUCUGAUGGGAUUAAGGGCCUG | 569 |
| SEQ ID NO: 180 | GUCUGUGCAGGGUAUUAUCAUCU | 609 |
| SEQ ID NO: 181 | UCUGAUGGGAUUAAGGGCCUGUA | 571 |
| SEQ ID NO: 182 | UACCAAGGCUUUAACGUGUCUGU | 592 |
| SEQ ID NO: 183 | AAUAUUCAUCUGACCAGUUUUCU | 1102 |
| SEQ ID NO: 184 | UGACUGCCUGGUUAAGAUCUACA | 546 |
| SEQ ID NO: 185 | GGGUUGACUUCCUAUCCAUUUGA | 745 |
| SEQ ID NO: 186 | UCUAUGACACUGCAAAGGGAAUG | 653 |
| SEQ ID NO: 187 | GGUUAAGAUCUACAAAUCUGAUG | 555 |
| SEQ ID NO: 188 | GCAGGGUAUUAUCAUCUACCGAG | 615 |
| SEQ ID NO: 189 | ACUGCCUGGUUAAGAUCUACAAA | 548 |
| SEQ ID NO: 190 | AUCUGAUGGGAUUAAGGGCCUGU | 570 |
| SEQ ID NO: 191 | UCGGUAUCUAUGACACUGCAAAG | 647 |
| SEQ ID NO: 192 | GUACCCGUCUAGCAGCUGAUGUG | 485 |
| SEQ ID NO: 193 | GGUAUCUAUGACACUGCAAAGGG | 649 |
| SEQ ID NO: 194 | AGCUGAUGUGGGUAAAGCUGGAG | 498 |
| SEQ ID NO: 195 | CGUCUAGCAGCUGAUGUGGGUAA | 490 |
| SEQ ID NO: 196 | AAGGGCCUGUACCAAGGCUUUAA | 583 |
| SEQ ID NO: 197 | UGCCCGUACCCGUCUAGCAGCUG | 480 |
| SEQ ID NO: 198 | AGCAGCUGAUGUGGGUAAAGCUG | 495 |
| SEQ ID NO: 199 | GCCCGUACCCGUCUAGCAGCUGA | 481 |
| SEQ ID NO: 200 | CAGCUGAUGUGGGUAAAGCUGGA | 497 |
| SEQ ID NO: 201 | AGGGUAUUAUCAUCUACCGAGCC | 617 |
| SEQ ID NO: 202 | UAUUAUCAUCUACCGAGCCGCCU | 621 |
| SEQ ID NO: 203 | CCGUACCCGUCUAGCAGCUGAUG | 483 |
| SEQ ID NO: 204 | CUGAUGGGAUUAAGGGCCUGUAC | 572 |
| SEQ ID NO: 205 | GUGUCUGUGCAGGGUAUUAUCAU | 607 |
| SEQ ID NO: 206 | CUGCGUGGUCCGUAUUCCCAAGG | 240 |
| SEQ ID NO: 207 | UGCGUGGUCCGUAUUCCCAAGGA | 241 |
| SEQ ID NO: 208 | CCUGGUUAAGAUCUACAAAUCUG | 552 |
| SEQ ID NO: 209 | GUAUGAUGAAAUCAAGAAGUACA | 942 |
| SEQ ID NO: 210 | AAGAUCUACAAAUCUGAUGGGAU | 559 |
| SEQ ID NO: 211 | CUGUGCAGGGUAUUAUCAUCUAC | 611 |
| SEQ ID NO: 212 | CUGUACCAAGGCUUUAACGUGUC | 589 |
| SEQ ID NO: 213 | UAGCAGCUGAUGUGGGUAAAGCU | 494 |
| SEQ ID NO: 214 | GCAUGGGUGGUGCUUUUGUGCUU | 914 |
| SEQ ID NO: 215 | CUCGGUGACUGCCUGGUUAAGAU | 541 |
| SEQ ID NO: 216 | GUCUAGCAGCUGAUGUGGGUAAA | 491 |
| SEQ ID NO: 217 | UCUACAAAUCUGAUGGGAUUAAG | 563 |
| SEQ ID NO: 218 | CUACUUCGGUAUCUAUGACACUG | 642 |
| SEQ ID NO: 219 | GCAGCUGAUGUGGGUAAAGCUGG | 496 |
| SEQ ID NO: 220 | CCCGUACCCGUCUAGCAGCUGAU | 482 |
| SEQ ID NO: 221 | UGUCUGUGCAGGGUAUUAUCAUC | 608 |
| SEQ ID NO: 222 | UGUGCUUGUCUUGUAUGAUGAAA | 930 |
| SEQ ID NO: 223 | GUACCAAGGCUUUAACGUGUCUG | 591 |
| SEQ ID NO: 224 | AAGGGUGCAUGGUCCAAUGUUCU | 886 |
| SEQ ID NO: 225 | GUGACUGCCUGGUUAAGAUCUAC | 545 |
| SEQ ID NO: 226 | CUGAUGUGGGUAAAGCUGGAGCU | 500 |
| SEQ ID NO: 227 | CGUGGUCCGUAUUCCCAAGGAGC | 243 |
| SEQ ID NO: 228 | UGACACUGCAAAGGGAAUGCUUC | 657 |
| SEQ ID NO: 229 | GGGAUUAAGGGCCUGUACCAAGG | 577 |
| SEQ ID NO: 230 | UCUGUGCAGGGUAUUAUCAUCUA | 610 |
| SEQ ID NO: 231 | UUAACGUGUCUGUGCAGGGUAUU | 602 |
| SEQ ID NO: 232 | AGGGCCUGUACCAAGGCUUUAAC | 584 |
| SEQ ID NO: 233 | UAUGACACUGCAAAGGGAAUGCU | 655 |
| SEQ ID NO: 234 | CCUCGGUGACUGCCUGGUUAAGA | 540 |
| SEQ ID NO: 235 | GUGCUUUUGUGCUUGUCUUGUAU | 923 |
| SEQ ID NO: 236 | UGGGUGGUGCUUUUGUGCUUGUC | 917 |
| SEQ ID NO: 237 | CCCGUCUAGCAGCUGAUGUGGGU | 488 |
| SEQ ID NO: 238 | UAAGGGCCUGUACCAAGGCUUUA | 582 |
| SEQ ID NO: 239 | UUGUGCUUGUCUUGUAUGAUGAA | 929 |
| SEQ ID NO: 240 | ACAAAUCUGAUGGGAUUAAGGGC | 566 |
| SEQ ID NO: 241 | AUCUACAAAUCUGAUGGGAUUAA | 562 |
| SEQ ID NO: 242 | UGCAGGGUAUUAUCAUCUACCGA | 614 |
| SEQ ID NO: 243 | GCCUGUACCAAGGCUUUAACGUG | 587 |
| SEQ ID NO: 244 | UAAGAUCUACAAAUCUGAUGGGA | 558 |
| SEQ ID NO: 245 | GCUUUUGUGCUUGUCUUGUAUGA | 925 |
| SEQ ID NO: 246 | CUACAAAUCUGAUGGGAUUAAGG | 564 |
| SEQ ID NO: 247 | UUCAAGGGUGCAUGGUCCAAUGU | 883 |
| SEQ ID NO: 248 | UGGGAUUAAGGGCCUGUACCAAG | 576 |
| SEQ ID NO: 249 | CUUCGGUAUCUAUGACACUGCAA | 645 |
| SEQ ID NO: 250 | ACCAAGGCUUUAACGUGUCUGUG | 593 |
| SEQ ID NO: 251 | GGCAUGGGUGGUGCUUUUGUGCU | 913 |
| SEQ ID NO: 252 | GUUAAGAUCUACAAAUCUGAUGG | 556 |
| SEQ ID NO: 253 | UACCCGUCUAGCAGCUGAUGUGG | 486 |
| SEQ ID NO: 254 | CUAUGACACUGCAAAGGGAAUGC | 654 |
| SEQ ID NO: 255 | UCUAGCAGCUGAUGUGGGUAAAG | 492 |
| SEQ ID NO: 256 | CUGCAAAGGGAAUGCUUCCGGAU | 662 |
| SEQ ID NO: 257 | UUGCCCGUACCCGUCUAGCAGCU | 479 |
| SEQ ID NO: 258 | GGGUAUUAUCAUCUACCGAGCCG | 618 |
| SEQ ID NO: 259 | CCCUCUUGAUUUUGCCCGUACCC | 468 |
| SEQ ID NO: 260 | CGUACCCGUCUAGCAGCUGAUGU | 484 |
| SEQ ID NO: 261 | UACAAAUCUGAUGGGAUUAAGGG | 565 |
| SEQ ID NO: 262 | GUGUACCCUCUUGAUUUUGCCCG | 463 |
| SEQ ID NO: 263 | GGCCUGUACCAAGGCUUUAACGU | 586 |
| SEQ ID NO: 264 | GAUGGGAUUAAGGGCCUGUACCA | 574 |
| SEQ ID NO: 265 | GUAUUAUCAUCUACCGAGCCGCC | 620 |
| SEQ ID NO: 266 | GGGCCUGUACCAAGGCUUUAACG | 585 |
| SEQ ID NO: 267 | UGAUGGGAUUAAGGGCCUGUACC | 573 |
| SEQ ID NO: 268 | UAACGUGUCUGUGCAGGGUAUUA | 603 |
| SEQ ID NO: 269 | AAGGCAUUAUAGACUGCGUGGUC | 227 |
| SEQ ID NO: 270 | ACUUCCCCACCCAGGCUCUUAAC | 314 |
| SEQ ID NO: 271 | CAAUGUCAUCAGAUACUUCCCCA | 300 |
| SEQ ID NO: 272 | AUGUCAUCAGAUACUUCCCCACC | 302 |
| SEQ ID NO: 273 | AAUGUCAUCAGAUACUUCCCCAC | 301 |
| SEQ ID NO: 274 | CCAAUGUCAUCAGAUACUUCCCC | 299 |
| SEQ ID NO: 275 | UACUUCCCCACCCAGGCUCUUAA | 313 |
| SEQ ID NO: 276 | AUCUACCGAGCCGCCUACUUCGG | 628 |

It is to be understood that SEQ ID NOs: 1 to 276 relate to human (Homo sapiens) mRNA sequences.

### DISEASE/CONDITIONS

The present invention further provides methods of treatment of a subject in need thereof. The treatment methods of the invention include administering, in a therapeutically effective amount, an inhibitor of expression of the SLC25A5 gene, such as an siRNA that targets an mRNA transcribed from the SLC25A5 gene, to a subject, e.g., a subject that would benefit from a reduction or inhibition of the expression of the SLC25A5 gene. Alternatively, the treatment methods of the invention include administering, in a therapeutically effective amount, an inhibitor of function of the gene product of SLC25A5 gene, i.e., the protein ANT2, to a subject, e.g., a subject that would benefit from a reduction or inhibition of the function of ANT2.

The disease to be treated is related to a metabolic disease or disorder, such as a metabolic disease or disorder associated with non-alcoholic fatty liver disease (NAFLD) and/or obesity and/or a disease or disorder associated with adipogenesis and/or adipogenesis.

The inhibitor according to the invention may be used in the prevention and/or treatment of a metabolic disease or disorder. As used herein, the term "metabolic disease" refers to a disease or condition affecting a metabolic process in a subject and is often caused by disruption of normal metabolism.

The patient to be treated may be a patient that already has a metabolic disease or disorder or that is at risk of developing a metabolic disease or disorder. That is, in certain embodiments, the inhibitor of the present invention may be used in the treatment and/or management of an existing metabolic disease or disorder. Treatment and/or management of an existing metabolic disease or disorder with the inhibitor of the present invention may prevent worsening of the metabolic disease or disorder and/or reverse the metabolic disease or disorder. In some instances, treatment of an existing metabolic disease or disorder with the inhibitor of the present invention may even cure the metabolic disease or disorder. In certain embodiments, the inhibitor of the present invention may be used to prevent manifestation of a metabolic disease or disorder in a patient that is at risk of developing a metabolic disease or disorder.

The skilled person is capable of diagnosing whether a patient has a metabolic disease or disorder or is at risk of developing a metabolic disease or disorder. For example, a metabolic disease or disorder may be diagnosed based weight gain and/or one or more blood markers, including, without limitation, blood glucose levels, blood insulin levels, blood free fatty acid levels, blood HbAlc levels, blood fibrinogen levels, blood cholesterol levels and blood triglyceride levels. The skilled person is aware of threshold values of one or more blood markers that indicate the presence of a metabolic disease or disorder or the risk of developing a metabolic disease or disorder.

In certain embodiments, the metabolic disease is fatty liver disease, in particular non-alcoholic fatty liver disease (NAFLD). As used herein "fatty-liver disease" refers to a disease wherein fat is excessively accumulated in the liver and can cause severe diseases such as chronic hepatitis and hepatic cirrhosis. In patients with fatty liver disease, lipids, particularly neutral fat, accumulate in hepatocytes to the extent that the amount exceeds the physiologically permissible range. From a biochemical point of view, a standard for judgment of fatty liver is that the weight of neutral fat is about 10% (100 mg/g wet weight) or more of the wet weight of hepatic tissue. Fatty liver disease is generally detected by observation of elevated serum levels of liver-specific enzymes such as the transaminases ALT and AST, which serve as indices of hepatocyte injury, as well as by presentation of symptoms, which include fatigue and pain in the region of the liver, though definitive diagnosis often requires a biopsy and may be supported by imaging such as ultrasound and/or MRI. The term "NAFLD" or "non-alcoholic fatty liver disease", as used herein, relates to a condition occurring when fat is deposited in the liver (steatosis) not due to excessive alcohol use. It is related to insulin resistance and the metabolic syndrome.

In a preferred embodiment, the fatty liver disease is Non-Alcoholic SteatoHepatitis (NASH). NASH, as used herein, refers to a liver disease characterized by an accumulation of fat (lipid droplets), along with inflammation and degeneration of hepatocytes. Once initiated, the disease is accompanied with a high risk of cirrhosis, a state wherein liver functions are altered that can progress to liver insufficiency. Thereafter, NASH often progresses to liver cancer.

In certain embodiments, the metabolic disease is obesity. The term "obesity" as used herein refers to a condition in which the natural energy reserve, stored in the fatty tissue of animals, in particular humans and other mammals, is increased to a point where it is associated with certain health conditions or increased mortality. The term "obese" as used herein is defined for an adult human as having a body mass index (BMI) greater than 30. Obesity is commonly associated with excessive body weight gain, in particular diet-induced body weight gain. "(Diet-induced) body weight gain" is defined herein as body weight gain resulting from an excessive dietary intake, including an excessive dietary intake of fat, in particular saturated fat, and optionally an excessive dietary intake of simple sugars, including sucrose and fructose. For a given subject, an excessive dietary intake, in particular of fat and optionally of simple sugars, refers to the consumption of an amount of diet, in particular of fat and optionally of simple sugars, higher than the amount necessary to meet the physiological needs and maintain the energy balance of said subject. The effect of a treatment on reduction of - or prevention - of diet-induced body weight gain in a subject can be assessed by comparing body weight gain observed in a subject receiving the treatment with those observed in the same subject without treatment receiving the same diet and having the same level of physical activity.

In certain embodiments, a patient is at risk of developing obesity if the patient has a BMI greater than 25. In certain embodiments, a patient is obese if the patient has a BMI greater than 30.

The term "body mass index" as used herein means the ratio of weight in kg divided by the height in metres, squared.

A "disease associated with adipogenesis" refers to a medical condition characterized by the abnormal proliferation and differentiation of adipocytes (fat cells) within the body, leading to an excessive accumulation of adipose tissue. This condition often results in health complications such as obesity, metabolic disorders, and associated comorbidities.

Within the present invention, a "reduction of adipogenesis" refers to a medical or pharmaceutical intervention designed to decrease the formation and accumulation of adipocytes within the body. A reduction in adipogenesis may be determined and/or quantified based on the size and/or number of adipocytes in a tissue sample obtained from a patient. Alternatively, or in addition, a reduction in adipogenesis may be determined and/or quantified by gene expression analysis, measurement of adipogenic markers (i.e., by ELISA), assessment of lipid accumulation in a sample and/or measurement of triglyceride levels in cells or tissues.

Thus, in a particular embodiment, the invention relates to an inhibitor suitable for use, or for use, in prevention and/or treatment of metabolic disease or disorder, such as a metabolic disease or disorder associated with non-alcoholic fatty liver disease (NAFLD) and/or obesity and/or a disease or disorder associated with adipogenesis and/or for use in reducing adipogenesis.

### INHIBITORS

Inhibitors of the invention include nucleic acids such as siRNAs, antibodies and antigen binding fragments thereof, e.g., monoclonal antibodies, polypeptides, antibody-drug conjugates, and small molecules. Preferred are nucleic acids such as siRNA.

Certain preferred features of inhibitors of the invention, where these are oligonucelosides such as siRNA, are given below.

In certain embodiments, the nucleic acid comprises a first strand comprising a sequence that is at least partially complementary to a portion of RNA transcribed from the *SLC25A5* gene (SEQ ID NO: 1381). In a preferred embodiment, the nucleic acid comprises a first strand comprising a sequence that is at least partially complementary to an SLC25A5 mRNA.

In certain embodiments, the nucleic acid for inhibiting expression of the *SLC25A5* gene comprises a duplex region that comprises a first strand and a second strand that is at least partially complementary to the first strand, wherein said first strand is:
(i) at least partially complementary to a portion of RNA transcribed from the *SLC25A5* gene, and
(ii) comprises at least 17 contiguous nucleosides differing by 0 or 1 nucleosides from any one of SEQ ID NO:277-552.

In certain embodiments, the first strand comprises nucleosides 2-18 of any one of the sequences set forth in SEQ ID NO: 277-552.

In certain embodiments, the first strand comprises any one of SEQ ID NO: 277-552.

In certain embodiments, the nucleic acid for inhibiting expression of the *SLC25A5* gene comprises a duplex region that comprises a first strand and a second strand that is at least partially complementary to the first strand, wherein said first strand is:
(i) at least partially complementary to a portion of RNA transcribed from the *SLC25A5* gene, and
(ii) comprises at least 21 contiguous nucleosides differing by 0 or 1 nucleosides from any one of SEQ ID NO: 277-552.

In certain embodiments, the first strand comprises nucleosides 2-22 of any one of the sequences set forth in SEQ ID NO: 277-552.

In certain embodiments, the second strand comprises a nucleoside sequence of at least 17 contiguous nucleosides differing by 0 or 1 nucleosides from any one of SEQ ID NO:553-828; wherein the second strand has a region of at least 85% complementarity over the 17 contiguous nucleosides to the first strand.

In certain embodiments, the second strand comprises any one of SEQ ID NO: 553-828.

In certain embodiments, the second strand comprises a nucleoside sequence of at least 19 contiguous nucleosides differing by 0 or 1 nucleosides from any one of SEQ ID NO: 553-828; wherein the second strand has a region of at least 85% complementarity over the 19 contiguous nucleosides to the first strand.

In certain embodiments, the second strand comprises a nucleoside sequence of at least 21 contiguous nucleosides differing by 0 or 1 nucleosides from any one of SEQ ID NO: 553-828; wherein the second strand has a region of at least 85% complementarity over the 21 contiguous nucleosides to the first strand.

In certain embodiments, the nucleic acid comprises a first strand that comprises, consists of, or consists essentially of a nucleoside sequence differing by 0 or 1 nucleosides from any one of SEQ ID NO: 277-552;
and a second strand that comprises, consists of, or consists essentially of a nucleoside sequence differing by 0 or 1 nucleosides from any one of SEQ ID NO: 553-828.

It is preferred herein that the duplex region is formed between a first (antisense) strand and a complementary second (sense) strand. Exemplary pairs of complementary antisense and sense strands are listed in Table 2 below:

**Table 2:**

| SEQ ID NO (AS) | Antisense Strand Base Sequence 5' → 3' (Shown as an Unmodified Nucleotide Sequence) | SEQ ID NO (SS) | Sense Strand Base Sequence 5' → 3' (Shown as an Unmodified Nucleotide Sequence) | Correspond! ng positions on ENST00000 317881.9 |
|---|---|---|---|---|
| SEQ ID NO: 277 | CCCAUUUUCAACCAGUAAAUAGU | SEQ ID NO: 553 | | 1072-1095 |
| SEQ ID NO: 278 | ACGCAGUCUAUAAUGCCUUUGUA | SEQ ID NO: 554 | | 223-246 |
| SEQ ID NO: 279 | GUGUACUUCUUGAUUUCAUCAUA | SEQ ID NO: 555 | | 943-966 |
| SEQ ID NO: 280 | AUACAAUUGAGUCCCAUCAUCAU | SEQ ID NO: 556 | | 1146-1169 |
| SEQ ID NO: 281 | GGACCACGCAGUCUAUAAUGCCU | SEQ ID NO: 557 | | 228-251 |
| SEQ ID NO: 282 | UUGUUAUUUAUCAGGAGUGACUG | SEQ ID NO: 558 | | 1178-1201 |
| SEQ ID NO: 283 | CAGUCUAUAAUGCCUUUGUAUUG | SEQ ID NO: 559 | | 220-243 |
| SEQ ID NO: 284 | AUAGAUACCGAAGUAGGCGGCUC | SEQ ID NO: 560 | | 635-658 |
| SEQ ID NO: 285 | UAGAUACCGAAGUAGGCGGCUCG | SEQ ID NO: 561 | | 634-657 |
| SEQ ID NO: 286 | GGAAUACGGACCACGCAGUCUAU | SEQ ID NO: 562 | | 235-258 |
| SEQ ID NO: 287 | CAGCUGAUGACGAUGUGAGUGUU | SEQ ID NO: 563 | | 691-714 |
| SEQ ID NO: 288 | AUUUAUCUUUGAAGGCGAAGUUA | SEQ ID NO: 564 | | 333-356 |
| SEQ ID NO: 289 | GUGUCAUAGAUACCGAAGUAGGC | SEQ ID NO: 565 | | 640-663 |
| SEQ ID NO: 290 | UUCCCAUUUUCAACCAGUAAAUA | SEQ ID NO: 566 | | 1074-1097 |
| SEQ ID NO: 291 | GCUGCUAGACGGGUACGGGCAAA | SEQ ID NO: 567 | | 478-501 |
| SEQ ID NO: 292 | CAGUUCCUUUGCGCCCUGACUGC | SEQ ID NO: 568 | | 792-815 |
| SEQ ID NO: 293 | CUUCCCAUUUUCAACCAGUAAAU | SEQ ID NO: 569 | | 1075-1098 |
| SEQ ID NO: 294 | AGAUACCGAAGUAGGCGGCUCGG | SEQ ID NO: 570 | | 633-656 |
| SEQ ID NO: 295 | UUAACCAGGCAGUCACCGAGGCC | SEQ ID NO: 571 | | 538-561 |
| SEQ ID NO: 296 | AUCAUCCAGCUGAUGACGAUGUG | SEQ ID NO: 572 | | 697-720 |
| SEQ ID NO: 297 | AUUUAUCAGGAGUGACUGAAAUA | SEQ ID NO: 573 | | 1173-1196 |
| SEQ ID NO: 298 | GACAGUCUGUGCGAUCAUCCAGC | SEQ ID NO: 574 | | 710-733 |
| SEQ ID NO: 299 | UCUAUAAUGCCUUUGUAUUGCUU | SEQ ID NO: 575 | | 217-240 |
| SEQ ID NO: 300 | UAACCAGGCAGUCACCGAGGCCU | SEQ ID NO: 576 | | 537-560 |
| SEQ ID NO: 301 | CCCUGCACAGACACGUUAAAGCC | SEQ ID NO: 577 | | 598-621 |
| SEQ ID NO: 302 | CACGCAGUCUAUAAUGCCUUUGU | SEQ ID NO: 578 | | 224-247 |
| SEQ ID NO: 303 | UAAUACAACAUGCCUGUUCACAG | SEQ ID NO: 579 | | 993-1016 |
| SEQ ID NO: 304 | UUAAGAGAAAACUGGUCAGAUGA | SEQ ID NO: 580 | | 1107-1130 |
| SEQ ID NO: 305 | UGCACAGACACGUUAAAGCCUUG | SEQ ID NO: 581 | | 595-618 |
| SEQ ID NO: 306 | GUUCCUUUGCGCCCUGACUGCAU | SEQ ID NO: 582 | | 790-813 |
| SEQ ID NO: 307 | GUGACAGUCUGUGCGAUCAUCCA | SEQ ID NO: 583 | | 712-735 |
| SEQ ID NO: 308 | ACCACGCAGUCUAUAAUGCCUUU | SEQ ID NO: 584 | | 226-249 |
| SEQ ID NO: 309 | CAGUGACAGUCUGUGCGAUCAUC | SEQ ID NO: 585 | | 714-737 |
| SEQ ID NO: 310 | UUGGGAAUACGGACCACGCAGUC | SEQ ID NO: 586 | | 238-261 |
| SEQ ID NO: 311 | GUCUAUAAUGCCUUUGUAUUGCU | SEQ ID NO: 587 | | 218-241 |
| SEQ ID NO: 312 | UGUGCGAUCAUCCAGCUGAUGAC | SEQ ID NO: 588 | | 703-726 |
| SEQ ID NO: 313 | ACGGACCACGCAGUCUAUAAUGC | SEQ ID NO: 589 | | 230-253 |
| SEQ ID NO: 314 | UGCUAGACGGGUACGGGCAAAAU | SEQ ID NO: 590 | | 476-499 |
| SEQ ID NO: 315 | UCAUCCAGCUGAUGACGAUGUGA | SEQ ID NO: 591 | | 696-719 |
| SEQ ID NO: 316 | CGGGUACGGGCAAAAUCAAGAGG | SEQ ID NO: 592 | | 469-492 |
| SEQ ID NO: 317 | AAUACGGACCACGCAGUCUAUAA | SEQ ID NO: 593 | | 233-256 |
| SEQ ID NO: 318 | UCAUAGAUACCGAAGUAGGCGGC | SEQ ID NO: 594 | | 637-660 |
| SEQ ID NO: 319 | GGGAAUACGGACCACGCAGUCUA | SEQ ID NO: 595 | | 236-259 |
| SEQ ID NO: 320 | GUGCGAUCAUCCAGCUGAUGACG | SEQ ID NO: 596 | | 702-725 |
| SEQ ID NO: 321 | UACGGACCACGCAGUCUAUAAUG | SEQ ID NO: 597 | | 231-254 |
| SEQ ID NO: 322 | UGUCAUAGAUACCGAAGUAGGCG | SEQ ID NO: 598 | | 639-662 |
| SEQ ID NO: 323 | UAUCAGGAGUGACUGAAAUAAAA | SEQ ID NO: 599 | | 1170-1193 |
| SEQ ID NO: 324 | UGCGAUCAUCCAGCUGAUGACGA | SEQ ID NO: 600 | | 701-724 |
| SEQ ID NO: 325 | CUAGACGGGUACGGGCAAAAUCA | SEQ ID NO: 601 | | 474-497 |
| SEQ ID NO: 326 | GAUCCGGAAGCAUUCCCUUUGCA | SEQ ID NO: 602 | | 663-686 |
| SEQ ID NO: 327 | GCACAGACACGUUAAAGCCUUGG | SEQ ID NO: 603 | | 594-617 |
| SEQ ID NO: 328 | CCACGCAGUCUAUAAUGCCUUUG | SEQ ID NO: 604 | | 225-248 |
| SEQ ID NO: 329 | GACGGGUACGGGCAAAAUCAAGA | SEQ ID NO: 605 | | 471-494 |
| SEQ ID NO: 330 | GAUACCGAAGUAGGCGGCUCGGU | SEQ ID NO: 606 | | 632-655 |
| SEQ ID NO: 331 | GUAGGCGGCUCGGUAGAUGAUAA | SEQ ID NO: 607 | | 623-646 |
| SEQ ID NO: 332 | AGACGGGUACGGGCAAAAUCAAG | SEQ ID NO: 608 | | 472-495 |
| SEQ ID NO: 333 | UAGGCGGCUCGGUAGAUGAUAAU | SEQ ID NO: 609 | | 622-645 |
| SEQ ID NO: 334 | AAGUAGGCGGCUCGGUAGAUGAU | SEQ ID NO: 610 | | 625-648 |
| SEQ ID NO: 335 | CUUUGAAGGCGAAGUUAAGAGCC | SEQ ID NO: 611 | | 327-350 |
| SEQ ID NO: 336 | CGAAGUUAAGAGCCUGGGUGGGG | SEQ ID NO: 612 | | 318-341 |
| SEQ ID NO: 337 | CCCUGCUCCUUGGGAAUACGGAC | SEQ ID NO: 613 | | 247-270 |
| SEQ ID NO: 338 | GAAGGACAGAACUCCCUGCUCCU | SEQ ID NO: 614 | | 260-283 |
| SEQ ID NO: 339 | AGAACUCCCUGCUCCUUGGGAAU | SEQ ID NO: 615 | | 253-276 |
| SEQ ID NO: 340 | CCUGCUCCUUGGGAAUACGGACC | SEQ ID NO: 616 | | 246-269 |
| SEQ ID NO: 341 | CAGAAGGACAGAACUCCCUGCUC | SEQ ID NO: 617 | | 262-285 |
| SEQ ID NO: 342 | CUGCUCCUUGGGAAUACGGACCA | SEQ ID NO: 618 | | 245-268 |
| SEQ ID NO: 343 | AGAAGGACAGAACUCCCUGCUCC | SEQ ID NO: 619 | | 261-284 |
| SEQ ID NO: 344 | AAGGACAGAACUCCCUGCUCCUU | SEQ ID NO: 620 | | 259-282 |
| SEQ ID NO: 345 | GAACUCCCUGCUCCUUGGGAAUA | SEQ ID NO: 621 | | 252-275 |
| SEQ ID NO: 346 | AGGACAGAACUCCCUGCUCCUUG | SEQ ID NO: 622 | | 258-281 |
| SEQ ID NO: 347 | GAAGUUAAGAGCCUGGGUGGGGA | SEQ ID NO: 623 | | 317-340 |
| SEQ ID NO: 348 | UCUUUGAAGGCGAAGUUAAGAGC | SEQ ID NO: 624 | | 328-351 |
| SEQ ID NO: 349 | UAUUUAUCAGGAGUGACUGAAAU | SEQ ID NO: 625 | | 1174-1197 |
| SEQ ID NO: 350 | GUUAUUUAUCAGGAGUGACUGAA | SEQ ID NO: 626 | | 1176-1199 |
| SEQ ID NO: 351 | UUAUUUAUCAGGAGUGACUGAAA | SEQ ID NO: 627 | | 1175-1198 |
| SEQ ID NO: 352 | AAAUACAAUUGAGUCCCAUCAUC | SEQ ID NO: 628 | | 1148-1171 |
| SEQ ID NO: 353 | AAAAUACAAUUGAGUCCCAUCAU | SEQ ID NO: 629 | | 1149-1172 |
| SEQ ID NO: 354 | AUUUGUUAUUUAUCAGGAGUGAC | SEQ ID NO: 630 | | 1180-1203 |
| SEQ ID NO: 355 | GAGAAAACUGGUCAGAUGAAUAU | SEQ ID NO: 631 | | 1103-1126 |
| SEQ ID NO: 356 | AUAUAAUACAACAUGCCUGUUCA | SEQ ID NO: 632 | | 996-1019 |
| SEQ ID NO: 357 | UGGGAUCCGGAAGCAUUCCCUUU | SEQ ID NO: 633 | | 666-689 |
| SEQ ID NO: 358 | UUGGGAUCCGGAAGCAUUCCCUU | SEQ ID NO: 634 | | 667-690 |
| SEQ ID NO: 359 | CAGGCAGUCACCGAGGCCUCGGA | SEQ ID NO: 635 | | 533-556 |
| SEQ ID NO: 360 | UAUAUAAUACAACAUGCCUGUUC | SEQ ID NO: 636 | | 997-1020 |
| SEQ ID NO: 361 | AGUCUAUAAUGCCUUUGUAUUGC | SEQ ID NO: 637 | | 219-242 |
| SEQ ID NO: 362 | UUUAUCAGGAGUGACUGAAAUAA | SEQ ID NO: 638 | | 1172-1195 |
| SEQ ID NO: 363 | AAUACAAUUGAGUCCCAUCAUCA | SEQ ID NO: 639 | | 1147-1170 |
| SEQ ID NO: 364 | CUUUAAGAGAAAACUGGUCAGAU | SEQ ID NO: 640 | | 1109-1132 |
| SEQ ID NO: 365 | GCUUUAAGAGAAAACUGGUCAGA | SEQ ID NO: 641 | | 1110-1133 |
| SEQ ID NO: 366 | AAUGCCUUUGUAUUGCUUAUCUG | SEQ ID NO: 642 | | 212-235 |
| SEQ ID NO: 367 | UAAUGCCUUUGUAUUGCUUAUCU | SEQ ID NO: 643 | | 213-236 |
| SEQ ID NO: 368 | UACCCUGCACAGACACGUUAAAG | SEQ ID NO: 644 | | 600-623 |
| SEQ ID NO: 369 | AUACCCUGCACAGACACGUUAAA | SEQ ID NO: 645 | | 601-624 |
| SEQ ID NO: 370 | AUCCAGCUGAUGACGAUGUGAGU | SEQ ID NO: 646 | | 694-717 |
| SEQ ID NO: 371 | UACAAUUGAGUCCCAUCAUCAUC | SEQ ID NO: 647 | | 1145-1168 |
| SEQ ID NO: 372 | UGGGAAUACGGACCACGCAGUCU | SEQ ID NO: 648 | | 237-260 |
| SEQ ID NO: 373 | UAUAAUACAACAUGCCUGUUCAC | SEQ ID NO: 649 | | 995-1018 |
| SEQ ID NO: 374 | UUAUCAGGAGUGACUGAAAUAAA | SEQ ID NO: 650 | | 1171-1194 |
| SEQ ID NO: 375 | AUAAUGCCUUUGUAUUGCUUAUC | SEQ ID NO: 651 | | 214-237 |
| SEQ ID NO: 376 | CCAGCUGAUGACGAUGUGAGUGU | SEQ ID NO: 652 | | 692-715 |
| SEQ ID NO: 377 | CAUAGAUACCGAAGUAGGCGGCU | SEQ ID NO: 653 | | 636-659 |
| SEQ ID NO: 378 | CAUCCAGCUGAUGACGAUGUGAG | SEQ ID NO: 654 | | 695-718 |
| SEQ ID NO: 379 | GUCAUAGAUACCGAAGUAGGCGG | SEQ ID NO: 655 | | 638-661 |
| SEQ ID NO: 380 | ACCCUGCACAGACACGUUAAAGC | SEQ ID NO: 656 | | 599-622 |
| SEQ ID NO: 381 | UGCAUCAUCAUGCGGCGGCGAAC | SEQ ID NO: 657 | | 772-795 |
| SEQ ID NO: 382 | CGGACCACGCAGUCUAUAAUGCC | SEQ ID NO: 658 | | 229-252 |
| SEQ ID NO: 383 | CUUGGGAAUACGGACCACGCAGU | SEQ ID NO: 659 | | 239-262 |
| SEQ ID NO: 384 | GAAGUAGGCGGCUCGGUAGAUGA | SEQ ID NO: 660 | | 626-649 |
| SEQ ID NO: 385 | CUGCUAGACGGGUACGGGCAAAA | SEQ ID NO: 661 | | 477-500 |
| SEQ ID NO: 386 | UCCAGCUGAUGACGAUGUGAGUG | SEQ ID NO: 662 | | 693-716 |
| SEQ ID NO: 387 | AUACGGACCACGCAGUCUAUAAU | SEQ ID NO: 663 | | 232-255 |
| SEQ ID NO: 388 | AACCAGGCAGUCACCGAGGCCUC | SEQ ID NO: 664 | | 536-559 |
| SEQ ID NO: 389 | UGACAGUCUGUGCGAUCAUCCAG | SEQ ID NO: 665 | | 711-734 |
| SEQ ID NO: 390 | UAGACGGGUACGGGCAAAAUCAA | SEQ ID NO: 666 | | 473-496 |
| SEQ ID NO: 391 | GCAGUGACAGUCUGUGCGAUCAU | SEQ ID NO: 667 | | 715-738 |
| SEQ ID NO: 392 | AGUGACAGUCUGUGCGAUCAUCC | SEQ ID NO: 668 | | 713-736 |
| SEQ ID NO: 393 | GCUAGACGGGUACGGGCAAAAUC | SEQ ID NO: 669 | | 475-498 |
| SEQ ID NO: 394 | AGUAGGCGGCUCGGUAGAUGAUA | SEQ ID NO: 670 | | 624-647 |
| SEQ ID NO: 395 | GAAUACGGACCACGCAGUCUAUA | SEQ ID NO: 671 | | 234-257 |
| SEQ ID NO: 396 | CUUUGUAUUGCUUAUCUGCAGUG | SEQ ID NO: 672 | | 207-230 |
| SEQ ID NO: 397 | UUUGUAUUGCUUAUCUGCAGUGA | SEQ ID NO: 673 | | 206-229 |
| SEQ ID NO: 398 | UUGUAUUGCUUAUCUGCAGUGAU | SEQ ID NO: 674 | | 205-228 |
| SEQ ID NO: 399 | UAUCUGCAGUGAUCUGCUUGCUG | SEQ ID NO: 675 | | 195-218 |
| SEQ ID NO: 400 | GUAUUGCUUAUCUGCAGUGAUCU | SEQ ID NO: 676 | | 203-226 |
| SEQ ID NO: 401 | UGUAUUGCUUAUCUGCAGUGAUC | SEQ ID NO: 677 | | 204-227 |
| SEQ ID NO: 402 | UGCUUAUCUGCAGUGAUCUGCUU | SEQ ID NO: 678 | | 199-222 |
| SEQ ID NO: 403 | UUGCUUAUCUGCAGUGAUCUGCU | SEQ ID NO: 679 | | 200-223 |
| SEQ ID NO: 404 | AUCUGCAGUGAUCUGCUUGCUGG | SEQ ID NO: 680 | | 194-217 |
| SEQ ID NO: 405 | UAUUGCUUAUCUGCAGUGAUCUG | SEQ ID NO: 681 | | 202-225 |
| SEQ ID NO: 406 | UUCUCCAAAUUUGUUAUUUAUCA | SEQ ID NO: 682 | | 1188-1211 |
| SEQ ID NO: 407 | CAAAUUUGUUAUUUAUCAGGAGU | SEQ ID NO: 683 | | 1183-1206 |
| SEQ ID NO: 408 | GAGGCCUCGGAAUUCCCUUUCAG | SEQ ID NO: 684 | | 521-544 |
| SEQ ID NO: 409 | AGAUAUGUUAUAUAAUACAACAU | SEQ ID NO: 685 | | 1005-1028 |
| SEQ ID NO: 410 | GCAGUCACCGAGGCCUCGGAAUU | SEQ ID NO: 686 | | 530-553 |
| SEQ ID NO: 411 | CGAGGCCUCGGAAUUCCCUUUCA | SEQ ID NO: 687 | | 522-545 |
| SEQ ID NO: 412 | CAAGAUAUGUUAUAUAAUACAAC | SEQ ID NO: 688 | | 1007-1030 |
| SEQ ID NO: 413 | AGUCACCGAGGCCUCGGAAUUCC | SEQ ID NO: 689 | | 528-551 |
| SEQ ID NO: 414 | CACCGAGGCCUCGGAAUUCCCUU | SEQ ID NO: 690 | | 525-548 |
| SEQ ID NO: 415 | CCUCGGAAUUCCCUUUCAGCUCC | SEQ ID NO: 691 | | 517-540 |
| SEQ ID NO: 416 | UCGGAAUUCCCUUUCAGCUCCAG | SEQ ID NO: 692 | | 515-538 |
| SEQ ID NO: 417 | GGCCUCGGAAUUCCCUUUCAGCU | SEQ ID NO: 693 | | 519-542 |
| SEQ ID NO: 418 | CUCGGAAUUCCCUUUCAGCUCCA | SEQ ID NO: 694 | | 516-539 |
| SEQ ID NO: 419 | GCCUCGGAAUUCCCUUUCAGCUC | SEQ ID NO: 695 | | 518-541 |
| SEQ ID NO: 420 | AUGUUAUAUAAUACAACAUGCCU | SEQ ID NO: 696 | | 1001-1024 |
| SEQ ID NO: 421 | AAGAUAUGUUAUAUAAUACAACA | SEQ ID NO: 697 | | 1006-1029 |
| SEQ ID NO: 422 | UCAAGAUAUGUUAUAUAAUACAA | SEQ ID NO: 698 | | 1008-1031 |
| SEQ ID NO: 423 | UGUUAUAUAAUACAACAUGCCUG | SEQ ID NO: 699 | | 1000-1023 |
| SEQ ID NO: 424 | CCGAGGCCUCGGAAUUCCCUUUC | SEQ ID NO: 700 | | 523-546 |
| SEQ ID NO: 425 | GUCACCGAGGCCUCGGAAUUCCC | SEQ ID NO: 701 | | 527-550 |
| SEQ ID NO: 426 | ACCGAGGCCUCGGAAUUCCCUUU | SEQ ID NO: 702 | | 524-547 |
| SEQ ID NO: 427 | UCACCGAGGCCUCGGAAUUCCCU | SEQ ID NO: 703 | | 526-549 |
| SEQ ID NO: 428 | AUUUCAUCAUACAAGACAAGCAC | SEQ ID NO: 704 | | 931-954 |
| SEQ ID NO: 429 | UGUAUUUAUCUUUGAAGGCGAAG | SEQ ID NO: 705 | | 336-359 |
| SEQ ID NO: 430 | AUUUGUAGAUCUUAACCAGGCAG | SEQ ID NO: 706 | | 549-572 |
| SEQ ID NO: 431 | UUCCCUUUGCAGUGUCAUAGAUA | SEQ ID NO: 707 | | 651-674 |
| SEQ ID NO: 432 | AUCAUACAAGACAAGCACAAAAG | SEQ ID NO: 708 | | 926-949 |
| SEQ ID NO: 433 | UAGAUCUUAACCAGGCAGUCACC | SEQ ID NO: 709 | | 544-567 |
| SEQ ID NO: 434 | AAGCAUUCCCUUUGCAGUGUCAU | SEQ ID NO: 710 | | 656-679 |
| SEQ ID NO: 435 | UUUGCAGUGUCAUAGAUACCGAA | SEQ ID NO: 711 | | 646-669 |
| SEQ ID NO: 436 | AGAGAAAACUGGUCAGAUGAAUA | SEQ ID NO: 712 | | 1104-1127 |
| SEQ ID NO: 437 | UGCAGUGUCAUAGAUACCGAAGU | SEQ ID NO: 713 | | 644-667 |
| SEQ ID NO: 438 | UCAGAUUUGUAGAUCUUAACCAG | SEQ ID NO: 714 | | 553-576 |
| SEQ ID NO: 439 | UCCCUUUGCAGUGUCAUAGAUAC | SEQ ID NO: 715 | | 650-673 |
| SEQ ID NO: 440 | GAACAUUGGACCAUGCACCCUUG | SEQ ID NO: 716 | | 885-908 |
| SEQ ID NO: 441 | AAUCCCAUCAGAUUUGUAGAUCU | SEQ ID NO: 717 | | 560-583 |
| SEQ ID NO: 442 | GCAGUGUCAUAGAUACCGAAGUA | SEQ ID NO: 718 | | 643-666 |
| SEQ ID NO: 443 | CAAGCACAAAAGCACCACCCAUG | SEQ ID NO: 719 | | 915-938 |
| SEQ ID NO: 444 | GCGGCUCGGUAGAUGAUAAUACC | SEQ ID NO: 720 | | 619-642 |
| SEQ ID NO: 445 | CUCCUUGGGAAUACGGACCACGC | SEQ ID NO: 721 | | 242-265 |
| SEQ ID NO: 446 | AUUCCCUUUGCAGUGUCAUAGAU | SEQ ID NO: 722 | | 652-675 |
| SEQ ID NO: 447 | CAUACAAGACAAGCACAAAAGCA | SEQ ID NO: 723 | | 924-947 |
| SEQ ID NO: 448 | AGACACGUUAAAGCCUUGGUACA | SEQ ID NO: 724 | | 590-613 |
| SEQ ID NO: 449 | UGAGAACAUUGGACCAUGCACCC | SEQ ID NO: 725 | | 888-911 |
| SEQ ID NO: 450 | GCUUGUAUUUAUCUUUGAAGGCG | SEQ ID NO: 726 | | 339-362 |
| SEQ ID NO: 451 | GCUCGGUAGAUGAUAAUACCCUG | SEQ ID NO: 727 | | 616-639 |
| SEQ ID NO: 452 | AGUGUCAUAGAUACCGAAGUAGG | SEQ ID NO: 728 | | 641-664 |
| SEQ ID NO: 453 | AUCAGAUUUGUAGAUCUUAACCA | SEQ ID NO: 729 | | 554-577 |
| SEQ ID NO: 454 | GGUAGAUGAUAAUACCCUGCACA | SEQ ID NO: 730 | | 612-635 |
| SEQ ID NO: 455 | CAGGCCCUUAAUCCCAUCAGAUU | SEQ ID NO: 731 | | 569-592 |
| SEQ ID NO: 456 | AGAUGAUAAUACCCUGCACAGAC | SEQ ID NO: 732 | | 609-632 |
| SEQ ID NO: 457 | UACAGGCCCUUAAUCCCAUCAGA | SEQ ID NO: 733 | | 571-594 |
| SEQ ID NO: 458 | ACAGACACGUUAAAGCCUUGGUA | SEQ ID NO: 734 | | 592-615 |
| SEQ ID NO: 459 | AGAAAACUGGUCAGAUGAAUAUU | SEQ ID NO: 735 | | 1102-1125 |
| SEQ ID NO: 460 | UGUAGAUCUUAACCAGGCAGUCA | SEQ ID NO: 736 | | 546-569 |
| SEQ ID NO: 461 | UCAAAUGGAUAGGAAGUCAACCC | SEQ ID NO: 737 | | 745-768 |
| SEQ ID NO: 462 | CAUUCCCUUUGCAGUGUCAUAGA | SEQ ID NO: 738 | | 653-676 |
| SEQ ID NO: 463 | CAUCAGAUUUGUAGAUCUUAACC | SEQ ID NO: 739 | | 555-578 |
| SEQ ID NO: 464 | CUCGGUAGAUGAUAAUACCCUGC | SEQ ID NO: 740 | | 615-638 |
| SEQ ID NO: 465 | UUUGUAGAUCUUAACCAGGCAGU | SEQ ID NO: 741 | | 548-571 |
| SEQ ID NO: 466 | ACAGGCCCUUAAUCCCAUCAGAU | SEQ ID NO: 742 | | 570-593 |
| SEQ ID NO: 467 | CUUUGCAGUGUCAUAGAUACCGA | SEQ ID NO: 743 | | 647-670 |
| SEQ ID NO: 468 | CACAUCAGCUGCUAGACGGGUAC | SEQ ID NO: 744 | | 485-508 |
| SEQ ID NO: 469 | CCCUUUGCAGUGUCAUAGAUACC | SEQ ID NO: 745 | | 649-672 |
| SEQ ID NO: 470 | CUCCAGCUUUACCCACAUCAGCU | SEQ ID NO: 746 | | 498-521 |
| SEQ ID NO: 471 | UUACCCACAUCAGCUGCUAGACG | SEQ ID NO: 747 | | 490-513 |
| SEQ ID NO: 472 | UUAAAGCCUUGGUACAGGCCCUU | SEQ ID NO: 748 | | 583-606 |
| SEQ ID NO: 473 | CAGCUGCUAGACGGGUACGGGCA | SEQ ID NO: 749 | | 480-503 |
| SEQ ID NO: 474 | CAGCUUUACCCACAUCAGCUGCU | SEQ ID NO: 750 | | 495-518 |
| SEQ ID NO: 475 | UCAGCUGCUAGACGGGUACGGGC | SEQ ID NO: 751 | | 481-504 |
| SEQ ID NO: 476 | UCCAGCUUUACCCACAUCAGCUG | SEQ ID NO: 752 | | 497-520 |
| SEQ ID NO: 477 | GGCUCGGUAGAUGAUAAUACCCU | SEQ ID NO: 753 | | 617-640 |
| SEQ ID NO: 478 | AGGCGGCUCGGUAGAUGAUAAUA | SEQ ID NO: 754 | | 621-644 |
| SEQ ID NO: 479 | CAUCAGCUGCUAGACGGGUACGG | SEQ ID NO: 755 | | 483-506 |
| SEQ ID NO: 480 | GUACAGGCCCUUAAUCCCAUCAG | SEQ ID NO: 756 | | 572-595 |
| SEQ ID NO: 481 | AUGAUAAUACCCUGCACAGACAC | SEQ ID NO: 757 | | 607-630 |
| SEQ ID NO: 482 | CCUUGGGAAUACGGACCACGCAG | SEQ ID NO: 758 | | 240-263 |
| SEQ ID NO: 483 | UCCUUGGGAAUACGGACCACGCA | SEQ ID NO: 759 | | 241-264 |
| SEQ ID NO: 484 | CAGAUUUGUAGAUCUUAACCAGG | SEQ ID NO: 760 | | 552-575 |
| SEQ ID NO: 485 | UGUACUUCUUGAUUUCAUCAUAC | SEQ ID NO: 761 | | 942-965 |
| SEQ ID NO: 486 | AUCCCAUCAGAUUUGUAGAUCUU | SEQ ID NO: 762 | | 559-582 |
| SEQ ID NO: 487 | GUAGAUGAUAAUACCCUGCACAG | SEQ ID NO: 763 | | 611-634 |
| SEQ ID NO: 488 | GACACGUUAAAGCCUUGGUACAG | SEQ ID NO: 764 | | 589-612 |
| SEQ ID NO: 489 | AGCUUUACCCACAUCAGCUGCUA | SEQ ID NO: 765 | | 494-517 |
| SEQ ID NO: 490 | AAGCACAAAAGCACCACCCAUGC | SEQ ID NO: 766 | | 914-937 |
| SEQ ID NO: 491 | AUCUUAACCAGGCAGUCACCGAG | SEQ ID NO: 767 | | 541-564 |
| SEQ ID NO: 492 | UUUACCCACAUCAGCUGCUAGAC | SEQ ID NO: 768 | | 491-514 |
| SEQ ID NO: 493 | CUUAAUCCCAUCAGAUUUGUAGA | SEQ ID NO: 769 | | 563-586 |
| SEQ ID NO: 494 | CAGUGUCAUAGAUACCGAAGUAG | SEQ ID NO: 770 | | 642-665 |
| SEQ ID NO: 495 | CCAGCUUUACCCACAUCAGCUGC | SEQ ID NO: 771 | | 496-519 |
| SEQ ID NO: 496 | AUCAGCUGCUAGACGGGUACGGG | SEQ ID NO: 772 | | 482-505 |
| SEQ ID NO: 497 | GAUGAUAAUACCCUGCACAGACA | SEQ ID NO: 773 | | 608-631 |
| SEQ ID NO: 498 | UUUCAUCAUACAAGACAAGCACA | SEQ ID NO: 774 | | 930-953 |
| SEQ ID NO: 499 | CAGACACGUUAAAGCCUUGGUAC | SEQ ID NO: 775 | | 591-614 |
| SEQ ID NO: 500 | AGAACAUUGGACCAUGCACCCUU | SEQ ID NO: 776 | | 886-909 |
| SEQ ID NO: 501 | GUAGAUCUUAACCAGGCAGUCAC | SEQ ID NO: 777 | | 545-568 |
| SEQ ID NO: 502 | AGCUCCAGCUUUACCCACAUCAG | SEQ ID NO: 778 | | 500-523 |
| SEQ ID NO: 503 | GCUCCUUGGGAAUACGGACCACG | SEQ ID NO: 779 | | 243-266 |
| SEQ ID NO: 504 | GAAGCAUUCCCUUUGCAGUGUCA | SEQ ID NO: 780 | | 657-680 |
| SEQ ID NO: 505 | CCUUGGUACAGGCCCUUAAUCCC | SEQ ID NO: 781 | | 577-600 |
| SEQ ID NO: 506 | UAGAUGAUAAUACCCUGCACAGA | SEQ ID NO: 782 | | 610-633 |
| SEQ ID NO: 507 | AAUACCCUGCACAGACACGUUAA | SEQ ID NO: 783 | | 602-625 |
| SEQ ID NO: 508 | GUUAAAGCCUUGGUACAGGCCCU | SEQ ID NO: 784 | | 584-607 |
| SEQ ID NO: 509 | AGCAUUCCCUUUGCAGUGUCAUA | SEQ ID NO: 785 | | 655-678 |
| SEQ ID NO: 510 | UCUUAACCAGGCAGUCACCGAGG | SEQ ID NO: 786 | | 540-563 |
| SEQ ID NO: 511 | AUACAAGACAAGCACAAAAGCAC | SEQ ID NO: 787 | | 923-946 |
| SEQ ID NO: 512 | GACAAGCACAAAAGCACCACCCA | SEQ ID NO: 788 | | 917-940 |
| SEQ ID NO: 513 | ACCCACAUCAGCUGCUAGACGGG | SEQ ID NO: 789 | | 488-511 |
| SEQ ID NO: 514 | UAAAGCCUUGGUACAGGCCCUUA | SEQ ID NO: 790 | | 582-605 |
| SEQ ID NO: 515 | UUCAUCAUACAAGACAAGCACAA | SEQ ID NO: 791 | | 929-952 |
| SEQ ID NO: 516 | GCCCUUAAUCCCAUCAGAUUUGU | SEQ ID NO: 792 | | 566-589 |
| SEQ ID NO: 517 | UUAAUCCCAUCAGAUUUGUAGAU | SEQ ID NO: 793 | | 562-585 |
| SEQ ID NO: 518 | UCGGUAGAUGAUAAUACCCUGCA | SEQ ID NO: 794 | | 614-637 |
| SEQ ID NO: 519 | CACGUUAAAGCCUUGGUACAGGC | SEQ ID NO: 795 | | 587-610 |
| SEQ ID NO: 520 | UCCCAUCAGAUUUGUAGAUCUUA | SEQ ID NO: 796 | | 558-581 |
| SEQ ID NO: 521 | UCAUACAAGACAAGCACAAAAGC | SEQ ID NO: 797 | | 925-948 |
| SEQ ID NO: 522 | CCUUAAUCCCAUCAGAUUUGUAG | SEQ ID NO: 798 | | 564-587 |
| SEQ ID NO: 523 | ACAUUGGACCAUGCACCCUUGAA | SEQ ID NO: 799 | | 883-906 |
| SEQ ID NO: 524 | CUUGGUACAGGCCCUUAAUCCCA | SEQ ID NO: 800 | | 576-599 |
| SEQ ID NO: 525 | UUGCAGUGUCAUAGAUACCGAAG | SEQ ID NO: 801 | | 645-668 |
| SEQ ID NO: 526 | CACAGACACGUUAAAGCCUUGGU | SEQ ID NO: 802 | | 593-616 |
| SEQ ID NO: 527 | AGCACAAAAGCACCACCCAUGCC | SEQ ID NO: 803 | | 913-936 |
| SEQ ID NO: 528 | CCAUCAGAUUUGUAGAUCUUAAC | SEQ ID NO: 804 | | 556-579 |
| SEQ ID NO: 529 | CCACAUCAGCUGCUAGACGGGUA | SEQ ID NO: 805 | | 486-509 |
| SEQ ID NO: 530 | GCAUUCCCUUUGCAGUGUCAUAG | SEQ ID NO: 806 | | 654-677 |
| SEQ ID NO: 531 | CUUUACCCACAUCAGCUGCUAGA | SEQ ID NO: 807 | | 492-515 |
| SEQ ID NO: 532 | AUCCGGAAGCAUUCCCUUUGCAG | SEQ ID NO: 808 | | 662-685 |
| SEQ ID NO: 533 | AGCUGCUAGACGGGUACGGGCAA | SEQ ID NO: 809 | | 479-502 |
| SEQ ID NO: 534 | CGGCUCGGUAGAUGAUAAUACCC | SEQ ID NO: 810 | | 618-641 |
| SEQ ID NO: 535 | GGGUACGGGCAAAAUCAAGAGGG | SEQ ID NO: 811 | | 468-491 |
| SEQ ID NO: 536 | ACAUCAGCUGCUAGACGGGUACG | SEQ ID NO: 812 | | 484-507 |
| SEQ ID NO: 537 | CCCUUAAUCCCAUCAGAUUUGUA | SEQ ID NO: 813 | | 565-588 |
| SEQ ID NO: 538 | CGGGCAAAAUCAAGAGGGUACAC | SEQ ID NO: 814 | | 463-486 |
| SEQ ID NO: 539 | ACGUUAAAGCCUUGGUACAGGCC | SEQ ID NO: 815 | | 586-609 |
| SEQ ID NO: 540 | UGGUACAGGCCCUUAAUCCCAUC | SEQ ID NO: 816 | | 574-597 |
| SEQ ID NO: 541 | GGCGGCUCGGUAGAUGAUAAUAC | SEQ ID NO: 817 | | 620-643 |
| SEQ ID NO: 542 | CGUUAAAGCCUUGGUACAGGCCC | SEQ ID NO: 818 | | 585-608 |
| SEQ ID NO: 543 | GGUACAGGCCCUUAAUCCCAUCA | SEQ ID NO: 819 | | 573-596 |
| SEQ ID NO: 544 | UAAUACCCUGCACAGACACGUUA | SEQ ID NO: 820 | | 603-626 |
| SEQ ID NO: 545 | GACCACGCAGUCUAUAAUGCCUU | SEQ ID NO: 821 | | 227-250 |
| SEQ ID NO: 546 | GUUAAGAGCCUGGGUGGGGAAGU | SEQ ID NO: 822 | | 314-337 |
| SEQ ID NO: 547 | UGGGGAAGUAUCUGAUGACAUUG | SEQ ID NO: 823 | | 300-323 |
| SEQ ID NO: 548 | GGUGGGGAAGUAUCUGAUGACAU | SEQ ID NO: 824 | | 302-325 |
| SEQ ID NO: 549 | GUGGGGAAGUAUCUGAUGACAUU | SEQ ID NO: 825 | | 301-324 |
| SEQ ID NO: 550 | GGGGAAGUAUCUGAUGACAUUGG | SEQ ID NO: 826 | | 299-322 |
| SEQ ID NO: 551 | UUAAGAGCCUGGGUGGGGAAGUA | SEQ ID NO: 827 | | 313-336 |
| SEQ ID NO: 552 | CCGAAGUAGGCGGCUCGGUAGAU | SEQ ID NO: 828 | | 628-651 |

In a particular embodiment, the invention relates to a nucleic acid comprising first and second strands that comprise, consist of, or consist essentially of a nucleoside sequence differing by 0 or 1 nucleosides from any one of the following first and second sequences:

| Unmodified first strand | Unmodified second strand |
|---|---|
| SEQ ID NO:304 | SEQ ID NO:580 |
| SEQ ID NO:323 | SEQ ID NO:599 |
| SEQ ID NO:439 | SEQ ID NO: 715 |
| SEQ ID NO:453 | SEQ ID NO:729 |
| SEQ ID NO:496 | SEQ ID NO:772 |

In a particular embodiment, the invention relates to a nucleic acid comprising first and second strands that comprise, consist of, or consist essentially of a nucleoside sequence differing by 0 or 1 nucleosides from any one of the following first and second sequences:

| Unmodified first strand | Unmodified second strand |
|---|---|
| SEQ ID NO:304 | SEQ ID NO:580 |

In certain embodiments, the nucleic acid for inhibiting expression of the *SLC25A5* gene comprises a duplex region that comprises a first strand and a second strand that is at least partially complementary to the first strand, wherein said first strand is:
(i) at least partially complementary to a portion of RNA transcribed from the *SLC25A5* gene, and
(ii) comprises at least 17 contiguous nucleosides differing by 0 or 1 nucleosides from any one of SEQ ID NO:829-1104.

In certain embodiments, the first strand comprises nucleosides 2-18 of any one of the sequences set forth in SEQ ID NO: 829-1104.

In certain embodiments, the nucleic acid for inhibiting expression of the *SLC25A5* gene comprises a duplex region that comprises a first strand and a second strand that is at least partially complementary to the first strand, wherein said first strand is:
(i) at least partially complementary to a portion of RNA transcribed from the *SLC25A5* gene, and
(ii) comprises at least 21 contiguous nucleosides differing by 0 or 1 nucleosides from any one of SEQ ID NO: 829-1104.

In certain embodiments, the first strand comprises nucleosides 2-22 of any one of the sequences set forth in SEQ ID NO: 829-1104.

In certain embodiments, the first strand comprises any one of SEQ ID NO: 829-1104.

The modification pattern of the nucleic acids as set forth in SEQ ID NO: 829-1104 is summarized in Table 3 below:

**Table 3**

| Antisense strand ID | Modified Antisense Strand 5' → 3' | SEQ ID NO (AS - mod) | Underlying Base Sequence 5' 4 3' (Shown as an Unmodified Nucleotide Sequence) | SEQ ID NO (AS - unmod) |
|---|---|---|---|---|
| ETX-S00003090 | CmsCfsCmAmUmUfUmUmCmAmAmCmC mAfGmUfAmAfAmUmAmsGmsUm | SEQ ID NO: 829 | CCCAUUUUCAACC AGUAAAUAGU | SEQ ID NO: 277 |
| ETX-S00003094 | AmsCfsGmCmAmGfUmCmUmAmUmAmA mUfGmCfCmUfUmUmGmsUmsAm | SEQ ID NO: 830 | ACGCAGUCUAUAA UGCCUUUGUA | SEQ ID NO: 278 |
| ETX-S00003098 | GmsUfsGmUmAmCfUmUmCmUmUmGmA mUfUmUfCmAfUmCmAmsUmsAm | SEQ ID NO: 831 | GUGUACUUCUUGA UUUCAUCAUA | SEQ ID NO: 279 |
| ETX-S00003102 | AmsUfsAmCmAmAfUmUmGmAmGmUmC mCfCmAfUmCfAmUmCmsAmsUm | SEQ ID NO: 832 | AUACAAUUGAGUC CCAUCAUCAU | SEQ ID NO: 280 |
| ETX-S00003106 | GmsGfsAmCmCmAfCmGmCmAmGmUmC mUfAmUfAmAfUmGmCmsCmsUm | SEQ ID NO: 833 | GGACCACGCAGUC UAUAAUGCCU | SEQ ID NO: 281 |
| ETX-S00003110 | UmsUfsGmUmUmAfUmUmUmAmUmCmA mGfGmAfGmUfGmAmCmsUmsGm | SEQ ID NO: 834 | UUGUUAUUUAUCA GGAGUGACUG | SEQ ID NO: 282 |
| ETX-S00003114 | CmsAfsGmUmCmUfAmUmAmAmUmGmC mCfUmUfUmGfUmAmUmsUmsGm | SEQ ID NO: 835 | CAGUCUAUAAUGC CUUUGUAUUG | SEQ ID NO: 283 |
| ETX-S00003118 | AmsUfsAmGmAmUfAmCmCmGmAmAmG mUfAmGfGmCfGmGmCmsUmsCm | SEQ ID NO: 836 | AUAGAUACCGAAG UAGGCGGCUC | SEQ ID NO: 284 |
| ETX-S00003122 | UmsAfsGmAmUmAfCmCmGmAmAmGmU mAfGmGfCmGfGmCmUmsCmsGm | SEQ ID NO: 837 | UAGAUACCGAAGU AGGCGGCUCG | SEQ ID NO: 285 |
| ETX-S00003126 | GmsGfsAmAmUmAfCmGmGmAmCmCmA mCfGmCfAmGfUmCmUmsAmsUm | SEQ ID NO: 838 | GGAAUACGGACCA CGCAGUCUAU | SEQ ID NO: 286 |
| ETX-S00003130 | CmsAfsGmCmUmGfAmUmGmAmCmGmA mUfGmUfGmAfGmUmGmsUmsUm | SEQ ID NO: 839 | CAGCUGAUGACGA UGUGAGUGUU | SEQ ID NO: 287 |
| ETX-S00003134 | AmsUfsUmUmAmUfCmUmUmUmGmAmA mGfGmCfGmAfAmGmUmsUmsAm | SEQ ID NO: 840 | AUUUAUCUUUGAA GGCGAAGUUA | SEQ ID NO: 288 |
| ETX-S00003138 | GmsUfsGmUmCmAfUmAmGmAmUmAmC mCfGmAfAmGfUmAmGmsGmsCm | SEQ ID NO: 841 | GUGUCAUAGAUAC CGAAGUAGGC | SEQ ID NO: 289 |
| ETX-S00003142 | UmsUfsCmCmCmAfUmUmUmUmCmAmA mCfCmAfGmUfAmAmAmsUmsAm | SEQ ID NO: 842 | UUCCCAUUUUCAA CCAGUAAAUA | SEQ ID NO: 290 |
| ETX-S00003146 | GmsCfsUmGmCmUfAmGmAmCmGmGmG mUfAmCfGmGfGmCmAmsAmsAm | SEQ ID NO: 843 | GCUGCUAGACGGG UACGGGCAAA | SEQ ID NO: 291 |
| ETX-S00003150 | CmsAfsGmUmUmCfCmUmUmUmGmCmG mCfCmCfUmGfAmCmUmsGmsCm | SEQ ID NO: 844 | CAGUUCCUUUGCG CCCUGACUGC | SEQ ID NO: 292 |
| ETX-S00003154 | CmsUfsUmCmCmCfAmUmUmUmUmCmA mAfCmCfAmGfUmAmAmsAmsUm | SEQ ID NO: 845 | CUUCCCAUUUUCA ACCAGUAAAU | SEQ ID NO: 293 |
| ETX-S00003158 | AmsGfsAmUmAmCfCmGmAmAmGmUmA mGfGmCfGmGfCmUmCmsGmsGm | SEQ ID NO: 846 | AGAUACCGAAGUA GGCGGCUCGG | SEQ ID NO: 294 |
| ETX-S00003162 | UmsUfsAmAmCmCfAmGmGmCmAmGmU mCfAmCfCmGfAmGmGmsCmsCm | SEQ ID NO: 847 | UUAACCAGGCAGU CACCGAGGCC | SEQ ID NO: 295 |
| ETX-S00003166 | AmsUfsCmAmUmCfCmAmGmCmUmGmA mUfGmAfCmGfAmUmGmsUmsGm | SEQ ID NO: 848 | AUCAUCCAGCUGA UGACGAUGUG | SEQ ID NO: 296 |
| ETX-S00003170 | AmsUfsUmUmAmUfCmAmGmGmAmGmU mGfAmCfUmGfAmAmAmsUmsAm | SEQ ID NO: 849 | AUUUAUCAGGAGU GACUGAAAUA | SEQ ID NO: 297 |
| ETX-S00003174 | GmsAfsCmAmGmUfCmUmGmUmGmCmG mAfUmCfAmUfCmCmAmsGmsCm | SEQ ID NO: 850 | GACAGUCUGUGCG AUCAUCCAGC | SEQ ID NO: 298 |
| ETX-S00003178 | UmsCfsUmAmUmAfAmUmGmCmCmUmU mUfGmUfAmUfUmGmCmsUmsUm | SEQ ID NO: 851 | UCUAUAAUGCCUU UGUAUUGCUU | SEQ ID NO: 299 |
| ETX-S00003182 | UmsAfsAmCmCmAfGmGmCmAmGmUmC mAfCmCfGmAfGmGmCmsCmsUm | SEQ ID NO: 852 | UAACCAGGCAGUC ACCGAGGCCU | SEQ ID NO: 300 |
| ETX-S00003186 | CmsCfsCmUmGmCfAmCmAmGmAmCmA mCfGmUfUmAfAmAmGmsCmsCm | SEQ ID NO: 853 | CCCUGCACAGACA CGUUAAAGCC | SEQ ID NO: 301 |
| ETX-S00003190 | CmsAfsCmGmCmAfGmUmCmUmAmUmA mAfUmGfCmCfUmUmUmsGmsUm | SEQ ID NO: 854 | CACGCAGUCUAUA AUGCCUUUGU | SEQ ID NO: 302 |
| ETX-S00003194 | UmsAfsAmUmAmCfAmAmCmAmUmGmC mCfUmGfUmUfCmAmCmsAmsGm | SEQ ID NO: 855 | UAAUACAACAUGC CUGUUCACAG | SEQ ID NO: 303 |
| ETX-S00003198 | UmsUfsAmAmGmAfGmAmAmAmAmCmU mGfGmUfCmAfGmAmUmsGmsAm | SEQ ID NO: 856 | UUAAGAGAAAACU GGUCAGAUGA | SEQ ID NO: 304 |
| ETX-S00003202 | UmsGfsCmAmCmAfGmAmCmAmCmGmU mUfAmAfAmGfCmCmUmsUmsGm | SEQ ID NO: 857 | UGCACAGACACGU UAAAGCCUUG | SEQ ID NO: 305 |
| ETX-S00003206 | GmsUfsUmCmCmUfUmUmGmCmGmCmC mCfUmGfAmCfUmGmCmsAmsUm | SEQ ID NO: 858 | GUUCCUUUGCGCC CUGACUGCAU | SEQ ID NO: 306 |
| ETX-S00003210 | GmsUfsGmAmCmAfGmUmCmUmGmUmG mCfGmAfUmCfAmUmCmsCmsAm | SEQ ID NO: 859 | GUGACAGUCUGUG CGAUCAUCCA | SEQ ID NO: 307 |
| ETX-S00003214 | AmsCfsCmAmCmGfCmAmGmUmCmUmA mUfAmAfUmGfCmCmUmsUmsUm | SEQ ID NO: 860 | ACCACGCAGUCUA UAAUGCCUUU | SEQ ID NO: 308 |
| ETX-S00003218 | CmsAfsGmUmGmAfCmAmGmUmCmUmG mUfGmCfGmAfUmCmAmsUmsCm | SEQ ID NO: 861 | CAGUGACAGUCUG UGCGAUCAUC | SEQ ID NO: 309 |
| ETX-S00003222 | UmsUfsGmGmGmAfAmUmAmCmGmGmA mCfCmAfCmGfCmAmGmsUmsCm | SEQ ID NO: 862 | UUGGGAAUACGGA CCACGCAGUC | SEQ ID NO: 310 |
| ETX-S00003226 | GmsUfsCmUmAmUfAmAmUmGmCmCmU mUfUmGfUmAfUmUmGmsCmsUm | SEQ ID NO: 863 | GUCUAUAAUGCCU UUGUAUUGCU | SEQ ID NO: 311 |
| ETX-S00003230 | UmsGfsUmGmCmGfAmUmCmAmUmCmC mAfGmCfUmGfAmUmGmsAmsCm | SEQ ID NO: 864 | UGUGCGAUCAUCC AGCUGAUGAC | SEQ ID NO: 312 |
| ETX-S00003234 | AmsCfsGmGmAmCfCmAmCmGmCmAmG mUfCmUfAmUfAmAmUmsGmsCm | SEQ ID NO: 865 | ACGGACCACGCAG UCUAUAAUGC | SEQ ID NO: 313 |
| ETX-S00003238 | UmsGfsCmUmAmGfAmCmGmGmGmUmA mCfGmGfGmCfAmAmAmsAmsUm | SEQ ID NO: 866 | UGCUAGACGGUA CGGGCAAAAU | SEQ ID NO: 314 |
| ETX-S00003242 | UmsCfsAmUmCmCfAmGmCmUmGmAmU mGfAmCfGmAfUmGmUmsGmsAm | SEQ ID NO: 867 | UCAUCCAGCUGAU GACGAUGUGA | SEQ ID NO: 315 |
| ETX-S00003246 | CmsGfsGmGmUmAfCmGmGmGmCmAmA mAfAmUfCmAfAmGmAmsGmsGm | SEQ ID NO: 868 | CGGGUACGGGCAA AAUCAAGAGG | SEQ ID NO: 316 |
| ETX-S00003250 | AmsAfsUmAmCmGfGmAmCmCmAmCmG mCfAmGfUmCfUmAmUmsAmsAm | SEQ ID NO: 869 | AAUACGGACCACG CAGUCUAUAA | SEQ ID NO: 317 |
| ETX-S00003254 | UmsCfsAmUmAmGfAmUmAmCmCmGmA mAfGmUfAmGfGmCmGmsGmsCm | SEQ ID NO: 870 | UCAUAGAUACCGA AGUAGGCGGC | SEQ ID NO: 318 |
| ETX-S00003258 | GmsGfsGmAmAmUfAmCmGmGmAmCmC mAfCmGfCmAfGmUmCmsUmsAm | SEQ ID NO: 871 | GGGAAUACGGACC ACGCAGUCUA | SEQ ID NO: 319 |
| ETX-S00003262 | GmsUfsGmCmGmAfUmCmAmUmCmCmA mGfCmUfGmAfUmGmAmsCmsGm | SEQ ID NO: 872 | GUGCGAUCAUCCA GCUGAUGACG | SEQ ID NO: 320 |
| ETX-S00003266 | UmsAfsCmGmGmAfCmCmAmCmGmCmA mGfUmCfUmAfUmAmAmsUmsGm | SEQ ID NO: 873 | UACGGACCACGCA GUCUAUAAUG | SEQ ID NO: 321 |
| ETX-S00003270 | UmsGfsUmCmAmUfAmGmAmUmAmCmC mGfAmAfGmUfAmGmGmsCmsGm | SEQ ID NO: 874 | UGUCAUAGAUACC GAAGUAGGCG | SEQ ID NO: 322 |
| ETX-S00003274 | UmsAfsUmCmAmGfGmAmGmUmGmAmC mUfGmAfAmAfUmAmAmsAmsAm | SEQ ID NO: 875 | UAUCAGGAGUGAC UGAAAUAAAA | SEQ ID NO: 323 |
| ETX-S00003278 | UmsGfsCmGmAmUfCmAmUmCmCmAmG mCfUmGfAmUfGmAmCmsGmsAm | SEQ ID NO: 876 | UGCGAUCAUCCAG CUGAUGACGA | SEQ ID NO: 324 |
| ETX-S00003282 | CmsUfsAmGmAmCfGmGmGmUmAmCmG mGfGmCfAmAfAmAmUmsCmsAm | SEQ ID NO: 877 | CUAGACGGGUACG GGCAAAAUCA | SEQ ID NO: 325 |
| ETX-S00003286 | GmsAfsUmCmCmGfGmAmAmGmCmAmU mUfCmCfCmUfUmUmGmsCmsAm | SEQ ID NO: 878 | GAUCCGGAAGCAU UCCCUUUGCA | SEQ ID NO: 326 |
| ETX-S00003290 | GmsCfsAmCmAmGfAmCmAmCmGmUmU mAfAmAfGmCfCmUmUmsGmsGm | SEQ ID NO: 879 | GCACAGACACGUU AAAGCCUUGG | SEQ ID NO: 327 |
| ETX-S00003294 | CmsCfsAmCmGmCfAmGmUmCmUmAmU mAfAmUfGmCfCmUmUmsUmsGm | SEQ ID NO: 880 | CCACGCAGUCUAU AAUGCCUUUG | SEQ ID NO: 328 |
| ETX-S00003298 | GmsAfsCmGmGmGfUmAmCmGmGmGmC mAfAmAfAmUfCmAmAmsGmsAm | SEQ ID NO: 881 | GACGGGUACGGGC AAAAUCAAGA | SEQ ID NO: 329 |
| ETX-S00003302 | GmsAfsUmAmCmCfGmAmAmGmUmAmG mGfCmGfGmCfUmCmGmsGmsUm | SEQ ID NO: 882 | GAUACCGAAGUAG GCGGCUCGGU | SEQ ID NO: 330 |
| ETX-S00003306 | GmsUfsAmGmGmCfGmGmCmUmCmGmG mUfAmGfAmUfGmAmUmsAmsAm | SEQ ID NO: 883 | GUAGGCGGCUCGG UAGAUGAUAA | SEQ ID NO: 331 |
| ETX-S00003310 | AmsGfsAmCmGmGfGmUmAmCmGmGmG mCfAmAfAmAfUmCmAmsAmsGm | SEQ ID NO: 884 | AGACGGGUACGGG CAAAAUCAAG | SEQ ID NO: 332 |
| ETX-S00003314 | UmsAfsGmGmCmGfGmCmUmCmGmGmU mAfGmAfUmGfAmUmAmsAmsUm | SEQ ID NO: 885 | UAGGCGGCUCGGU AGAUGAUAAU | SEQ ID NO: 333 |
| ETX-S00003318 | AmsAfsGmUmAmGfGmCmGmGmCmUmC mGfGmUfAmGfAmUmGmsAmsUm | SEQ ID NO: 886 | AAGUAGGCGGCUC GGUAGAUGAU | SEQ ID NO: 334 |
| ETX-S00003322 | CmsUfsUmUmGmAfAmGmGmCmGmAmA mGfUmUfAmAfGmAmGmsCmsCm | SEQ ID NO: 887 | CUUUGAAGGCGAA GUUAAGAGCC | SEQ ID NO: 335 |
| ETX-S00003326 | CmsGfsAmAmGmUfUmAmAmGmAmGmC mCfUmGfGmGfUmGmGmsGmsGm | SEQ ID NO: 888 | CGAAGUUAAGAGC CUGGGUGGGG | SEQ ID NO: 336 |
| ETX-S00003330 | CmsCfsCmUmGmCfUmCmCmUmUmGmG mGfAmAfUmAfCmGmGmsAmsCm | SEQ ID NO: 889 | CCCUGCUCCUUGG GAAUACGGAC | SEQ ID NO: 337 |
| ETX-S00003334 | GmsAfsAmGmGmAfCmAmGmAmAmCmU mCfCmCfUmGfCmUmCmsCmsUm | SEQ ID NO: 890 | GAAGGACAGAACU CCCUGCUCCU | SEQ ID NO: 338 |
| ETX-S00003338 | AmsGfsAmAmCmUfCmCmCmUmGmCmU mCfCmUfUmGfGmGmAmsAmsUm | SEQ ID NO: 891 | AGAACUCCCUGCU CCUUGGGAAU | SEQ ID NO: 339 |
| ETX-S00003342 | CmsCfsUmGmCmUfCmCmUmUmGmGmG mAfAmUfAmCfGmGmAmsCmsCm | SEQ ID NO: 892 | CCUGCUCCUUGGG AAUACGGACC | SEQ ID NO: 340 |
| ETX-S00003346 | CmsAfsGmAmAmGfGmAmCmAmGmAmA mCfUmCfCmCfUmGmCmsUmsCm | SEQ ID NO: 893 | CAGAAGGACAGAA CUCCCUGCUC | SEQ ID NO: 341 |
| ETX-S00003350 | CmsUfsGmCmUmCfCmUmUmGmGmGmA mAfUmAfCmGfGmAmCmsCmsAm | SEQ ID NO: 894 | CUGCUCCUUGGA AUACGGACCA | SEQ ID NO: 342 |
| ETX-S00003354 | AmsGfsAmAmGmGfAmCmAmGmAmAmC mUfCmCfCmUfGmCmUmsCmsCm | SEQ ID NO: 895 | AGAAGGACAGAAC UCCCUGCUCC | SEQ ID NO: 343 |
| ETX-S00003358 | AmsAfsGmGmAmCfAmGmAmAmCmUmC mCfCmUfGmCfUmCmCmsUmsUm | SEQ ID NO: 896 | AAGGACAGAACUC CCUGCUCCUU | SEQ ID NO: 344 |
| ETX-S00003362 | GmsAfsAmCmUmCfCmCmUmGmCmUmC mCfUmUfGmGfGmAmAmsUmsAm | SEQ ID NO: 897 | GAACUCCCUGCUC CUUGGGAAUA | SEQ ID NO: 345 |
| ETX-S00003366 | AmsGfsGmAmCmAfGmAmAmCmUmCmC mCfUmGfCmUfCmCmUmsUmsGm | SEQ ID NO: 898 | AGGACAGAACUCC CUGCUCCUUG | SEQ ID NO: 346 |
| ETX-S00003370 | GmsAfsAmGmUmUfAmAmGmAmGmCmC mUfGmGfGmUfGmGmGmsGmsAm | SEQ ID NO: 899 | GAAGUUAAGAGCC UGGGUGGGGA | SEQ ID NO: 347 |
| ETX-S00003374 | UmsCfsUmUmUmGfAmAmGmGmCmGmA mAfGmUfUmAfAmGmAmsGmsCm | SEQ ID NO: 900 | UCUUUGAAGGCGA AGUUAAGAGC | SEQ ID NO: 348 |
| ETX-S00003378 | UmsAfsUmUmUmAfUmCmAmGmGmAmG mUfGmAfCmUfGmAmAmsAmsUm | SEQ ID NO: 901 | UAUUUAUCAGGAG UGACUGAAAU | SEQ ID NO: 349 |
| ETX-S00003382 | GmsUfsUmAmUmUfUmAmUmCmAmGmG mAfGmUfGmAfCmUmGmsAmsAm | SEQ ID NO: 902 | GUUAUUUAUCAGG AGUGACUGAA | SEQ ID NO: 350 |
| ETX-S00003386 | UmsUfsAmUmUmUfAmUmCmAmGmGmA mGfUmGfAmCfUmGmAmsAmsAm | SEQ ID NO: 903 | UUAUUUAUCAGGA GUGACUGAAA | SEQ ID NO: 351 |
| ETX-S00003390 | AmsAfsAmUmAmCfAmAmUmUmGmAmG mUfCmCfCmAfUmCmAmsUmsCm | SEQ ID NO: 904 | AAAUACAAUUGAG UCCCAUCAUC | SEQ ID NO: 352 |
| ETX-S00003394 | AmsAfsAmAmUmAfCmAmAmUmUmGmA mGfUmCfCmCfAmUmCmsAmsUm | SEQ ID NO: 905 | AAAAUACAAUUGA GUCCCAUCAU | SEQ ID NO: 353 |
| ETX-S00003398 | AmsUfsUmUmGmUfUmAmUmUmUmAmU mCfAmGfGmAfGmUmGmsAmsCm | SEQ ID NO: 906 | AUUUGUUAUUUAU CAGGAGUGAC | SEQ ID NO: 354 |
| ETX-S00003402 | GmsAfsGmAmAmAfAmCmUmGmGmUmC mAfGmAfUmGfAmAmUmsAmsUm | SEQ ID NO: 907 | GAGAAAACUGGUC AGAUGAAUAU | SEQ ID NO: 355 |
| ETX-S00003406 | AmsUfsAmUmAmAfUmAmCmAmAmCmA mUfGmCfCmUfGmUmUmsCmsAm | SEQ ID NO: 908 | AUAUAAUACAACA UGCCUGUUCA | SEQ ID NO: 356 |
| ETX-S00003410 | UmsGfsGmGmAmUfCmCmGmGmAmAmG mCfAmUfUmCfCmCmUmsUmsUm | SEQ ID NO: 909 | UGGGAUCCGGAAG CAUUCCCUUU | SEQ ID NO: 357 |
| ETX-S00003414 | UmsUfsGmGmGmAfUmCmCmGmGmAmA mGfCmAfUmUfCmCmCmsUmsUm | SEQ ID NO: 910 | UUGGGAUCCGGAA GCAUUCCCUU | SEQ ID NO: 358 |
| ETX-S00003418 | CmsAfsGmGmCmAfGmUmCmAmCmCmG mAfGmGfCmCfUmCmGmsGmsAm | SEQ ID NO: 911 | CAGGCAGUCACCG AGGCCUCGGA | SEQ ID NO: 359 |
| ETX-S00003422 | UmsAfsUmAmUmAfAmUmAmCmAmAmC mAfUmGfCmCfUmGmUmsUmsCm | SEQ ID NO: 912 | UAUAUAAUACAAC AUGCCUGUUC | SEQ ID NO: 360 |
| ETX-S00003426 | AmsGfsUmCmUmAfUmAmAmUmGmCmC mUfUmUfGmUfAmUmUmsGmsCm | SEQ ID NO: 913 | AGUCUAUAAUGCC UUUGUAUUGC | SEQ ID NO: 361 |
| ETX-S00003430 | UmsUfsUmAmUmCfAmGmGmAmGmUmG mAfCmUfGmAfAmAmUmsAmsAm | SEQ ID NO: 914 | UUUAUCAGGAGUG ACUGAAAUAA | SEQ ID NO: 362 |
| ETX-S00003434 | AmsAfsUmAmCmAfAmUmUmGmAmGmU mCfCmCfAmUfCmAmUmsCmsAm | SEQ ID NO: 915 | AAUACAAUUGAGU CCCAUCAUCA | SEQ ID NO: 363 |
| ETX-S00003438 | CmsUfsUmUmAmAfGmAmGmAmAmAmA mCfUmGfGmUfCmAmGmsAmsUm | SEQ ID NO: 916 | CUUUAAGAGAAAA CUGGUCAGAU | SEQ ID NO: 364 |
| ETX-S00003442 | GmsCfsUmUmUmAfAmGmAmGmAmAmA mAfCmUfGmGfUmCmAmsGmsAm | SEQ ID NO: 917 | GCUUUAAGAGAAA ACUGGUCAGA | SEQ ID NO: 365 |
| ETX-S00003446 | AmsAfsUmGmCmCfUmUmUmGmUmAmU mUfGmCfUmUfAmUmCmsUmsGm | SEQ ID NO: 918 | AAUGCCUUUGUAU UGCUUAUCUG | SEQ ID NO: 366 |
| ETX-S00003450 | UmsAfsAmUmGmCfCmUmUmUmGmUmA mUfUmGfCmUfUmAmUmsCmsUm | SEQ ID NO: 919 | UAAUGCCUUUGUA UUGCUUAUCU | SEQ ID NO: 367 |
| ETX-S00003454 | UmsAfsCmCmCmUfGmCmAmCmAmGmA mCfAmCfGmUfUmAmAmsAmsGm | SEQ ID NO: 920 | UACCCUGCACAGA CACGUUAAAG | SEQ ID NO: 368 |
| ETX-S00003458 | AmsUfsAmCmCmCfUmGmCmAmCmAmG mAfCmAfCmGfUmUmAmsAmsAm | SEQ ID NO: 921 | AUACCCUGCACAG ACACGUUAAA | SEQ ID NO: 369 |
| ETX-S00003462 | AmsUfsCmCmAmGfCmUmGmAmUmGmA mCfGmAfUmGfUmGmAmsGmsUm | SEQ ID NO: 922 | AUCCAGCUGAUGA CGAUGUGAGU | SEQ ID NO: 370 |
| ETX-S00003466 | UmsAfsCmAmAmUfUmGmAmGmUmCmC mCfAmUfCmAfUmCmAmsUmsCm | SEQ ID NO: 923 | UACAAUUGAGUCC CAUCAUCAUC | SEQ ID NO: 371 |
| ETX-S00003470 | UmsGfsGmGmAmAfUmAmCmGmGmAmC mCfAmCfGmCfAmGmUmsCmsUm | SEQ ID NO: 924 | UGGGAAUACGGAC CACGCAGUCU | SEQ ID NO: 372 |
| ETX-S00003474 | UmsAfsUmAmAmUfAmCmAmAmCmAmU mGfCmCfUmGfUmUmCmsAmsCm | SEQ ID NO: 925 | UAUAAUACAACAU GCCUGUUCAC | SEQ ID NO: 373 |
| ETX-S00003478 | UmsUfsAmUmCmAfGmGmAmGmUmGmA mCfUmGfAmAfAmUmAmsAmsAm | SEQ ID NO: 926 | UUAUCAGGAGUGA CUGAAAUAAA | SEQ ID NO: 374 |
| ETX-S00003482 | AmsUfsAmAmUmGfCmCmUmUmUmGmU mAfUmUfGmCfUmUmAmsUmsCm | SEQ ID NO: 927 | AUAAUGCCUUUGU AUUGCUUAUC | SEQ ID NO: 375 |
| ETX-S00003486 | CmsCfsAmGmCmUfGmAmUmGmAmCmG mAfUmGfUmGfAmGmUmsGmsUm | SEQ ID NO: 928 | CCAGCUGAUGACG AUGUGAGUGU | SEQ ID NO: 376 |
| ETX-S00003490 | CmsAfsUmAmGmAfUmAmCmCmGmAmA mGfUmAfGmGfCmGmGmsCmsUm | SEQ ID NO: 929 | CAUAGAUACCGAA GUAGGCGGCU | SEQ ID NO: 377 |
| ETX-S00003494 | CmsAfsUmCmCmAfGmCmUmGmAmUmG mAfCmGfAmUfGmUmGmsAmsGm | SEQ ID NO: 930 | CAUCCAGCUGAUG ACGAUGUGAG | SEQ ID NO: 378 |
| ETX-S00003498 | GmsUfsCmAmUmAfGmAmUmAmCmCmG mAfAmGfUmAfGmGmCmsGmsGm | SEQ ID NO: 931 | GUCAUAGAUACCG AAGUAGGCGG | SEQ ID NO: 379 |
| ETX-S00003502 | AmsCfsCmCmUmGfCmAmCmAmGmAmC mAfCmGfUmUfAmAmAmsGmsCm | SEQ ID NO: 932 | ACCCUGCACAGAC ACGUUAAAGC | SEQ ID NO: 380 |
| ETX-S00003506 | UmsGfsCmAmUmCfAmUmCmAmUmGmC mGfGmCfGmGfCmGmAmsAmsCm | SEQ ID NO: 933 | UGCAUCAUCAUGC GGCGGCGAAC | SEQ ID NO: 381 |
| ETX-S00003510 | CmsGfsGmAmCmCfAmCmGmCmAmGmU mCfUmAfUmAfAmUmGmsCmsCm | SEQ ID NO: 934 | CGGACCACGCAGU CUAUAAUGCC | SEQ ID NO: 382 |
| ETX-S00003514 | CmsUfsUmGmGmGfAmAmUmAmCmGmG mAfCmCfAmCfGmCmAmsGmsUm | SEQ ID NO: 935 | CUUGGGAAUACGG ACCACGCAGU | SEQ ID NO: 383 |
| ETX-S00003518 | GmsAfsAmGmUmAfGmGmCmGmGmCmU mCfGmGfUmAfGmAmUmsGmsAm | SEQ ID NO: 936 | GAAGUAGGCGGCU CGGUAGAUGA | SEQ ID NO: 384 |
| ETX-S00003522 | CmsUfsGmCmUmAfGmAmCmGmGmGmU mAfCmGfGmGfCmAmAmsAmsAm | SEQ ID NO: 937 | CUGCUAGACGGU ACGGGCAAAA | SEQ ID NO: 385 |
| ETX-S00003526 | UmsCfsCmAmGmCfUmGmAmUmGmAmC mGfAmUfGmUfGmAmGmsUmsGm | SEQ ID NO: 938 | UCCAGCUGAUGAC GAUGUGAGUG | SEQ ID NO: 386 |
| ETX-S00003530 | AmsUfsAmCmGmGfAmCmCmAmCmGmC mAfGmUfCmUfAmUmAmsAmsUm | SEQ ID NO: 939 | AUACGGACCACGC AGUCUAUAAU | SEQ ID NO: 387 |
| ETX-S00003534 | AmsAfsCmCmAmGfGmCmAmGmUmCmA mCfCmGfAmGfGmCmCmsUmsCm | SEQ ID NO: 940 | AACCAGGCAGUCA CCGAGGCCUC | SEQ ID NO: 388 |
| ETX-S00003538 | UmsGfsAmCmAmGfUmCmUmGmUmGmC mGfAmUfCmAfUmCmCmsAmsGm | SEQ ID NO: 941 | UGACAGUCUGUGC GAUCAUCCAG | SEQ ID NO: 389 |
| ETX-S00003542 | UmsAfsGmAmCmGfGmGmUmAmCmGmG mGfCmAfAmAfAmUmCmsAmsAm | SEQ ID NO: 942 | UAGACGGGUACGG GCAAAAUCAA | SEQ ID NO: 390 |
| ETX-S00003546 | GmsCfsAmGmUmGfAmCmAmGmUmCmU mGfUmGfCmGfAmUmCmsAmsUm | SEQ ID NO: 943 | GCAGUGACAGUCU GUGCGAUCAU | SEQ ID NO: 391 |
| ETX-S00003550 | AmsGfsUmGmAmCfAmGmUmCmUmGmU mGfCmGfAmUfCmAmUmsCmsCm | SEQ ID NO: 944 | AGUGACAGUCUGU GCGAUCAUCC | SEQ ID NO: 392 |
| ETX-S00003554 | GmsCfsUmAmGmAfCmGmGmUmAmC mGfGmGfCmAfAmAmAmsUmsCm | SEQ ID NO: 945 | GCUAGACGGGUAC GGGCAAAAUC | SEQ ID NO: 393 |
| ETX-S00003558 | AmsGfsUmAmGmGfCmGmGmCmUmCmG mGfUmAfGmAfUmGmAmsUmsAm | SEQ ID NO: 946 | AGUAGGCGGCUCG GUAGAUGAUA | SEQ ID NO: 394 |
| ETX-S00003562 | GmsAfsAmUmAmCfGmGmAmCmCmAmC mGfCmAfGmUfCmUmAmsUmsAm | SEQ ID NO: 947 | GAAUACGGACCAC GCAGUCUAUA | SEQ ID NO: 395 |
| ETX-S00003566 | CmsUfsUmUmGmUfAmUmUmGmCmUmU mAfUmCfUmGfCmAmGmsUmsGm | SEQ ID NO: 948 | CUUUGUAUUGCUU AUCUGCAGUG | SEQ ID NO: 396 |
| ETX-S00003570 | UmsUfsUmGmUmAfUmUmGmCmUmUmA mUfCmUfGmCfAmGmUmsGmsAm | SEQ ID NO: 949 | UUUGUAUUGCUUA UCUGCAGUGA | SEQ ID NO: 397 |
| ETX-S00003574 | UmsUfsGmUmAmUfUmGmCmUmUmAmU mCfUmGfCmAfGmUmGmsAmsUm | SEQ ID NO: 950 | UUGUAUUGCUUAU CUGCAGUGAU | SEQ ID NO: 398 |
| ETX-S00003578 | UmsAfsUmCmUmGfCmAmGmUmGmAmU mCfUmGfCmUfUmGmCmsUmsGm | SEQ ID NO: 951 | UAUCUGCAGUGAU CUGCUUGCUG | SEQ ID NO: 399 |
| ETX-S00003582 | GmsUfsAmUmUmGfCmUmUmAmUmCmU mGfCmAfGmUfGmAmUmsCmsUm | SEQ ID NO: 952 | GUAUUGCUUAUCU GCAGUGAUCU | SEQ ID NO: 400 |
| ETX-S00003586 | UmsGfsUmAmUmUfGmCmUmUmAmUmC mUfGmCfAmGfUmGmAmsUmsCm | SEQ ID NO: 953 | UGUAUUGCUUAUC UGCAGUGAUC | SEQ ID NO: 401 |
| ETX-S00003590 | UmsGfsCmUmUmAfUmCmUmGmCmAmG mUfGmAfUmCfUmGmCmsUmsUm | SEQ ID NO: 954 | UGCUUAUCUGCAG UGAUCUGCUU | SEQ ID NO: 402 |
| ETX-S00003594 | UmsUfsGmCmUmUfAmUmCmUmGmCmA mGfUmGfAmUfCmUmGmsCmsUm | SEQ ID NO: 955 | UUGCUUAUCUGCA GUGAUCUGCU | SEQ ID NO: 403 |
| ETX-S00003598 | AmsUfsCmUmGmCfAmGmUmGmAmUmC mUfGmCfUmUfGmCmUmsGmsGm | SEQ ID NO: 956 | AUCUGCAGUGAUC UGCUUGCUGG | SEQ ID NO: 404 |
| ETX-S00003602 | UmsAfsUmUmGmCfUmUmAmUmCmUmG mCfAmGfUmGfAmUmCmsUmsGm | SEQ ID NO: 957 | UAUUGCUUAUCUG CAGUGAUCUG | SEQ ID NO: 405 |
| ETX-S00003606 | UmsUfsCmUmCmCfAmAmAmUmUmUmG mUfUmAfUmUfUmAmUmsCmsAm | SEQ ID NO: 958 | UUCUCCAAAUUUG UUAUUUAUCA | SEQ ID NO: 406 |
| ETX-S00003610 | CmsAfsAmAmUmUfUmGmUmUmAmUmU mUfAmUfCmAfGmGmAmsGmsUm | SEQ ID NO: 959 | CAAAUUUGUUAUU UAUCAGGAGU | SEQ ID NO: 407 |
| ETX-S00003614 | GmsAfsGmGmCmCfUmCmGmGmAmAmU mUfCmCfCmUfUmUmCmsAmsGm | SEQ ID NO: 960 | GAGGCCUCGGAAU UCCCUUUCAG | SEQ ID NO: 408 |
| ETX-S00003618 | AmsGfsAmUmAmUfGmUmUmAmUmAmU mAfAmUfAmCfAmAmCmsAmsUm | SEQ ID NO: 961 | AGAUAUGUUAUAU AAUACAACAU | SEQ ID NO: 409 |
| ETX-S00003622 | GmsCfsAmGmUmCfAmCmCmGmAmGmG mCfCmUfCmGfGmAmAmsUmsUm | SEQ ID NO: 962 | GCAGUCACCGAGG CCUCGGAAUU | SEQ ID NO: 410 |
| ETX-S00003626 | CmsGfsAmGmGmCfCmUmCmGmGmAmA mUfUmCfCmCfUmUmUmsCmsAm | SEQ ID NO: 963 | CGAGGCCUCGGAA UUCCCUUUCA | SEQ ID NO: 411 |
| ETX-S00003630 | CmsAfsAmGmAmUfAmUmGmUmUmAmU mAfUmAfAmUfAmCmAmsAmsCm | SEQ ID NO: 964 | CAAGAUAUGUUAU AUAAUACAAC | SEQ ID NO: 412 |
| ETX-S00003634 | AmsGfsUmCmAmCfCmGmAmGmGmCmC mUfCmGfGmAfAmUmUmsCmsCm | SEQ ID NO: 965 | AGUCACCGAGGCC UCGGAAUUCC | SEQ ID NO: 413 |
| ETX-S00003638 | CmsAfsCmCmGmAfGmGmCmCmUmCmG mGfAmAfUmUfCmCmCmsUmsUm | SEQ ID NO: 966 | CACCGAGGCCUCG GAAUUCCCUU | SEQ ID NO: 414 |
| ETX-S00003642 | CmsCfsUmCmGmGfAmAmUmUmCmCmC mUfUmUfCmAfGmCmUmsCmsCm | SEQ ID NO: 967 | CCUCGGAAUUCCC UUUCAGCUCC | SEQ ID NO: 415 |
| ETX-S00003646 | UmsCfsGmGmAmAfUmUmCmCmCmUmU mUfCmAfGmCfUmCmCmsAmsGm | SEQ ID NO: 968 | UCGGAAUUCCCUU UCAGCUCCAG | SEQ ID NO: 416 |
| ETX-S00003650 | GmsGfsCmCmUmCfGmGmAmAmUmUmC mCfCmUfUmUfCmAmGmsCmsUm | SEQ ID NO: 969 | GGCCUCGGAAUUC CCUUUCAGCU | SEQ ID NO: 417 |
| ETX-S00003654 | CmsUfsCmGmGmAfAmUmUmCmCmCmU mUfUmCfAmGfCmUmCmsCmsAm | SEQ ID NO: 970 | CUCGGAAUUCCCU UUCAGCUCCA | SEQ ID NO: 418 |
| ETX-S00003658 | GmsCfsCmUmCmGfGmAmAmUmUmCmC mCfUmUfUmCfAmGmCmsUmsCm | SEQ ID NO: 971 | GCCUCGGAAUUCC CUUUCAGCUC | SEQ ID NO: 419 |
| ETX-S00003662 | AmsUfsGmUmUmAfUmAmUmAmAmUmA mCfAmAfCmAfUmGmCmsCmsUm | SEQ ID NO: 972 | AUGUUAUAUAAUA CAACAUGCCU | SEQ ID NO: 420 |
| ETX-S00003666 | AmsAfsGmAmUmAfUmGmUmUmAmUmA mUfAmAfUmAfCmAmAmsCmsAm | SEQ ID NO: 973 | AAGAUAUGUUAUA UAAUACAACA | SEQ ID NO: 421 |
| ETX-S00003670 | UmsCfsAmAmGmAfUmAmUmGmUmUmA mUfAmUfAmAfUmAmCmsAmsAm | SEQ ID NO: 974 | UCAAGAUAUGUUA UAUAAUACAA | SEQ ID NO: 422 |
| ETX-S00003674 | UmsGfsUmUmAmUfAmUmAmAmUmAmC mAfAmCfAmUfGmCmCmsUmsGm | SEQ ID NO: 975 | UGUUAUAUAAUAC AACAUGCCUG | SEQ ID NO: 423 |
| ETX-S00003678 | CmsCfsGmAmGmGfCmCmUmCmGmGmA mAfUmUfCmCfCmUmUmsUmsCm | SEQ ID NO: 976 | CCGAGGCCUCGGA AUUCCCUUUC | SEQ ID NO: 424 |
| ETX-S00003682 | GmsUfsCmAmCmCfGmAmGmGmCmCmU mCfGmGfAmAfUmUmCmsCmsCm | SEQ ID NO: 977 | GUCACCGAGGCCU CGGAAUUCCC | SEQ ID NO: 425 |
| ETX-S00003686 | AmsCfsCmGmAmGfGmCmCmUmCmGmG mAfAmUfUmCfCmCmUmsUmsUm | SEQ ID NO: 978 | ACCGAGGCCUCGG AAUUCCCUUU | SEQ ID NO: 426 |
| ETX-S00003690 | UmsCfsAmCmCmGfAmGmGmCmCmUmC mGfGmAfAmUfUmCmCmsCmsUm | SEQ ID NO: 979 | UCACCGAGGCCUC GGAAUUCCCU | SEQ ID NO: 427 |
| ETX-S00003694 | AmsUfsUmUmCmAfUmCmAmUmAmCmA mAfGmAfCmAfAmGmCmsAmsCm | SEQ ID NO: 980 | AUUUCAUCAUACA AGACAAGCAC | SEQ ID NO: 428 |
| ETX-S00003698 | UmsGfsUmAmUmUfUmAmUmCmUmUmU mGfAmAfGmGfCmGmAmsAmsGm | SEQ ID NO: 981 | UGUAUUUAUCUUU GAAGGCGAAG | SEQ ID NO: 429 |
| ETX-S00003702 | AmsUfsUmUmGmUfAmGmAmUmCmUmU mAfAmCfCmAfGmGmCmsAmsGm | SEQ ID NO: 982 | AUUUGUAGAUCUU AACCAGGCAG | SEQ ID NO: 430 |
| ETX-S00003706 | UmsUfsCmCmCmUfUmUmGmCmAmGmU mGfUmCfAmUfAmGmAmsUmsAm | SEQ ID NO: 983 | UUCCCUUUGCAGU GUCAUAGAUA | SEQ ID NO: 431 |
| ETX-S00003710 | AmsUfsCmAmUmAfCmAmAmGmAmCmA mAfGmCfAmCfAmAmAmsAmsGm | SEQ ID NO: 984 | AUCAUACAAGACA AGCACAAAAG | SEQ ID NO: 432 |
| ETX-S00003714 | UmsAfsGmAmUmCfUmUmAmAmCmCmA mGfGmCfAmGfUmCmAmsCmsCm | SEQ ID NO: 985 | UAGAUCUUAACCA GGCAGUCACC | SEQ ID NO: 433 |
| ETX-S00003718 | AmsAfsGmCmAmUfUmCmCmCmUmUmU mGfCmAfGmUfGmUmCmsAmsUm | SEQ ID NO: 986 | AAGCAUUCCCUUU GCAGUGUCAU | SEQ ID NO: 434 |
| ETX-S00003722 | UmsUfsUmGmCmAfGmUmGmUmCmAmU mAfGmAfUmAfCmCmGmsAmsAm | SEQ ID NO: 987 | UUUGCAGUGUCAU AGAUACCGAA | SEQ ID NO: 435 |
| ETX-S00003726 | AmsGfsAmGmAmAfAmAmCmUmGmGmU mCfAmGfAmUfGmAmAmsUmsAm | SEQ ID NO: 988 | AGAGAAAACUGGU CAGAUGAAUA | SEQ ID NO: 436 |
| ETX-S00003730 | UmsGfsCmAmGmUfGmUmCmAmUmAmG mAfUmAfCmCfGmAmAmsGmsUm | SEQ ID NO: 989 | UGCAGUGUCAUAG AUACCGAAGU | SEQ ID NO: 437 |
| ETX-S00003734 | UmsCfsAmGmAmUfUmUmGmUmAmGmA mUfCmUfUmAfAmCmCmsAmsGm | SEQ ID NO: 990 | UCAGAUUUGUAGA UCUUAACCAG | SEQ ID NO: 438 |
| ETX-S00003738 | UmsCfsCmCmUmUfUmGmCmAmGmUmG mUfCmAfUmAfGmAmUmsAmsCm | SEQ ID NO: 991 | UCCCUUUGCAGUG UCAUAGAUAC | SEQ ID NO: 439 |
| ETX-S00003742 | GmsAfsAmCmAmUfUmGmGmAmCmCmA mUfGmCfAmCfCmCmUmsUmsGm | SEQ ID NO: 992 | GAACAUUGGACCA UGCACCCUUG | SEQ ID NO: 440 |
| ETX-S00003746 | AmsAfsUmCmCmCfAmUmCmAmGmAmU mUfUmGfUmAfGmAmUmsCmsUm | SEQ ID NO: 993 | AAUCCCAUCAGAU UUGUAGAUCU | SEQ ID NO: 441 |
| ETX-S00003750 | GmsCfsAmGmUmGfUmCmAmUmAmGmA mUfAmCfCmGfAmAmGmsUmsAm | SEQ ID NO: 994 | GCAGUGUCAUAGA UACCGAAGUA | SEQ ID NO: 442 |
| ETX-S00003754 | CmsAfsAmGmCmAfCmAmAmAmAmGmC mAfCmCfAmCfCmCmAmsUmsGm | SEQ ID NO: 995 | CAAGCACAAAAGC ACCACCCAUG | SEQ ID NO: 443 |
| ETX-S00003758 | GmsCfsGmGmCmUfCmGmGmUmAmGmA mUfGmAfUmAfAmUmAmsCmsCm | SEQ ID NO: 996 | GCGGCUCGGUAGA UGAUAAUACC | SEQ ID NO: 444 |
| ETX-S00003762 | CmsUfsCmCmUmUfGmGmGmAmAmUmA mCfGmGfAmCfCmAmCmsGmsCm | SEQ ID NO: 997 | CUCCUUGGAAUA CGGACCACGC | SEQ ID NO: 445 |
| ETX-S00003766 | AmsUfsUmCmCmCfUmUmUmGmCmAmG mUfGmUfCmAfUmAmGmsAmsUm | SEQ ID NO: 998 | AUUCCCUUUGCAG UGUCAUAGAU | SEQ ID NO: 446 |
| ETX-S00003770 | CmsAfsUmAmCmAfAmGmAmCmAmAmG mCfAmCfAmAfAmAmGmsCmsAm | SEQ ID NO: 999 | CAUACAAGACAAG CACAAAAGCA | SEQ ID NO: 447 |
| ETX-S00003774 | AmsGfsAmCmAmCfGmUmUmAmAmAmG mCfCmUfUmGfGmUmAmsCmsAm | SEQ ID NO: 1000 | AGACACGUUAAAG CCUUGGUACA | SEQ ID NO: 448 |
| ETX-S00003778 | UmsGfsAmGmAmAfCmAmUmUmGmGmA mCfCmAfUmGfCmAmCmsCmsCm | SEQ ID NO: 1001 | UGAGAACAUUGGA CCAUGCACCC | SEQ ID NO: 449 |
| ETX-S00003782 | GmsCfsUmUmGmUfAmUmUmAmUmC mUfUmUfGmAfAmGmGmsCmsGm | SEQ ID NO: 1002 | GCUUGUAUUUAUC UUUGAAGGCG | SEQ ID NO: 450 |
| ETX-S00003786 | GmsCfsUmCmGmGfUmAmGmAmUmGmA mUfAmAfUmAfCmCmCmsUmsGm | SEQ ID NO: 1003 | GCUCGGUAGAUGA UAAUACCCUG | SEQ ID NO: 451 |
| ETX-S00003790 | AmsGfsUmGmUmCfAmUmAmGmAmUmA mCfCmGfAmAfGmUmAmsGmsGm | SEQ ID NO: 1004 | AGUGUCAUAGAUA CCGAAGUAGG | SEQ ID NO: 452 |
| ETX-S00003794 | AmsUfsCmAmGmAfUmUmUmGmUmAmG mAfUmCfUmUfAmAmCmsCmsAm | SEQ ID NO: 1005 | AUCAGAUUUGUAG AUCUUAACCA | SEQ ID NO: 453 |
| ETX-S00003798 | GmsGfsUmAmGmAfUmGmAmUmAmAmU mAfCmCfCmUfGmCmAmsCmsAm | SEQ ID NO: 1006 | GGUAGAUGAUAAU ACCCUGCACA | SEQ ID NO: 454 |
| ETX-S00003802 | CmsAfsGmGmCmCfCmUmUmAmAmUmC mCfCmAfUmCfAmGmAmsUmsUm | SEQ ID NO: 1007 | CAGGCCCUUAAUC CCAUCAGAUU | SEQ ID NO: 455 |
| ETX-S00003806 | AmsGfsAmUmGmAfUmAmAmUmAmCmC mCfUmGfCmAfCmAmGmsAmsCm | SEQ ID NO: 1008 | AGAUGAUAAUACC CUGCACAGAC | SEQ ID NO: 456 |
| ETX-S00003810 | UmsAfsCmAmGmGfCmCmCmUmUmAmA mUfCmCfCmAfUmCmAmsGmsAm | SEQ ID NO: 1009 | UACAGGCCCUUAA UCCCAUCAGA | SEQ ID NO: 457 |
| ETX-S00003814 | AmsCfsAmGmAmCfAmCmGmUmUmAmA mAfGmCfCmUfUmGmGmsUmsAm | SEQ ID NO: 1010 | ACAGACACGUUAA AGCCUUGGUA | SEQ ID NO: 458 |
| ETX-S00003818 | AmsGfsAmAmAmAfCmUmGmGmUmCmA mGfAmUfGmAfAmUmAmsUmsUm | SEQ ID NO: 1011 | AGAAAACUGGUCA GAUGAAUAUU | SEQ ID NO: 459 |
| ETX-S00003822 | UmsGfsUmAmGmAfUmCmUmUmAmAmC mCfAmGfGmCfAmGmUmsCmsAm | SEQ ID NO: 1012 | UGUAGAUCUUAAC CAGGCAGUCA | SEQ ID NO: 460 |
| ETX-S00003826 | UmsCfsAmAmAmUfGmGmAmUmAmGmG mAfAmGfUmCfAmAmCmsCmsCm | SEQ ID NO: 1013 | UCAAAUGGAUAGG AAGUCAACCC | SEQ ID NO: 461 |
| ETX-S00003830 | CmsAfsUmUmCmCfCmUmUmUmGmCmA mGfUmGfUmCfAmUmAmsGmsAm | SEQ ID NO: 1014 | CAUUCCCUUUGCA GUGUCAUAGA | SEQ ID NO: 462 |
| ETX-S00003834 | CmsAfsUmCmAmGfAmUmUmGmUmA mGfAmUfCmUfUmAmAmsCmsCm | SEQ ID NO: 1015 | CAUCAGAUUUGUA GAUCUUAACC | SEQ ID NO: 463 |
| ETX-S00003838 | CmsUfsCmGmGmUfAmGmAmUmGmAmU mAfAmUfAmCfCmCmUmsGmsCm | SEQ ID NO: 1016 | CUCGGUAGAUGAU AAUACCCUGC | SEQ ID NO: 464 |
| ETX-S00003842 | UmsUfsUmGmUmAfGmAmUmCmUmUmA mAfCmCfAmGfGmCmAmsGmsUm | SEQ ID NO: 1017 | UUUGUAGAUCUUA ACCAGGCAGU | SEQ ID NO: 465 |
| ETX-S00003846 | AmsCfsAmGmGmCfCmCmUmUmAmAmU mCfCmCfAmUfCmAmGmsAmsUm | SEQ ID NO: 1018 | ACAGGCCCUUAAU CCCAUCAGAU | SEQ ID NO: 466 |
| ETX-S00003850 | CmsUfsUmUmGmCfAmGmUmGmUmCmA mUfAmGfAmUfAmCmCmsGmsAm | SEQ ID NO: 1019 | CUUUGCAGUGUCA UAGAUACCGA | SEQ ID NO: 467 |
| ETX-S00003854 | CmsAfsCmAmUmCfAmGmCmUmGmCmU mAfGmAfCmGfGmGmUmsAmsCm | SEQ ID NO: 1020 | CACAUCAGCUGCU AGACGGUAC | SEQ ID NO: 468 |
| ETX-S00003858 | CmsCfsCmUmUmUfGmCmAmGmUmGmU mCfAmUfAmGfAmUmAmsCmsCm | SEQ ID NO: 1021 | CCCUUUGCAGUGU CAUAGAUACC | SEQ ID NO: 469 |
| ETX-S00003862 | CmsUfsCmCmAmGfCmUmUmUmAmCmC mCfAmCfAmUfCmAmGmsCmsUm | SEQ ID NO: 1022 | CUCCAGCUUUACC CACAUCAGCU | SEQ ID NO: 470 |
| ETX-S00003866 | UmsUfsAmCmCmCfAmCmAmUmCmAmG mCfUmGfCmUfAmGmAmsCmsGm | SEQ ID NO: 1023 | UUACCCACAUCAG CUGCUAGACG | SEQ ID NO: 471 |
| ETX-S00003870 | UmsUfsAmAmAmGfCmCmUmUmGmGmU mAfCmAfGmGfCmCmCmsUmsUm | SEQ ID NO: 1024 | UUAAAGCCUUGGU ACAGGCCCUU | SEQ ID NO: 472 |
| ETX-S00003874 | CmsAfsGmCmUmGfCmUmAmGmAmCmG mGfGmUfAmCfGmGmGmsCmsAm | SEQ ID NO: 1025 | CAGCUGCUAGACG GGUACGGGCA | SEQ ID NO: 473 |
| ETX-S00003878 | CmsAfsGmCmUmUfUmAmCmCmCmAmC mAfUmCfAmGfCmUmGmsCmsUm | SEQ ID NO: 1026 | CAGCUUUACCCAC AUCAGCUGCU | SEQ ID NO: 474 |
| ETX-S00003882 | UmsCfsAmGmCmUfGmCmUmAmGmAmC mGfGmGfUmAfCmGmGmsGmsCm | SEQ ID NO: 1027 | UCAGCUGCUAGAC GGGUACGGGC | SEQ ID NO: 475 |
| ETX-S00003886 | UmsCfsCmAmGmCfUmUmUmAmCmCmC mAfCmAfUmCfAmGmCmsUmsGm | SEQ ID NO: 1028 | UCCAGCUUUACCC ACAUCAGCUG | SEQ ID NO: 476 |
| ETX-S00003890 | GmsGfsCmUmCmGfGmUmAmGmAmUmG mAfUmAfAmUfAmCmCmsCmsUm | SEQ ID NO: 1029 | GGCUCGGUAGAUG AUAAUACCCU | SEQ ID NO: 477 |
| ETX-S00003894 | AmsGfsGmCmGmGfCmUmCmGmGmUmA mGfAmUfGmAfUmAmAmsUmsAm | SEQ ID NO: 1030 | AGGCGGCUCGGUA GAUGAUAAUA | SEQ ID NO: 478 |
| ETX-S00003898 | CmsAfsUmCmAmGfCmUmGmCmUmAmG mAfCmGfGmGfUmAmCmsGmsGm | SEQ ID NO: 1031 | CAUCAGCUGCUAG ACGGGUACGG | SEQ ID NO: 479 |
| ETX-S00003902 | GmsUfsAmCmAmGfGmCmCmCmUmUmA mAfUmCfCmCfAmUmCmsAmsGm | SEQ ID NO: 1032 | GUACAGGCCCUUA AUCCCAUCAG | SEQ ID NO: 480 |
| ETX-S00003906 | AmsUfsGmAmUmAfAmUmAmCmCmCmU mGfCmAfCmAfGmAmCmsAmsCm | SEQ ID NO: 1033 | AUGAUAAUACCCU GCACAGACAC | SEQ ID NO: 481 |
| ETX-S00003910 | CmsCfsUmUmGmGfGmAmAmUmAmCmG mGfAmCfCmAfCmGmCmsAmsGm | SEQ ID NO: 1034 | CCUUGGGAAUACG GACCACGCAG | SEQ ID NO: 482 |
| ETX-S00003914 | UmsCfsCmUmUmGfGmGmAmAmUmAmC mGfGmAfCmCfAmCmGmsCmsAm | SEQ ID NO: 1035 | UCCUUGGAAUAC GGACCACGCA | SEQ ID NO: 483 |
| ETX-S00003918 | CmsAfsGmAmUmUfUmGmUmAmGmAmU mCfUmUfAmAfCmCmAmsGmsGm | SEQ ID NO: 1036 | CAGAUUUGUAGAU CUUAACCAGG | SEQ ID NO: 484 |
| ETX-S00003922 | UmsGfsUmAmCmUfUmCmUmUmGmAmU mUfUmCfAmUfCmAmUmsAmsCm | SEQ ID NO: 1037 | UGUACUUCUUGAU UUCAUCAUAC | SEQ ID NO: 485 |
| ETX-S00003926 | AmsUfsCmCmCmAfUmCmAmGmAmUmU mUfGmUfAmGfAmUmCmsUmsUm | SEQ ID NO: 1038 | AUCCCAUCAGAUU UGUAGAUCUU | SEQ ID NO: 486 |
| ETX-S00003930 | GmsUfsAmGmAmUfGmAmUmAmAmUmA mCfCmCfUmGfCmAmCmsAmsGm | SEQ ID NO: 1039 | GUAGAUGAUAAUA CCCUGCACAG | SEQ ID NO: 487 |
| ETX-S00003934 | GmsAfsCmAmCmGfUmUmAmAmAmGmC mCfUmUfGmGfUmAmCmsAmsGm | SEQ ID NO: 1040 | GACACGUUAAAGC CUUGGUACAG | SEQ ID NO: 488 |
| ETX-S00003938 | AmsGfsCmUmUmUfAmCmCmCmAmCmA mUfCmAfGmCfUmGmCmsUmsAm | SEQ ID NO: 1041 | AGCUUUACCCACA UCAGCUGCUA | SEQ ID NO: 489 |
| ETX-S00003942 | AmsAfsGmCmAmCfAmAmAmAmGmCmA mCfCmAfCmCfCmAmUmsGmsCm | SEQ ID NO: 1042 | AAGCACAAAAGCA CCACCCAUGC | SEQ ID NO: 490 |
| ETX-S00003946 | AmsUfsCmUmUmAfAmCmCmAmGmGmC mAfGmUfCmAfCmCmGmsAmsGm | SEQ ID NO: 1043 | AUCUUAACCAGGC AGUCACCGAG | SEQ ID NO: 491 |
| ETX-S00003950 | UmsUfsUmAmCmCfCmAmCmAmUmCmA mGfCmUfGmCfUmAmGmsAmsCm | SEQ ID NO: 1044 | UUUACCCACAUCA GCUGCUAGAC | SEQ ID NO: 492 |
| ETX-S00003954 | CmsUfsUmAmAmUfCmCmCmAmUmCmA mGfAmUfUmUfGmUmAmsGmsAm | SEQ ID NO: 1045 | CUUAAUCCCAUCA GAUUUGUAGA | SEQ ID NO: 493 |
| ETX-S00003958 | CmsAfsGmUmGmUfCmAmUmAmGmAmU mAfCmCfGmAfAmGmUmsAmsGm | SEQ ID NO: 1046 | CAGUGUCAUAGAU ACCGAAGUAG | SEQ ID NO: 494 |
| ETX-S00003962 | CmsCfsAmGmCmUfUmUmAmCmCmCmA mCfAmUfCmAfGmCmUmsGmsCm | SEQ ID NO: 1047 | CCAGCUUUACCCA CAUCAGCUGC | SEQ ID NO: 495 |
| ETX-S00003966 | AmsUfsCmAmGmCfUmGmCmUmAmGmA mCfGmGfGmUfAmCmGmsGmsGm | SEQ ID NO: 1048 | AUCAGCUGCUAGA CGGGUACGGG | SEQ ID NO: 496 |
| ETX-S00003970 | GmsAfsUmGmAmUfAmAmUmAmCmCmC mUfGmCfAmCfAmGmAmsCmsAm | SEQ ID NO: 1049 | GAUGAUAAUACCC UGCACAGACA | SEQ ID NO: 497 |
| ETX-S00003974 | UmsUfsUmCmAmUfCmAmUmAmCmAmA mGfAmCfAmAfGmCmAmsCmsAm | SEQ ID NO: 1050 | UUUCAUCAUACAA GACAAGCACA | SEQ ID NO: 498 |
| ETX-S00003978 | CmsAfsGmAmCmAfCmGmUmUmAmAmA mGfCmCfUmUfGmGmUmsAmsCm | SEQ ID NO: 1051 | CAGACACGUUAAA GCCUUGGUAC | SEQ ID NO: 499 |
| ETX-S00003982 | AmsGfsAmAmCmAfUmUmGmGmAmCmC mAfUmGfCmAfCmCmCmsUmsUm | SEQ ID NO: 1052 | AGAACAUUGGACC AUGCACCCUU | SEQ ID NO: 500 |
| ETX-S00003986 | GmsUfsAmGmAmUfCmUmUmAmAmCmC mAfGmGfCmAfGmUmCmsAmsCm | SEQ ID NO: 1053 | GUAGAUCUUAACC AGGCAGUCAC | SEQ ID NO: 501 |
| ETX-S00003990 | AmsGfsCmUmCmCfAmGmCmUmUmUmA mCfCmCfAmCfAmUmCmsAmsGm | SEQ ID NO: 1054 | AGCUCCAGCUUUA CCCACAUCAG | SEQ ID NO: 502 |
| ETX-S00003994 | GmsCfsUmCmCmUfUmGmGmGmAmAmU mAfCmGfGmAfCmCmAmsCmsGm | SEQ ID NO: 1055 | GCUCCUUGGGAAU ACGGACCACG | SEQ ID NO: 503 |
| ETX-S00003998 | GmsAfsAmGmCmAfUmUmCmCmCmUmU mUfGmCfAmGfUmGmUmsCmsAm | SEQ ID NO: 1056 | GAAGCAUUCCCUU UGCAGUGUCA | SEQ ID NO: 504 |
| ETX-S00004002 | CmsCfsUmUmGmGfUmAmCmAmGmGmC mCfCmUfUmAfAmUmCmsCmsCm | SEQ ID NO: 1057 | CCUUGGUACAGGC CCUUAAUCCC | SEQ ID NO: 505 |
| ETX-S00004006 | UmsAfsGmAmUmGfAmUmAmAmUmAmC mCfCmUfGmCfAmCmAmsGmsAm | SEQ ID NO: 1058 | UAGAUGAUAAUAC CCUGCACAGA | SEQ ID NO: 506 |
| ETX-S00004010 | AmsAfsUmAmCmCfCmUmGmCmAmCmA mGfAmCfAmCfGmUmUmsAmsAm | SEQ ID NO: 1059 | AAUACCCUGCACA GACACGUUAA | SEQ ID NO: 507 |
| ETX-S00004014 | GmsUfsUmAmAmAfGmCmCmUmUmGmG mUfAmCfAmGfGmCmCmsCmsUm | SEQ ID NO: 1060 | GUUAAAGCCUUGG UACAGGCCCU | SEQ ID NO: 508 |
| ETX-S00004018 | AmsGfsCmAmUmUfCmCmCmUmUmUmG mCfAmGfUmGfUmCmAmsUmsAm | SEQ ID NO: 1061 | AGCAUUCCCUUUG CAGUGUCAUA | SEQ ID NO: 509 |
| ETX-S00004022 | UmsCfsUmUmAmAfCmCmAmGmGmCmA mGfUmCfAmCfCmGmAmsGmsGm | SEQ ID NO: 1062 | UCUUAACCAGGCA GUCACCGAGG | SEQ ID NO: 510 |
| ETX-S00004026 | AmsUfsAmCmAmAfGmAmCmAmAmGmC mAfCmAfAmAfAmGmCmsAmsCm | SEQ ID NO: 1063 | AUACAAGACAAGC ACAAAAGCAC | SEQ ID NO: 511 |
| ETX-S00004030 | GmsAfsCmAmAmGfCmAmCmAmAmAmA mGfCmAfCmCfAmCmCmsCmsAm | SEQ ID NO: 1064 | GACAAGCACAAAA GCACCACCCA | SEQ ID NO: 512 |
| ETX-S00004034 | AmsCfsCmCmAmCfAmUmCmAmGmCmU mGfCmUfAmGfAmCmGmsGmsGm | SEQ ID NO: 1065 | ACCCACAUCAGCU GCUAGACGGG | SEQ ID NO: 513 |
| ETX-S00004038 | UmsAfsAmAmGmCfCmUmUmGmGmUmA mCfAmGfGmCfCmCmUmsUmsAm | SEQ ID NO: 1066 | UAAAGCCUUGGUA CAGGCCCUUA | SEQ ID NO: 514 |
| ETX-S00004042 | UmsUfsCmAmUmCfAmUmAmCmAmAmG mAfCmAfAmGfCmAmCmsAmsAm | SEQ ID NO: 1067 | UUCAUCAUACAAG ACAAGCACAA | SEQ ID NO: 515 |
| ETX-S00004046 | GmsCfsCmCmUmUfAmAmUmCmCmCmA mUfCmAfGmAfUmUmUmsGmsUm | SEQ ID NO: 1068 | GCCCUUAAUCCCA UCAGAUUUGU | SEQ ID NO: 516 |
| ETX-S00004050 | UmsUfsAmAmUmCfCmCmAmUmCmAmG mAfUmUfUmGfUmAmGmsAmsUm | SEQ ID NO: 1069 | UUAAUCCCAUCAG AUUUGUAGAU | SEQ ID NO: 517 |
| ETX-S00004054 | UmsCfsGmGmUmAfGmAmUmGmAmUmA mAfUmAfCmCfCmUmGmsCmsAm | SEQ ID NO: 1070 | UCGGUAGAUGAUA AUACCCUGCA | SEQ ID NO: 518 |
| ETX-S00004058 | CmsAfsCmGmUmUfAmAmAmGmCmCmU mUfGmGfUmAfCmAmGmsGmsCm | SEQ ID NO: 1071 | CACGUUAAAGCCU UGGUACAGGC | SEQ ID NO: 519 |
| ETX-S00004062 | UmsCfsCmCmAmUfCmAmGmAmUmUmU mGfUmAfGmAfUmCmUmsUmsAm | SEQ ID NO: 1072 | UCCCAUCAGAUUU GUAGAUCUUA | SEQ ID NO: 520 |
| ETX-S00004066 | UmsCfsAmUmAmCfAmAmGmAmCmAmA mGfCmAfCmAfAmAmAmsGmsCm | SEQ ID NO: 1073 | UCAUACAAGACAA GCACAAAAGC | SEQ ID NO: 521 |
| ETX-S00004070 | CmsCfsUmUmAmAfUmCmCmCmAmUmC mAfGmAfUmUfUmGmUmsAmsGm | SEQ ID NO: 1074 | CCUUAAUCCCAUC AGAUUUGUAG | SEQ ID NO: 522 |
| ETX-S00004074 | AmsCfsAmUmUmGfGmAmCmCmAmUmG mCfAmCfCmCfUmUmGmsAmsAm | SEQ ID NO: 1075 | ACAUUGGACCAUG CACCCUUGAA | SEQ ID NO: 523 |
| ETX-S00004078 | CmsUfsUmGmGmUfAmCmAmGmGmCmC mCfUmUfAmAfUmCmCmsCmsAm | SEQ ID NO: 1076 | CUUGGUACAGGCC CUUAAUCCCA | SEQ ID NO: 524 |
| ETX-S00004082 | UmsUfsGmCmAmGfUmGmUmCmAmUmA mGfAmUfAmCfCmGmAmsAmsGm | SEQ ID NO: 1077 | UUGCAGUGUCAUA GAUACCGAAG | SEQ ID NO: 525 |
| ETX-S00004086 | CmsAfsCmAmGmAfCmAmCmGmUmUmA mAfAmGfCmCfUmUmGmsGmsUm | SEQ ID NO: 1078 | CACAGACACGUUA AAGCCUUGGU | SEQ ID NO: 526 |
| ETX-S00004090 | AmsGfsCmAmCmAfAmAmAmGmCmAmC mCfAmCfCmCfAmUmGmsCmsCm | SEQ ID NO: 1079 | AGCACAAAAGCAC CACCCAUGCC | SEQ ID NO: 527 |
| ETX-S00004094 | CmsCfsAmUmCmAfGmAmUmUmUmGmU mAfGmAfUmCfUmUmAmsAmsCm | SEQ ID NO: 1080 | CCAUCAGAUUUGU AGAUCUUAAC | SEQ ID NO: 528 |
| ETX-S00004098 | CmsCfsAmCmAmUfCmAmGmCmUmGmC mUfAmGfAmCfGmGmGmsUmsAm | SEQ ID NO: 1081 | CCACAUCAGCUGC UAGACGGUA | SEQ ID NO: 529 |
| ETX-S00004102 | GmsCfsAmUmUmCfCmCmUmUmUmGmC mAfGmUfGmUfCmAmUmsAmsGm | SEQ ID NO: 1082 | GCAUUCCCUUUGC AGUGUCAUAG | SEQ ID NO: 530 |
| ETX-S00004106 | CmsUfsUmUmAmCfCmCmAmCmAmUmC mAfGmCfUmGfCmUmAmsGmsAm | SEQ ID NO: 1083 | CUUUACCCACAUC AGCUGCUAGA | SEQ ID NO: 531 |
| ETX-S00004110 | AmsUfsCmCmGmGfAmAmGmCmAmUmU mCfCmCfUmUfUmGmCmsAmsGm | SEQ ID NO: 1084 | AUCCGGAAGCAUU CCCUUUGCAG | SEQ ID NO: 532 |
| ETX-S00004114 | AmsGfsCmUmGmCfUmAmGmAmCmGmG mGfUmAfCmGfGmGmCmsAmsAm | SEQ ID NO: 1085 | AGCUGCUAGACGG GUACGGGCAA | SEQ ID NO: 533 |
| ETX-S00004118 | CmsGfsGmCmUmCfGmGmUmAmGmAmU mGfAmUfAmAfUmAmCmsCmsCm | SEQ ID NO: 1086 | CGGCUCGGUAGAU GAUAAUACCC | SEQ ID NO: 534 |
| ETX-S00004122 | GmsGfsGmUmAmCfGmGmGmCmAmAmA mAfUmCfAmAfGmAmGmsGmsGm | SEQ ID NO: 1087 | GGGUACGGGCAAA AUCAAGAGGG | SEQ ID NO: 535 |
| ETX-S00004126 | AmsCfsAmUmCmAfGmCmUmGmCmUmA mGfAmCfGmGfGmUmAmsCmsGm | SEQ ID NO: 1088 | ACAUCAGCUGCUA GACGGGUACG | SEQ ID NO: 536 |
| ETX-S00004130 | CmsCfsCmUmUmAfAmUmCmCmCmAmU mCfAmGfAmUfUmUmGmsUmsAm | SEQ ID NO: 1089 | CCCUUAAUCCCAU CAGAUUUGUA | SEQ ID NO: 537 |
| ETX-S00004134 | CmsGfsGmGmCmAfAmAmAmUmCmAmA mGfAmGfGmGfUmAmCmsAmsCm | SEQ ID NO: 1090 | CGGGCAAAAUCAA GAGGGUACAC | SEQ ID NO: 538 |
| ETX-S00004138 | AmsCfsGmUmUmAfAmAmGmCmCmUmU mGfGmUfAmCfAmGmGmsCmsCm | SEQ ID NO: 1091 | ACGUUAAAGCCUU GGUACAGGCC | SEQ ID NO: 539 |
| ETX-S00004142 | UmsGfsGmUmAmCfAmGmGmCmCmCmU mUfAmAfUmCfCmCmAmsUmsCm | SEQ ID NO: 1092 | UGGUACAGGCCCU UAAUCCCAUC | SEQ ID NO: 540 |
| ETX-S00004146 | GmsGfsCmGmGmCfUmCmGmGmUmAmG mAfUmGfAmUfAmAmUmsAmsCm | SEQ ID NO: 1093 | GGCGGCUCGGUAG AUGAUAAUAC | SEQ ID NO: 541 |
| ETX-S00004150 | CmsGfsUmUmAmAfAmGmCmCmUmUmG mGfUmAfCmAfGmGmCmsCmsCm | SEQ ID NO: 1094 | CGUUAAAGCCUUG GUACAGGCCC | SEQ ID NO: 542 |
| ETX-S00004154 | GmsGfsUmAmCmAfGmGmCmCmCmUmU mAfAmUfCmCfCmAmUmsCmsAm | SEQ ID NO: 1095 | GGUACAGGCCCUU AAUCCCAUCA | SEQ ID NO: 543 |
| ETX-S00004158 | UmsAfsAmUmAmCfCmCmUmGmCmAmC mAfGmAfCmAfCmGmUmsUmsAm | SEQ ID NO: 1096 | UAAUACCCUGCAC AGACACGUUA | SEQ ID NO: 544 |
| ETX-S00004162 | GmsAfsCmCmAmCfGmCmAmGmUmCmU mAfUmAfAmUfGmCmCmsUmsUm | SEQ ID NO: 1097 | GACCACGCAGUCU AUAAUGCCUU | SEQ ID NO: 545 |
| ETX-S00004166 | GmsUfsUmAmAmGfAmGmCmCmUmGmG mGfUmGfGmGfGmAmAmsGmsUm | SEQ ID NO: 1098 | GUUAAGAGCCUGG GUGGGAAGU | SEQ ID NO: 546 |
| ETX-S00004170 | UmsGfsGmGmGmAfAmGmUmAmUmCmU mGfAmUfGmAfCmAmUmsUmsGm | SEQ ID NO: 1099 | UGGGGAAGUAUCU GAUGACAUUG | SEQ ID NO: 547 |
| ETX-S00004174 | GmsGfsUmGmGmGfGmAmAmGmUmAmU mCfUmGfAmUfGmAmCmsAmsUm | SEQ ID NO: 1100 | GGUGGGGAAGUAU CUGAUGACAU | SEQ ID NO: 548 |
| ETX-S00004178 | GmsUfsGmGmGmGfAmAmGmUmAmUmC mUfGmAfUmGfAmCmAmsUmsUm | SEQ ID NO: 1101 | GUGGGGAAGUAUC UGAUGACAUU | SEQ ID NO: 549 |
| ETX-S00004182 | GmsGfsGmGmAmAfGmUmAmUmCmUmG mAfUmGfAmCfAmUmUmsGmsGm | SEQ ID NO: 1102 | GGGGAAGUAUCUG AUGACAUUGG | SEQ ID NO: 550 |
| ETX-S00004186 | UmsUfsAmAmGmAfGmCmCmUmGmGmG mUfGmGfGmGfAmAmGmsUmsAm | SEQ ID NO: 1103 | UUAAGAGCCUGGG UGGGGAAGUA | SEQ ID NO: 551 |
| ETX-S00004190 | CmsCfsGmAmAmGfUmAmGmGmCmGmG mCfUmCfGmGfUmAmGmsAmsUm | SEQ ID NO: 1104 | CCGAAGUAGGCGG CUCGGUAGAU | SEQ ID NO: 552 |

In certain embodiments, the second strand comprises a nucleoside sequence of at least 17 contiguous nucleosides differing by 0 or 1 nucleosides from any one of SEQ ID NO: 1105-1380; wherein the second strand has a region of at least 85% complementarity over the 17 contiguous nucleosides to the first strand.

In certain embodiments, the second strand comprises a nucleoside sequence of at least 19 contiguous nucleosides differing by 0 or 1 nucleosides from any one of SEQ ID NO: 1105-1380; wherein the second strand has a region of at least 85% complementarity over the 19 contiguous nucleosides to the first strand.

In certain embodiments, the second strand comprises a nucleoside sequence of at least 21 contiguous nucleosides differing by 0 or 1 nucleosides from any one of SEQ ID NO: 1105-1380; wherein the second strand has a region of at least 85% complementarity over the 21 contiguous nucleosides to the first strand.

In certain embodiments, the second strand comprises any one of SEQ ID NO: 1105-1380.

The modification pattern of the nucleic acids as set forth in SEQ ID NO: 1105-1380 is summarized in Table 4 below:

**Table 4**

| Sense strand ID | Modified Sense Strand 5' → 3' | SEQ ID NO (SS - mod) | Underlying Base Sequence 5' → 3' (Shown as an Unmodified Nucleotide Sequence) | SEQ ID NO (SS - unmod) |
|---|---|---|---|---|
| ETX-S00003089 | iaiaUmsAmsUmUmUmAmCmUmGfGfUfU mGmAmAmAmAmUmGmGmGm | SEQ ID NO: 1105 | UAUUUACUGGUUG AAAAUGGG | SEQ ID NO: 553 |
| ETX-S00003093 | iaiaCmsAmsAmAmGmGmCmAmUfUfAfU mAmGmAmCmUmGmCmGmUm | SEQ ID NO: 1106 | CAAAGGCAUUAUA GACUGCGU | SEQ ID NO: 554 |
| ETX-S00003097 | iaiaUmsGmsAmUmGmAmAmAmUfCfAfA mGmAmAmGmUmAmCmAmCm | SEQ ID NO: 1107 | UGAUGAAAUCAAG AAGUACAC | SEQ ID NO: 555 |
| ETX-S00003101 | iaiaGmsAmsUmGmAmUmGmGfAfCfU mCmAmAmUmUmGmUmAmUm | SEQ ID NO: 1108 | GAUGAUGGACUC AAUUGUAU | SEQ ID NO: 556 |
| ETX-S00003105 | iaiaGmsCmsAmUmUmAmUmAmGfAfCfU mGmCmGmUmGmGmUmCmCm | SEQ ID NO: 1109 | GCAUUAUAGACUG CGUGGUCC | SEQ ID NO: 557 |
| ETX-S00003109 | iaiaGmsUmsCmAmCmUmCmCmUfGfAfUm AmAmAmUmAmAmCmAmAm | SEQ ID NO: 1110 | GUCACUCCUGAUA AAUAACAA | SEQ ID NO: 558 |
| ETX-S00003113 | iaiaAmsUmsAmCmAmAmAmGmGfCfAfU mUmAmUmAmGmAmCmUmGm | SEQ ID NO: 1111 | AUACAAAGGCAUU AUAGACUG | SEQ ID NO: 559 |
| ETX-S00003117 | iaiaGmsCmsCmGmCmCmUmAmCfUfUfCm GmGmUmAmUmCmUmAmUm | SEQ ID NO: 1112 | GCCGCCUACUUCG GUAUCUAU | SEQ ID NO: 560 |
| ETX-S00003121 | iaiaAmsGmsCmCmGmCmCmUmAfCfUfUm CmGmGmUmAmUmCmUmAm | SEQ ID NO: 1113 | AGCCGCCUACUUC GGUAUCUA | SEQ ID NO: 561 |
| ETX-S00003125 | iaiaAmsGmsAmCmUmGmCmGmUfGfGfU mCmCmGmUmAmUmUmCmCm | SEQ ID NO: 1114 | AGACUGCGUGGUC CGUAUUCC | SEQ ID NO: 562 |
| ETX-S00003129 | iaiaCmsAmsCmUmCmAmCmAmUfCfGfUm CmAmUmCmAmGmCmUmGm | SEQ ID NO: 1115 | CACUCACAUCGUC AUCAGCUG | SEQ ID NO: 563 |
| ETX-S00003133 | iaiaAmsCmsUmUmCmGmCmCmUfUfCfAm AmAmGmAmUmAmAmAmUm | SEQ ID NO: 1116 | ACUUCGCCUUCAA AGAUAAAU | SEQ ID NO: 564 |
| ETX-S00003137 | iaiaCmsUmsAmCmUmUmCmGmGfUfAfUm CmUmAmUmGmAmCmAmCm | SEQ ID NO: 1117 | CUACUUCGGUAUC UAUGACAC | SEQ ID NO: 565 |
| ETX-S00003141 | iaiaUmsUmsUmAmCmUmGmGmUfUfGfA mAmAmAmUmGmGmGmAmAm | SEQ ID NO: 1118 | UUUACUGGUUGAA AAUGGGAA | SEQ ID NO: 566 |
| ETX-S00003145 | iaiaUmsGmsCmCmCmGmUmAmCfCfCfGm UmCmUmAmGmCmAmGmCm | SEQ ID NO: 1119 | UGCCCGUACCCGU CUAGCAGC | SEQ ID NO: 567 |
| ETX-S00003149 | iaiaAmsGmsUmCmAmGmGmGmCfGfCfAm AmAmGmGmAmAmCmUmGm | SEQ ID NO: 1120 | AGUCAGGGCGCAA AGGAACUG | SEQ ID NO: 568 |
| ETX-S00003153 | iaiaUmsUmsAmCmUmGmGmUmUfGfAfA mAmAmUmGmGmGmAmAmGm | SEQ ID NO: 1121 | UUACUGGUUGAAA AUGGGAAG | SEQ ID NO: 569 |
| ETX-S00003157 | iaiaGmsAmsGmCmCmGmCmCmUfAfCfUm UmCmGmGmUmAmUmCmUm | SEQ ID NO: 1122 | GAGCCGCCUACUU CGGUAUCU | SEQ ID NO: 570 |
| ETX-S00003161 | iaiaCmsCmsUmCmGmGmUmGmAfCfUfGm CmCmUmGmGmUmUmAmAm | SEQ ID NO: 1123 | CCUCGGUGACUGC CUGGUUAA | SEQ ID NO: 571 |
| ETX-S00003165 | iaiaCmsAmsUmCmGmUmCmAmUfCfAfGm CmUmGmGmAmUmGmAmUm | SEQ ID NO: 1124 | CAUCGUCAUCAGC UGGAUGAU | SEQ ID NO: 572 |
| ETX-S00003169 | iaiaUmsUmsUmCmAmGmUmCmAfCfUfCm CmUmGmAmUmAmAmAmUm | SEQ ID NO: 1125 | UUUCAGUCACUCC UGAUAAAU | SEQ ID NO: 573 |
| ETX-S00003173 | iaiaUmsGmsGmAmUmGmAmUmCfGfCfA mCmAmGmAmCmUmGmUmCm | SEQ ID NO: 1126 | UGGAUGAUCGCAC AGACUGUC | SEQ ID NO: 574 |
| ETX-S00003177 | iaiaGmsCmsAmAmUmAmCmAmAfAfGfG mCmAmUmUmAmUmAmGmAm | SEQ ID NO: 1127 | GCAAUACAAAGGC AUUAUAGA | SEQ ID NO: 575 |
| ETX-S00003181 | iaiaGmsCmsCmUmCmGmGmUmGfAfCfUm GmCmCmUmGmGmUmUmAm | SEQ ID NO: 1128 | GCCUCGGUGACUG CCUGGUUA | SEQ ID NO: 576 |
| ETX-S00003185 | iaiaCmsUmsUmUmAmAmCmGmUfGfUfCm UmGmUmGmCmAmGmGmGm | SEQ ID NO: 1129 | CUUUAACGUGUCU GUGCAGGG | SEQ ID NO: 577 |
| ETX-S00003189 | iaiaAmsAmsAmGmGmCmAmUmUfAfUfA mGmAmCmUmGmCmGmUmGm | SEQ ID NO: 1130 | AAAGGCAUUAUAG ACUGCGUG | SEQ ID NO: 578 |
| ETX-S00003193 | iaiaGmsUmsGmAmAmCmAmGmGfCfAfU mGmUmUmGmUmAmUmUmAm | SEQ ID NO: 1131 | GUGAACAGGCAUG UUGUAUUA | SEQ ID NO: 579 |
| ETX-S00003197 | iaiaAmsUmsCmUmGmAmCmCmAfGfUfUm UmUmCmUmCmUmUmAmAm | SEQ ID NO: 1132 | AUCUGACCAGUUU UCUCUUAA | SEQ ID NO: 580 |
| ETX-S00003201 | iaiaAmsGmsGmCmUmUmUmAmAfCfGfU mGmUmCmUmGmUmGmCmAm | SEQ ID NO: 1133 | AGGCUUUAACGUG UCUGUGCA | SEQ ID NO: 581 |
| ETX-S00003205 | iaiaGmsCmsAmGmUmCmAmGmGfGfCfGm CmAmAmAmGmGmAmAmCm | SEQ ID NO: 1134 | GCAGUCAGGGCGC AAAGGAAC | SEQ ID NO: 582 |
| ETX-S00003209 | iaiaGmsAmsUmGmAmUmCmGmCfAfCfAm GmAmCmUmGmUmCmAmCm | SEQ ID NO: 1135 | GAUGAUCGCACAG ACUGUCAC | SEQ ID NO: 583 |
| ETX-S00003213 | iaiaAmsGmsGmCmAmUmUmAmUfAfGfA mCmUmGmCmGmUmGmGmUm | SEQ ID NO: 1136 | AGGCAUUAUAGAC UGCGUGGU | SEQ ID NO: 584 |
| ETX-S00003217 | iaiaUmsGmsAmUmCmGmCmAmCfAfGfAm CmUmGmUmCmAmCmUmGm | SEQ ID NO: 1137 | UGAUCGCACAGAC UGUCACUG | SEQ ID NO: 585 |
| ETX-S00003221 | iaiaCmsUmsGmCmGmUmGmGmUfCfCfGm UmAmUmUmCmCmCmAmAm | SEQ ID NO: 1138 | CUGCGUGGUCCGU AUUCCCAA | SEQ ID NO: 586 |
| ETX-S00003225 | iaiaCmsAmsAmUmAmCmAmAmAfGfGfCm AmUmUmAmUmAmGmAmCm | SEQ ID NO: 1139 | CAAUACAAAGGCA UUAUAGAC | SEQ ID NO: 587 |
| ETX-S00003229 | iaiaCmsAmsUmCmAmGmCmUmGfGfAfUm GmAmUmCmGmCmAmCmAm | SEQ ID NO: 1140 | CAUCAGCUGGAUG AUCGCACA | SEQ ID NO: 588 |
| ETX-S00003233 | iaiaAmsUmsUmAmUmAmGmAmCfUfGfC mGmUmGmGmUmCmCmGmUm | SEQ ID NO: 1141 | AUUAUAGACUGCG UGGUCCGU | SEQ ID NO: 589 |
| ETX-S00003237 | iaiaUmsUmsUmGmCmCmCmGmUfAfCfCm CmGmUmCmUmAmGmCmAm | SEQ ID NO: 1142 | UUUGCCCGUACCC GUCUAGCA | SEQ ID NO: 590 |
| ETX-S00003241 | iaiaAmsCmsAmUmCmGmUmCmAfUfCfAm GmCmUmGmGmAmUmGmAm | SEQ ID NO: 1143 | ACAUCGUCAUCAG CUGGAUGA | SEQ ID NO: 591 |
| ETX-S00003245 | iaiaUmsCmsUmUmGmAmUmUmUfUfGfC mCmCmGmUmAmCmCmCmGm | SEQ ID NO: 1144 | UCUUGAUUUUGCC CGUACCCG | SEQ ID NO: 592 |
| ETX-S00003249 | iaiaAmsUmsAmGmAmCmUmGmCfGfUfG mGmUmCmCmGmUmAmUmUm | SEQ ID NO: 1145 | AUAGACUGCGUGG UCCGUAUU | SEQ ID NO: 593 |
| ETX-S00003253 | iaiaCmsGmsCmCmUmAmCmUmUfCfGfGm UmAmUmCmUmAmUmGmAm | SEQ ID NO: 1146 | CGCCUACUUCGGU AUCUAUGA | SEQ ID NO: 594 |
| ETX-S00003257 | iaiaGmsAmsCmUmGmCmGmUmGfGfUfCm CmGmUmAmUmUmCmCmCm | SEQ ID NO: 1147 | GACUGCGUGGUCC GUAUUCCC | SEQ ID NO: 595 |
| ETX-S00003261 | iaiaUmsCmsAmUmCmAmGmCmUfGfGfAm UmGmAmUmCmGmCmAmCm | SEQ ID NO: 1148 | UCAUCAGCUGGAU GAUCGCAC | SEQ ID NO: 596 |
| ETX-S00003265 | iaiaUmsUmsAmUmAmGmAmCmUfGfCfG mUmGmGmUmCmCmGmUmAm | SEQ ID NO: 1149 | UUAUAGACUGCGU GGUCCGUA | SEQ ID NO: 597 |
| ETX-S00003269 | iaiaCmsCmsUmAmCmUmUmCmGfGfUfAm UmCmUmAmUmGmAmCmAm | SEQ ID NO: 1150 | CCUACUUCGGUAU CUAUGACA | SEQ ID NO: 598 |
| ETX-S00003273 | iaiaUmsUmsAmUmUmUmCmAmGfUfCfA mCmUmCmCmUmGmAmUmAm | SEQ ID NO: 1151 | UUAUUUCAGUCAC UCCUGAUA | SEQ ID NO: 599 |
| ETX-S00003277 | iaiaGmsUmsCmAmUmCmAmGmCfUfGfGm AmUmGmAmUmCmGmCmAm | SEQ ID NO: 1152 | GUCAUCAGCUGGA UGAUCGCA | SEQ ID NO: 600 |
| ETX-S00003281 | iaiaAmsUmsUmUmUmGmCmCmCfGfUfAm CmCmCmGmUmCmUmAmGm | SEQ ID NO: 1153 | AUUUUGCCCGUAC CCGUCUAG | SEQ ID NO: 601 |
| ETX-S00003285 | iaiaCmsAmsAmAmGmGmGmAmAfUfGfC mUmUmCmCmGmGmAmUmCm | SEQ ID NO: 1154 | CAAAGGAAUGCU UCCGGAUC | SEQ ID NO: 602 |
| ETX-S00003289 | iaiaAmsAmsGmGmCmUmUmUmAfAfCfG mUmGmUmCmUmGmUmGmCm | SEQ ID NO: 1155 | AAGGCUUUAACGU GUCUGUGC | SEQ ID NO: 603 |
| ETX-S00003293 | iaiaAmsAmsGmGmCmAmUmUmAfUfAfG mAmCmUmGmCmGmUmGmGm | SEQ ID NO: 1156 | AAGGCAUUAUAGA CUGCGUGG | SEQ ID NO: 604 |
| ETX-S00003297 | iaiaUmsUmsGmAmUmUmUmUmGfCfCfCm GmUmAmCmCmCmGmUmCm | SEQ ID NO: 1157 | UUGAUUUUGCCCG UACCCGUC | SEQ ID NO: 605 |
| ETX-S00003301 | iaiaCmsGmsAmGmCmCmGmCmCfUfAfCm UmUmCmGmGmUmAmUmCm | SEQ ID NO: 1158 | CGAGCCGCCUACU UCGGUAUC | SEQ ID NO: 606 |
| ETX-S00003305 | iaiaAmsUmsCmAmUmCmUmAmCfCfGfAm GmCmCmGmCmCmUmAmCm | SEQ ID NO: 1159 | AUCAUCUACCGAG CCGCCUAC | SEQ ID NO: 607 |
| ETX-S00003309 | iaiaUmsGmsAmUmUmUmUmGmCfCfCfGm UmAmCmCmCmGmUmCmUm | SEQ ID NO: 1160 | UGAUUUUGCCCGU ACCCGUCU | SEQ ID NO: 608 |
| ETX-S00003313 | iaiaUmsAmsUmCmAmUmCmUmAfCfCfGm AmGmCmCmGmCmCmUmAm | SEQ ID NO: 1161 | UAUCAUCUACCGA GCCGCCUA | SEQ ID NO: 609 |
| ETX-S00003317 | iaiaCmsAmsUmCmUmAmCmCmGfAfGfCm CmGmCmCmUmAmCmUmUm | SEQ ID NO: 1162 | CAUCUACCGAGCC GCCUACUU | SEQ ID NO: 610 |
| ETX-S00003321 | iaiaCmsUmsCmUmUmAmAmCmUfUfCfGm CmCmUmUmCmAmAmAmGm | SEQ ID NO: 1163 | CUCUUAACUUCGC CUUCAAAG | SEQ ID NO: 611 |
| ETX-S00003325 | iaiaCmsCmsAmCmCmCmAmGmGfCfUfCm UmUmAmAmCmUmUmCmGm | SEQ ID NO: 1164 | CCACCCAGGCUCU UAACUUCG | SEQ ID NO: 612 |
| ETX-S00003329 | iaiaCmsCmsGmUmAmUmUmCmCfCfAfAm GmGmAmGmCmAmGmGmGm | SEQ ID NO: 1165 | CCGUAUUCCCAAG GAGCAGGG | SEQ ID NO: 613 |
| ETX-S00003333 | iaiaGmsAmsGmCmAmGmGmGmAfGfUfU mCmUmGmUmCmCmUmUmCm | SEQ ID NO: 1166 | GAGCAGGGAGUUC UGUCCUUC | SEQ ID NO: 614 |
| ETX-S00003337 | iaiaUmsCmsCmCmAmAmGmGmAfGfCfAm GmGmGmAmGmUmUmCmUm | SEQ ID NO: 1167 | UCCCAAGGAGCAG GGAGUUCU | SEQ ID NO: 615 |
| ETX-S00003341 | iaiaUmsCmsCmGmUmAmUmUmCfCfCfAm AmGmGmAmGmCmAmGmGm | SEQ ID NO: 1168 | UCCGUAUUCCCAA GGAGCAGG | SEQ ID NO: 616 |
| ETX-S00003345 | iaiaGmsCmsAmGmGmGmAmGmUfUfCfU mGmUmCmCmUmUmCmUmGm | SEQ ID NO: 1169 | GCAGGGAGUUCUG UCCUUCUG | SEQ ID NO: 617 |
| ETX-S00003349 | iaiaGmsUmsCmCmGmUmAmUmUfCfCfCm AmAmGmGmAmGmCmAmGm | SEQ ID NO: 1170 | GUCCGUAUUCCCA AGGAGCAG | SEQ ID NO: 618 |
| ETX-S00003353 | iaiaAmsGmsCmAmGmGmGmAmGfUfUfC mUmGmUmCmCmUmUmCmUm | SEQ ID NO: 1171 | AGCAGGGAGUUCU GUCCUUCU | SEQ ID NO: 619 |
| ETX-S00003357 | iaiaGmsGmsAmGmCmAmGmGmGfAfGfU mUmCmUmGmUmCmCmUmUm | SEQ ID NO: 1172 | GGAGCAGGGAGUU CUGUCCUU | SEQ ID NO: 620 |
| ETX-S00003361 | iaiaUmsUmsCmCmCmAmAmGmGfAfGfCm AmGmGmGmAmGmUmUmCm | SEQ ID NO: 1173 | UUCCCAAGGAGCA GGGAGUUC | SEQ ID NO: 621 |
| ETX-S00003365 | iaiaAmsGmsGmAmGmCmAmGmGfGfAfG mUmUmCmUmGmUmCmCmUm | SEQ ID NO: 1174 | AGGAGCAGGGAGU UCUGUCCU | SEQ ID NO: 622 |
| ETX-S00003369 | iaiaCmsCmsCmAmCmCmCmAmGfGfCfUm CmUmUmAmAmCmUmUmCm | SEQ ID NO: 1175 | CCCACCCAGGCUC UUAACUUC | SEQ ID NO: 623 |
| ETX-S00003373 | iaiaUmsCmsUmUmAmAmCmUmUfCfGfCm CmUmUmCmAmAmAmGmAm | SEQ ID NO: 1176 | UCUUAACUUCGCC UUCAAAGA | SEQ ID NO: 624 |
| ETX-S00003377 | iaiaUmsUmsCmAmGmUmCmAmCfUfCfCm UmGmAmUmAmAmAmUmAm | SEQ ID NO: 1177 | UUCAGUCACUCCU GAUAAAUA | SEQ ID NO: 625 |
| ETX-S00003381 | iaiaCmsAmsGmUmCmAmCmUmCfCfUfGm AmUmAmAmAmUmAmAmCm | SEQ ID NO: 1178 | CAGUCACUCCUGA UAAAUAAC | SEQ ID NO: 626 |
| ETX-S00003385 | iaiaUmsCmsAmGmUmCmAmCmUfCfCfUm GmAmUmAmAmAmUmAmAm | SEQ ID NO: 1179 | UCAGUCACUCCUG AUAAAUAA | SEQ ID NO: 627 |
| ETX-S00003389 | iaiaUmsGmsAmUmGmGmGmAmCfUfCfA mAmUmUmGmUmAmUmUmUm | SEQ ID NO: 1180 | UGAUGGGACUCAA UUGUAUUU | SEQ ID NO: 628 |
| ETX-S00003393 | iaiaGmsAmsUmGmGmGmAmCmUfCfAfA mUmUmGmUmAmUmUmUmUm | SEQ ID NO: 1181 | GAUGGGACUCAAU UGUAUUUU | SEQ ID NO: 629 |
| ETX-S00003397 | iaiaCmsAmsCmUmCmCmUmGmAfUfAfAm AmUmAmAmCmAmAmAmUm | SEQ ID NO: 1182 | CACUCCUGAUAAA UAACAAAU | SEQ ID NO: 630 |
| ETX-S00003401 | iaiaAmsUmsUmCmAmUmCmUmGfAfCfCm AmGmUmUmUmUmCmUmCm | SEQ ID NO: 1183 | AUUCAUCUGACCA GUUUUCUC | SEQ ID NO: 631 |
| ETX-S00003405 | iaiaAmsAmsCmAmGmGmCmAmUfGfUfU mGmUmAmUmUmAmUmAmUm | SEQ ID NO: 1184 | AACAGGCAUGUUG UAUUAUAU | SEQ ID NO: 632 |
| ETX-S00003409 | iaiaAmsGmsGmGmAmAmUmGmCfUfUfC mCmGmGmAmUmCmCmCmAm | SEQ ID NO: 1185 | AGGGAAUGCUUCC GGAUCCCA | SEQ ID NO: 633 |
| ETX-S00003413 | iaiaGmsGmsGmAmAmUmGmCmUfUfCfCm GmGmAmUmCmCmCmAmAm | SEQ ID NO: 1186 | GGGAAUGCUUCCG GAUCCCAA | SEQ ID NO: 634 |
| ETX-S00003417 | iaiaCmsGmsAmGmGmCmCmUmCfGfGfUm GmAmCmUmGmCmCmUmGm | SEQ ID NO: 1187 | CGAGGCCUCGGUG ACUGCCUG | SEQ ID NO: 635 |
| ETX-S00003421 | iaiaAmsCmsAmGmGmCmAmUmGfUfUfG mUmAmUmUmAmUmAmUmAm | SEQ ID NO: 1188 | ACAGGCAUGUUGU AUUAUAUA | SEQ ID NO: 636 |
| ETX-S00003425 | iaiaAmsAmsUmAmCmAmAmAmGfGfCfA mUmUmAmUmAmGmAmCmUm | SEQ ID NO: 1189 | AAUACAAAGGCAU UAUAGACU | SEQ ID NO: 637 |
| ETX-S00003429 | iaiaAmsUmsUmUmCmAmGmUmCfAfCfUm CmCmUmGmAmUmAmAmAm | SEQ ID NO: 1190 | AUUUCAGUCACUC CUGAUAAA | SEQ ID NO: 638 |
| ETX-S00003433 | iaiaAmsUmsGmAmUmGmGmGmAfCfUfC mAmAmUmUmGmUmAmUmUm | SEQ ID NO: 1191 | AUGAUGGGACUCA AUUGUAUU | SEQ ID NO: 639 |
| ETX-S00003437 | iaiaCmsUmsGmAmCmCmAmGmUfUfUfUm CmUmCmUmUmAmAmAmGm | SEQ ID NO: 1192 | CUGACCAGUUUC UCUUAAAG | SEQ ID NO: 640 |
| ETX-S00003441 | iaiaUmsGmsAmCmCmAmGmUmUfUfUfCm UmCmUmUmAmAmAmGmCm | SEQ ID NO: 1193 | UGACCAGUUUCU CUUAAAGC | SEQ ID NO: 641 |
| ETX-S00003445 | iaiaGmsAmsUmAmAmGmCmAmAfUfAfC mAmAmAmGmGmCmAmUmUm | SEQ ID NO: 1194 | GAUAAGCAAUACA AAGGCAUU | SEQ ID NO: 642 |
| ETX-S00003449 | iaiaAmsUmsAmAmGmCmAmAmUfAfCfA mAmAmGmGmCmAmUmUmAm | SEQ ID NO: 1195 | AUAAGCAAUACAA AGGCAUUA | SEQ ID NO: 643 |
| ETX-S00003453 | iaiaUmsUmsAmAmCmGmUmGmUfCfUfG mUmGmCmAmGmGmGmUmAm | SEQ ID NO: 1196 | UUAACGUGUCUGU GCAGGUA | SEQ ID NO: 644 |
| ETX-S00003457 | iaiaUmsAmsAmCmGmUmGmUmCfUfGfU mGmCmAmGmGmGmUmAmUm | SEQ ID NO: 1197 | UAACGUGUCUGUG CAGGGUAU | SEQ ID NO: 645 |
| ETX-S00003461 | iaiaUmsCmsAmCmAmUmCmGmUfCfAfUm CmAmGmCmUmGmGmAmUm | SEQ ID NO: 1198 | UCACAUCGUCAUC AGCUGGAU | SEQ ID NO: 646 |
| ETX-S00003465 | iaiaUmsGmsAmUmGmAmUmGmGfGfAfC mUmCmAmAmUmUmGmUmAm | SEQ ID NO: 1199 | UGAUGAUGGACU CAAUUGUA | SEQ ID NO: 647 |
| ETX-S00003469 | iaiaAmsCmsUmGmCmGmUmGmGfUfCfCm GmUmAmUmUmCmCmCmAm | SEQ ID NO: 1200 | ACUGCGUGGUCCG UAUUCCCA | SEQ ID NO: 648 |
| ETX-S00003473 | iaiaGmsAmsAmCmAmGmGmCmAfUfGfU mUmGmUmAmUmUmAmUmAm | SEQ ID NO: 1201 | GAACAGGCAUGUU GUAUUAUA | SEQ ID NO: 649 |
| ETX-S00003477 | iaiaUmsAmsUmUmUmCmAmGmUfCfAfCm UmCmCmUmGmAmUmAmAm | SEQ ID NO: 1202 | UAUUUCAGUCACU CCUGAUAA | SEQ ID NO: 650 |
| ETX-S00003481 | iaiaUmsAmsAmGmCmAmAmUmAfCfAfA mAmGmGmCmAmUmUmAmUm | SEQ ID NO: 1203 | UAAGCAAUACAAA GGCAUUAU | SEQ ID NO: 651 |
| ETX-S00003485 | iaiaAmsCmsUmCmAmCmAmUmCfGfUfCm AmUmCmAmGmCmUmGmGm | SEQ ID NO: 1204 | ACUCACAUCGUCA UCAGCUGG | SEQ ID NO: 652 |
| ETX-S00003489 | iaiaCmsCmsGmCmCmUmAmCmUfUfCfGm GmUmAmUmCmUmAmUmGm | SEQ ID NO: 1205 | CCGCCUACUUCGG UAUCUAUG | SEQ ID NO: 653 |
| ETX-S00003493 | iaiaCmsAmsCmAmUmCmGmUmCfAfUfCm AmGmCmUmGmGmAmUmGm | SEQ ID NO: 1206 | CACAUCGUCAUCA GCUGGAUG | SEQ ID NO: 654 |
| ETX-S00003497 | iaiaGmsCmsCmUmAmCmUmUmCfGfGfUm AmUmCmUmAmUmGmAmCm | SEQ ID NO: 1207 | GCCUACUUCGGUA UCUAUGAC | SEQ ID NO: 655 |
| ETX-S00003501 | iaiaUmsUmsUmAmAmCmGmUmGfUfCfU mGmUmGmCmAmGmGmGmUm | SEQ ID NO: 1208 | UUUAACGUGUCUG UGCAGGGU | SEQ ID NO: 656 |
| ETX-S00003505 | iaiaUmsCmsGmCmCmGmCmCmGfCfAfUm GmAmUmGmAmUmGmCmAm | SEQ ID NO: 1209 | UCGCCGCCGCAUG AUGAUGCA | SEQ ID NO: 657 |
| ETX-S00003509 | iaiaCmsAmsUmUmAmUmAmGmAfCfUfG mCmGmUmGmGmUmCmCmGm | SEQ ID NO: 1210 | CAUUAUAGACUGC GUGGUCCG | SEQ ID NO: 658 |
| ETX-S00003513 | iaiaUmsGmsCmGmUmGmGmUmCfCfGfUm AmUmUmCmCmCmAmAmGm | SEQ ID NO: 1211 | UGCGUGGUCCGUA UUCCCAAG | SEQ ID NO: 659 |
| ETX-S00003517 | iaiaAmsUmsCmUmAmCmCmGmAfGfCfCm GmCmCmUmAmCmUmUmCm | SEQ ID NO: 1212 | AUCUACCGAGCCG CCUACUUC | SEQ ID NO: 660 |
| ETX-S00003521 | iaiaUmsUmsGmCmCmCmGmUmAfCfCfCm GmUmCmUmAmGmCmAmGm | SEQ ID NO: 1213 | UUGCCCGUACCCG UCUAGCAG | SEQ ID NO: 661 |
| ETX-S00003525 | iaiaCmsUmsCmAmCmAmUmCmGfUfCfAm UmCmAmGmCmUmGmGmAm | SEQ ID NO: 1214 | CUCACAUCGUCAU CAGCUGGA | SEQ ID NO: 662 |
| ETX-S00003529 | iaiaUmsAmsUmAmGmAmCmUmGfCfGfU mGmGmUmCmCmGmUmAmUm | SEQ ID NO: 1215 | UAUAGACUGCGUG GUCCGUAU | SEQ ID NO: 663 |
| ETX-S00003533 | iaiaGmsGmsCmCmUmCmGmGmUfGfAfCm UmGmCmCmUmGmGmUmUm | SEQ ID NO: 1216 | GGCCUCGGUGACU GCCUGGUU | SEQ ID NO: 664 |
| ETX-S00003537 | iaiaGmsGmsAmUmGmAmUmCmGfCfAfCm AmGmAmCmUmGmUmCmAm | SEQ ID NO: 1217 | GGAUGAUCGCACA GACUGUCA | SEQ ID NO: 665 |
| ETX-S00003541 | iaiaGmsAmsUmUmUmUmGmCmCfCfGfUm AmCmCmCmGmUmCmUmAm | SEQ ID NO: 1218 | GAUUUUGCCCGUA CCCGUCUA | SEQ ID NO: 666 |
| ETX-S00003545 | iaiaGmsAmsUmCmGmCmAmCmAfGfAfCm UmGmUmCmAmCmUmGmCm | SEQ ID NO: 1219 | GAUCGCACAGACU GUCACUGC | SEQ ID NO: 667 |
| ETX-S00003549 | iaiaAmsUmsGmAmUmCmGmCmAfCfAfGm AmCmUmGmUmCmAmCmUm | SEQ ID NO: 1220 | AUGAUCGCACAGA CUGUCACU | SEQ ID NO: 668 |
| ETX-S00003553 | iaiaUmsUmsUmUmGmCmCmCmGfUfAfCm CmCmGmUmCmUmAmGmCm | SEQ ID NO: 1221 | UUUUGCCCGUACC CGUCUAGC | SEQ ID NO: 669 |
| ETX-S00003557 | iaiaUmsCmsAmUmCmUmAmCmCfGfAfGm CmCmGmCmCmUmAmCmUm | SEQ ID NO: 1222 | UCAUCUACCGAGC CGCCUACU | SEQ ID NO: 670 |
| ETX-S00003561 | iaiaUmsAmsGmAmCmUmGmCmGfUfGfG mUmCmCmGmUmAmUmUmCm | SEQ ID NO: 1223 | UAGACUGCGUGGU CCGUAUUC | SEQ ID NO: 671 |
| ETX-S00003565 | iaiaCmsUmsGmCmAmGmAmUmAfAfGfCm AmAmUmAmCmAmAmAmGm | SEQ ID NO: 1224 | CUGCAGAUAAGCA AUACAAAG | SEQ ID NO: 672 |
| ETX-S00003569 | iaiaAmsCmsUmGmCmAmGmAmUfAfAfG mCmAmAmUmAmCmAmAmAm | SEQ ID NO: 1225 | ACUGCAGAUAAGC AAUACAAA | SEQ ID NO: 673 |
| ETX-S00003573 | iaiaCmsAmsCmUmGmCmAmGmAfUfAfAm GmCmAmAmUmAmCmAmAm | SEQ ID NO: 1226 | CACUGCAGAUAAG CAAUACAA | SEQ ID NO: 674 |
| ETX-S00003577 | iaiaGmsCmsAmAmGmCmAmGmAfUfCfAm CmUmGmCmAmGmAmUmAm | SEQ ID NO: 1227 | GCAAGCAGAUCAC UGCAGAUA | SEQ ID NO: 675 |
| ETX-S00003581 | iaiaAmsUmsCmAmCmUmGmCmAfGfAfUm AmAmGmCmAmAmUmAmCm | SEQ ID NO: 1228 | AUCACUGCAGAUA AGCAAUAC | SEQ ID NO: 676 |
| ETX-S00003585 | iaiaUmsCmsAmCmUmGmCmAmGfAfUfAm AmGmCmAmAmUmAmCmAm | SEQ ID NO: 1229 | UCACUGCAGAUAA GCAAUACA | SEQ ID NO: 677 |
| ETX-S00003589 | iaiaGmsCmsAmGmAmUmCmAmCfUfGfCm AmGmAmUmAmAmGmCmAm | SEQ ID NO: 1230 | GCAGAUCACUGCA GAUAAGCA | SEQ ID NO: 678 |
| ETX-S00003593 | iaiaCmsAmsGmAmUmCmAmCmUfGfCfAm GmAmUmAmAmGmCmAmAm | SEQ ID NO: 1231 | CAGAUCACUGCAG AUAAGCAA | SEQ ID NO: 679 |
| ETX-S00003597 | iaiaAmsGmsCmAmAmGmCmAmGfAfUfCm AmCmUmGmCmAmGmAmUm | SEQ ID NO: 1232 | AGCAAGCAGAUCA CUGCAGAU | SEQ ID NO: 680 |
| ETX-S00003601 | iaiaGmsAmsUmCmAmCmUmGmCfAfGfAm UmAmAmGmCmAmAmUmAm | SEQ ID NO: 1233 | GAUCACUGCAGAU AAGCAAUA | SEQ ID NO: 681 |
| ETX-S00003605 | iaiaAmsUmsAmAmAmUmAmAmCfAfAfA mUmUmUmGmGmAmGmAmAm | SEQ ID NO: 1234 | AUAAAUAACAAAU UUGGAGAA | SEQ ID NO: 682 |
| ETX-S00003609 | iaiaUmsCmsCmUmGmAmUmAmAfAfUfA mAmCmAmAmAmUmUmUmGm | SEQ ID NO: 1235 | UCCUGAUAAAUAA CAAAUUUG | SEQ ID NO: 683 |
| ETX-S00003613 | iaiaGmsAmsAmAmGmGmGmAmAfUfUfC mCmGmAmGmGmCmCmUmCm | SEQ ID NO: 1236 | GAAAGGAAUUCC GAGGCCUC | SEQ ID NO: 684 |
| ETX-S00003617 | iaiaGmsUmsUmGmUmAmUmUmAfUfAfU mAmAmCmAmUmAmUmCmUm | SEQ ID NO: 1237 | GUUGUAUUAUAUA ACAUAUCU | SEQ ID NO: 685 |
| ETX-S00003621 | iaiaUmsUmsCmCmGmAmGmGmCfCfUfCm GmGmUmGmAmCmUmGmCm | SEQ ID NO: 1238 | UUCCGAGGCCUCG GUGACUGC | SEQ ID NO: 686 |
| ETX-S00003625 | iaiaAmsAmsAmGmGmGmAmAmUfUfCfC mGmAmGmGmCmCmUmCmGm | SEQ ID NO: 1239 | AAAGGGAAUUCCG AGGCCUCG | SEQ ID NO: 687 |
| ETX-S00003629 | iaiaUmsGmsUmAmUmUmAmUmAfUfAfA mCmAmUmAmUmCmUmUmGm | SEQ ID NO: 1240 | UGUAUUAUAUAAC AUAUCUUG | SEQ ID NO: 688 |
| ETX-S00003633 | iaiaAmsAmsUmUmCmCmGmAmGfGfCfCm UmCmGmGmUmGmAmCmUm | SEQ ID NO: 1241 | AAUUCCGAGGCCU CGGUGACU | SEQ ID NO: 689 |
| ETX-S00003637 | iaiaGmsGmsGmAmAmUmUmCmCfGfAfG mGmCmCmUmCmGmGmUmGm | SEQ ID NO: 1242 | GGGAAUUCCGAGG CCUCGGUG | SEQ ID NO: 690 |
| ETX-S00003641 | iaiaAmsGmsCmUmGmAmAmAmGfGfGfA mAmUmUmCmCmGmAmGmGm | SEQ ID NO: 1243 | AGCUGAAAGGAA UUCCGAGG | SEQ ID NO: 691 |
| ETX-S00003645 | iaiaGmsGmsAmGmCmUmGmAmAfAfGfG mGmAmAmUmUmCmCmGmAm | SEQ ID NO: 1244 | GGAGCUGAAAGGG AAUUCCGA | SEQ ID NO: 692 |
| ETX-S00003649 | iaiaCmsUmsGmAmAmAmGmGmGfAfAfU mUmCmCmGmAmGmGmCmCm | SEQ ID NO: 1245 | CUGAAAGGGAAUU CCGAGGCC | SEQ ID NO: 693 |
| ETX-S00003653 | iaiaGmsAmsGmCmUmGmAmAmAfGfGfG mAmAmUmUmCmCmGmAmGm | SEQ ID NO: 1246 | GAGCUGAAAGGGA AUUCCGAG | SEQ ID NO: 694 |
| ETX-S00003657 | iaiaGmsCmsUmGmAmAmAmGmGfGfAfA mUmUmCmCmGmAmGmGmCm | SEQ ID NO: 1247 | GCUGAAAGGAAU UCCGAGGC | SEQ ID NO: 695 |
| ETX-S00003661 | iaiaGmsCmsAmUmGmUmUmGmUfAfUfU mAmUmAmUmAmAmCmAmUm | SEQ ID NO: 1248 | GCAUGUUGUAUUA UAUAACAU | SEQ ID NO: 696 |
| ETX-S00003665 | iaiaUmsUmsGmUmAmUmUmAmUfAfUfA mAmCmAmUmAmUmCmUmUm | SEQ ID NO: 1249 | UUGUAUUAUAUAA CAUAUCUU | SEQ ID NO: 697 |
| ETX-S00003669 | iaiaGmsUmsAmUmUmAmUmAmUfAfAfC mAmUmAmUmCmUmUmGmAm | SEQ ID NO: 1250 | GUAUUAUAUAACA UAUCUUGA | SEQ ID NO: 698 |
| ETX-S00003673 | iaiaGmsGmsCmAmUmGmUmUmGfUfAfU mUmAmUmAmUmAmAmCmAm | SEQ ID NO: 1251 | GGCAUGUUGUAUU AUAUAACA | SEQ ID NO: 699 |
| ETX-S00003677 | iaiaAmsAmsGmGmGmAmAmUmUfCfCfG mAmGmGmCmCmUmCmGmGm | SEQ ID NO: 1252 | AAGGGAAUUCCGA GGCCUCGG | SEQ ID NO: 700 |
| ETX-S00003681 | iaiaGmsAmsAmUmUmCmCmGmAfGfGfCm CmUmCmGmGmUmGmAmCm | SEQ ID NO: 1253 | GAAUUCCGAGGCC UCGGUGAC | SEQ ID NO: 701 |
| ETX-S00003685 | iaiaAmsGmsGmGmAmAmUmUmCfCfGfA mGmGmCmCmUmCmGmGmUm | SEQ ID NO: 1254 | AGGGAAUUCCGAG GCCUCGGU | SEQ ID NO: 702 |
| ETX-S00003689 | iaiaGmsGmsAmAmUmUmCmCmGfAfGfG mCmCmUmCmGmGmUmGmAm | SEQ ID NO: 1255 | GGAAUUCCGAGGC CUCGGUGA | SEQ ID NO: 703 |
| ETX-S00003693 | iaiaGmsCmsUmUmGmUmCmUmUfGfUfA mUmGmAmUmGmAmAmAmUm | SEQ ID NO: 1256 | GCUUGUCUUGUAU GAUGAAAU | SEQ ID NO: 704 |
| ETX-S00003697 | iaiaUmsCmsGmCmCmUmUmCmAfAfAfGm AmUmAmAmAmUmAmCmAm | SEQ ID NO: 1257 | UCGCCUUCAAAGA UAAAUACA | SEQ ID NO: 705 |
| ETX-S00003701 | iaiaGmsCmsCmUmGmGmUmUmAfAfGfA mUmCmUmAmCmAmAmAmUm | SEQ ID NO: 1258 | GCCUGGUUAAGAU CUACAAAU | SEQ ID NO: 706 |
| ETX-S00003705 | iaiaUmsCmsUmAmUmGmAmCmAfCfUfGm CmAmAmAmGmGmGmAmAm | SEQ ID NO: 1259 | UCUAUGACACUGC AAAGGAA | SEQ ID NO: 707 |
| ETX-S00003709 | iaiaUmsUmsUmGmUmGmCmUmUfGfUfC mUmUmGmUmAmUmGmAmUm | SEQ ID NO: 1260 | UUUGUGCUUGUCU UGUAUGAU | SEQ ID NO: 708 |
| ETX-S00003713 | iaiaUmsGmsAmCmUmGmCmCmUfGfGfUm UmAmAmGmAmUmCmUmAm | SEQ ID NO: 1261 | UGACUGCCUGGUU AAGAUCUA | SEQ ID NO: 709 |
| ETX-S00003717 | iaiaGmsAmsCmAmCmUmGmCmAfAfAfGm GmGmAmAmUmGmCmUmUm | SEQ ID NO: 1262 | GACACUGCAAAGG GAAUGCUU | SEQ ID NO: 710 |
| ETX-S00003721 | iaiaCmsGmsGmUmAmUmCmUmAfUfGfA mCmAmCmUmGmCmAmAmAm | SEQ ID NO: 1263 | CGGUAUCUAUGAC ACUGCAAA | SEQ ID NO: 711 |
| ETX-S00003725 | iaiaUmsUmsCmAmUmCmUmGmAfCfCfAm GmUmUmUmUmCmUmCmUm | SEQ ID NO: 1264 | UUCAUCUGACCAG UUUUCUCU | SEQ ID NO: 712 |
| ETX-S00003729 | iaiaUmsUmsCmGmGmUmAmUmCfUfAfU mGmAmCmAmCmUmGmCmAm | SEQ ID NO: 1265 | UUCGGUAUCUAUG ACACUGCA | SEQ ID NO: 713 |
| ETX-S00003733 | iaiaGmsGmsUmUmAmAmGmAmUfCfUfA mCmAmAmAmUmCmUmGmAm | SEQ ID NO: 1266 | GGUUAAGAUCUAC AAAUCUGA | SEQ ID NO: 714 |
| ETX-S00003737 | iaiaAmsUmsCmUmAmUmGmAmCfAfCfUm GmCmAmAmAmGmGmGmAm | SEQ ID NO: 1267 | AUCUAUGACACUG CAAAGGA | SEQ ID NO: 715 |
| ETX-S00003741 | iaiaAmsGmsGmGmUmGmCmAmUfGfGfU mCmCmAmAmUmGmUmUmCm | SEQ ID NO: 1268 | AGGGUGCAUGGUC CAAUGUUC | SEQ ID NO: 716 |
| ETX-S00003745 | iaiaAmsUmsCmUmAmCmAmAmAfUfCfUm GmAmUmGmGmGmAmUmUm | SEQ ID NO: 1269 | AUCUACAAAUCUG AUGGGAUU | SEQ ID NO: 717 |
| ETX-S00003749 | iaiaCmsUmsUmCmGmGmUmAmUfCfUfAm UmGmAmCmAmCmUmGmCm | SEQ ID NO: 1270 | CUUCGGUAUCUAU GACACUGC | SEQ ID NO: 718 |
| ETX-S00003753 | iaiaUmsGmsGmGmUmGmGmUmGfCfUfU mUmUmGmUmGmCmUmUmGm | SEQ ID NO: 1271 | UGGGUGGUGCUUU UGUGCUUG | SEQ ID NO: 719 |
| ETX-S00003757 | iaiaUmsAmsUmUmAmUmCmAmUfCfUfA mCmCmGmAmGmCmCmGmCm | SEQ ID NO: 1272 | UAUUAUCAUCUAC CGAGCCGC | SEQ ID NO: 720 |
| ETX-S00003761 | iaiaGmsUmsGmGmUmCmCmGmUfAfUfU mCmCmCmAmAmGmGmAmGm | SEQ ID NO: 1273 | GUGGUCCGUAUUC CCAAGGAG | SEQ ID NO: 721 |
| ETX-S00003765 | iaiaCmsUmsAmUmGmAmCmAmCfUfGfCm AmAmAmGmGmGmAmAmUm | SEQ ID NO: 1274 | CUAUGACACUGCA AAGGGAAU | SEQ ID NO: 722 |
| ETX-S00003769 | iaiaCmsUmsUmUmUmGmUmGmCfUfUfG mUmCmUmUmGmUmAmUmGm | SEQ ID NO: 1275 | CUUUUGUGCUUGU CUUGUAUG | SEQ ID NO: 723 |
| ETX-S00003773 | iaiaUmsAmsCmCmAmAmGmGmCfUfUfUm AmAmCmGmUmGmUmCmUm | SEQ ID NO: 1276 | UACCAAGGCUUUA ACGUGUCU | SEQ ID NO: 724 |
| ETX-S00003777 | iaiaGmsUmsGmCmAmUmGmGmUfCfCfAm AmUmGmUmUmCmUmCmAm | SEQ ID NO: 1277 | GUGCAUGGUCCAA UGUUCUCA | SEQ ID NO: 725 |
| ETX-S00003781 | iaiaCmsCmsUmUmCmAmAmAmGfAfUfAm AmAmUmAmCmAmAmGmCm | SEQ ID NO: 1278 | CCUUCAAAGAUAA AUACAAGC | SEQ ID NO: 726 |
| ETX-S00003785 | iaiaGmsGmsGmUmAmUmUmAmUfCfAfU mCmUmAmCmCmGmAmGmCm | SEQ ID NO: 1279 | GGGUAUUAUCAUC UACCGAGC | SEQ ID NO: 727 |
| ETX-S00003789 | iaiaUmsAmsCmUmUmCmGmGmUfAfUfCm UmAmUmGmAmCmAmCmUm | SEQ ID NO: 1280 | UACUUCGGUAUCU AUGACACU | SEQ ID NO: 728 |
| ETX-S00003793 | iaiaGmsUmsUmAmAmGmAmUmCfUfAfC mAmAmAmUmCmUmGmAmUm | SEQ ID NO: 1281 | GUUAAGAUCUACA AAUCUGAU | SEQ ID NO: 729 |
| ETX-S00003797 | iaiaUmsGmsCmAmGmGmGmUmAfUfUfA mUmCmAmUmCmUmAmCmCm | SEQ ID NO: 1282 | UGCAGGGUAUUAU CAUCUACC | SEQ ID NO: 730 |
| ETX-S00003801 | iaiaUmsCmsUmGmAmUmGmGmGfAfUfU mAmAmGmGmGmCmCmUmGm | SEQ ID NO: 1283 | UCUGAUGGGAUUA AGGGCCUG | SEQ ID NO: 731 |
| ETX-S00003805 | iaiaCmsUmsGmUmGmCmAmGmGfGfUfA mUmUmAmUmCmAmUmCmUm | SEQ ID NO: 1284 | CUGUGCAGGGUAU UAUCAUCU | SEQ ID NO: 732 |
| ETX-S00003809 | iaiaUmsGmsAmUmGmGmGmAmUfUfAfA mGmGmGmCmCmUmGmUmAm | SEQ ID NO: 1285 | UGAUGGGAUUAAG GGCCUGUA | SEQ ID NO: 733 |
| ETX-S00003813 | iaiaCmsCmsAmAmGmGmCmUmUfUfAfAm CmGmUmGmUmCmUmGmUm | SEQ ID NO: 1286 | CCAAGGCUUUAAC GUGUCUGU | SEQ ID NO: 734 |
| ETX-S00003817 | iaiaUmsAmsUmUmCmAmUmCmUfGfAfCm CmAmGmUmUmUmUmCmUm | SEQ ID NO: 1287 | UAUUCAUCUGACC AGUUUUCU | SEQ ID NO: 735 |
| ETX-S00003821 | iaiaAmsCmsUmGmCmCmUmGmGfUfUfAm AmGmAmUmCmUmAmCmAm | SEQ ID NO: 1288 | ACUGCCUGGUUAA GAUCUACA | SEQ ID NO: 736 |
| ETX-S00003825 | iaiaGmsUmsUmGmAmCmUmUmCfCfUfAm UmCmCmAmUmUmUmGmAm | SEQ ID NO: 1289 | GUUGACUUCCUAU CCAUUUGA | SEQ ID NO: 737 |
| ETX-S00003829 | iaiaUmsAmsUmGmAmCmAmCmUfGfCfAm AmAmGmGmGmAmAmUmGm | SEQ ID NO: 1290 | UAUGACACUGCAA AGGGAAUG | SEQ ID NO: 738 |
| ETX-S00003833 | iaiaUmsUmsAmAmGmAmUmCmUfAfCfA mAmAmUmCmUmGmAmUmGm | SEQ ID NO: 1291 | UUAAGAUCUACAA AUCUGAUG | SEQ ID NO: 739 |
| ETX-S00003837 | iaiaAmsGmsGmGmUmAmUmUmAfUfCfA mUmCmUmAmCmCmGmAmGm | SEQ ID NO: 1292 | AGGGUAUUAUCAU CUACCGAG | SEQ ID NO: 740 |
| ETX-S00003841 | iaiaUmsGmsCmCmUmGmGmUmUfAfAfG mAmUmCmUmAmCmAmAmAm | SEQ ID NO: 1293 | UGCCUGGUUAAGA UCUACAAA | SEQ ID NO: 741 |
| ETX-S00003845 | iaiaCmsUmsGmAmUmGmGmGmAfUfUfA mAmGmGmGmCmCmUmGmUm | SEQ ID NO: 1294 | CUGAUGGAUUAA GGGCCUGU | SEQ ID NO: 742 |
| ETX-S00003849 | iaiaGmsGmsUmAmUmCmUmAmUfGfAfC mAmCmUmGmCmAmAmAmGm | SEQ ID NO: 1295 | GGUAUCUAUGACA CUGCAAAG | SEQ ID NO: 743 |
| ETX-S00003853 | iaiaAmsCmsCmCmGmUmCmUmAfGfCfAm GmCmUmGmAmUmGmUmGm | SEQ ID NO: 1296 | ACCCGUCUAGCAG CUGAUGUG | SEQ ID NO: 744 |
| ETX-S00003857 | iaiaUmsAmsUmCmUmAmUmGmAfCfAfCm UmGmCmAmAmAmGmGmGm | SEQ ID NO: 1297 | UAUCUAUGACACU GCAAAGGG | SEQ ID NO: 745 |
| ETX-S00003861 | iaiaCmsUmsGmAmUmGmUmGmGfGfUfA mAmAmGmCmUmGmGmAmGm | SEQ ID NO: 1298 | CUGAUGUGGGUAA AGCUGGAG | SEQ ID NO: 746 |
| ETX-S00003865 | iaiaUmsCmsUmAmGmCmAmGmCfUfGfAm UmGmUmGmGmGmUmAmAm | SEQ ID NO: 1299 | UCUAGCAGCUGAU GUGGGUAA | SEQ ID NO: 747 |
| ETX-S00003869 | iaiaGmsGmsGmCmCmUmGmUmAfCfCfAm AmGmGmCmUmUmUmAmAm | SEQ ID NO: 1300 | GGGCCUGUACCAA GGCUUUAA | SEQ ID NO: 748 |
| ETX-S00003873 | iaiaCmsCmsCmGmUmAmCmCmCfGfUfCm UmAmGmCmAmGmCmUmGm | SEQ ID NO: 1301 | CCCGUACCCGUCU AGCAGCUG | SEQ ID NO: 749 |
| ETX-S00003877 | iaiaCmsAmsGmCmUmGmAmUmGfUfGfG mGmUmAmAmAmGmCmUmGm | SEQ ID NO: 1302 | CAGCUGAUGUGGG UAAAGCUG | SEQ ID NO: 750 |
| ETX-S00003881 | iaiaCmsCmsGmUmAmCmCmCmGfUfCfUm AmGmCmAmGmCmUmGmAm | SEQ ID NO: 1303 | CCGUACCCGUCUA GCAGCUGA | SEQ ID NO: 751 |
| ETX-S00003885 | iaiaGmsCmsUmGmAmUmGmUmGfGfGfU mAmAmAmGmCmUmGmGmAm | SEQ ID NO: 1304 | GCUGAUGUGGGUA AAGCUGGA | SEQ ID NO: 752 |
| ETX-S00003889 | iaiaGmsGmsUmAmUmUmAmUmCfAfUfC mUmAmCmCmGmAmGmCmCm | SEQ ID NO: 1305 | GGUAUUAUCAUCU ACCGAGCC | SEQ ID NO: 753 |
| ETX-S00003893 | iaiaUmsUmsAmUmCmAmUmCmUfAfCfCm GmAmGmCmCmGmCmCmUm | SEQ ID NO: 1306 | UUAUCAUCUACCG AGCCGCCU | SEQ ID NO: 754 |
| ETX-S00003897 | iaiaGmsUmsAmCmCmCmGmUmCfUfAfGm CmAmGmCmUmGmAmUmGm | SEQ ID NO: 1307 | GUACCCGUCUAGC AGCUGAUG | SEQ ID NO: 755 |
| ETX-S00003901 | iaiaGmsAmsUmGmGmGmAmUmUfAfAfG mGmGmCmCmUmGmUmAmCm | SEQ ID NO: 1308 | GAUGGGAUUAAGG GCCUGUAC | SEQ ID NO: 756 |
| ETX-S00003905 | iaiaGmsUmsCmUmGmUmGmCmAfGfGfG mUmAmUmUmAmUmCmAmUm | SEQ ID NO: 1309 | GUCUGUGCAGGGU AUUAUCAU | SEQ ID NO: 757 |
| ETX-S00003909 | iaiaGmsCmsGmUmGmGmUmCmCfGfUfAm UmUmCmCmCmAmAmGmGm | SEQ ID NO: 1310 | GCGUGGUCCGUAU UCCCAAGG | SEQ ID NO: 758 |
| ETX-S00003913 | iaiaCmsGmsUmGmGmUmCmCmGfUfAfUm UmCmCmCmAmAmGmGmAm | SEQ ID NO: 1311 | CGUGGUCCGUAUU CCCAAGGA | SEQ ID NO: 759 |
| ETX-S00003917 | iaiaUmsGmsGmUmUmAmAmGmAfUfCfU mAmCmAmAmAmUmCmUmGm | SEQ ID NO: 1312 | UGGUUAAGAUCUA CAAAUCUG | SEQ ID NO: 760 |
| ETX-S00003921 | iaiaAmsUmsGmAmUmGmAmAmAfUfCfA mAmGmAmAmGmUmAmCmAm | SEQ ID NO: 1313 | AUGAUGAAAUCAA GAAGUACA | SEQ ID NO: 761 |
| ETX-S00003925 | iaiaGmsAmsUmCmUmAmCmAmAfAfUfCm UmGmAmUmGmGmGmAmUm | SEQ ID NO: 1314 | GAUCUACAAAUCU GAUGGGAU | SEQ ID NO: 762 |
| ETX-S00003929 | iaiaGmsUmsGmCmAmGmGmGmUfAfUfU mAmUmCmAmUmCmUmAmCm | SEQ ID NO: 1315 | GUGCAGGGUAUUA UCAUCUAC | SEQ ID NO: 763 |
| ETX-S00003933 | iaiaGmsUmsAmCmCmAmAmGmGfCfUfUm UmAmAmCmGmUmGmUmCm | SEQ ID NO: 1316 | GUACCAAGGCUUU AACGUGUC | SEQ ID NO: 764 |
| ETX-S00003937 | iaiaGmsCmsAmGmCmUmGmAmUfGfUfG mGmGmUmAmAmAmGmCmUm | SEQ ID NO: 1317 | GCAGCUGAUGUGG GUAAAGCU | SEQ ID NO: 765 |
| ETX-S00003941 | iaiaAmsUmsGmGmGmUmGmGmUfGfCfU mUmUmUmGmUmGmCmUmUm | SEQ ID NO: 1318 | AUGGGUGGUGCUU UUGUGCUU | SEQ ID NO: 766 |
| ETX-S00003945 | iaiaCmsGmsGmUmGmAmCmUmGfCfCfUm GmGmUmUmAmAmGmAmUm | SEQ ID NO: 1319 | CGGUGACUGCCUG GUUAAGAU | SEQ ID NO: 767 |
| ETX-S00003949 | iaiaCmsUmsAmGmCmAmGmCmUfGfAfUm GmUmGmGmGmUmAmAmAm | SEQ ID NO: 1320 | CUAGCAGCUGAUG UGGGUAAA | SEQ ID NO: 768 |
| ETX-S00003953 | iaiaUmsAmsCmAmAmAmUmCmUfGfAfU mGmGmGmAmUmUmAmAmGm | SEQ ID NO: 1321 | UACAAAUCUGAUG GGAUUAAG | SEQ ID NO: 769 |
| ETX-S00003957 | iaiaAmsCmsUmUmCmGmGmUmAfUfCfUm AmUmGmAmCmAmCmUmGm | SEQ ID NO: 1322 | ACUUCGGUAUCUA UGACACUG | SEQ ID NO: 770 |
| ETX-S00003961 | iaiaAmsGmsCmUmGmAmUmGmUfGfGfG mUmAmAmAmGmCmUmGmGm | SEQ ID NO: 1323 | AGCUGAUGUGGGU AAAGCUGG | SEQ ID NO: 771 |
| ETX-S00003965 | iaiaCmsGmsUmAmCmCmCmGmUfCfUfAm GmCmAmGmCmUmGmAmUm | SEQ ID NO: 1324 | CGUACCCGUCUAG CAGCUGAU | SEQ ID NO: 772 |
| ETX-S00003969 | iaiaUmsCmsUmGmUmGmCmAmGfGfGfU mAmUmUmAmUmCmAmUmCm | SEQ ID NO: 1325 | UCUGUGCAGGUA UUAUCAUC | SEQ ID NO: 773 |
| ETX-S00003973 | iaiaUmsGmsCmUmUmGmUmCmUfUfGfU mAmUmGmAmUmGmAmAmAm | SEQ ID NO: 1326 | UGCUUGUCUUGUA UGAUGAAA | SEQ ID NO: 774 |
| ETX-S00003977 | iaiaAmsCmsCmAmAmGmGmCmUfUfUfAm AmCmGmUmGmUmCmUmGm | SEQ ID NO: 1327 | ACCAAGGCUUUAA CGUGUCUG | SEQ ID NO: 775 |
| ETX-S00003981 | iaiaGmsGmsGmUmGmCmAmUmGfGfUfC mCmAmAmUmGmUmUmCmUm | SEQ ID NO: 1328 | GGGUGCAUGGUCC AAUGUUCU | SEQ ID NO: 776 |
| ETX-S00003985 | iaiaGmsAmsCmUmGmCmCmUmGfGfUfUm AmAmGmAmUmCmUmAmCm | SEQ ID NO: 1329 | GACUGCCUGGUUA AGAUCUAC | SEQ ID NO: 777 |
| ETX-S00003989 | iaiaGmsAmsUmGmUmGmGmGmUfAfAfA mGmCmUmGmGmAmGmCmUm | SEQ ID NO: 1330 | GAUGUGGUAAAG CUGGAGCU | SEQ ID NO: 778 |
| ETX-S00003993 | iaiaUmsGmsGmUmCmCmGmUmAfUfUfCm CmCmAmAmGmGmAmGmCm | SEQ ID NO: 1331 | UGGUCCGUAUUCC CAAGGAGC | SEQ ID NO: 779 |
| ETX-S00003997 | iaiaAmsCmsAmCmUmGmCmAmAfAfGfGm GmAmAmUmGmCmUmUmCm | SEQ ID NO: 1332 | ACACUGCAAAGGG AAUGCUUC | SEQ ID NO: 780 |
| ETX-S00004001 | iaiaGmsAmsUmUmAmAmGmGmGfCfCfU mGmUmAmCmCmAmAmGmGm | SEQ ID NO: 1333 | GAUUAAGGCCUG UACCAAGG | SEQ ID NO: 781 |
| ETX-S00004005 | iaiaUmsGmsUmGmCmAmGmGmGfUfAfU mUmAmUmCmAmUmCmUmAm | SEQ ID NO: 1334 | UGUGCAGGGUAUU AUCAUCUA | SEQ ID NO: 782 |
| ETX-S00004009 | iaiaAmsAmsCmGmUmGmUmCmUfGfUfG mCmAmGmGmGmUmAmUmUm | SEQ ID NO: 1335 | AACGUGUCUGUGC AGGGUAUU | SEQ ID NO: 783 |
| ETX-S00004013 | iaiaGmsGmsCmCmUmGmUmAmCfCfAfAm GmGmCmUmUmUmAmAmCm | SEQ ID NO: 1336 | GGCCUGUACCAAG GCUUUAAC | SEQ ID NO: 784 |
| ETX-S00004017 | iaiaUmsGmsAmCmAmCmUmGmCfAfAfAm GmGmGmAmAmUmGmCmUm | SEQ ID NO: 1337 | UGACACUGCAAAG GGAAUGCU | SEQ ID NO: 785 |
| ETX-S00004021 | iaiaUmsCmsGmGmUmGmAmCmUfGfCfCm UmGmGmUmUmAmAmGmAm | SEQ ID NO: 1338 | UCGGUGACUGCCU GGUUAAGA | SEQ ID NO: 786 |
| ETX-S00004025 | iaiaGmsCmsUmUmUmUmGmUmGfCfUfU mGmUmCmUmUmGmUmAmUm | SEQ ID NO: 1339 | GCUUUUGUGCUUG UCUUGUAU | SEQ ID NO: 787 |
| ETX-S00004029 | iaiaGmsGmsUmGmGmUmGmCmUfUfUfU mGmUmGmCmUmUmGmUmCm | SEQ ID NO: 1340 | GGUGGUGCUUUUG UGCUUGUC | SEQ ID NO: 788 |
| ETX-S00004033 | iaiaCmsGmsUmCmUmAmGmCmAfGfCfUm GmAmUmGmUmGmGmGmUm | SEQ ID NO: 1341 | CGUCUAGCAGCUG AUGUGGGU | SEQ ID NO: 789 |
| ETX-S00004037 | iaiaAmsGmsGmGmCmCmUmGmUfAfCfCm AmAmGmGmCmUmUmUmAm | SEQ ID NO: 1342 | AGGGCCUGUACCA AGGCUUUA | SEQ ID NO: 790 |
| ETX-S00004041 | iaiaGmsUmsGmCmUmUmGmUmCfUfUfG mUmAmUmGmAmUmGmAmAm | SEQ ID NO: 1343 | GUGCUUGUCUUGU AUGAUGAA | SEQ ID NO: 791 |
| ETX-S00004045 | iaiaAmsAmsAmUmCmUmGmAmUfGfGfG mAmUmUmAmAmGmGmGmCm | SEQ ID NO: 1344 | AAAUCUGAUGGGA UUAAGGGC | SEQ ID NO: 792 |
| ETX-S00004049 | iaiaCmsUmsAmCmAmAmAmUmCfUfGfAm UmGmGmGmAmUmUmAmAm | SEQ ID NO: 1345 | CUACAAAUCUGAU GGGAUUAA | SEQ ID NO: 793 |
| ETX-S00004053 | iaiaCmsAmsGmGmGmUmAmUmUfAfUfC mAmUmCmUmAmCmCmGmAm | SEQ ID NO: 1346 | CAGGGUAUUAUCA UCUACCGA | SEQ ID NO: 794 |
| ETX-S00004057 | iaiaCmsUmsGmUmAmCmCmAmAfGfGfCm UmUmUmAmAmCmGmUmGm | SEQ ID NO: 1347 | CUGUACCAAGGCU UUAACGUG | SEQ ID NO: 795 |
| ETX-S00004061 | iaiaAmsGmsAmUmCmUmAmCmAfAfAfU mCmUmGmAmUmGmGmGmAm | SEQ ID NO: 1348 | AGAUCUACAAAUC UGAUGGGA | SEQ ID NO: 796 |
| ETX-S00004065 | iaiaUmsUmsUmUmGmUmGmCmUfUfGfU mCmUmUmGmUmAmUmGmAm | SEQ ID NO: 1349 | UUUUGUGCUUGUC UUGUAUGA | SEQ ID NO: 797 |
| ETX-S00004069 | iaiaAmsCmsAmAmAmUmCmUmGfAfUfG mGmGmAmUmUmAmAmGmGm | SEQ ID NO: 1350 | ACAAAUCUGAUGG GAUUAAGG | SEQ ID NO: 798 |
| ETX-S00004073 | iaiaCmsAmsAmGmGmGmUmGmCfAfUfG mGmUmCmCmAmAmUmGmUm | SEQ ID NO: 1351 | CAAGGGUGCAUGG UCCAAUGU | SEQ ID NO: 799 |
| ETX-S00004077 | iaiaGmsGmsAmUmUmAmAmGmGfGfCfC mUmGmUmAmCmCmAmAmGm | SEQ ID NO: 1352 | GGAUUAAGGCCU GUACCAAG | SEQ ID NO: 800 |
| ETX-S00004081 | iaiaUmsCmsGmGmUmAmUmCmUfAfUfG mAmCmAmCmUmGmCmAmAm | SEQ ID NO: 1353 | UCGGUAUCUAUGA CACUGCAA | SEQ ID NO: 801 |
| ETX-S00004085 | iaiaCmsAmsAmGmGmCmUmUmUfAfAfCm GmUmGmUmCmUmGmUmGm | SEQ ID NO: 1354 | CAAGGCUUUAACG UGUCUGUG | SEQ ID NO: 802 |
| ETX-S00004089 | iaiaCmsAmsUmGmGmGmUmGmGfUfGfC mUmUmUmUmGmUmGmCmUm | SEQ ID NO: 1355 | CAUGGGUGGUGCU UUUGUGCU | SEQ ID NO: 803 |
| ETX-S00004093 | iaiaUmsAmsAmGmAmUmCmUmAfCfAfA mAmUmCmUmGmAmUmGmGm | SEQ ID NO: 1356 | UAAGAUCUACAAA UCUGAUGG | SEQ ID NO: 804 |
| ETX-S00004097 | iaiaCmsCmsCmGmUmCmUmAmGfCfAfGm CmUmGmAmUmGmUmGmGm | SEQ ID NO: 1357 | CCCGUCUAGCAGC UGAUGUGG | SEQ ID NO: 805 |
| ETX-S00004101 | iaiaAmsUmsGmAmCmAmCmUmGfCfAfAm AmGmGmGmAmAmUmGmCm | SEQ ID NO: 1358 | AUGACACUGCAAA GGGAAUGC | SEQ ID NO: 806 |
| ETX-S00004105 | iaiaUmsAmsGmCmAmGmCmUmGfAfUfG mUmGmGmGmUmAmAmAmGm | SEQ ID NO: 1359 | UAGCAGCUGAUGU GGGUAAAG | SEQ ID NO: 807 |
| ETX-S00004109 | iaiaGmsCmsAmAmAmGmGmGmAfAfUfG mCmUmUmCmCmGmGmAmUm | SEQ ID NO: 1360 | GCAAAGGGAAUGC UUCCGGAU | SEQ ID NO: 808 |
| ETX-S00004113 | iaiaGmsCmsCmCmGmUmAmCmCfCfGfUm CmUmAmGmCmAmGmCmUm | SEQ ID NO: 1361 | GCCCGUACCCGUC UAGCAGCU | SEQ ID NO: 809 |
| ETX-S00004117 | iaiaGmsUmsAmUmUmAmUmCmAfUfCfU mAmCmCmGmAmGmCmCmGm | SEQ ID NO: 1362 | GUAUUAUCAUCUA CCGAGCCG | SEQ ID NO: 810 |
| ETX-S00004121 | iaiaCmsUmsCmUmUmGmAmUmUfUfUfG mCmCmCmGmUmAmCmCmCm | SEQ ID NO: 1363 | CUCUUGAUUUUGC CCGUACCC | SEQ ID NO: 811 |
| ETX-S00004125 | iaiaUmsAmsCmCmCmGmUmCmUfAfGfCm AmGmCmUmGmAmUmGmUm | SEQ ID NO: 1364 | UACCCGUCUAGCA GCUGAUGU | SEQ ID NO: 812 |
| ETX-S00004129 | iaiaCmsAmsAmAmUmCmUmGmAfUfGfG mGmAmUmUmAmAmGmGmGm | SEQ ID NO: 1365 | CAAAUCUGAUGGG AUUAAGGG | SEQ ID NO: 813 |
| ETX-S00004133 | iaiaGmsUmsAmCmCmCmUmCmUfUfGfAm UmUmUmUmGmCmCmCmGm | SEQ ID NO: 1366 | GUACCCUCUUGAU UUUGCCCG | SEQ ID NO: 814 |
| ETX-S00004137 | iaiaCmsCmsUmGmUmAmCmCmAfAfGfGm CmUmUmUmAmAmCmGmUm | SEQ ID NO: 1367 | CCUGUACCAAGGC UUUAACGU | SEQ ID NO: 815 |
| ETX-S00004141 | iaiaUmsGmsGmGmAmUmUmAmAfGfGfG mCmCmUmGmUmAmCmCmAm | SEQ ID NO: 1368 | UGGGAUUAAGGGC CUGUACCA | SEQ ID NO: 816 |
| ETX-S00004145 | iaiaAmsUmsUmAmUmCmAmUmCfUfAfCm CmGmAmGmCmCmGmCmCm | SEQ ID NO: 1369 | AUUAUCAUCUACC GAGCCGCC | SEQ ID NO: 817 |
| ETX-S00004149 | iaiaGmsCmsCmUmGmUmAmCmCfAfAfGm GmCmUmUmUmAmAmCmGm | SEQ ID NO: 1370 | GCCUGUACCAAGG CUUUAACG | SEQ ID NO: 818 |
| ETX-S00004153 | iaiaAmsUmsGmGmGmAmUmUmAfAfGfG mGmCmCmUmGmUmAmCmCm | SEQ ID NO: 1371 | AUGGGAUUAAGGG CCUGUACC | SEQ ID NO: 819 |
| ETX-S00004157 | iaiaAmsCmsGmUmGmUmCmUmGfUfGfCm AmGmGmGmUmAmUmUmAm | SEQ ID NO: 1372 | ACGUGUCUGUGCA GGGUAUUA | SEQ ID NO: 820 |
| ETX-S00004161 | iaiaGmsGmsCmAmUmUmAmUmAfGfAfC mUmGmCmGmUmGmGmUmCm | SEQ ID NO: 1373 | GGCAUUAUAGACU GCGUGGUC | SEQ ID NO: 821 |
| ETX-S00004165 | iaiaUmsUmsCmCmCmCmAmCmCfCfAfGm GmCmUmCmUmUmAmAmCm | SEQ ID NO: 1374 | UUCCCCACCCAGG CUCUUAAC | SEQ ID NO: 822 |
| ETX-S00004169 | iaiaAmsUmsGmUmCmAmUmCmAfGfAfU mAmCmUmUmCmCmCmCmAm | SEQ ID NO: 1375 | AUGUCAUCAGAUA CUUCCCCA | SEQ ID NO: 823 |
| ETX-S00004173 | iaiaGmsUmsCmAmUmCmAmGmAfUfAfCm UmUmCmCmCmCmAmCmCm | SEQ ID NO: 1376 | GUCAUCAGAUACU UCCCCACC | SEQ ID NO: 824 |
| ETX-S00004177 | iaiaUmsGmsUmCmAmUmCmAmGfAfUfA mCmUmUmCmCmCmCmAmCm | SEQ ID NO: 1377 | UGUCAUCAGAUAC UUCCCCAC | SEQ ID NO: 825 |
| ETX-S00004181 | iaiaAmsAmsUmGmUmCmAmUmCfAfGfA mUmAmCmUmUmCmCmCmCm | SEQ ID NO: 1378 | AAUGUCAUCAGAU ACUUCCCC | SEQ ID NO: 826 |
| ETX-S00004185 | iaiaCmsUmsUmCmCmCmCmAmCfCfCfAm GmGmCmUmCmUmUmAmAm | SEQ ID NO: 1379 | CUUCCCCACCCAG GCUCUUAA | SEQ ID NO: 827 |
| ETX-S00004189 | iaiaCmsUmsAmCmCmGmAmGmCfCfGfCm CmUmAmCmUmUmCmGmGm | SEQ ID NO: 1380 | CUACCGAGCCGCC UACUUCGG | SEQ ID NO: 828 |

As used herein, and in particular in Tables 3 and 4, the following abbreviations are used for modified nucleosides:
Am stands for 2'-O-methyl-adenosine, Cm stands for 2'-O-methyl-cytidine, Gm stands for 2'-O-methyl-guanosine, Um stands for 2'-O-methyl-uridine, Af stands for 2'-Fluoro-adenosine, Cf stands for 2'-Fluoro-cytidine, Gf stands for 2'-Fluoro-guanosine and Uf stands for 2'-Fluoro-uridine.

Furthermore, the letter "s" is used as abbreviation for a phosphorothioate linkage between two consecutive (modified) nucleosides. For example, the abbreviation "AmsAm" is used for two consecutive 2'-O-methyl-adenosine nucleosides that are linked via a 3'5' phosphorothioate linkage. No abbreviation is used for nucleosides that are linked via a standard 3'5' phosphodiester linkage. For example, the abbreviation "AmAm" is used for two consecutive 2'-O-methyl-adenosine nucleosides that are linked via a 3'5' phosphodiester linkage.

In certain embodiments, the nucleic acid comprises a first strand that comprises, consists of, or consists essentially of a (modified) nucleoside sequence differing by 0 or 1 nucleosides from any one of SEQ ID NO: 829-1104;
and a second strand that comprises, consists of, or consists essentially of a (modified) nucleoside sequence differing by 0 or 1 nucleosides from any one of SEQ ID NO: 1105-1380.

Preferred combinations of complementary modified antisense (first) and sense (second) strands are listed below in Table 5:

**Table 5**

| Duplex ID | Antisense strand ID | Sense strand ID |
|---|---|---|
| ETX-M00001351 | ETX-S00003090 | ETX-S00003089 |
| ETX-M00001352 | ETX-S00003094 | ETX-S00003093 |
| ETX-M00001353 | ETX-S00003098 | ETX-S00003097 |
| ETX-M00001354 | ETX-S00003102 | ETX-S00003101 |
| ETX-M00001355 | ETX-S00003106 | ETX-S00003105 |
| ETX-M00001356 | ETX-S00003110 | ETX-S00003109 |
| ETX-M00001357 | ETX-S00003114 | ETX-S00003113 |
| ETX-M00001358 | ETX-S00003118 | ETX-S00003117 |
| ETX-M00001359 | ETX-S00003122 | ETX-S00003121 |
| ETX-M00001360 | ETX-S00003126 | ETX-S00003125 |
| ETX-M00001361 | ETX-S00003130 | ETX-S00003129 |
| ETX-M00001362 | ETX-S00003134 | ETX-S00003133 |
| ETX-M00001363 | ETX-S00003138 | ETX-S00003137 |
| ETX-M00001364 | ETX-S00003142 | ETX-S00003141 |
| ETX-M00001365 | ETX-S00003146 | ETX-S00003145 |
| ETX-M00001366 | ETX-S00003150 | ETX-S00003149 |
| ETX-M00001367 | ETX-S00003154 | ETX-S00003153 |
| ETX-M00001368 | ETX-S00003158 | ETX-S00003157 |
| ETX-M00001369 | ETX-S00003162 | ETX-S00003161 |
| ETX-M00001370 | ETX-S00003166 | ETX-S00003165 |
| ETX-M00001371 | ETX-S00003170 | ETX-S00003169 |
| ETX-M00001372 | ETX-S00003174 | ETX-S00003173 |
| ETX-M00001373 | ETX-S00003178 | ETX-S00003177 |
| ETX-M00001374 | ETX-S00003182 | ETX-S00003181 |
| ETX-M00001375 | ETX-S00003186 | ETX-S00003185 |
| ETX-M00001376 | ETX-S00003190 | ETX-S00003189 |
| ETX-M00001377 | ETX-S00003194 | ETX-S00003193 |
| ETX-M00001378 | ETX-S00003198 | ETX-S00003197 |
| ETX-M00001379 | ETX-S00003202 | ETX-S00003201 |
| ETX-M00001380 | ETX-S00003206 | ETX-S00003205 |
| ETX-M00001381 | ETX-S00003210 | ETX-S00003209 |
| ETX-M00001382 | ETX-S00003214 | ETX-S00003213 |
| ETX-M00001383 | ETX-S00003218 | ETX-S00003217 |
| ETX-M00001384 | ETX-S00003222 | ETX-S00003221 |
| ETX-M00001385 | ETX-S00003226 | ETX-S00003225 |
| ETX-M00001386 | ETX-S00003230 | ETX-S00003229 |
| ETX-M00001387 | ETX-S00003234 | ETX-S00003233 |
| ETX-M00001388 | ETX-S00003238 | ETX-S00003237 |
| ETX-M00001389 | ETX-S00003242 | ETX-S00003241 |
| ETX-M00001390 | ETX-S00003246 | ETX-S00003245 |
| ETX-M00001391 | ETX-S00003250 | ETX-S00003249 |
| ETX-M00001392 | ETX-S00003254 | ETX-S00003253 |
| ETX-M00001393 | ETX-S00003258 | ETX-S00003257 |
| ETX-M00001394 | ETX-S00003262 | ETX-S00003261 |
| ETX-M00001395 | ETX-S00003266 | ETX-S00003265 |
| ETX-M00001396 | ETX-S00003270 | ETX-S00003269 |
| ETX-M00001397 | ETX-S00003274 | ETX-S00003273 |
| ETX-M00001398 | ETX-S00003278 | ETX-S00003277 |
| ETX-M00001399 | ETX-S00003282 | ETX-S00003281 |
| ETX-M00001400 | ETX-S00003286 | ETX-S00003285 |
| ETX-M00001401 | ETX-S00003290 | ETX-S00003289 |
| ETX-M00001402 | ETX-S00003294 | ETX-S00003293 |
| ETX-M00001403 | ETX-S00003298 | ETX-S00003297 |
| ETX-M00001404 | ETX-S00003302 | ETX-S00003301 |
| ETX-M00001405 | ETX-S00003306 | ETX-S00003305 |
| ETX-M00001406 | ETX-S00003310 | ETX-S00003309 |
| ETX-M00001407 | ETX-S00003314 | ETX-S00003313 |
| ETX-M00001408 | ETX-S00003318 | ETX-S00003317 |
| ETX-M00001409 | ETX-S00003322 | ETX-S00003321 |
| ETX-M00001410 | ETX-S00003326 | ETX-S00003325 |
| ETX-M00001411 | ETX-S00003330 | ETX-S00003329 |
| ETX-M00001412 | ETX-S00003334 | ETX-S00003333 |
| ETX-M00001413 | ETX-S00003338 | ETX-S00003337 |
| ETX-M00001414 | ETX-S00003342 | ETX-S00003341 |
| ETX-M00001415 | ETX-S00003346 | ETX-S00003345 |
| ETX-M00001416 | ETX-S00003350 | ETX-S00003349 |
| ETX-M00001417 | ETX-S00003354 | ETX-S00003353 |
| ETX-M00001418 | ETX-S00003358 | ETX-S00003357 |
| ETX-M00001419 | ETX-S00003362 | ETX-S00003361 |
| ETX-M00001420 | ETX-S00003366 | ETX-S00003365 |
| ETX-M00001421 | ETX-S00003370 | ETX-S00003369 |
| ETX-M00001422 | ETX-S00003374 | ETX-S00003373 |
| ETX-M00001423 | ETX-S00003378 | ETX-S00003377 |
| ETX-M00001424 | ETX-S00003382 | ETX-S00003381 |
| ETX-M00001425 | ETX-S00003386 | ETX-S00003385 |
| ETX-M00001426 | ETX-S00003390 | ETX-S00003389 |
| ETX-M00001427 | ETX-S00003394 | ETX-S00003393 |
| ETX-M00001428 | ETX-S00003398 | ETX-S00003397 |
| ETX-M00001429 | ETX-S00003402 | ETX-S00003401 |
| ETX-M00001430 | ETX-S00003406 | ETX-S00003405 |
| ETX-M00001431 | ETX-S00003410 | ETX-S00003409 |
| ETX-M00001432 | ETX-S00003414 | ETX-S00003413 |
| ETX-M00001433 | ETX-S00003418 | ETX-S00003417 |
| ETX-M00001434 | ETX-S00003422 | ETX-S00003421 |
| ETX-M00001435 | ETX-S00003426 | ETX-S00003425 |
| ETX-M00001436 | ETX-S00003430 | ETX-S00003429 |
| ETX-M00001437 | ETX-S00003434 | ETX-S00003433 |
| ETX-M00001438 | ETX-S00003438 | ETX-S00003437 |
| ETX-M00001439 | ETX-S00003442 | ETX-S00003441 |
| ETX-M00001440 | ETX-S00003446 | ETX-S00003445 |
| ETX-M00001441 | ETX-S00003450 | ETX-S00003449 |
| ETX-M00001442 | ETX-S00003454 | ETX-S00003453 |
| ETX-M00001443 | ETX-S00003458 | ETX-S00003457 |
| ETX-M00001444 | ETX-S00003462 | ETX-S00003461 |
| ETX-M00001445 | ETX-S00003466 | ETX-S00003465 |
| ETX-M00001446 | ETX-S00003470 | ETX-S00003469 |
| ETX-M00001447 | ETX-S00003474 | ETX-S00003473 |
| ETX-M00001448 | ETX-S00003478 | ETX-S00003477 |
| ETX-M00001449 | ETX-S00003482 | ETX-S00003481 |
| ETX-M00001450 | ETX-S00003486 | ETX-S00003485 |
| ETX-M00001451 | ETX-S00003490 | ETX-S00003489 |
| ETX-M00001452 | ETX-S00003494 | ETX-S00003493 |
| ETX-M00001453 | ETX-S00003498 | ETX-S00003497 |
| ETX-M00001454 | ETX-S00003502 | ETX-S00003501 |
| ETX-M00001455 | ETX-S00003506 | ETX-S00003505 |
| ETX-M00001456 | ETX-S00003510 | ETX-S00003509 |
| ETX-M00001457 | ETX-S00003514 | ETX-S00003513 |
| ETX-M00001458 | ETX-S00003518 | ETX-S00003517 |
| ETX-M00001459 | ETX-S00003522 | ETX-S00003521 |
| ETX-M00001460 | ETX-S00003526 | ETX-S00003525 |
| ETX-M00001461 | ETX-S00003530 | ETX-S00003529 |
| ETX-M00001462 | ETX-S00003534 | ETX-S00003533 |
| ETX-M00001463 | ETX-S00003538 | ETX-S00003537 |
| ETX-M00001464 | ETX-S00003542 | ETX-S00003541 |
| ETX-M00001465 | ETX-S00003546 | ETX-S00003545 |
| ETX-M00001466 | ETX-S00003550 | ETX-S00003549 |
| ETX-M00001467 | ETX-S00003554 | ETX-S00003553 |
| ETX-M00001468 | ETX-S00003558 | ETX-S00003557 |
| ETX-M00001469 | ETX-S00003562 | ETX-S00003561 |
| ETX-M00001470 | ETX-S00003566 | ETX-S00003565 |
| ETX-M00001471 | ETX-S00003570 | ETX-S00003569 |
| ETX-M00001472 | ETX-S00003574 | ETX-S00003573 |
| ETX-M00001473 | ETX-S00003578 | ETX-S00003577 |
| ETX-M00001474 | ETX-S00003582 | ETX-S00003581 |
| ETX-M00001475 | ETX-S00003586 | ETX-S00003585 |
| ETX-M00001476 | ETX-S00003590 | ETX-S00003589 |
| ETX-M00001477 | ETX-S00003594 | ETX-S00003593 |
| ETX-M00001478 | ETX-S00003598 | ETX-S00003597 |
| ETX-M00001479 | ETX-S00003602 | ETX-S00003601 |
| ETX-M00001480 | ETX-S00003606 | ETX-S00003605 |
| ETX-M00001481 | ETX-S00003610 | ETX-S00003609 |
| ETX-M00001482 | ETX-S00003614 | ETX-S00003613 |
| ETX-M00001483 | ETX-S00003618 | ETX-S00003617 |
| ETX-M00001484 | ETX-S00003622 | ETX-S00003621 |
| ETX-M00001485 | ETX-S00003626 | ETX-S00003625 |
| ETX-M00001486 | ETX-S00003630 | ETX-S00003629 |
| ETX-M00001487 | ETX-S00003634 | ETX-S00003633 |
| ETX-M00001488 | ETX-S00003638 | ETX-S00003637 |
| ETX-M00001489 | ETX-S00003642 | ETX-S00003641 |
| ETX-M00001490 | ETX-S00003646 | ETX-S00003645 |
| ETX-M00001491 | ETX-S00003650 | ETX-S00003649 |
| ETX-M00001492 | ETX-S00003654 | ETX-S00003653 |
| ETX-M00001493 | ETX-S00003658 | ETX-S00003657 |
| ETX-M00001494 | ETX-S00003662 | ETX-S00003661 |
| ETX-M00001495 | ETX-S00003666 | ETX-S00003665 |
| ETX-M00001496 | ETX-S00003670 | ETX-S00003669 |
| ETX-M00001497 | ETX-S00003674 | ETX-S00003673 |
| ETX-M00001498 | ETX-S00003678 | ETX-S00003677 |
| ETX-M00001499 | ETX-S00003682 | ETX-S00003681 |
| ETX-M00001500 | ETX-S00003686 | ETX-S00003685 |
| ETX-M00001501 | ETX-S00003690 | ETX-S00003689 |
| ETX-M00001502 | ETX-S00003694 | ETX-S00003693 |
| ETX-M00001503 | ETX-S00003698 | ETX-S00003697 |
| ETX-M00001504 | ETX-S00003702 | ETX-S00003701 |
| ETX-M00001505 | ETX-S00003706 | ETX-S00003705 |
| ETX-M00001506 | ETX-S00003710 | ETX-S00003709 |
| ETX-M00001507 | ETX-S00003714 | ETX-S00003713 |
| ETX-M00001508 | ETX-S00003718 | ETX-S00003717 |
| ETX-M00001509 | ETX-S00003722 | ETX-S00003721 |
| ETX-M00001510 | ETX-S00003726 | ETX-S00003725 |
| ETX-M00001511 | ETX-S00003730 | ETX-S00003729 |
| ETX-M00001512 | ETX-S00003734 | ETX-S00003733 |
| ETX-M00001513 | ETX-S00003738 | ETX-S00003737 |
| ETX-M00001514 | ETX-S00003742 | ETX-S00003741 |
| ETX-M00001515 | ETX-S00003746 | ETX-S00003745 |
| ETX-M00001516 | ETX-S00003750 | ETX-S00003749 |
| ETX-M00001517 | ETX-S00003754 | ETX-S00003753 |
| ETX-M00001518 | ETX-S00003758 | ETX-S00003757 |
| ETX-M00001519 | ETX-S00003762 | ETX-S00003761 |
| ETX-M00001520 | ETX-S00003766 | ETX-S00003765 |
| ETX-M00001521 | ETX-S00003770 | ETX-S00003769 |
| ETX-M00001522 | ETX-S00003774 | ETX-S00003773 |
| ETX-M00001523 | ETX-S00003778 | ETX-S00003777 |
| ETX-M00001524 | ETX-S00003782 | ETX-S00003781 |
| ETX-M00001525 | ETX-S00003786 | ETX-S00003785 |
| ETX-M00001526 | ETX-S00003790 | ETX-S00003789 |
| ETX-M00001527 | ETX-S00003794 | ETX-S00003793 |
| ETX-M00001528 | ETX-S00003798 | ETX-S00003797 |
| ETX-M00001529 | ETX-S00003802 | ETX-S00003801 |
| ETX-M00001530 | ETX-S00003806 | ETX-S00003805 |
| ETX-M00001531 | ETX-S00003810 | ETX-S00003809 |
| ETX-M00001532 | ETX-S00003814 | ETX-S00003813 |
| ETX-M00001533 | ETX-S00003818 | ETX-S00003817 |
| ETX-M00001534 | ETX-S00003822 | ETX-S00003821 |
| ETX-M00001535 | ETX-S00003826 | ETX-S00003825 |
| ETX-M00001536 | ETX-S00003830 | ETX-S00003829 |
| ETX-M00001537 | ETX-S00003834 | ETX-S00003833 |
| ETX-M00001538 | ETX-S00003838 | ETX-S00003837 |
| ETX-M00001539 | ETX-S00003842 | ETX-S00003841 |
| ETX-M00001540 | ETX-S00003846 | ETX-S00003845 |
| ETX-M00001541 | ETX-S00003850 | ETX-S00003849 |
| ETX-M00001542 | ETX-S00003854 | ETX-S00003853 |
| ETX-M00001543 | ETX-S00003858 | ETX-S00003857 |
| ETX-M00001544 | ETX-S00003862 | ETX-S00003861 |
| ETX-M00001545 | ETX-S00003866 | ETX-S00003865 |
| ETX-M00001546 | ETX-S00003870 | ETX-S00003869 |
| ETX-M00001547 | ETX-S00003874 | ETX-S00003873 |
| ETX-M00001548 | ETX-S00003878 | ETX-S00003877 |
| ETX-M00001549 | ETX-S00003882 | ETX-S00003881 |
| ETX-M00001550 | ETX-S00003886 | ETX-S00003885 |
| ETX-M00001551 | ETX-S00003890 | ETX-S00003889 |
| ETX-M00001552 | ETX-S00003894 | ETX-S00003893 |
| ETX-M00001553 | ETX-S00003898 | ETX-S00003897 |
| ETX-M00001554 | ETX-S00003902 | ETX-S00003901 |
| ETX-M00001555 | ETX-S00003906 | ETX-S00003905 |
| ETX-M00001556 | ETX-S00003910 | ETX-S00003909 |
| ETX-M00001557 | ETX-S00003914 | ETX-S00003913 |
| ETX-M00001558 | ETX-S00003918 | ETX-S00003917 |
| ETX-M00001559 | ETX-S00003922 | ETX-S00003921 |
| ETX-M00001560 | ETX-S00003926 | ETX-S00003925 |
| ETX-M00001561 | ETX-S00003930 | ETX-S00003929 |
| ETX-M00001562 | ETX-S00003934 | ETX-S00003933 |
| ETX-M00001563 | ETX-S00003938 | ETX-S00003937 |
| ETX-M00001564 | ETX-S00003942 | ETX-S00003941 |
| ETX-M00001565 | ETX-S00003946 | ETX-S00003945 |
| ETX-M00001566 | ETX-S00003950 | ETX-S00003949 |
| ETX-M00001567 | ETX-S00003954 | ETX-S00003953 |
| ETX-M00001568 | ETX-S00003958 | ETX-S00003957 |
| ETX-M00001569 | ETX-S00003962 | ETX-S00003961 |
| ETX-M00001570 | ETX-S00003966 | ETX-S00003965 |
| ETX-M00001571 | ETX-S00003970 | ETX-S00003969 |
| ETX-M00001572 | ETX-S00003974 | ETX-S00003973 |
| ETX-M00001573 | ETX-S00003978 | ETX-S00003977 |
| ETX-M00001574 | ETX-S00003982 | ETX-S00003981 |
| ETX-M00001575 | ETX-S00003986 | ETX-S00003985 |
| ETX-M00001576 | ETX-S00003990 | ETX-S00003989 |
| ETX-M00001577 | ETX-S00003994 | ETX-S00003993 |
| ETX-M00001578 | ETX-S00003998 | ETX-S00003997 |
| ETX-M00001579 | ETX-S00004002 | ETX-S00004001 |
| ETX-M00001580 | ETX-S00004006 | ETX-S00004005 |
| ETX-M00001581 | ETX-S00004010 | ETX-S00004009 |
| ETX-M00001582 | ETX-S00004014 | ETX-S00004013 |
| ETX-M00001583 | ETX-S00004018 | ETX-S00004017 |
| ETX-M00001584 | ETX-S00004022 | ETX-S00004021 |
| ETX-M00001585 | ETX-S00004026 | ETX-S00004025 |
| ETX-M00001586 | ETX-S00004030 | ETX-S00004029 |
| ETX-M00001587 | ETX-S00004034 | ETX-S00004033 |
| ETX-M00001588 | ETX-S00004038 | ETX-S00004037 |
| ETX-M00001589 | ETX-S00004042 | ETX-S00004041 |
| ETX-M00001590 | ETX-S00004046 | ETX-S00004045 |
| ETX-M00001591 | ETX-S00004050 | ETX-S00004049 |
| ETX-M00001592 | ETX-S00004054 | ETX-S00004053 |
| ETX-M00001593 | ETX-S00004058 | ETX-S00004057 |
| ETX-M00001594 | ETX-S00004062 | ETX-S00004061 |
| ETX-M00001595 | ETX-S00004066 | ETX-S00004065 |
| ETX-M00001596 | ETX-S00004070 | ETX-S00004069 |
| ETX-M00001597 | ETX-S00004074 | ETX-S00004073 |
| ETX-M00001598 | ETX-S00004078 | ETX-S00004077 |
| ETX-M00001599 | ETX-S00004082 | ETX-S00004081 |
| ETX-M00001600 | ETX-S00004086 | ETX-S00004085 |
| ETX-M00001601 | ETX-S00004090 | ETX-S00004089 |
| ETX-M00001602 | ETX-S00004094 | ETX-S00004093 |
| ETX-M00001603 | ETX-S00004098 | ETX-S00004097 |
| ETX-M00001604 | ETX-S00004102 | ETX-S00004101 |
| ETX-M00001605 | ETX-S00004106 | ETX-S00004105 |
| ETX-M00001606 | ETX-S00004110 | ETX-S00004109 |
| ETX-M00001607 | ETX-S00004114 | ETX-S00004113 |
| ETX-M00001608 | ETX-S00004118 | ETX-S00004117 |
| ETX-M00001609 | ETX-S00004122 | ETX-S00004121 |
| ETX-M00001610 | ETX-S00004126 | ETX-S00004125 |
| ETX-M00001611 | ETX-S00004130 | ETX-S00004129 |
| ETX-M00001612 | ETX-S00004134 | ETX-S00004133 |
| ETX-M00001613 | ETX-S00004138 | ETX-S00004137 |
| ETX-M00001614 | ETX-S00004142 | ETX-S00004141 |
| ETX-M00001615 | ETX-S00004146 | ETX-S00004145 |
| ETX-M00001616 | ETX-S00004150 | ETX-S00004149 |
| ETX-M00001617 | ETX-S00004154 | ETX-S00004153 |
| ETX-M00001618 | ETX-S00004158 | ETX-S00004157 |
| ETX-M00001619 | ETX-S00004162 | ETX-S00004161 |
| ETX-M00001620 | ETX-S00004166 | ETX-S00004165 |
| ETX-M00001621 | ETX-S00004170 | ETX-S00004169 |
| ETX-M00001622 | ETX-S00004174 | ETX-S00004173 |
| ETX-M00001623 | ETX-S00004178 | ETX-S00004177 |
| ETX-M00001624 | ETX-S00004182 | ETX-S00004181 |
| ETX-M00001625 | ETX-S00004186 | ETX-S00004185 |
| ETX-M00001626 | ETX-S00004190 | ETX-S00004189 |

For duplexes of Table 5:
ETX-M00001351 - ETX-M00001626 preferably have a duplex structure according to Figure 8b.

In a particularly preferred embodiment, the invention relates to a nucleic acid comprising first and second strands that comprise, consist of, or consist essentially of a nucleoside sequence differing by 0 or 1 nucleosides from any one of the following first and second sequences:

| Duplex ID | Modified first strand | Modified second strand |
|---|---|---|
| ETX-M00001378 | ETX-S00003198 (SEQ ID NO: 856) | ETX-S00003197 (SEQ ID NO: 1132) |
| ETX-M00001397 | ETX-S00003274 (SEQ ID NO: 875) | ETX-S00003273 (SEQ ID NO: 1151) |
| ETX-M00001513 | ETX-S00003738 (SEQ ID NO: 991) | ETX-S00003737 (SEQ ID NO: 1267) |
| ETX-M00001527 | ETX-S00003794 (SEQ ID NO: 1005) | ETX-S00003793 (SEQ ID NO: 1281) |
| ETX-M00001570 | ETX-S00003966 (SEQ ID NO: 1048) | ETX-S00003965 (SEQ ID NO: 1324) |

In an even more preferred embodiment, the invention relates to a nucleic acid comprising first and second strands that comprise, consist of, or consist essentially of a nucleoside sequence differing by 0 or 1 nucleosides from any one of the following first and second sequences:

| Duplex ID | Modified first strand | Modified second strand |
|---|---|---|
| ETX-M00001378 | ETX-S00003198 (SEQ ID NO: 856) | ETX-S00003197 (SEQ ID NO: 1132) |

In case of ambiguity between the sequences in this specification and the sequences in the attached sequence listing, the sequences provided herein are considered to be the correct sequences.

### ABASIC NUCLEOTIDES

In certain embodiments, there are 1, e.g. 2, e.g. 3, e.g. 4 or more abasic nucleosides present in nucleic acids according to the invention. Abasic nucleosides are modified nucleosides because they lack the base normally seen at position 1 of the sugar moiety. Typically, there will be a hydrogen at position 1 of the sugar moiety of the abasic nucleosides present in a nucleic acid according to the present invention.

The abasic nucleosides are in the terminal region of the second strand, preferably located within the terminal 5 nucleosides of the end of the strand. The terminal region may be the terminal 5 nucleosides, which includes abasic nucleosides.

The second strand may comprise, as preferred features (which are all specifically contemplated in combination unless mutually exclusive):
2, or more than 2, abasic nucleosides in a terminal region of the second strand; and/or
2, or more than 2, abasic nucleosides in either the 5' or 3' terminal region of the second strand; and/or
2, or more than 2, abasic nucleosides in either the 5' or 3' terminal region of the second strand, wherein the abasic nucleosides are present in an overhang as herein described; and/or
2, or more than 2, consecutive abasic nucleosides in a terminal region of the second strand, wherein preferably one such abasic nucleosides is a terminal nucleosides; and/or
2, or more than 2, consecutive abasic nucleosides in either the 5' or 3' terminal region of the second strand, wherein preferably one such abasic nucleosides is a terminal nucleosides in either the 5' or 3' terminal region of the second strand; and/or
a reversed internucleoside linkage connects at least one abasic nucleoside to an adjacent basic nucleoside in a terminal region of the second strand; and/or
a reversed internucleoside linkage connects at least one abasic nucleoside to an adjacent basic nucleoside in either the 5' or 3' terminal region of the second strand; and/or
an abasic nucleoside as the penultimate nucleoside which is connected via the reversed linkage to the nucleoside which is not the terminal nucleoside (called the antepenultimate nucleoside herein); and/or
abasic nucleosides as the 2 terminal nucleosides connected via a 5'-3' linkage when reading the strand in the direction towards the terminus comprising the terminal nucleosides;
abasic nucleosides as the 2 terminal nucleosides connected via a 3'-5' linkage when reading the strand in the direction towards the terminus comprising the terminal nucleosides;
abasic nucleosides as the terminal 2 positions, wherein the penultimate nucleoside is connected via the reversed linkage to the antepenultimate nucleoside, and wherein the reversed linkage is a 5-5' reversed linkage or a 3'-3' reversed linkage;
abasic nucleosides as the terminal 2 positions, wherein the penultimate nucleoside is connected via the reversed linkage to the antepenultimate nucleoside, and wherein either
   (1) the reversed linkage is a 5-5' reversed linkage and the linkage between the terminal and penultimate abasic nucleosides is 3'5' when reading towards the terminus comprising the terminal and penultimate abasic nucleosides; or
   (2) the reversed linkage is a 3-3' reversed linkage and the linkage between the terminal and penultimate abasic nucleosides is 5'3' when reading towards the terminus comprising the terminal and penultimate abasic nucleosides.

Preferably there is an abasic nucleoside at the terminus of the second strand.

Preferably there are 2 or at least 2 abasic nucleosides in the terminal region of the second strand, preferably at the terminal and penultimate positions.

Preferably 2 or more abasic nucleosides are consecutive, for example all abasic nucleosides may be consecutive. For example, the terminal 1 or terminal 2 or terminal 3 or terminal 4 nucelotides may be abasic nucleosides.

An abasic nucleoside may also be linked to an adjacent nucleoside through a 5'-3' phosphodiester linkage or reversed linkage unless there is only 1 abasic nucleoside at the terminus, in which case it will have a reversed linkage to the adjacent nucleoside.

A reversed linkage (which may also be referred to as an inverted linkage, which is also seen in the art), comprises either a 5'-5', a 3-'3', a 3'-2' or a 2'-3' phosphodiester linkage between the adjacent sugar moieties of the nucleosides.

Abasic nucleosides which are not terminal will have 2 phosphodiester bonds, one with each adjacent nucleoside, and these may be a reversed linkage or may be a 5'-3 phosphodiester bond or may be one of each.

A preferred embodiment comprises 2 abasic nucleosides at the terminal and penultimate positions of the second strand, and wherein the reversed internucleoside linkage is located between the penultimate (abasic) nucleoside and the antepenultimate nucleoside.

Preferably there are 2 abasic nucleosides at the terminal and penultimate positions of the second strand and the penultimate nucleoside is linked to the antepenultimate nucleoside through a reversed internucleoside linkage and is linked to the terminal nucleoside through a 5'-3' or 3'-5' phosphodiester linkage (reading in the direction of the terminus of the molecule).

Different preferred features are as follows:
The reversed internucleoside linkage is a 3'-3' reversed linkage. The reversed internucleoside linkage is at a terminal region which is distal to the 5' terminal phosphate of the second strand.
The reversed internucleoside linkage is a 5'-5' reversed linkage. The reversed internucleoside linkage is at a terminal region which is distal to the 3' terminal hydroxide of the second strand.
In certain embodiments, the second strand comprises 2 consecutive abasic nucleosides in the 5' terminal region of the second strand, wherein one such abasic nucleoside is a terminal nucleoside at the 5' terminal region of the second strand and the other abasic nucleoside is a penultimate nucleoside at the 5' terminal region of the second strand, wherein: (a) said penultimate abasic nucleoside is connected to an adjacent first basic nucleoside in an adjacent 5' near terminal region through a reversed internucleoside linkage; and (b) the reversed linkage is a 5-5' reversed linkage; and (c) the linkage between the terminal and penultimate abasic nucleosides is 3'5' when reading towards the terminus comprising the terminal and penultimate abasic nucleosides. More typically, (i) the first strand and the second strand each has a length of 23 nucleosides; (ii) two phosphorothioate internucleoside linkages are respectively between three consecutive positions in said 5' near terminal region of the second strand, wherein a first phosphorothioate internucleoside linkage is present between said adjacent first basic nucleoside of (a) and an adjacent second basic nucleoside in said 5' near terminal region of the second strand, and a second phosphorothioate internucleoside linkage is present between said adjacent second basic nucleoside and an adjacent third basic nucleoside in said 5' near terminal region of the second strand; (iii) two phosphorothioate internucleoside linkages are respectively between three consecutive positions in both 5' and 3' terminal regions of the first strand, whereby a terminal nucleoside respectively at each of the 5' and 3' terminal regions of said first strand is each attached to a respective 5' and 3' adjacent penultimate nucleoside by a phosphorothioate internucleoside linkage, and each first 5' and 3' penultimate nucleoside is attached to a respective 5' and 3' adjacent antepenultimate nucleoside by a phosphorothioate internucleoside linkage; and (iv) the second strand of the nucleic acid is conjugated directly or indirectly to one or more ligand moieties at the 3' terminal region of the second strand.

Alternatively the second strand comprises 2 consecutive abasic nucleosides preferably in an overhang in the 3' terminal region of the second strand, wherein one such abasic nucleoside is a terminal nucleoside at the 3' terminal region of the second strand and the other abasic nucleoside is a penultimate nucleoside at the 3' terminal region of the second strand, wherein: (a) said penultimate abasic nucleoside is connected to an adjacent first basic nucleoside in an adjacent 3' near terminal region through a reversed internucleoside linkage; and (b) the reversed linkage is a 3-3' reversed linkage; and (c) the linkage between the terminal and penultimate abasic nucleosides is 5'-3' when reading towards the terminus comprising the terminal and penultimate abasic nucleosides. More typically, (i) the first strand and the second strand each has a length of 23 nucleosides; (ii) two phosphorothioate internucleoside linkages are respectively between three consecutive positions in said 3' near terminal region of the second strand, wherein a first phosphorothioate internucleoside linkage is present between said adjacent first basic nucleoside of (a) and an adjacent second basic nucleoside in said 3' near terminal region of the second strand, and a second phosphorothioate internucleoside linkage is present between said adjacent second basic nucleoside and an adj acent third basic nucleoside in said 3' near terminal region of the second strand; (iii) two phosphorothioate internucleoside linkages are respectively between three consecutive positions in both 5' and 3' terminal regions of the first strand, whereby a terminal nucleoside respectively at each of the 5' and 3' terminal regions of said first strand is each attached to a respective 5' and 3' adjacent penultimate nucleoside by a phosphorothioate internucleoside linkage, and each first 5' and 3' penultimate nucleoside is attached to a respective 5' and 3' adjacent antepenultimate nucleoside by a phosphorothioate internucleoside linkage; and (iv) the second strand of the nucleic acid is conjugated directly or indirectly to one or more ligand moieties at the 5' terminal region of the second strand.

Examples of the structures are as follows (where the specific RNA nucleosides shown are not limiting and could be any RNA nucleoside):
A A 3'-3' reversed bond (and also showing the 5'-3 direction of the last phosphodiester bond between the two abasic molecules reading towards the terminus of the molecule) 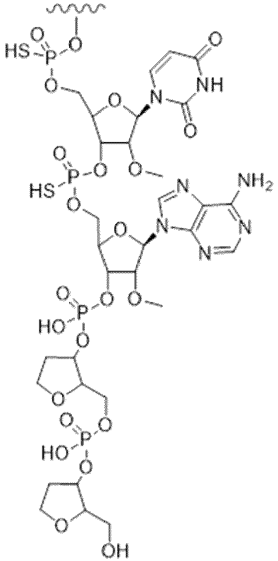
B Illustrating a 5'-5' reversed bond (and also showing the 3'-5' direction of the last phosphodiester bond between the two abasic molecules reading towards the terminus of the molecule)

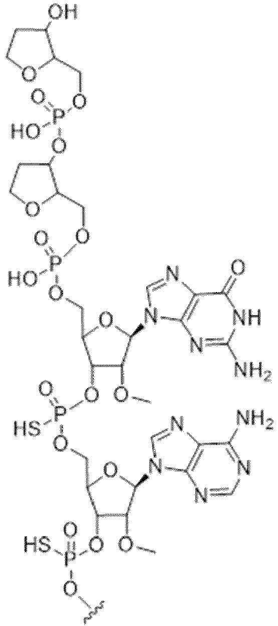

The abasic nucleoside or abasic nucleosides present in the nucleic acid are provided in the presence of a reversed internucleoside linkage or linkages, namely a 5'-5' or a 3 '-3' reversed internucleoside linkage. A reversed linkage occurs as a result of a change of orientation of an adjacent nucleoside sugar, such that the sugar will have a 3' - 5' orientation as opposed to the conventional 5' - 3' orientation (with reference to the numbering of ring atoms on the nucleoside sugars). The abasic nucleoside or nucleosides as present in the nucleic acids of the invention preferably include such inverted nucleoside sugars.

In the case of a terminal nucleoside having an inverted orientation, then this will result in an "inverted" end configuration for the overall nucleic acid. Whilst certain structures drawn and referenced herein are represented using conventional 5' - 3' direction (with reference to the numbering of ring atoms on the nucleoside sugars), it will be appreciated that the presence of a terminal nucleoside having a change of orientation and a proximal 3'-3' reversed linkage, will result in a nucleic acid having an overall 5'- 5' end structure (i.e. the conventional 3' end nucleoside becomes a 5' end nucleoside). Alternatively, it will be appreciated that the presence of a terminal nucleoside having a change of orientation and a proximal 5'-5' reversed linkage will result in a nucleic acid with an overall 3'- 3' end structure.

The proximal 3'-3' or 5'-5' reversed linkage as herein described, may comprise the reversed linkage being directly adjacent / attached to a terminal nucleoside having an inverted orientation, such as a single terminal nucleoside having an inverted orientation. Alternatively, the proximal 3'-3' or 5'-5' reversed linkage as herein described, may comprise the reversed linkage being adjacent 2, or more than 2, nucleosides having an inverted orientation, such as 2, or more than 2, terminal region nucleosides having an inverted orientation, such as the terminal and penultimate nucleosides. In this way, the reversed linkage may be attached to a penultimate nucleoside having an inverted orientation. While a skilled addressee will appreciate that inverted orientations as described above can result in nucleic acid molecules having overall 3' - 3' or 5'- 5' end structures as described herein, it will also be appreciated that with the presence of one or more additional reversed linkages and / or nucleosides having an inverted orientation, then the overall nucleic acid may have 3' - 5' end structures corresponding to the conventionally positioned 5' / 3' ends.

In one aspect the nucleic acid may have a 3'-3' reversed linkage, and the terminal sugar moiety may comprise a 5' OH rather than a 5' phosphate group at the 5' position of that terminal sugar.

A skilled person would therefore clearly understand that 5'-5', 3'-3' and 3'-5' (reading in the direction of that terminus) end variants of the more conventional 5'-3' structures (with reference to the numbering of ring atoms on the end nucleoside sugars) drawn herein are included in the scope of the disclosure, where a reversed linkage or linkages is / are present.

In the situation of e.g. a reversed internucleoside linkage and / or one or more nucleosides having an inverted orientation creating an inverted end, and where the relative position of a linkage (e.g. to a linker) or the location of an internal feature (such as a modified nucleoside) is defined relative to the 5' or 3' end of the nucleic acid, then the 5' or 3' end is the conventional 5' or 3' end which would have existed had a reversed linkage not been in place, and wherein the conventional 5' or 3' end is determined by consideration of the directionality of the majority of the internal nucleoside linkages and / or nucleoside orientation within the nucleic acid. It is possible to tell from these internal bonds and / or nucleoside orientation which ends of the nucleic acid would constitute the conventional 5' and 3' ends (with reference to the numbering of ring atoms on the end nucleoside sugars) of the molecule absent the reversed linkage.

For example, in the structure shown below there are abasic residues in the first 2 positions located at the "5'" end. Where the terminal nucleoside has an inverted orientation then the "5"' end indicated in the diagram below, which is the conventional 5' end, can in fact comprise a 3' OH in view of the inverted nucleoside at the terminal position. Nevertheless the majority of the molecule will comprise conventional internucleoside linkages that run from the 3' OH of the sugar to the 5' phosphate of the next sugar, when reading in the standard 5' [P04] to 3' [OH] direction of a nucleic acid molecule (with reference to the numbering of ring atoms on the nucleoside sugars), which can be used to determine the conventional 5' and 3' ends that would be found absent the inverted end configuration.
A 5' A-A-Me-Me-Me-Me-Me-Me-F-Me-F-F-F-Me-Me-Me-Me-Me-Me-Me-Me-Me-Me 3'

The reversed bond is preferably located at the end of the nucleic acid e.g. RNA which is distal to a ligand moiety, such as a GalNAc containing portion, of the molecule.

GalNAc-siRNA constructs with a 5'-GalNAc on the sense strand can have a reversed linkage on the opposite end of the sense strand.

GalNAc-siRNA constructs with a 3'-GalNAc on the sense strand can have a reversed linkage on the opposite end of the sense strand.

### NUCLEIC ACID LENGTHS

In one aspect the i) the first strand of the nucleic acid has a length in the range of 17 to 30 nucleosides, preferably 19 to 25 nucleosides, more preferably 19 or 23 nucleosides; and / or ii) the second strand of the nucleic acid has a length in the range of 17 to 30 nucleosides, preferably 19 to 25 nucleosides, more preferably 19 or 21 nucleosides.

Typically the duplex region of the nucleic acid is between 17 and 30 nucleosides in length, more preferably is 19 or 21 nucleosides in length. Similarly, the region of complementarity between the first strand and the portion of RNA transcribed from the *SLC25A5* gene is between 17 and 30 nucleosides in length. Generally, the duplex structure of the nucleic acid e.g. an iRNA is about 15 to 30 base pairs in length, e.g., 15-29, 15-28, 15-27, 15-26, 15-25, 15-24, 15-23, 15-22, 15-21, 15-20, 15-19, 15-18, 15-17, 18-30, 18-29, 18-28, 18-27, 18-26, 18-25, 18-24, 18-23, 18-22, 18-21, 18-20, 19-30, 19-29, 19-28, 19-27, 19-26, 19-25, 19-24, 19- 23, 19-22, 19-21, 19-20, 20-30, 20-29, 20-28, 20-27, 20-26, 20-25, 20-24,20-23, 20-22, 20-21, 21-30, 21-29, 21-28, 21-27, 21-26, 21-25, 21-24, 21-23, or 21-22 base pairs in length. Ranges and lengths intermediate to the above recited ranges and lengths are also contemplated to be part of the invention.

Similarly, the region of complementarity of an antisense sequence to a target sequence and/or the region of complementarity of an antisense sequence to a sense sequence is about 15 to 30 nucleosides in length, e.g., 15-29, 15-28, 15-27, 15-26, 15-25, 15-24, 15-23, 15-22, 15-21, 15-20, 15-19, 15-18, 15-17, 18-30, 18-29, 18-28, 18-27, 18-26, 18-25, 18-24, 18-23, 18-22, 18-21, 18-20, 19-30, 19-29, 19-28, 19-27, 19-26, 19-25, 19-24, 19-23, 19-22, 19-21, 19-20, 20-30, 20-29, 20-28, 20-27, 20-26, 20-25, 20- 24,20-23, 20-22, 20-21, 21-30, 21-29, 21-28, 21-27, 21-26, 21-25, 21-24, 21-23, or 21-22 nucleosides in length. Ranges and lengths intermediate to the above recited ranges and lengths are also contemplated to be part of the invention.

In certain preferred embodiments, the region of complementarity of an antisense sequence to a target sequence and/or the region of complementarity of an antisense sequence to a sense sequence is at least 17 nucleosides in length. For example, the region of complementarity between the antisense strand and the target is 19 to 21 nucleosides in length, for example, the region of complementarity is 21 nucleosides in length.

In preferred embodiments, each strand is no more than 30 nucleosides in length.

In certain preferred embodiments, the duplex structure of the nucleic acid e.g. an siRNA is 19 or 21 base pairs in length.

A nucleic acid e.g. a dsRNA as described herein can further include one or more single-stranded nucleoside overhangs e.g., 1-4, 2-4, 1-3, 2-3, 1, 2, 3, or 4 nucleosides. A nucleoside overhang can comprise or consist of a nucleoside/nucleoside analog, including a deoxynucleoside/nucleoside. The overhang(s) can be on the sense strand, the antisense strand, or any combination thereof. Furthermore, the nucleoside(s) of an overhang can be present on the 5'-end, 3'- end, or both ends of an antisense or sense strand of a nucleic acid e.g. a dsRNA.

In certain preferred embodiments, at least one strand comprises a 3' overhang of at least 1 nucleoside, e.g. , at least one strand comprises a 3' overhang of at least 2 nucleosides. The overhang is suitably on the antisense/ guide strand and/or the sense / passenger strand.

### NUCLEIC ACID MODIFICATIONS

In certain embodiments, the nucleic acid e.g. an RNA of the invention e.g., a dsiRNA, does not comprise further modifications, e.g., chemical modifications or conjugations known in the art and described herein.

In other preferred embodiments, the nucleic acid e.g. RNA of the invention, e.g., a dsiRNA, is further chemically modified to enhance stability or other beneficial characteristics.

In certain embodiments of the invention, substantially all of the nucleosides are modified.

The nucleic acids featured in the invention can be synthesized or modified by methods well established in the art, such as those described in "Current protocols in nucleic acid chemistry," Beaucage, S.L. et al. (Edrs.), John Wiley & Sons, Inc., New York, NY, USA, which is hereby incorporated herein by reference.

Modifications include, for example, end modifications, e.g., 5'-end modifications (phosphorylation, conjugation, inverted linkages) or 3 '-end modifications (conjugation, DNA nucleosides within an RNA, or RNA nucleosides within a DNA, inverted linkages, etc.); base modifications, e.g., replacement with stabilizing bases, destabilizing bases, or bases that base pair with an expanded repertoire of partners, conjugated bases; sugar modifications (e.g. , at the 2'-position or 4'- position) or replacement of the sugar; or backbone modifications, including modification or replacement of the phosphodiester linkages.

Specific examples of nucleic acids such as siRNA compounds useful in the embodiments described herein include, but are not limited to RNAs containing modified backbones or no natural internucleoside linkages. Nucleic acids such as RNAs having modified backbones include, among others, those that do not have a phosphorus atom in the backbone. For the purposes of this specification, and as sometimes referenced in the art, modified nucleic acids e.g., RNAs that do not have a phosphorus atom in their internucleoside backbone can also be considered to be oligonucleosides. In some embodiments, a modified nucleic acid e.g., an siRNA will have a phosphorus atom in its internucleoside backbone.

Modified nucleic acid e.g. RNA backbones include, for example, phosphorothioates, chiral phosphorothioates, phosphorodithioates, phosphotriesters, aminoalkylphosphotriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates having normal 3'-5' linkages, 2'-5'-linked analogs of these, and those having inverted polarity wherein the adjacent pairs of nucleoside units are linked 5'-3' or 5'-2'. Various salts, mixed salts and free acid forms are also included.

Modified nucleic acids e.g. RNAs can also contain one or more substituted sugar moieties. The nucleic acids e.g. siRNAs, e.g., dsiRNAs, featured herein can include one of the following at the 2'-position: OH; F; O-, S-, or N-alkyl; O-, S-, or N-alkenyl; O-, S- or N-alkynyl; or O-alkyl-O-alkyl, wherein the alkyl, alkenyl and alkynyl can be substituted or unsubstituted. 2' O- methyl and 2' -F are preferred modifications.

In certain preferred embodiments, the nucleic acid comprises at least one modified nucleoside.

The nucleic acid of the invention may comprise one or more modified nucleosides on the first strand and/or the second strand.

In some embodiments, substantially all of the nucleosides of the sense strand and all of the nucleosides of the antisense strand comprise a modification.

In some embodiments, all of the nucleosides of the sense strand and substantially all of the nucleosides of the antisense strand comprise a modification.

In some embodiments, all of the nucleosides of the sense strand and all of the nucleosides of the antisense strand comprise a modification.

In one embodiment, at least one of the modified nucleosides is selected from the group consisting of a deoxy- nucleoside, a 3 '-terminal deoxy-thymine (dT) nucleoside, a 2'-O-methyl modified nucleoside (also called herein 2'-Me, where Me is a methoxy), a 2'-fluoro modified nucleoside, a 2'-deoxy- modified nucleoside, a locked nucleoside, an unlocked nucleoside, a conformationally restricted nucleoside, a constrained ethyl nucleoside, an abasic nucleoside, a 2' -amino- modified nucleoside, a 2'- O-allyl- modified nucleoside, 2' -C-alkyl- modified nucleoside, 2'-hydroxly-modified nucleoside, a 2'- methoxyethyl modified nucleoside, a 2'-O-alkyl-modified nucleoside, a morpholino nucleoside, a phosphoramidate, a non-natural base comprising nucleoside, a tetrahydropyran modified nucleoside, a 1 ,5-anhydrohexitol modified nucleoside, a cyclohexenyl modified nucleoside, a nucleoside comprising a phosphorothioate group, a nucleoside comprising a methylphosphonate group, a nucleoside comprising a 5 '-phosphate, and a nucleoside comprising a 5 '-phosphate mimic. In another embodiment, the modified nucleosides comprise a short sequence of 3 '-terminal deoxy-thymine nucleosides (dT).

Modifications on the nucleosides may preferably be selected from the group including, but not limited to, LNA, HNA, CeNA, 2 -methoxyethyl, 2'-O-alkyl, 2 -O-allyl, 2'-C-allyl, 2'-fluoro, 2'-deoxy, 2'- hydroxyl, and combinations thereof. In another embodiment, the modifications on the nucleosides are 2-O-methyl ("2-Me") or 2'-fluoro modifications.

One preferred modification is a modification at the 2'-OH group of the ribose sugar, optionally selected from 2'-Me or 2'-F modifications.

In certain embodiments, the nucleic acid e.g., siRNA agent further comprises at least one phosphorothioate or methylphosphonate internucleoside linkage. For example the phosphorothioate or methylphosphonate internucleoside linkage can be at the 3 '-terminus or in the terminal region of one strand, i.e., the sense strand or the antisense strand; or at the ends of both strands, the sense strand and the antisense strand.

In certain embodiments, the phosphorothioate or methylphosphonate internucleoside linkage is at the 5 'terminus or in the terminal region of one strand, i.e., the sense strand or the antisense strand; or at the ends of both strands, the sense strand and the antisense strand.

In certain embodiments, a phosphorothioate or a methylphosphonate internucleoside linkage is at both the 5'- and 3 '-terminus or in the terminal region of one strand, i.e., the sense strand or the antisense strand; or at the ends of both strands, the sense strand and the antisense strand.

Any nucleic acid may comprise one or more phosphorothioate (PS) modifications within the nucleic acid, such as at least two PS internucleoside bonds at the ends of a strand.

At least one of the oligoribonucleoside strands preferably comprises at least two consecutive phosphorothioate modifications in the last 3 nucleosides of the oligonucleoside.

The invention therefore also relates to: A nucleic acid disclosed herein which comprises phosphorothioate internucleoside linkages respectively between at least two or three consecutive positions, such as in a 5' and/or 3' terminal region and/or near terminal region of the second strand, whereby said near terminal region is preferably adjacent said terminal region wherein said one or more abasic nucleosides of said second strand is / are located.

A nucleic acid disclosed herein which comprises phosphorothioate internucleoside linkages respectively between at least two or three consecutive positions in a 5' and / or 3' terminal region of the first strand, whereby preferably the terminal position at the 5' and / or 3' terminal region of said first strand is attached to its adjacent position by a phosphorothioate internucleoside linkage.

The nucleic acid strand may be an RNA comprising a phosphorothioate internucleoside linkage between the three nucleosides contiguous with 2 terminally located abasic nucleosides.

A preferred nucleic acid is a double stranded RNA comprising 2 adjacent abasic nucleosides at the 5' terminus of the second strand and a ligand moiety comprising one or more GalNAc ligand moieties at the opposite 3' end of the second strand. Further preferred, the same nucleic acid may also comprise a phosphorothioate bond between nucelotides at positions 3-4 and 4-5 of the second strand, reading from the position 1 of the second strand.

Position 1 of the first or the second strand is the nucleoside which is the closest to the end of the nucleic acid (ignoring any abasic nucleosides) and that is joined to an adjacent nucleoside (at Position 2) via a 3' to 5' internal bond, with reference to the bonds between the sugar moieties of the backbone, and reading in a direction away from that end of the molecule.

It can therefore be seen that "position 1 of the sense strand" is the 5' most nucleoside (not including abasic nucleosides) at the conventional 5' end of the sense strand. Typically, the nucleoside at this position 1 of the sense strand will be equivalent to the 5' nucleoside of the selected target nucleic acid sequence, and more generally the sense strand will have equivalent nucleosides to those of the target nucleic acid sequence starting from this position 1 of the sense strand, whilst also allowing for acceptable mismatches between the sequences.

As used herein, "position 1 of the antisense strand" is the 5' most nucleoside (not including abasic nucleosides) at the conventional 5' end of the antisense strand. As hereinbefore described, there will be a region of complementarity between the sense and antisense strands, and in this way the antisense strand will also have a region of complementarity to the target nucleic acid sequence as referred to above.

Preferred modifications that can be used with sequences according to the present invention can be as follows:
Modification 1:
   First strand modification:
      NmsNfsNmNfNmNfNmNfNfNmNmNmNmNfNmNfNmNmNmNmNmsNmsNm (5' to 3')
   Second strand modification:
      iaiaNmsNmsNmNmNmNmNfNfNfNfNfNmNmNmNmNmNmNmNfNmNm (5' to 3')
Modification 2:
   First strand modification:
      NmsNfsNmNfNmNfNmNfNfNmNmNmNmNfNmNfNmNmNmNmNmsNmsNm (5' to 3')
   Second strand modification:
      iaiaNmsNmsNmNmNmNfNfNmNfNfNfNfNmNmNmNmNmNmNmNmNm (5' to 3')
Modification 3:
   First strand modification:
      NmsNfsNmNfNmNfNmNfNfNmNmNmNmNfNmNfNmNmNmNmNmsNmsNm (5' to 3')
   Second strand modification:
      iaiaNmsNmsNmNmNmNmNfNmNfNfNfNfNmNmNmNmNmNmNmNmNm (5' to 3')
Modification 4:
   First strand modification:
      NmsNfsNmNfNmNfNmNmNmNmNmNmNmNfNmNfNmNmNmNmNmsNmsNm (5' to 3')
   Second strand modification:
      iaiaNmsNmsNmNmNmNmNfNmNfNfNfNfNmNmNmNmNmNmNmNmNm (5' to 3')
Modification 5:
   First strand modification:
      NmsNfsNmNmNmNfNmNmNfNmNmNmNmNfNmNfNmNmNmNmNmsNmsNm (5' to 3')
   Second strand modification:
      iaiaNmsNmsNmNmNmNmNmNmNfNfNfNmNmNmNmNmNmNmNmNmNm (5' to 3')
Modification 6:
   First strand modification:
      NmsNfsNmNmNmNfNmNmNmNmNmNmNmNfNmNfNmNfNmNmNmsNmsNm (5' to 3')
   Second strand modification:
      iaiaNmsNmsNmNmNmNmNmNmNfNfNfNmNmNmNmNmNmNmNmNmNm (5' to 3')
Modification 7:
   First strand modification:
      NmsNfsNmNmNmNyNmNmNmNmNmNmNmNfNmNfNmNmNmNmNmsNmsNm (5' to 3')
   Second strand modification:
      iaiaNmsNmsNmNmNmNmNmNmNfNfNfNmNmNmNmNmNmNmNmNmNm (5' to 3')
Modification 8:
   First strand modification:
      NmsNfsNmNmNmNyNmNfNfNmNmNmNmNfNmNfNmNmNmNmNmsNmsNm (5' to 3')
   Second strand modification:
      iaiaNmsNmsNmNmNmNmNmNmNfNfNfNmNmNmNmNmNmNmNmNmNm (5' to 3')
Modification 9:
   First strand modification:
      NmsNfsNmNmNmNfNmNmNmNmNmNmNmNfNmNfNmNmNmNfNmsNmsNm (5' to 3')
   Second strand modification:
      iaiaNmsNmsNmNmNmNmNmNmNfNfNfNmNmNmNmNmNmNmNmNmNm (5' to 3')
Modification 10:
   First strand modification:
      NmsNfsNmNfNmNfNmNmNmNmNmNmNmNfNmNfNmNmNmNmNmsNmsNm (5' to 3')
   Second strand modification:
      iaiaNmsNmsNmNmNmNmNmNmNfNfNfNmNmNmNmNmNmNmNmNmNm (5' to 3')
Modification 11:
   First strand modification:
      NmsNfsNmNfNmNfNmNfNfNmNmNmNmNfNmNfNmNmNmNmNmsNmsNm (5' to 3')
   Second strand modification:
      iaiaNmsNmsNmNmNmNmNmNmNfNfNfNmNmNmNmNmNmNmNmNmNm (5' to 3')
Modification 12:
   First strand modification:
      NmsNfsNmNmNmNfNmNfNfNmNmNmNmNfNmNfNmNfNmNmNmsNmsNm (5' to 3')
   Second strand modification:
      iaiaNmsNmsNmNmNmNmNmNmNfNfNfNmNmNmNmNmNmNmNmNmNm (5' to 3')
Modification 13:
   First strand modification:
      NmsNfsNmNmNmNfNmNfNfNmNmNmNmNfNmNfNmNmNmNfNmsNmsNm (5' to 3')
   Second strand modification:
      iaiaNmsNmsNmNmNmNmNmNmNfNfNfNmNmNmNmNmNmNmNmNmNm (5' to 3')
   wherein in each of the above modifications:
      ia represents an inverted abasic nucleoside;
      Nm represents a 2'Me ribose modified nucleoside;
      Nf represents a 2'F ribose modified nucleoside;
      Ny represents a nucleoside with a thermally destabilizing modification, preferably wherein the destabilizing modification is selected from a modified unlocked nucleic acid (UNA) and a glycol nucleic acid (GNA), more preferably a glycol nucleic acid, most preferably an (S)-glycol nucleic acid;
      s represents a phosphorothioate internucleoside bond.

A particularly preferred modification that can be used with sequences according to the present invention can be:
Modification 6:
   First strand modification:
      NmsNfsNmNmNmNfNmNmNmNmNmNmNmNfNmNfNmNfNmNmNmsNmsNm (5' to 3')
   Second strand modification:
      iaiaNmsNmsNmNmNmNmNmNmNfNfNfNmNmNmNmNmNmNmNmNmNm (5' to 3')
   wherein in each of the above modifications:
      ia represents an inverted abasic nucleoside;
      Nm represents a 2'Me ribose modified nucleoside;
      Nf represents a 2'F ribose modified nucleoside; and
      s represents a phosphorothioate internucleoside bond.

### CONJUGATION OF NUCLEIC ACID TO LIGAND

Another modification of a nucleic acid e.g. RNA e.g. an siRNA of the invention involves linking the nucleic acid e.g. the siRNA to one or more ligand moieties e.g. to enhance the activity, cellular distribution, or cellular uptake of the nucleic acid e.g. siRNA e.g., into a cell.

In some embodiments, the ligand moiety described can be attached to a nucleic acid e.g. an siRNA oligonucleoside, via a linker that can be cleavable or non-cleavable. The term "linker" or "linking group" means an organic moiety that connects two parts of a compound, e.g., covalently attaches two parts of a compound.

The ligand can be attached to the 3' or 5' end of the sense strand.

The ligand is preferably conjugated to 3' end of the sense strand of the nucleic acid e.g. an siRNA agent.

The invention therefore relates in a further aspect to a conjugate for inhibiting expression of a target e.g. a target gene, in a cell, said conjugate comprising a nucleic acid portion and one or more ligand moieties, said nucleic acid portion comprising a nucleic acid as disclosed herein.

In one aspect the second strand of the nucleic acid is conjugated directly or indirectly (e.g. via a linker) to the one or more ligand moiety(s), wherein said ligand moiety is typically present at a terminal region of the second strand, preferably at the 3' terminal region thereof.

In certain embodiments, the ligand moiety comprises a GalNAc or GalNAc derivative attached to the nucleic acid e.g. dsiRNA through a linker.

Therefore, the invention relates to a conjugate wherein the ligand moiety comprises
i) one or more GalNAc ligands; and/or
ii) one or more GalNAc ligand derivatives; and/or
iii) one or more GalNAc ligands conjugated to said nucleic acid through a linker.

Said GalNAc ligand may be conjugated directly or indirectly to the 5' or 3' terminal region of the second strand of the nucleic acid, preferably at the 3' terminal region thereof.

GalNAc ligands are well known in the art and described in, inter alia, EP3775207A1.

In some embodiments, the ligand moiety comprises one or more ligands.

In some embodiments, the ligand moiety comprises one or more carbohydrate ligands.

In some embodiments, the one or more carbohydrates can be a monosaccharide, disaccharide, trisaccharide, tetrasaccharide, oligosaccharide and / or polysaccharide.

In some embodiments, the one or more carbohydrates comprise one or more galactose moieties, one or more lactose moieties, one or more N-AcetylGalactosamine moieties, and / or one or more mannose moieties.

In some embodiments, the one or more carbohydrates comprise one or more N-Acetyl-Galactosamine moieties.

In some embodiments, the compounds as described anywhere herein comprise two or three N-AcetylGalactosamine moieties.

In some embodiments, the one or more ligands are attached in a linear configuration, or in a branched configuration, for example each configuration being respectively attached to a branch point in an overall linker.

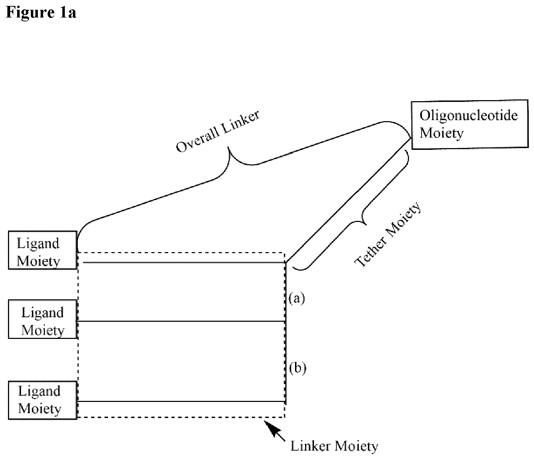

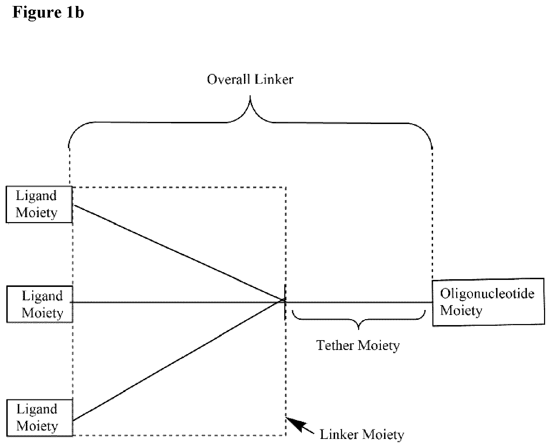

Exemplary linear configurations and Exemplary branched configurations are shown in Figures 1a and 1b:
In Fig 1a, (linear), (a) and / or (b) can typically represent connecting bonds or groups, such as phosphate or phosphorothioate groups.

In Fig 1b, (branched), in some embodiments, the one or more ligands are attached as a biantennary or triantennary branched configuration. Typically, a triantennary branched configuration can be preferred, such as an N-AcetylGalactosamine triantennary branched configuration.

### LINKER

Exemplary compounds of the invention comprise a `linker moiety', such as that as depicted in Formula (I), that is part of an overall 'linker'. 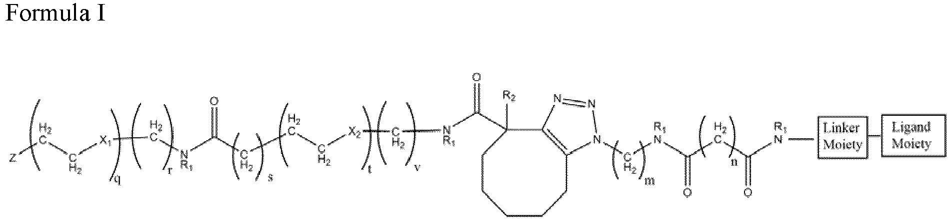 wherein:
R₁ at each occurrence is independently selected from the group consisting of hydrogen, methyl and ethyl;
R₂ is selected from the group consisting of hydrogen, hydroxy, -OC₁₋₃alkyl, -C(=O)OC₁₋₃alkyl, halo and nitro;
X₁ and X₂ at each occurrence are independently selected from the group consisting of methylene, oxygen and sulfur;
m is an integer of from 1 to 6;
n is an integer of from 1 to 10;
q, r, s, t, v are independently integers from 0 to 4, with the proviso that:
   (i) q and r cannot both be 0 at the same time; and
   (ii) s, t and v cannot all be 0 at the same time;
Z is an oligonucleoside moiety.

As will be further understood in the art, exemplary compounds of the invention comprise an overall linker that is located between the oligonucleoside moiety and the ligand moiety of these compounds. The overall linker, thereby 'links' the oligonucleoside moiety and the ligand moiety to each other.

The overall linker is often notionally envisaged as comprising one or more linker building blocks. For example, there is a linker portion that is depicted as the `linker moiety' as represented in Formula (I) positioned adjacent the ligand moiety and attaching the ligand moiety, typically via a branch point, directly or indirectly to the oligonucleoside moiety. The linker moiety as depicted in Formula (I) can also often be referred to as the `ligand arm or arms' of the overall linker. There can also, but not always, be a further linker portion between the oligonucleoside moiety and the branch point, that is often referred to as the `tether moiety' of the overall linker, `tethering' the oligonucleoside moiety to the remainder of the conjugated compound. Such `ligand arms' and / or `linker moieties' and / or `tether moieties' can be envisaged by reference to the linear and / or branched configurations as set out above.

As can be seen from the claims, and the reminder of the patent specification, the scope of the present invention extends to linear or branched configurations, and with no limitation as to the number of individual ligands that might be present. Furthermore, the addressee will also be aware that there are many structures that could be used as the linker moiety, based on the state of the art and the expertise of an oligonucleoside chemist.

The remainder of the overall linker (other than the linker moiety) as set out in the claims, and the remainder of the patent specification, is shown by its chemical constituents in Formula (I), which the inventors consider to be particularly unique to the current invention. In more general terms, however, these chemical constituents could be described as a `tether moiety' as hereinbefore described, wherein the `tether moiety' is that portion of the overall linker which comprises the group of atoms between Z, namely the oligonucleoside moiety, and the linker moiety as depicted in Formula (I).

### Tether moiety of Formula I

In relation to Formula (I), the `tether moiety' comprises the group of atoms between Z, namely the oligonucleoside moiety, and the linker moiety.

In some embodiments, R₁ is hydrogen at each occurrence. In some embodiments, R₁ is methyl. In some embodiments, R₁ is ethyl.

In some embodiments, R₂ is hydroxy. In some embodiments, R₂ is halo. In some embodiments, R₂ is fluoro. In some embodiments, R₂ is chloro. In some embodiments, R₂ is bromo. In some embodiments, R₂ is iodo. In some embodiments, R₂ is nitro.

In some embodiments, X₁ is methylene. In some embodiments, X₁ is oxygen. In some embodiments, X₁ is sulfur.

In some embodiments, X₂ is methylene. In some embodiments, X₂ is oxygen. In some embodiments, X₂ is sulfur.

In some embodiments, m = 3.

In some embodiments, n = 6.

In some embodiments, X₁ is oxygen and X₂ is methylene. In some embodiments, both X₁ and X₂ are methylene.

In some embodiments, q = 1, r = 2, s = 1, t = 1, v = 1. In some embodiments, q = 1, r = 3, s = 1, t = 1, v = 1.

In some embodiments, R₁ is hydrogen at each occurrence, n = 6, m = 3, R₂ is fluoro, X₂ is methylene, v = 1, t = 1, s = 1, X₁ is methylene, q = 1 and r = 2.

Thus, in some embodiments, exemplary compounds of the invention comprise the following structure:

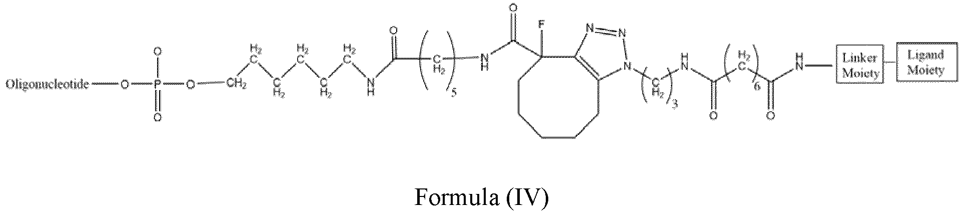

In some embodiments, R₁ is hydrogen at each occurrence, n = 6, m = 3, R₂ is fluoro, X₂ is methylene, v = 1, t = 1, s = 1, X₁ is oxygen, q = 1 and r = 2.

Thus, in some embodiments, exemplary compounds of the invention comprise the following structure:

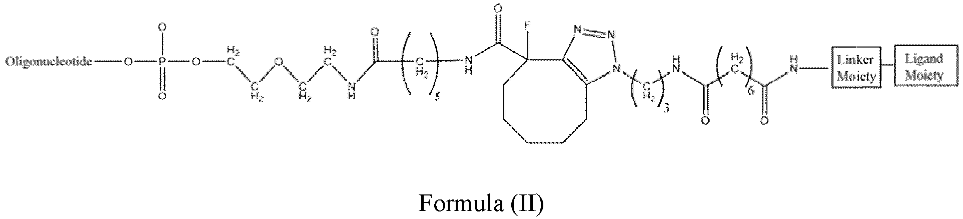

### Alternative tether moieties

During the synthesis of compounds of the present invention, alternative tether moiety structures may arise. In some embodiments, alternative tether moieties have a change of one or more atoms in the tether moiety of the overall linker compared to tether moieties described anywhere herein.

In some embodiments, the alternative tether moiety is a compound of Formula (I) as described anywhere herein, wherein R₂ is hydroxy.

In some embodiments, R₁ is hydrogen at each occurrence, n = 6, m = 3, R₂ is hydroxy, X₂ is methylene, v = 1, t = 1, s = 1, X₁ is methylene, q = 1 and r = 2.

Thus, in some embodiments, compounds of the invention comprise the following structure:

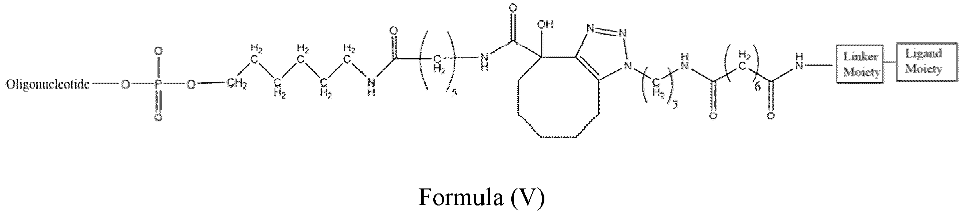

In some embodiments, R₁ is hydrogen at each occurrence, n = 6, m = 3, R₂ is hydroxy, X₂ is methylene, v = 1, t = 1, s = 1, X₁ is oxygen, q = 1 and r = 2.

Thus, in some embodiments, compounds of the invention comprise the following structure:

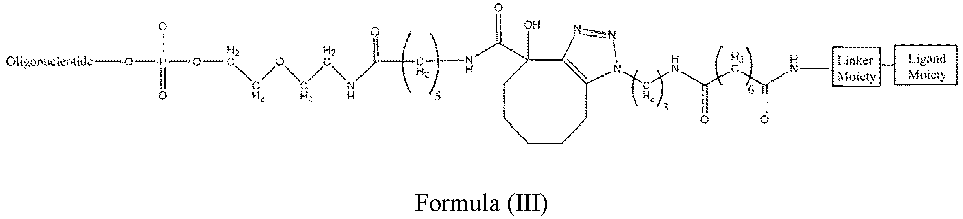

### Linker moiety

In relation to Formula (I), the `linker moiety' as depicted in Formula (I) comprises the group of atoms located between the tether moiety as described anywhere herein, and the ligand moiety as described anywhere herein.

In some embodiments: 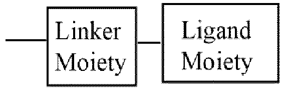 as depicted in Formula (I) as described anywhere herein is any of Formulae (VIa), (VIb) or (VIc), preferably Formula (VIa): 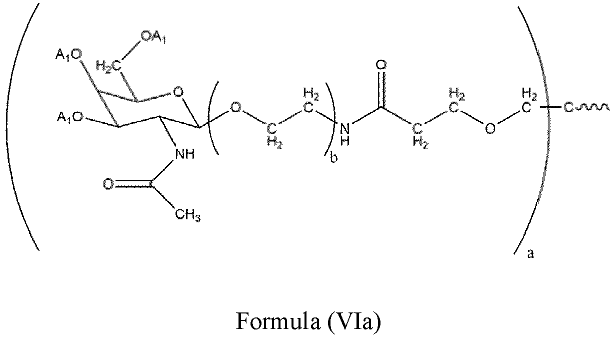 wherein:
A_{I} is hydrogen, or a suitable hydroxy protecting group;
a is an integer of 2 or 3; and
b is an integer of 2 to 5; or 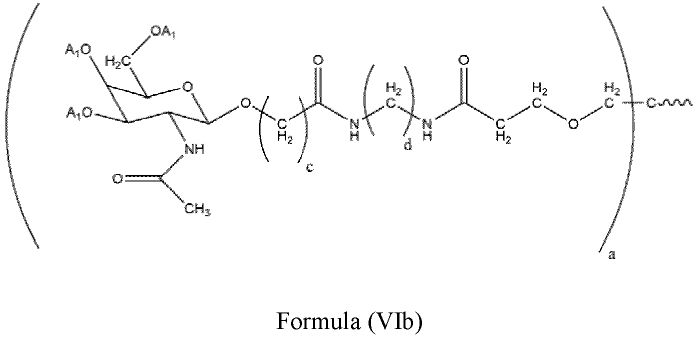
wherein:
   A_{I} is hydrogen, or a suitable hydroxy protecting group;
   a is an integer of 2 or 3; and
   c and d are independently integers of 1 to 6; or 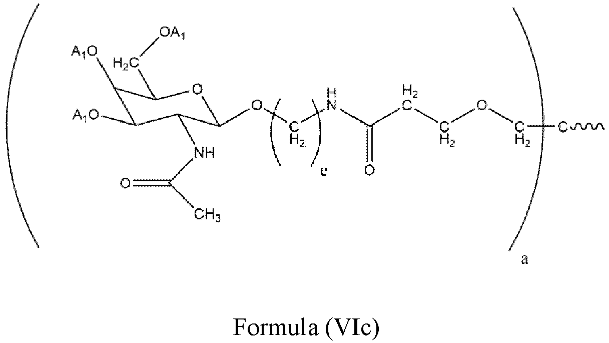
   wherein:
      A_{I} is hydrogen, or a suitable hydroxy protecting group;
      a is an integer of 2 or 3; and
      e is an integer of 2 to 10.

In some embodiments, the moiety: 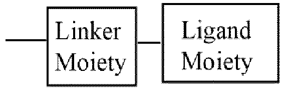 as depicted in Formula (I) is Formula (VIa): 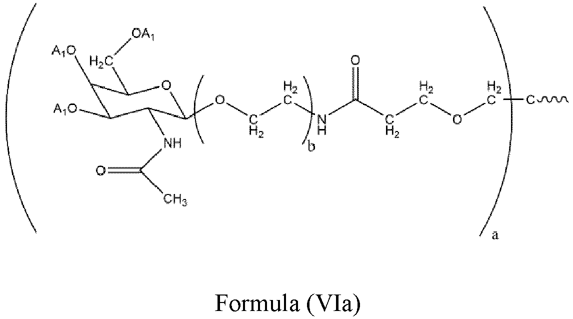 wherein:
A_{I} is hydrogen, or a suitable hydroxy protecting group;
a is 3; and
b is an integer of 3.

In some embodiments, the moiety: 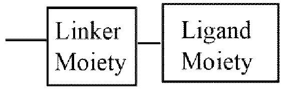 as depicted in Formula (I) as described anywhere herein is Formula (VII): 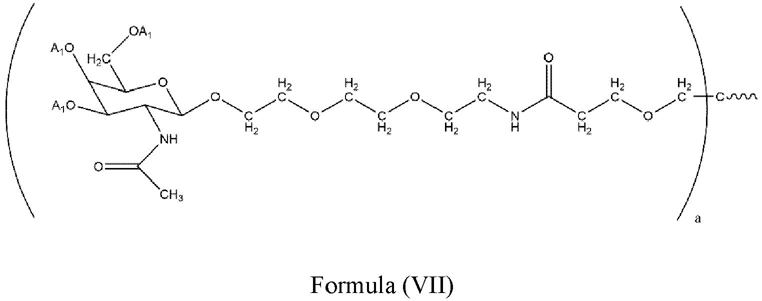 wherein:
A_{I} is hydrogen;
a is an integer of 2 or 3, preferably 3.

Other exemplary compounds of the invention comprise a `linker moiety', as depicted in Formula (I*), that is part of an overall 'linker'. 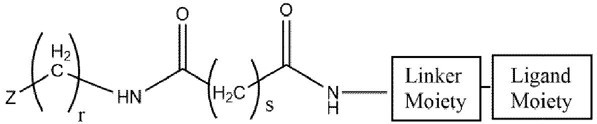 Formula I*

Where:
r and s are independently an integer selected from 1 to 16; and
Z is an oligonucleoside moiety.

As will be further understood in the art, exemplary compounds of the invention comprise an overall linker that is located between the oligonucleoside moiety and the ligand moiety of these compounds. The overall linker, thereby 'links' the oligonucleoside moiety and the ligand moiety to each other.

The overall linker is often notionally envisaged as comprising one or more linker building blocks. For example, there is a linker portion that is depicted as the `linker moiety' as represented in Formula (I*) positioned adjacent the ligand moiety and attaching the ligand moiety, typically via a branch point, directly or indirectly to the oligonucleoside moiety. The linker moiety as depicted in Formula (I*) can also often be referred to as the `ligand arm or arms' of the overall linker. There can also, but not always, be a further linker portion between the oligonucleoside moiety and the branch point, that is often referred to as the `tether moiety' of the overall linker, `tethering' the oligonucleoside moiety to the remainder of the conjugated compound. Such `ligand arms' and / or `linker moieties' and / or `tether moieties' can be envisaged by reference to the linear and / or branched configurations as set out above.

As can be seen from the claims, and the reminder of the patent specification, the scope of the present invention extends to linear or branched configurations, and with no limitation as to the number of individual ligands that might be present. Furthermore, the addressee will also be aware that there are many structures that could be used as the linker moiety, based on the state of the art and the expertise of an oligonucleoside chemist.

The remainder of the overall linker (other than the linker moiety) as set out in the claims, and the remainder of the patent specification, is shown by its chemical constituents in Formula (I), which the inventors consider to be particularly unique to the current invention. In more general terms, however, these chemical constituents could be described as a `tether moiety' as hereinbefore described, wherein the `tether moiety' is that portion of the overall linker which comprises the group of atoms between Z, namely the oligonucleoside moiety, and the linker moiety as depicted in Formula (I).

### Tether moiety

In relation to Formula (I*), the `tether moiety' comprises the group of atoms between Z, namely the oligonucleoside moiety, and the linker moiety.

In some embodiments, s is an integer selected from 4 to 12. In some embodiments, s is 6.

In some embodiments, r is an integer selected from 4 to 14. In some embodiments, r is 6. In some embodiments, r is 12.

In some embodiments, r is 12 and s is 6.

Thus, in some embodiments, exemplary compounds of the invention comprise the following structure:

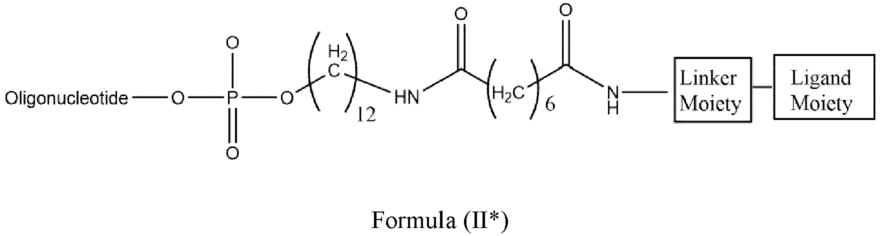

In some embodiments, r is 6 and s is 6.

Thus, in some embodiments, exemplary compounds of the invention comprise the following structure:

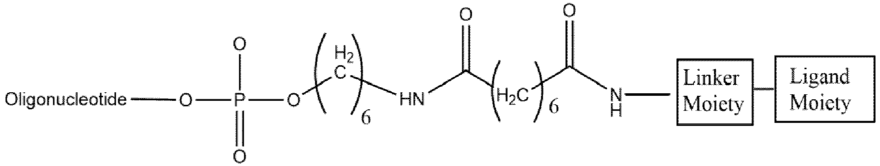

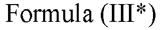

### Linker moiety

In relation to Formula (I*), the `linker moiety' as depicted in Formula (I) comprises the group of atoms located between the tether moiety as described anywhere herein, and the ligand moiety as described anywhere herein.

In some embodiments, the moiety: 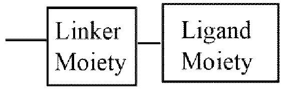 as depicted in Formula (I*) as described anywhere herein is any of Formulae (IV*), (V*) or (VI*), preferably Formula (IV*): 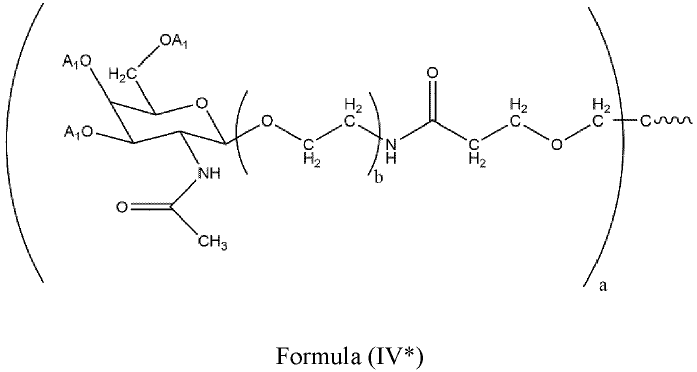 wherein:
A_{I} is hydrogen, or a suitable hydroxy protecting group;
a is an integer of 2 or 3; and
b is an integer of 2 to 5; or 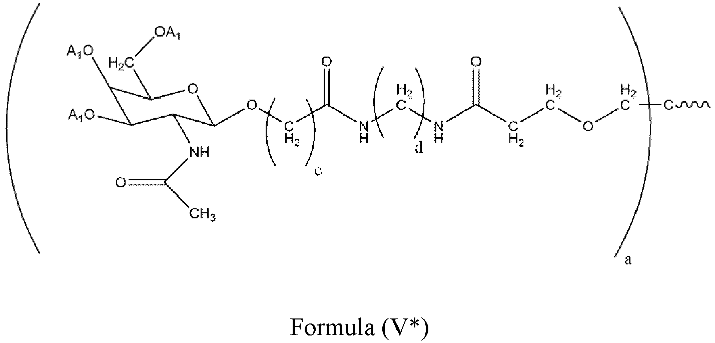
wherein:
   A_{I} is hydrogen, or a suitable hydroxy protecting group;
   a is an integer of 2 or 3; and
   c and d are independently integers of 1 to 6; or 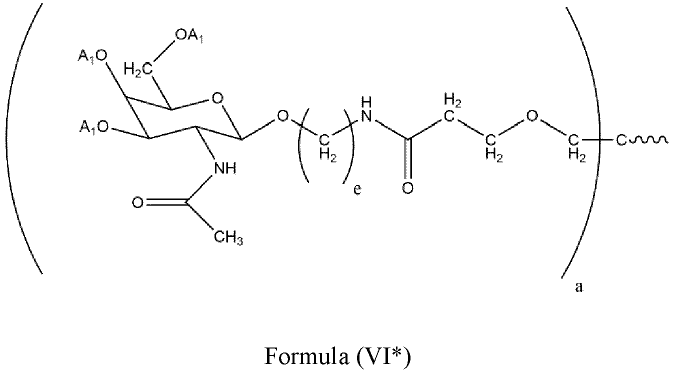
wherein:
   A_{I} is hydrogen, or a suitable hydroxy protecting group;
   a is an integer of 2 or 3; and
   e is an integer of 2 to 10.

In some embodiments, the moiety: 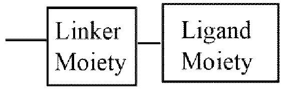 as depicted in Formula (I) is Formula (VIa*): 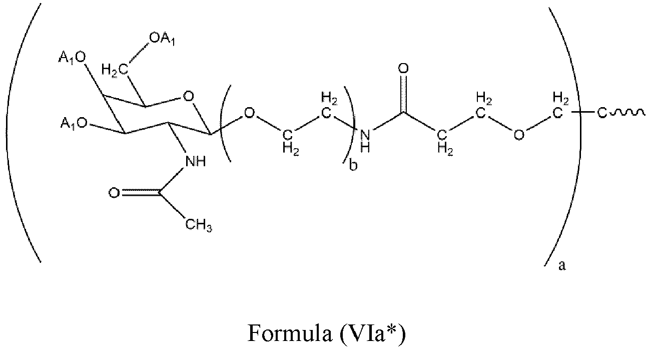 wherein:
A_{I} is hydrogen, or a suitable hydroxy protecting group;
a is 3; and
b is an integer of 3.

In some embodiments, the moiety: 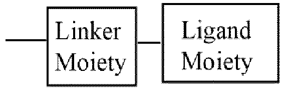 as depicted in Formula (I) as described anywhere herein is Formula (VII*): 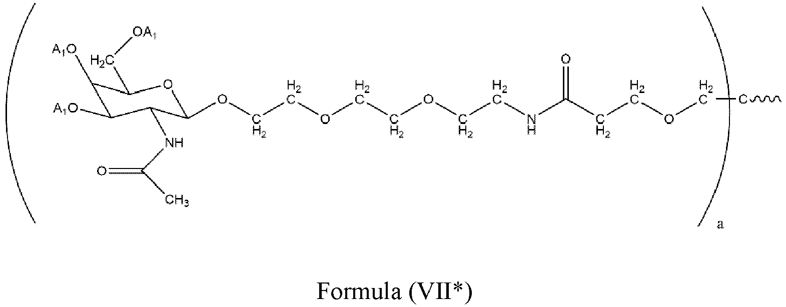 wherein:
A_{I} is hydrogen;
a is an integer of 2 or 3.

In some embodiments, a = 2. In some embodiments, a = 3. In some embodiments, b = 3.

### VECTOR AND CELL

In one aspect, the invention provides a cell containing a nucleic acid, such as inhibitory RNA [RNAi] as described herein.

In one aspect, the invention provides a cell comprising a vector as described herein.

In one aspect the invention provides a vector comprising an oligonucleotide inhibitor, e.g.an iRNA e.g. siRNA.

### PHARMACEUTICALLY ACCEPTABLE COMPOSITIONS

In one aspect, the invention provides a pharmaceutical composition for inhibiting expression of a target gene, the composition comprising an inhibitor such as an oligomer such as a nucleic acid as disclosed herein.

The pharmaceutically acceptable composition may comprise an excipient and or carrier.

Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) lubricating agents, such as magnesium stearate, sodium lauryl sulfate and talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen- free water; (17) isotonic saline; (18) Ringer's solution; (19) ethyl alcohol; (20) pH buffered solutions; (21) polyesters, polycarbonates and/or poly anhydrides; (22) bulking agents, such as polypeptides and amino acids (23) serum component, such as serum albumin, HDL and LDL; and (22) other non-toxic compatible substances employed in pharmaceutical formulations.

Typical pharmaceutical carriers include, but are not limited to, binding agents (e.g., pregelatinized maize starch, polyvinylpyrrolidone or hydroxypropyl methylcellulose, etc.); fillers (e.g., lactose and other sugars, microcrystalline cellulose, pectin, gelatin, calcium sulfate, ethyl cellulose, polyacrylates or calcium hydrogen phosphate, etc.); lubricants (e.g. , magnesium stearate, talc, silica, colloidal silicon dioxide, stearic acid, metallic stearates, hydrogenated vegetable oils, corn starch, polyethylene glycols, sodium benzoate, sodium acetate, etc.); disintegrants (e.g. , starch, sodium starch glycolate, etc.); and wetting agents (e.g., sodium lauryl sulphate, etc).

Pharmaceutically acceptable organic or inorganic excipients suitable for non-parenteral administration which do not deleteriously react with nucleic acids can also be used to formulate the compositions of the present invention. Suitable pharmaceutically acceptable excipients include, but are not limited to, water, salt solutions, alcohols, polyethylene glycols, gelatin, lactose, amylose, magnesium stearate, talc, silicic acid, viscous paraffin, hydroxymethylcellulose, polyvinylpyrrolidone, and the like.

Formulations for topical administration of nucleic acids can include sterile and non-sterile aqueous solutions, non-aqueous solutions in common solvents such as alcohols, or solutions of the nucleic acids in liquid or solid oil bases. The solutions can also contain buffers, diluents and other suitable additives. Pharmaceutically acceptable organic or inorganic excipients suitable for non- parenteral administration which do not deleteriously react with nucleic acids can be used.

In one embodiment, the nucleic acid or composition is administered in an unbuffered solution. In certain embodiments, the unbuffered solution is saline or water. In other embodiments, the nucleic acid e.g. RNAi agent is administered in a buffered solution. In such embodiments, the buffer solution can comprise acetate, citrate, prolamine, carbonate, or phosphate, or any combination thereof. For example, the buffer solution can be phosphate buffered saline (PBS).

### DOSAGES

The pharmaceutical compositions of the invention may be administered in dosages sufficient to inhibit expression of a gene or modify the expression or function of a target. In general, where the composition comprising a nucleic acid, a suitable dose of a nucleic acid e.g. an siRNA of the invention will be in the range of about 0.001 to about 200.0 milligrams per kilogram body weight of the recipient per day, generally in the range of about 1 to 50 mg per kilogram body weight per day. Typically, a suitable dose of a nucleic acid e.g. an siRNA of the invention will be in the range of about 0.1 mg/kg to about 5.0 mg/kg, e.g., about 0.3 mg/kg and about 3.0 mg/kg.

A repeat-dose regimen may include administration of a therapeutic amount of a nucleic acid e.g. siRNA on a regular basis, such as every other day or once a year. In certain embodiments, the nucleic acid e.g. siRNA is administered about once per month to about once per quarter (i.e., about once every three months).

In various embodiments, the nucleic acid e.g. siRNA agent is administered at a dose of about 0.01 mg/kg to about 10 mg/kg or about 0.5 mg/kg to about 50 mg/kg. In some embodiments, the nucleic acid e.g. siRNA agent is administered at a dose of about 10 mg/kg to about 30 mg/kg. In certain embodiments, the nucleic acid e.g. siRNA agent is administered at a dose selected from about 0.5 mg/kg 1 mg/kg, 1.5 mg/kg, 3 mg/kg, 5 mg/kg, 10 mg/kg, and 30 mg/kg. In certain embodiments, the nucleic acid e.g. siRNA agent is administered about once per week, once per month, once every other two months, or once a quarter (i.e., once every three months) at a dose of about 0.1 mg/kg to about 5.0 mg/kg. In certain embodiments, the nucleic acid e.g. siRNA agent is administered to the subject once a week. In certain embodiments, the nucleic acid e.g. siRNA agent is administered to the subject once a month. In certain embodiments, the nucleic acid e.g. siRNA agent is administered once per quarter (i.e., every three months).

After an initial treatment regimen, the treatments can be administered on a less frequent basis. For example, after administration weekly or biweekly for three months, administration can be repeated once per month, for six months, or a year; or longer.

The pharmaceutical composition can be administered once daily, or administered as two, three, or more sub-doses at appropriate intervals throughout the day or even using continuous infusion or delivery through a controlled release formulation. In that case, the nucleic acid e.g. siRNA contained in each sub-dose must be correspondingly smaller in order to achieve the total daily dosage. The dosage unit can also be compounded for delivery over several days, e.g., using a conventional sustained release formulation which provides sustained release of the nucleic acid e.g. siRNA over a several day period. Sustained release formulations are well known in the art and are particularly useful for delivery of agents at a particular site, such as could be used with the agents of the present invention. In this embodiment, the dosage unit contains a corresponding multiple of the daily dose.

In other embodiments, a single dose of the pharmaceutical compositions can be long lasting, such that subsequent doses are administered at not more than 3, 4, or 5 day intervals, or at not more than 1, 2, 3, or 4 week intervals. In some embodiments of the invention, a single dose of the pharmaceutical compositions of the invention is administered once per week. In other embodiments of the invention, a single dose of the pharmaceutical compositions of the invention is administered bimonthly. In certain embodiments, the siRNA is administered about once per month to about once per quarter (i.e., about once every three months), or even every 6 months or 12 months.

Estimates of effective dosages and *in vivo* half-lives for the individual nucleic acid e.g. siRNAs encompassed by the invention can be made using conventional methodologies or on the basis of *in vivo* testing using an appropriate animal model, as known in the art.

The pharmaceutical compositions of the present invention can be administered in a number of ways depending upon whether local or systemic treatment is desired and upon the area to be treated. Administration can be topical {e.g., by a transdermal patch), pulmonary, e.g., by inhalation or insufflation of powders or aerosols, including by nebulizer; intratracheal, intranasal, epidermal and transdermal, oral or parenteral. Parenteral administration includes intravenous, intraarterial, subcutaneous, intraperitoneal, or intramuscular injection or infusion; subdermal, e.g., via an implanted device; or intracranial, e.g., by intraparenchymal, intrathecal or intraventricular administration. In certain preferred embodiments, the compositions are administered by intravenous infusion or injection. In certain embodiments, the compositions are administered by subcutaneous injection.

In one embodiment, the nucleic acid e.g. siRNA agent is administered to the subject subcutaneously.

The inhibitor e.g. nucleic acid e.g. siRNA can be delivered in a manner to target a particular tissue (e.g. in particular liver cells).

### METHODS FOR INHIBITING GENE EXPRESSION OR INHIBITION OF TARGET EXPRESSION OR FUNCTION IN VITRO

The present invention also provides methods of inhibiting expression of SLC25A5 gene in a cell. The methods include contacting a cell with a nucleic acid of the invention e.g. siRNA agent, such as double stranded siRNA agent, in an amount effective to inhibit expression of the SLC25A5 gene in the cell, thereby inhibiting expression of the SLC25A5 gene in the cell. It is to be noted that a nucleic acid "for inhibiting the expression of SLC25A5" is a nucleic acid that is capable of inhibiting SLC25A5 expression, preferably as described herein below.

Contacting of a cell with the nucleic acid e.g. an siRNA, such as a double stranded siRNA agent, may be done in vitro or in vivo. Contacting a cell in vivo with nucleic acid e.g. includes contacting a cell or group of cells within a subject, e.g., a human subject, with the nucleic acid e.g. siRNA. Combinations of in vitro and in vivo methods of contacting a cell are also possible. Contacting a cell may be direct or indirect, as discussed above. Furthermore, contacting a cell may be accomplished via a targeting ligand moiety, including any ligand moiety described herein or known in the art. In preferred embodiments, the targeting ligand moiety is a carbohydrate moiety, e.g. a GalNAc3 ligand, or any other ligand moiety that directs the siRNA agent to a site of interest.

The term "inhibiting," as used herein, is used interchangeably with "reducing," "silencing," "downregulating", "suppressing", and other similar terms, and includes any level of inhibition.

In some embodiments of the methods of the invention, expression of SLC25A5 gene is inhibited by at least 30%, 35%, 40%, 45%, 50%, 55%, 60%, 65%, 70%, 75%, 80%, 85%, 90%, or 95%, or to below the level of detection of the assay, preferably when determined by qPCR as described herein and/or when the siRNA is introduced into the target cell by transfection. In certain embodiments, the methods include a clinically relevant inhibition of expression of SLC25A5 target gene e.g. as demonstrated by a clinically relevant outcome after treatment of a subject with an agent to reduce the expression of the gene.

In some embodiments, when transfected into the cells, the nucleic acid of the invention inhibits expression of the SLC25A5 gene with an IC50 value lower than 2500 pM, 2400 pM, 2300 pM, 2200 pM, 2100 pM, 2000 pM, 1900 pM, 1800 pM, 1700 pM, 1600 pM, 1500 pM, 1400 pM, 1300 pM, 1200 pM, 1100 pM, 1000 pM, 900 pM, 800 pM, 700 pM, 600 pM, 500 pM, 400 pM, 300 pM, 200 pM or 100 pM, preferably when determined by qPCR, more preferably by reverse transcriptase (RT)-qPCR, as described herein.

In a preferred embodiment, when transfected into the cells, the nucleic acid of the invention inhibits expression of the SLC25A5 gene with an IC50 value lower than 2500 pM. In a more preferred embodiment, when transfected into the cells, the nucleic acid of the invention inhibits expression of the SLC25A5 gene with an IC50 value lower than 1000 pM. In an even more preferred embodiment, when transfected into the cells, the nucleic acid of the invention inhibits expression of the SLC25A5 gene with an IC50 value lower than 500 pM. In a most preferred embodiment, when transfected into the cells, the nucleic acid of the invention inhibits expression of the SLC25A5 gene with an IC50 value lower than 100 pM.

Inhibition of expression of the SLC25A5 gene may be quantified by the following method:
Huh7 cells (human hepatocyte-derived cell line, obtained from JCRB Cell Bank) may be maintained in Dulbecco's Modified Eagle Medium (DMEM) supplemented with 10% FBS and 1% non-essential amino acids at 37°C in an atmosphere of 5% CO₂. Cells may then be transfected with siRNA duplexes targeting SLC25A5 mRNA or a negative control siRNA (siRNA-control; sense strand 5'-GCCUGUACCAAGGCUUUAA-3' (SEQ ID NO: 1383), antisense strand 5'-UUAAAGCCUUGGUACAGGC-3' (SEQ ID NO: 1382)) using six 10-fold serial dilutions over a final duplex concentration range of 3 nM to 0.03 pM. Transfection may be carried out by adding 9.7 µL Opti-MEM (ThermoFisher) plus 0.3 µL Lipofectamine RNAiMAX (ThermoFisher) to 10 µL of each siRNA duplex. The mixture may be incubated at room temperature for 15 minutes before being added to 100 µL of complete growth medium containing 20,000 Huh7 cells. Cells may be incubated for 24 hours at 37°C/5% CO₂ prior to total RNA purification using a RNeasy 96 Kit (Qiagen). Each duplex may be tested by transfection in duplicate wells in a single experiment.
cDNA synthesis may be performed using FastKing RT (with gDNase) Kit (Tiangen). Real-time quantitative PCR (qPCR) may be performed on an ABI Prism 7900HT or ABI QuantStudio 7 with primers specific for human SLC25A5 (forward: ACTGACATCATGTACACAGGCAC (SEQ ID NO: 1384), reverse: ACCCATGCCTCTGAGAACATT (SEQ ID NO: 1385)) and human GAPDH (forward: GAAGGTGAAGGTCGGAGTC (SEQ ID NO: 1386), reverse: GAAGATGGTGATGGGATTTC (SEQ ID NO: 1387)) using a SensiFAST SYBR Hi-ROX kit (Meridian).
qPCR may be performed in duplicate on cDNA derived from each well and the mean cycle threshold (Ct) calculated. Relative SLC25A5 expression may be calculated from mean Ct values using the comparative Ct (ΔΔCt) method, normalised to GAPDH and relative to untreated cells. Maximum percent inhibition of SLC25A5 expression and IC50 values may be calculated using a four parameter (variable slope) model using GraphPad Prism 9.

Alternatively or in addition, a pEC50 value may be calculated to quantify and or compare the inhibitory potential of the siRNAs according to the invention.

In some embodiments, when transfected into the cells, the nucleic acid of the invention inhibits expression of the SLC25A5 gene with an pEC50 value lower than 5, 6, 7, 8, 9 or 10, preferably when determined by qPCR, more preferably by reverse transcriptase (RT)-qPCR, as described herein.

For that, Huh7 cells (human hepatocyte-derived cell line, obtained from JCRB Cell Bank) may be maintained in Dulbecco's Modified Eagle Medium (DMEM) supplemented with 10% FBS and 1% non-essential amino acids at 37°C, 5% CO₂, 95% humidity. Cells may be transfected with siRNA duplexes targeting either SLC25A5 mRNA or a negative control siRNA (siRNA-control; sense strand 5'-GCCUGUACCAAGGCUUUAA-3' (SEQ ID NO: 1383), antisense strand 5'-UUAAAGCCUUGGUACAGGC-3' (SEQ ID NO: 1382)) in a 6-point, log dose response curve to give final in assay concentrations of 3nM to 0.03pM. Transfection may be carried out by diluting Lipofectamine RNAiMAX (ThermoFisher) in Opti-MEM (ThermoFisher) medium at a ratio of 48.5:1.5. This solution may be added to an equal volume of siRNA, diluted to the required concentration in phosphate buffered saline. The lipofectamine RNAiMAX and siRNA mixture may be incubated at room temperature for 15 minutes before 20 µL may be added to wells of a 96 well plate. Huh7 cells may be dissociated from flasks using trypsin and resuspended at a density of 200,000 cells/mL. 100 µL of Huh7 cell suspension may be added to each well of the siRNA-containing 96-well plates. Cells may be incubated for 24 hours at 37°C, 5% CO₂,95% humidity. Each siRNA may be tested in triplicate wells and on two separate days for a total of six replicates.

Intracellular RNA may be isolated using an Rneasy kit (Qiagen) according to the manufacturer's instructions. cDNA synthesis may be performed using a FastKing RT kit, with gDNase (Tiangen). Target cDNA may be the quantified by qPCR on an ABI Prism 7900HT or ABI QuantStudio 7 with primers specific for human SLC25A5 (forward: ACTGACATCATGTACACAGGCAC (SEQ ID NO: 1384), reverse: ACCCATGCCTCTGAGAACATT (SEQ ID NO: 1385)) and human GAPDH (forward: GAAGGTGAAGGTCGGAGTC (SEQ ID NO: 1386), reverse: GAAGATGGTGATGGGATTTC (SEQ ID NO: 1387)) using a SensiFAST SYBR Hi-ROX kit (Meridian).

qPCR may be performed in duplicate on cDNA derived from each well and the mean Ct calculated. Relative SLC25A5 expression may be calculated from mean Ct values using the comparative Ct (ΔΔCt) method, normalised to GAPDH and relative to untreated cells. Maximum percent inhibition of SLC25A5 expression and pEC50 values (-logic of the EC50) may be calculated using a four parameter (variable slope) model using NumPy (Python).

Alternatively or in addition, inhibition of expression of the SLC25A5 gene may be characterized by a reduction of mean relative expression of the SLC25A5 gene.

In some embodiments, when cells are transfected with 0.1 nM of the nucleic acid of the invention, the mean relative expression of SLC25A5 is below 1, 0.9, 0.8, 0.7, 0.6, 0.5, or 0.4, preferably when determined by qPCR, more preferably by reverse transcriptase (RT)-qPCR, as described herein.

In some embodiments, when cells are transfected with 5 nM of the nucleic acid of the invention, the mean relative expression of SLC25A5 is below 1, 0.9, 0.8, 0.7, 0.6, 0.5, 0.4 or 0.3, preferably when determined by qPCR, more preferably by reverse transcriptase (RT)-qPCR, as described herein.

Mean relative expression of the SLC25A5 gene may be quantified by the following method:

Huh7 cells (human hepatocyte-derived cell line, obtained from JCRB Cell Bank) may be maintained in Dulbecco's Modified Eagle Medium (DMEM) supplemented with 10% FBS at 37°C in at atmosphere of 5% CO₂. Cells may be transfected with siRNA duplexes targeting SLC25A5 mRNA or a negative control siRNA (siRNA-control; sense strand 5'-GCCUGUACCAAGGCUUUAA-3' (SEQ ID NO: 1383), antisense strand 5'-UUAAAGCCUUGGUACAGGC-3' (SEQ ID NO: 1382)) at a final duplex concentration of 5 nM and 0.1 nM. Transfection may be carried out by adding 9.7 µL Opti-MEM (ThermoFisher) plus 0.3 µL Lipofectamine RNAiMAX (ThermoFisher) to 10 µL of each siRNA duplex. The mixture may be incubated at room temperature for 15 minutes before being added to 100 µL of complete growth medium containing 20,000 Huh7 cells. Cells may be incubated for 24 hours at 37°C/5% CO₂ prior to total RNA purification using a Rneasy 96 Kit (Qiagen). Each duplex may be tested by transfection in duplicate wells in two independent experiments.

cDNA synthesis may be performed using FastKing RT (with gDNase) Kit (Tiangen). Real-time quantitative PCR (qPCR) may be performed on an ABI Prism 7900HT or ABI QuantStudio 7 with primers specific for human SLC25A5 (forward: ACTGACATCATGTACACAGGCAC (SEQ ID NO: 1384), reverse: ACCCATGCCTCTGAGAACATT (SEQ ID NO: 1385)) and human GAPDH (forward: GAAGGTGAAGGTCGGAGTC (SEQ ID NO: 1386), reverse: GAAGATGGTGATGGGATTTC (SEQ ID NO: 1387)) using a SensiFAST SYBR Hi-ROX kit (Meridian) .

qPCR may be performed in duplicate on cDNA derived from each well and the mean Ct calculated. Relative SLC25A5 expression may be calculated from mean Ct values using the comparative Ct (ΔΔCt) method, normalised to GAPDH and relative to untreated cells.

Inhibition of the expression of SLC25A5 gene may be manifested by a reduction of the amount of mRNA of the target SLC25A5 gene in comparison to a suitable control.

In other embodiments, inhibition of the expression of SLC25A5 gene may be assessed in terms of a reduction of a parameter that is functionally linked to gene expression, e.g , protein expression or signaling pathways.

### METHODS OF TREATING OR PREVENTING DISEASES ASSOCIATED WITH GENE EXPRESSION/ EXPRESSION OF FUNCTION OF A TARGET.

The present invention also provides methods of using nucleic acid e.g. an siRNA of the invention or a composition containing nucleic acid e.g. an siRNA of the invention to reduce or inhibit gene expression in a cell or reduce expression or function of a target. The methods include contacting the cell with a nucleic acid e.g. dsiRNA of the invention and maintaining the cell for a time sufficient to obtain degradation of the mRNA transcript of a gene, thereby inhibiting expression of the gene in the cell. Reduction in gene expression or function of a target can be assessed by any methods known in the art. In a preferred embodiment, the gene is *SLC25A5.*

In the methods of the invention the cell may be contacted *in vitro* or *in vivo,* i.e., the cell may be within a subject.

A cell suitable for treatment using the methods of the invention may be any cell that expresses a gene of interest or target of interest associated with disease.

The *in vivo* methods of the invention may include administering to a subject a composition containing a nucleic acid of the invention e.g. an iRNA, where the nucleic acid e.g. siRNA includes a nucleoside sequence that is complementary to at least a part of an RNA transcript of the gene of the mammal to be treated, or complementary to another nucleic acid the expression and /or function of which is associated with diseases.

The present invention further provides methods of treatment of a subject in need thereof. The treatment methods of the invention include administering a nucleic acid such as an siRNA of the invention to a subject, e.g., a subject that would benefit from a reduction or inhibition of the expression of a gene and/or expression and/or function of a target, in a therapeutically effective amount e.g. a nucleic acid such as an siRNA targeting a gene or a pharmaceutical composition comprising the nucleic acid targeting a gene.

A nucleic acid e.g. siRNA of the invention may be administered as a "free" nucleic acid or "free" siRNA, administered in the absence of a pharmaceutical composition. The naked nucleic acid may be in a suitable buffer solution. The buffer solution may comprise acetate, citrate, prolamine, carbonate, or phosphate, or any combination thereof. In one embodiment, the buffer solution is phosphate buffered saline (PBS). The pH and osmolarity of the buffer solution can be adjusted such that it is suitable for administering to a subject.

Alternatively, a nucleic acid e.g. siRNA of the invention may be administered as a pharmaceutical composition, such as a dsiRNA liposomal formulation.

In one embodiment, the method includes administering a composition featured herein such that expression of the target gene is decreased, such as for about 1, 2, 3, 4, 5, 6, 7, 8, 12, 16, 18, 24 hours, 28, 32, or about 36 hours. In one embodiment, expression of the target gene is decreased for an extended duration, e.g., at least about two, three, four days or more, e.g., about one week, two weeks, three weeks, or four weeks or longer, e.g., about 1 month, 2 months, or 3 months.

Subjects can be administered a therapeutic amount of nucleic acid e.g. siRNA, such as about 0.01 mg/kg to about 200 mg/kg.

The nucleic acid e.g. siRNA can be administered by intravenous infusion over a period of time, on a regular basis. In certain embodiments, after an initial treatment regimen, the treatments can be administered on a less frequent basis. Administration of the siRNA can reduce gene product levels of a target gene, e.g., in a cell or tissue of the patient by at least about 20%, 25%, 30%, 35%, 40%, 45%, 50%, 55%, 60%, 65%, 70%, 75%, 80%, 85%, 90%, or 95%, or below the level of detection of the assay method used. In certain embodiments, administration results in clinical stabilization or preferably clinically relevant reduction of at least one sign or symptom of a gene-associated disorder.

Alternatively, the nucleic acid e.g. siRNA can be administered subcutaneously, i.e., by subcutaneous injection. One or more injections may be used to deliver the desired daily dose of nucleic acid e.g. s iRNA to a subject. The injections may be repeated over a period of time. The administration may be repeated on a regular basis. In certain embodiments, after an initial treatment regimen, the treatments can be administered on a less frequent basis. A repeat-dose regimen may include administration of a therapeutic amount of nucleic acid on a regular basis, such as every other day or to once a year. In certain embodiments, the nucleic acid is administered about once per month to about once per quarter (i.e., about once every three months). Administration of the siRNA can reduce gene product levels of a target gene , e.g., in a cell or tissue of the patient by at least about 20%, 25%, 30%, 35%, 40%, 45%, 50%, 55%, 60%, 65%, 70%, 75%, 80%, 85%, 90%, or 95%, or below the level of detection of the assay method used.

### COMBINATION THERAPIES

The inhibitor of the present invention may be combined with other therapeutic agents for use in therapy, in particular for use in treatment of any of the metabolic diseases or disorders disclosed herein.

In certain embodiments, the inhibitor of the present invention, such as any of the siRNA molecules disclosed herein, may be combined with a GLP-1 agonist, including, without limitation, GLP-1/GIP dual agonists, GLP-1/FGF21 dual agonists, GLP-1/GCGR dual agonists and GLP-1/GIP/GCGR triple agonists, and/or a THR-beta agonist.

That is, in certain embodiments, the inhibitor of the invention, in particular the siRNA molecules disclosed herein, may be combined with a GLP-1 agonist for the treatment of metabolic diseases or disorders. In a particular embodiment, the inhibitor of the invention, in particular the siRNA molecules disclosed herein, may be combined with a GLP-1 agonist for the treatment of fatty liver diseases, in particular NAFLD and/or NASH.

The term "GLP-1 agonist" as used herein refers to a compound, which fully or partially activates the human GLP-1 receptor. The term is thus equal to the term "GLP-1 receptor agonist" used in other documents. The term GLP-1 agonist as well as the specific GLP-1 agonists described herein also encompass salt forms thereof.

It follows that the GLP-1 agonist should display "GLP-1 activity" which refers to the ability of the compound, i.e. a GLP-1 analogue or a compound comprising a GLP-1 analogue, to bind to the GLP-1 receptor and initiate a signal transduction pathway resulting in insulinotropic action or other physiological effects as is known in the art. In some embodiments the "GLP-1 agonist" binds to a GLP-1 receptor, e.g., with an affinity constant (K_{D}) or activate the receptor with a potency (EC₅o) of below 1 mM, e.g. below 100 nM as measured by methods known in the art (see e.g. WO 98/08871) and exhibits insulinotropic activity, where insulinotropic activity may be measured in vivo or in vitro assays known to those of ordinary skill in the art. For example, the GLP-1 agonist may be administered to an animal with increased blood glucose (e.g. obtained using an Intravenous Glucose Tolerance Test (IVGTT). A person skilled in the art will be able to determine a suitable glucose dosage and a suitable blood sampling regime, e.g. depending on the species of the animal, for the IVGTT) and measure the plasma insulin concentration overtime. Suitable assays have been described in such as WO2015/155151.

The term half maximal effective concentration (EC₅o) generally refers to the concentration which induces a response halfway between the baseline and maximum, by reference to the dose response curve. EC₅o is used as a measure of the potency of a compound and represents the concentration where 50% of its maximal effect is observed. Due to the albumin binding effects of GLP-1 agonists comprising a substituent as described herein, it is important to pay attention to if the assay includes human serum albumin or not.

The in vitro potency of the GLP-1 agonist may be determined as described in WO 2015/155151 , example 29 without Human Serum Albumin (HSA), and the EC₅o determined. The lower the EC₅o value, the better the potency. In one embodiment, the potency (EC50) as determined (without HSA) is 5-1000 pM, such as 10-750 pM, 10-500 pM or 10-200 pM. In one embodiment the EC50 (without HSA) is at most 500 pM, such as at most 300 pM, such as at most 200 pM.

In one embodiment the EC50 (without HSA) is comparable to human GLP-1 (7-37).

In one embodiment the EC50 (without HSA) is at most 50 pM. In a further such embodiment the EC50 is at most 40 pM, such as at most 30 pM such as at most 20 pM, such as at most 10 pM. In one embodiment the EC50 is around 10 pM

Also, or alternatively, the binding of the GLP-1 agonist to albumin may be measured using the in vitro potency assay of Example 29 of WO 2015/155151 including HSA. An increase of the in vitro potency, EC₅o value, in the presence of serum albumin reflects the affinity to serum albumin.

In one embodiment the potency (EC50) as determined (with 1 % HSA) is 5-1000 pM, such as 100-750 pM, 200-500 pM or 100-400 pM. In one embodiment the EC50 (with 1 % HSA) is at most 750 pM, such as at most 500 pM, such as at most 400 pM, such as at most 300 or such as at most 250 pM.

If desired, the fold variation in relation to a known GLP-1 receptor agonist may be calculated as EC50(test analogue)/EC50(known analogue), and if this ratio is such as 0.5- 1.5, or 0.8-1.2 the potencies are considered to be equivalent.

In one embodiment the potency, EC50 (without HSA), is equivalent to the potency of liraglutide. In one embodiment the potency, EC50 (without HSA), is equivalent to the potency of semaglutide.

In some embodiments the GLP-1 agonist is a GLP-1 analogue, optionally comprising "one subsituent". The term "analogue" as used herein referring to a GLP-1 peptide (hereafter "peptide") means a peptide wherein at least one amino acid residue of the peptide has been substituted with another amino acid residue and/or wherein at least one amino acid residue has been deleted from the peptide and/or wherein at least one amino acid residue has been added to the peptide and/or wherein at least one amino acid residue of the peptide has been modified. Such addition or deletion of amino acid residues may take place at the N- terminal of the peptide and/or at the C-terminal of the peptide.

In some embodiments the term "GLP-1 analogue" or "analogue of GLP-1" as used herein refers to a peptide, or a compound, which is a variant of the human Glucagon-Like Peptide-1 (GLP-1 (7-37)). GLP-1 (7-37) has the sequence HAEGTFTSDV S SYLEGQAAKEFIA WL VKGRG (SEQ ID NO: 1388). In some embodiments the term "variant" refers to a compound which comprises one or more amino acid substitutions, deletions, additions and/or insertions.

In one embodiment the GLP-1 agonist exhibits at least 60%, 65%, 70%, 80% or 90% sequence identity to GLP-1 (7-37) over the entire length of GLP-1 (7-37).

In general, the term GLP-1 agonist is meant to encompass the GLP-1 agonist and any pharmaceutically acceptable salt, amide, or ester thereof. In some embodiments the composition comprises the GLP-1 agonist or a pharmaceutically acceptable salt, amide, or ester thereof. In some embodiments the composition comprises the GLP-1 agonist and one or more pharmaceutically acceptable counter ions.

In certain embodiments, the inhibitor of the present invention, in particular the siRNA molecules of the present invention, may be administered with a GLP-1 agonist selected from one or more of the GLP-1 agonists disclosed in WO93/19175, WO96/29342, WO98/08871, WO99/43707, WO99/43706, WO99/43341 , WO99/43708, WO2005/027978, WO2005/058954, WO2005/058958, WO2006/005667, WO2006/037810, WO2006/037811 , WO2006/097537, WO2006/097538, WO2008/023050, WO2009/030738, WO2009/030771, WO2009/030774 and WO2021/219710, which are incorporated herein by reference in their entirety.

In certain embodiments, the inhibitor of the present invention, in particular the siRNA molecules of the present invention, may be administered with a GLP-1 agonist selected from the group consisting of: Dulaglutide (Trulicity^{®}), Exenatide (Byetta^{®}), Exenatide extended-release (Bydureon^{®}), Liraglutide (Victoza^{®}), Lixisenatide (Adlyxin^{®}), Semaglutide injection (Ozempic^{®}), and Semaglutide tablets (Rybelsus^{®}).

In some embodiments, the GLP-1 agonist is semaglutide having a formula of N- epsilon26-[2-(2-{2-[2-(2-{2-[(S)-4-carboxy-4-(17-carboxy-heptadecanoylamino)butyrylamino]ethoxy}ethoxy) acetylamino]ethoxy}ethoxy)acetyl] [Aib8,Arg34]GLP-1 (7-37).

The term "GLP-1 agonist" as used herein also encompasses dual or triple agonists that can activate more than one receptor. These dual or triple agonists may be molecules that have the ability to activate the GLP-1 receptor and at least one further receptor. In some embodiments, the dual or triple agonist may be a chimeric molecule comprising a first portion that activates the GLP-1 receptor and further portions that activate further receptors. In certain embodiments, the GLP-1 agonist is a dual or triple agonist that, in addition to the GLP-1 receptor, further activates one or more of: a glucose-dependent insulinotropic polypeptide (GIP) receptor, a Fibroblast growth factor 21 (FGF21) receptor, and a glucagon receptor (GCGR).

In certain embodiments, the inhibitor of the invention, in particular the siRNA molecules disclosed herein, may be combined with a GLP-1/GIP dual agonist for the treatment of metabolic diseases or disorders. In a particular embodiment, the inhibitor of the invention, in particular the siRNA molecules disclosed herein, may be combined with a GLP-1/GIP dual agonist for the treatment of fatty liver diseases, in particular NAFLD and/or NASH.

The term "GLP-1/GIP dual agonist" as used in the context of the present invention refers to a substance or ligand that can activate the GLP-1 receptor and the glucose-dependent insulinotropic polypeptide (GIP) receptor. GLP-1/GIP receptor co-agonists and their potential medical uses are described in several patent applications such as WO 2010/011439, WO 2013/164483, WO 2014/192284, WO 2015/067715, WO 2015/022420, WO 2015/086728, WO 2015/086729, WO 2016/111971, WO 2020/023386, US 9745360, US 2014/162945, US 2014/0357552, WO 2021/150673, WO 2021/260530, WO 2022/018185, and WO 2022/079639, which are fully incorporated herein by reference.

In certain embodiments, the inhibitor of the invention, in particular the siRNA molecules disclosed herein, may be combined with the GLP-1/GIP dual agonist tirzepatide (Mounjaro^{®}) for the treatment of metabolic diseases or disorders. In a particular embodiment, the inhibitor of the invention, in particular the siRNA molecules disclosed herein, may be combined with the GLP-1/GIP dual agonist tirzepatide (Mounjaro^{®}) for the treatment of fatty liver diseases, in particular NAFLD and/or NASH.

As used herein, "tirzepatide" means a GLP-1/GIP dual agonist peptide as described in U.S. Pat. No. 9,474,780 and described by CAS Registry Number: 2023788-19-2. Tirzepatide is described in Example 1 of U.S. Pat. No. 9,474,780, with the following sequence:
YX₁EGTFTSDYSIX₂LDKIAQKAFVQWLMGGPSSGAPPPS (SEQ ID NO: 1389)
wherein X₁ is α-amino isobutyric acid (Aib); X₂ is Aib; K at position 20 is chemically modified through conjugation to the epsilon-amino group of the K side-chain with (2-[2-(2-Amino-ethoxy)-ethoxy]-acetyl)₂-(γGlu)₁-CO-(CH₂)₁₈-CO₂H; and the C-terminal amino acid is amidated as a C-terminal primary amide.

In certain embodiments, the inhibitor of the invention, in particular the siRNA molecules disclosed herein, may be combined with the FGF-21 analogue (analog) efruxifermin for the treatment of metabolic diseases or disorders. In a particular embodiment, the inhibitor of the invention, in particular the siRNA molecules disclosed herein, may be combined with the FGF-21 analogue efruxifermin for the treatment of fatty liver diseases, in particular NAFLD and/or NASH.

In certain embodiments, the inhibitor of the invention, in particular the siRNA molecules disclosed herein, may be combined with a GLP-1/ FGF21 dual agonist for the treatment of metabolic diseases or disorders. In a particular embodiment, the inhibitor of the invention, in particular the siRNA molecules disclosed herein, may be combined with a GLP-1/FGF21 dual agonist for the treatment of fatty liver diseases, in particular NAFLD and/or NASH.

The term "GLP-1/FGF21 dual agonist" as used in the context of the present invention refers to a substance or ligand that can activate the GLP-1 receptor and the Fibroblast growth factor 21 (FGF21) receptor. Preferably, the GLP-1/FGF21 dual agonist is a chimeric molecule comprising a GLP-1 agonist as defined herein above and the molecule FGF21 or a functionally active variant or analogue thereof. In certain embodiments, the GLP-1/FGF21 dual agonist may further comprise an antibody Fc region.

GLP-1/ FGF21 receptor co-agonists and their potential medical uses are described in several patent applications such as WO 2010/142665, WO2014/037373, WO 2018/115401, WO 2018/166461, WO 2019/243557, and WO 2022/002408, which are fully incorporated herein by reference.

In certain embodiments, the inhibitor of the invention, in particular the siRNA molecules disclosed herein, may be combined with a GLP-1/ GCGR dual agonist for the treatment of metabolic diseases or disorders. In a particular embodiment, the inhibitor of the invention, in particular the siRNA molecules disclosed herein, may be combined with a GLP-1/GCGR dual agonist for the treatment of fatty liver diseases, in particular NAFLD and/or NASH.

The term "GLP-1/ GCGR dual agonist" as used in the context of the present invention refers to a substance or ligand that can activate the GLP-1 receptor and the glucagon receptor (GCGR). GLP-1/GCGR receptor co-agonists and their potential medical uses are described in several patent applications such as, WO 2008/101017, WO 2009/155258, WO 2011/075393, WO 2011/160630, WO 2014/056872, WO 2014/091316, WO 2015/086733, WO 2017/181452, WO 2018/100174, WO 2019/030268, WO 2019/060660 and WO 2023/006923, which are fully incorporated herein by reference.

In certain embodiments, the inhibitor of the invention, in particular the siRNA molecules disclosed herein, may be combined with the GLP-1/ GCGR dual agonist Survodutide (BI 456906) for the treatment of metabolic diseases or disorders. In a particular embodiment, the inhibitor of the invention, in particular the siRNA molecules disclosed herein, may be combined with the GLP-1/ GCGR dual agonist Survodutide (BI 456906) for the treatment of fatty liver diseases, in particular NAFLD and/or NASH.

In certain embodiments, the inhibitor of the invention, in particular the siRNA molecules disclosed herein, may be combined with a GLP-1/GIP/GCGR triple agonist for the treatment of metabolic diseases or disorders. In a particular embodiment, the inhibitor of the invention, in particular the siRNA molecules disclosed herein, may be combined with a GLP-1/GIP/GCGR dual agonist for the treatment of fatty liver diseases, in particular NAFLD and/or NASH.

The term " GLP-1/GIP/GCGR triple agonist" as used in the context of the present invention refers to a substance or ligand that can activate the GLP-1 receptor, the GIP receptor and the glucagon receptor (GCGR). GLP-1/GIP/GCGR receptor co-agonists and their potential medical uses are described in several patent applications such as, WO 2014/096150, WO 2015/067716, WO 2019/125292, WO 2022/090447, and WO 2022/268029, which are fully incorporated herein by reference.

In certain embodiments, the inhibitor of the invention, in particular the siRNA molecules disclosed herein, may be combined with Retatrutid (LY-3437943) for the treatment of metabolic diseases or disorders. In a particular embodiment, the inhibitor of the invention, in particular the siRNA molecules disclosed herein, may be combined with Retatrutid (LY-3437943) for the treatment of fatty liver diseases, in particular NAFLD and/or NASH.

The person skilled in the art is aware of ways to formulate GLP-1 agonists, including dual and triple agonists, for any suitable route of administration. In certain embodiments, the GLP-1 agonist may be administered orally or by injection, e.g., by subcutaneous injection.

In certain embodiments, the inhibitor of the invention, in particular the siRNA molecules disclosed herein, may be combined with a THR-beta agonist for the treatment of metabolic diseases or disorders. In a particular embodiment, the inhibitor of the invention, in particular the siRNA molecules disclosed herein, may be combined with a THR-beta agonist for the treatment of fatty liver diseases, in particular NAFLD and/or NASH.

The term "THR-beta agonist" as used herein refers to a compound, which fully or partially activates the human thyroid hormone receptor-β. The term THR-beta agonist as well as the specific THR-beta agonists described herein also encompass salt forms thereof.

Various THR-beta agonists have been described in the art and have been summarized, inter alia, by Zucchi (Thyroid Hormone Analogues: An Update, Thyroid. August 2020; 30(8): 1099-1105), which is incorporated herein by reference in its entirety.

In certain embodiments, the THR-beta agonist that is to be administered in combination with the inhibitor of the invention is MGL-3196 (resmetirom), KB-2115 (eprotirome), GC-1 (sobetirome) or MB07344/VK2809, as, for example, described by Zucchi.

In certain embodiments, the THR-beta agonist is MGL-3196 (resmetirom) or any of the compounds disclosed in WO 2014/043706, which is incorporated herein by reference in its entirety. Resmetirom is a thyroid hormone receptor (THR) β-selective agonist. Disclosures related to resmetirom can further be found in US Patent No. 9,266,861, US Patent Application Serial No. 16/343,065, and PCT Application Serial No. PCT/US2019/040276, the contents of each of which are incorporated herein by reference in their entireties.

In certain embodiments, the THR-beta agonist is KB-2115 (eprotirome) or any of the compounds disclosed in WO 2007/110226 or WO 2009/077147, which are incorporated herein by reference in their entirety.

The person skilled in the art is aware of ways to formulate THR-beta agonists for any suitable route of administration.

In certain embodiments, the inhibitor of the invention, in particular the siRNA molecules disclosed herein, may be combined with a GLP-1 agonist, such as any one of the GLP-1 agonists disclosed herein above, including dual or triple agonists, and a THR-beta agonist, such as any one of the THR-beta agonists disclosed herein above, for the treatment of metabolic diseases or disorders. In a particular embodiment, the inhibitor of the invention, in particular the siRNA molecules disclosed herein, may be combined with a GLP-1 agonist, such as any one of the GLP-1 agonists disclosed herein above, including dual or triple agonists, and a THR-beta agonist, such as any one of the THR-beta agonists disclosed herein above, for the treatment of fatty liver diseases, in particular NAFLD and/or NASH.

The inhibitor of the invention, in particular the siRNA molecules disclosed herein, may alternatively, or in addition, be combined and/or co-administered with one or more of: an amylin receptor agonist (such as pramlintide), and/or a dual amylin + calcitonin receptor agonist, and/or a glucagon receptor agonist, and/or an FXR receptor agonist (such as cilofexor or obeticholic acid), and/or an FGF-21 analogue or FGF-21 receptor agonist (such as efruxifermin), and/or an FGF-19 analogue or FGF-19 receptor agonist (such as aldafermin), and/or a galectin 3 inhibitor (such as belapectin), and/or a PPARα agonist (such as elafibrinor), and/or a PPARγ agonist (such as pioglitazone or rosiglitazone), and/or a mixed PPARα and/or δ and/or γ agonist, and/or a pan PPARαγδ agonist (such as lanafibranor), and/or an acetyl CoA desaturase activator, and/or an ASK1 inhibitor (such as selonsertib), and/or an LOXL2 inhibitor (such as simtuzumab), and/or a dual CCR2/5 inhibitor (such as cenicriviroc), and/or an inhibitor of an enzyme in the de novo lipogenesis (DNL) pathway including citrate/isocitrate carrier (CIC), ATP-citrate lyase (ACLY), acetyl-CoA carboxylase (ACC) and fatty acid synthase (FAS), and/or an inhibitor of an enzyme in the cholesterol biosynthesis pathway (such as an HMGCoA reductase inhibitor, such as atorvastatin); preferably for the treatment of any of the metabolic diseases or disorders disclosed herein, more preferably for the treatment of NAFLD.

In certain embodiments, the inhibitor of the invention, in particular the siRNA molecules disclosed herein, may be used for the treatment of any of the metabolic diseases or disorders disclosed herein, more preferably for the treatment of NAFLD, in combination with any of the molecules disclosed in Fig.1 of Nathani and Bansal (Gastroenterol Hepatol (NY). 2023 Jul; 19(7): 371-381; incorporated by reference), in particular one or more of: a THR-beta agonist (such as resmetirom, VK2809 or TERN-501), a PPAR agonist (such as Lanifibranor, Saroglitazar, or Elafibranor), a GLP-1 agonist (such as Liraglutide, Semaglutide or Tirzepatide), a CCR2/5 inhibitor (such as Cenicriviroc), an ASK1 inhibitor (such as Selonsertib), an ACC inhibitor (such as Firsocostat or PF-05221304), an SCD inhibitor (such as Aramchol), an FGF21 analogue (such as Efruxifermin or Pegbelfermin), a Galectin-3 inhibitor (such as Belapectin), a LOXL2 inhibitor (such as Simtuzumab), an FXR agonists (such as Obeticholic acid, Tropifexor, Cilofexor, EDP-305, or MET409), and/or an FGF19 analogue (such as Aldafermina).

In certain embodiments, the inhibitor of the invention, in particular the siRNA molecules disclosed herein, may be used for the treatment of any of the metabolic diseases or disorders disclosed herein, more preferably for the treatment of NAFLD, in combination with any of the molecules disclosed in Batchuluun et al. (Nat Rev Drug Discov, 2022, 21(4):283-305. doi: 10.1038/s41573-021-00367-2.; incorporated by reference), in particular one or more of: a citrate/isocitrate carrier (CIC) inhibitor (such as Benzenetricarboxylate, CPTI-1 or CPTI-2), an ATP-citrate lyase (ACLY) inhibitor (such as Bempedoic acid, Hydroxycitrate, BMS-303141, Emodin derivates, Furan carboxylate derivates, MEDICA 16, SB-204990, or NDI-091143), an acetyl-CoA carboxylase (ACC) inhibitor (such as Firsocostat, PF-05221304, PF-05175157, MK-4074, A-908292, Carboxamide derivative-1k, CP-640186, Monocyclic derivate-1q, ND-654, ND-646, Olefin derivate-2e, (S)-9c, Soraphen A, TOFA, or WZ66), and/or a fatty acid synthase (FAS) inhibitor (such as Orlistat, TVB-2640, FT-4101, BI-99179, Cerulenin, C75, Fasnall, GSK2194069, IPI-9119, MP-ML-24-N1, or TVB-3166).

A combination therapy, or a "combination" contemplated herein includes the co-administration of an inhibitor according to the invention, in particular an siRNA according to the invention, with one or more additional therapeutic agent, preferably one or more of the additional therapeutic agents disclosed herein above. The inhibitor according to the invention may be administered prior to, after, or at the same time as the one or more additional therapeutic agent.

In certain embodiments, the inhibitor according to the invention, in particular the siRNA according to the invention, may be co-administered with a GLP-1 agonist, including GLP-1/GIP dual agonists, GLP-1/FGF21 dual agonists, GLP-1/GCGR dual agonists and GLP-1/GIP/GCGR triple agonists, and/or a THR-beta agonist, wherein the inhibitor according to the invention may be administered prior to, after, or at the same time as the GLP-1 agonist and/or the THR-beta agonist.

In an exemplary embodiment, the co-administration includes administering an siRNA according to the invention and any of the GLP-1 agonists, including GLP-1/GIP dual agonists, GLP-1/FGF21 dual agonists, GLP-1/GCGR dual agonists and GLP-1/GIP/GCGR triple agonists, disclosed herein or incorporated herein by reference. In another exemplary embodiment, the co-administration includes administering an siRNA according to the invention and the GLP-1 agonist semaglutide. In another exemplary embodiment, the co-administration includes administering an siRNA according to the invention and the GLP-1/GIP dual agonist terzapetide.

In yet another exemplary embodiment, the co-administration includes administering an siRNA according to the invention and any of the THR-beta agonists disclosed herein or incorporated herein by reference. In another exemplary embodiment, the co-administration includes administering an siRNA according to the invention and the THR-beta agonist resmetirom.

In another exemplary embodiment, the co-administration includes administering an siRNA according to the invention, the GLP-1 agonists semaglutide or terzapetide, and the THR-beta agonist resmetirom.

The combination therapy of the disclosure comprising the inhibitor according to the invention and at least one more additional therapeutic agent may be administered via any route. In some embodiments, the inhibitor according to the invention and the at least one more additional therapeutic agent may be delivered orally, subcutaneously, intravenously, intranasally, transdermally, intraperitoneally, intramuscularly, intrapulmonary, vaginally, rectally, or intraocularly. In exemplary embodiments, the inhibitor according to the invention may be administered intravenously (IV) and/or subcutaneously, and the GLP-1 agonist may be administered subcutaneously.

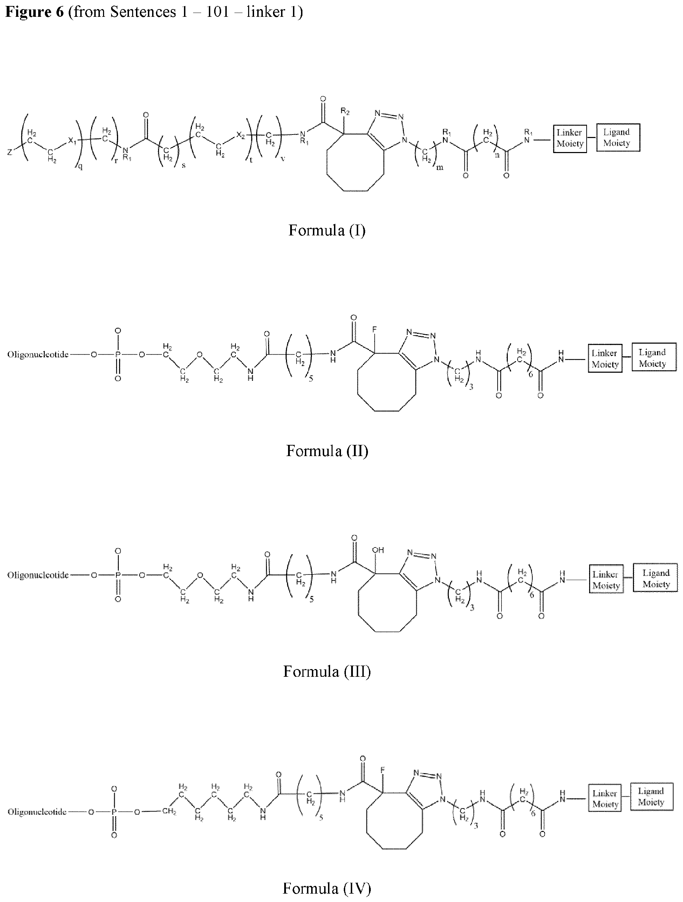

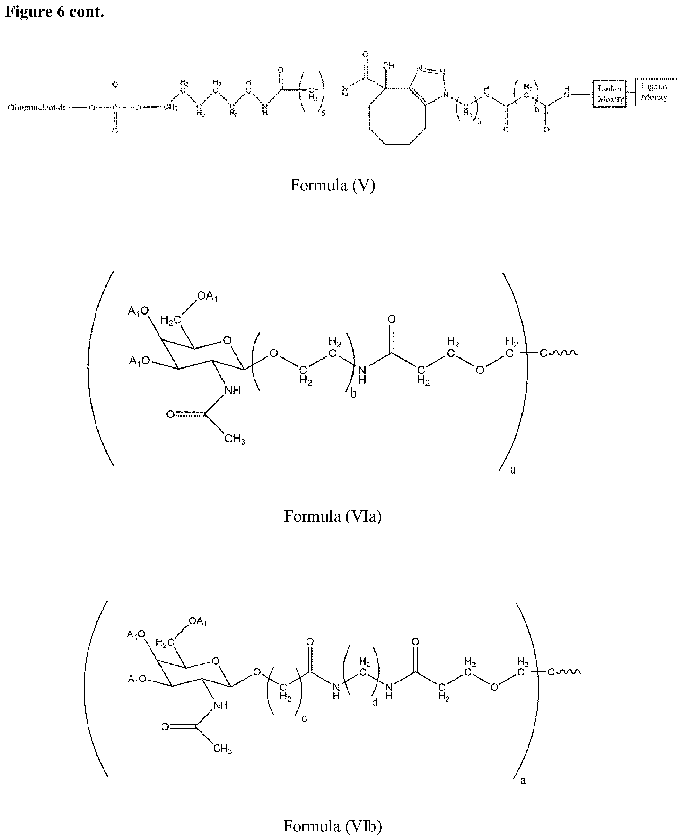

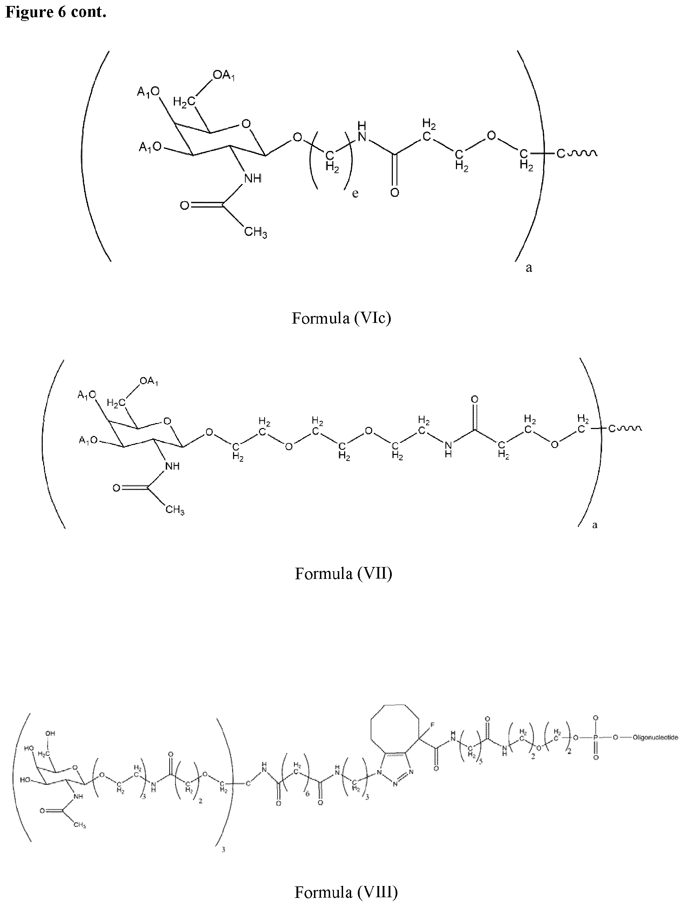

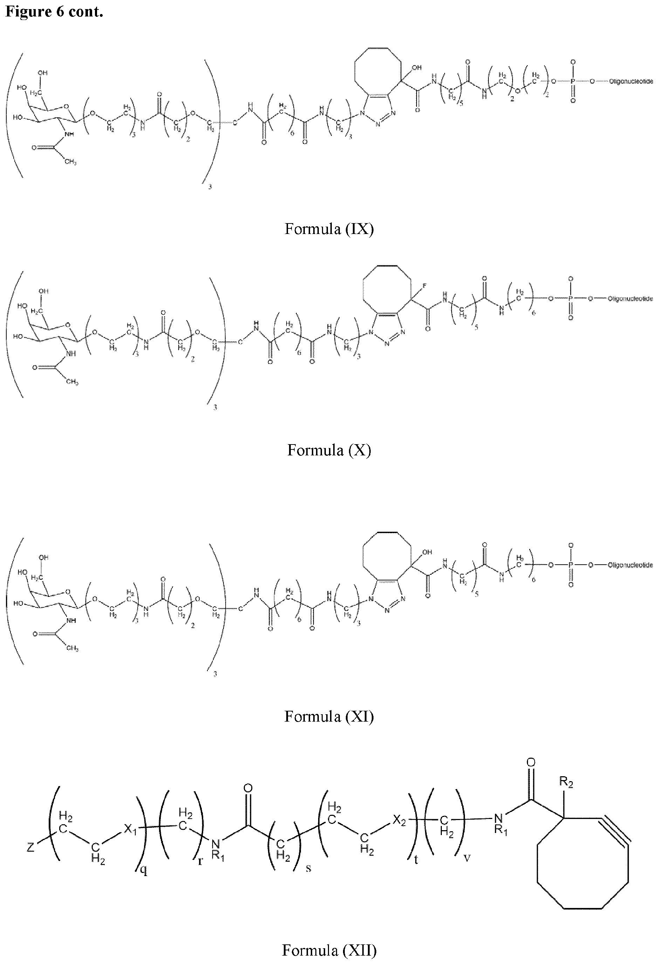

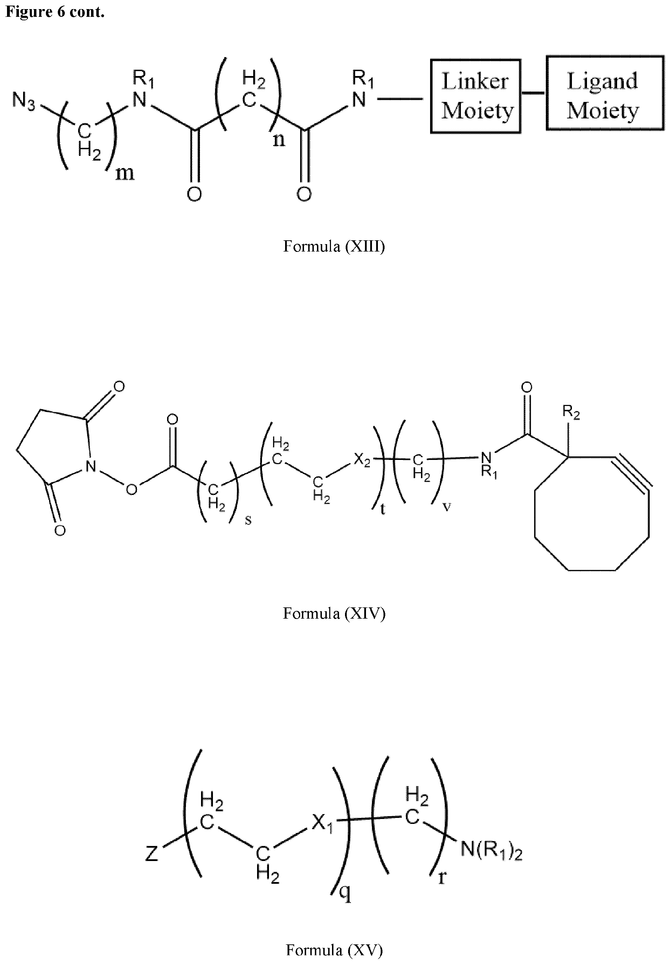

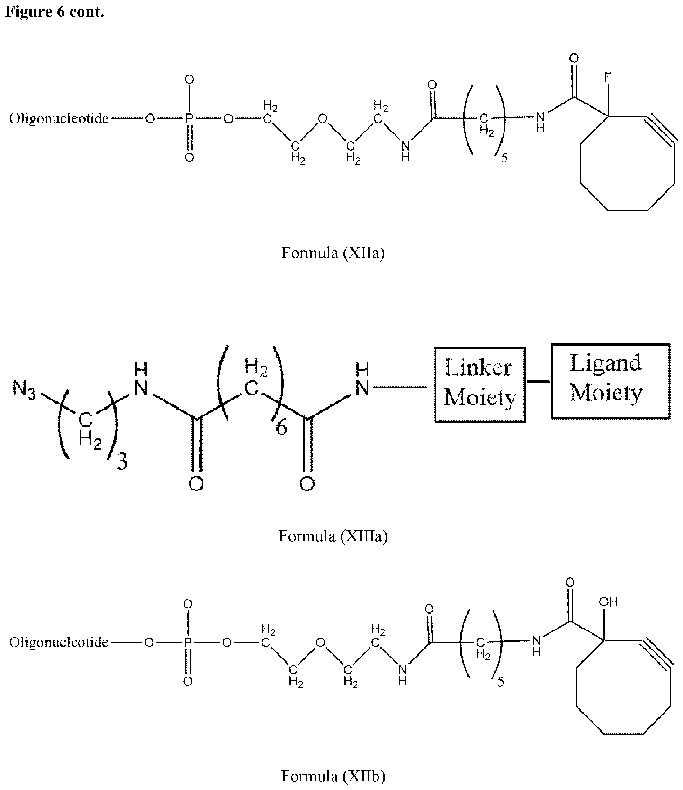

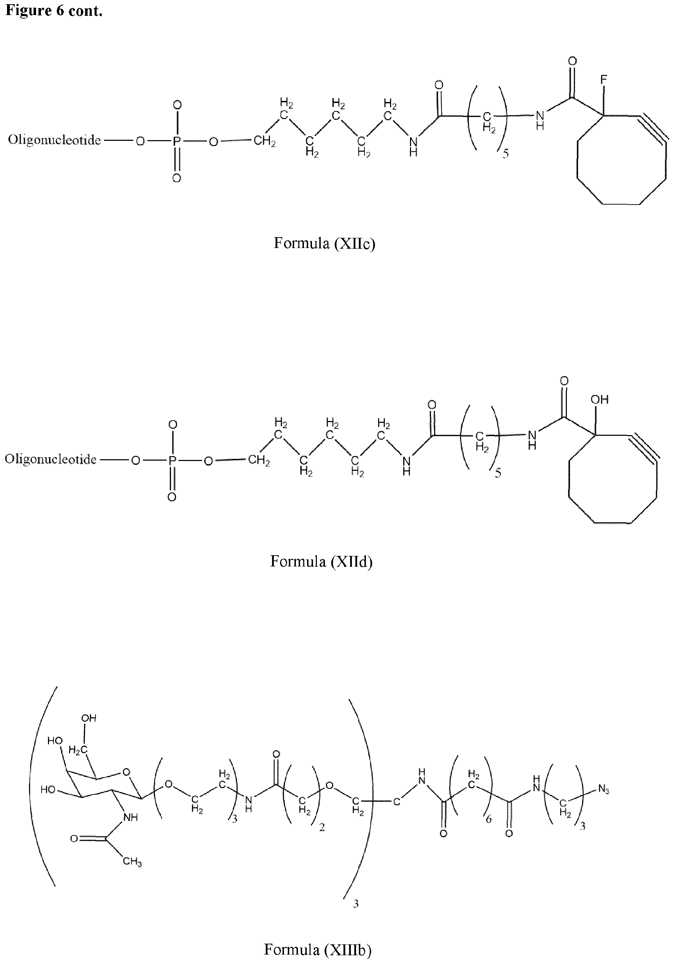

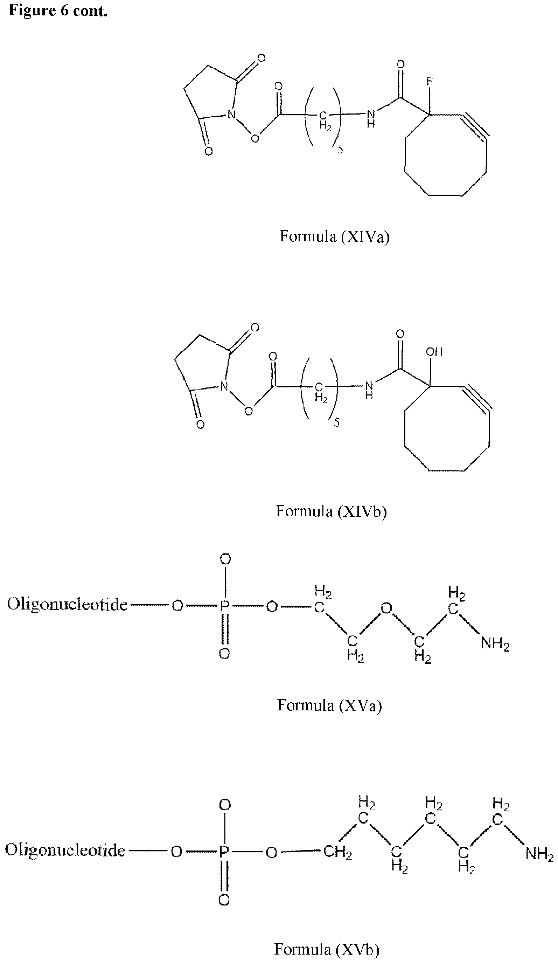

In one aspect the present invention may be applied in the compounds, processes, compositions or uses of the following Sentences numbered 1-101 (wherein reference to any Formula in the Sentences 1-101 refers only to those Formulas that are defined within Sentences 1-101. These formulae are reproduced in Figure 6)
1. A compound comprising the following structure: 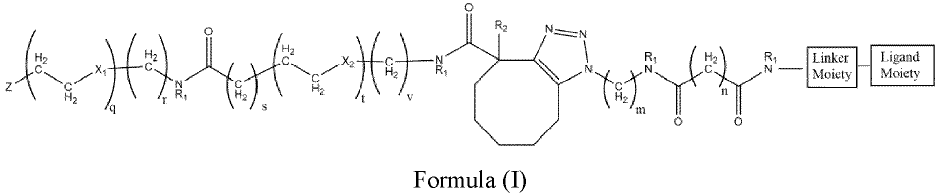 wherein:
   R₁ at each occurrence is independently selected from the group consisting of hydrogen, methyl and ethyl;
   R₂ is selected from the group consisting of hydrogen, hydroxy, -OC₁₋₃alkyl, -C(=O)OC₁₋₃alkyl, halo and nitro;
   X₁ and X₂ at each occurrence are independently selected from the group consisting of methylene, oxygen and sulfur;
   m is an integer of from 1 to 6;
   n is an integer of from 1 to 10;
   q, r, s, t, v are independently integers from 0 to 4, with the proviso that:
      (i) q and r cannot both be 0 at the same time; and
      (ii) s, t and v cannot all be 0 at the same time;
   Z is an oligonucleoside moiety.
2. A compound according to Sentence 1, wherein R₁ is hydrogen at each occurrence.
3. A compound according to Sentence 1, wherein R₁ is methyl.
4. A compound according to Sentence 1, wherein R₁ is ethyl.
5. A compound according to any of Sentences 1 to 4, wherein R₂ is hydroxy.
6. A compound according to any of Sentences 1 to 4, wherein R₂ is halo.
7. A compound according to Sentence 6, wherein R₂ is fluoro.
8. A compound according to Sentence 6, wherein R₂ is chloro.
9. A compound according to Sentence 6, wherein R₂ is bromo.
10. A compound according to Sentence 6, wherein R₂ is iodo.
11. A compound according to Sentence 6, wherein R₂ is nitro.
12. A compound according to any of Sentences 1 to 11, wherein X₁ is methylene.
13. A compound according to any of Sentences 1 to 11, wherein X₁ is oxygen.
14. A compound according to any of Sentences 1 to 11, wherein X₁ is sulfur.
15. A compound according to any of Sentences 1 to 14, wherein X₂ is methylene.
16. A compound according to any of Sentences 1 to 15, wherein X₂ is oxygen.
17. A compound according to any of Sentences 1 to 16, wherein X₂ is sulfur.
18. A compound according to any of Sentences 1 to 17, wherein m = 3.
19. A compound according to any of Sentences 1 to 18, wherein n = 6.
20. A compound according to Sentences 13 and 15, wherein X₁ is oxygen and X₂ is methylene, and preferably wherein:
   q = 1,
   r = 2,
   s = 1,
   t = 1,
   v = 1.
21. A compound according to Sentences 12 and 15, wherein both X₁ and X₂ are methylene, and preferably wherein:
   q = 1,
   r = 3,
   s = 1,
   t = 1,
   v = 1.
22. A compound according to any of Sentences 1 to 21, wherein Z is: 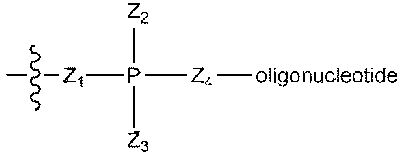 wherein:
   Z₁, Z₂, Z₃, Z₄ are independently at each occurrence oxygen or sulfur; and
   one the bonds between P and Z₂, and P and Z₃ is a single bond and the other bond is a double bond.
23. A compound according to Sentence 22, wherein said oligonucleoside is an RNA compound capable of modulating, preferably inhibiting, expression of a target gene.
24. A compound according to Sentence 23, wherein said RNA compound comprises an RNA duplex comprising first and second strands, wherein the first strand is at least partially complementary to an RNA sequence of a target gene, and the second strand is at least partially complementary to said first strand, and wherein each of the first and second strands have 5' and 3' ends.
25. A compound according to Sentence 24, wherein the RNA compound is attached at the 5' end of its second strand to the adjacent phosphate.
26. A compound according to Sentence 24, wherein the RNA compound is attached at the 3' end of its second strand to the adjacent phosphate.
27. A compound of Formula (II): 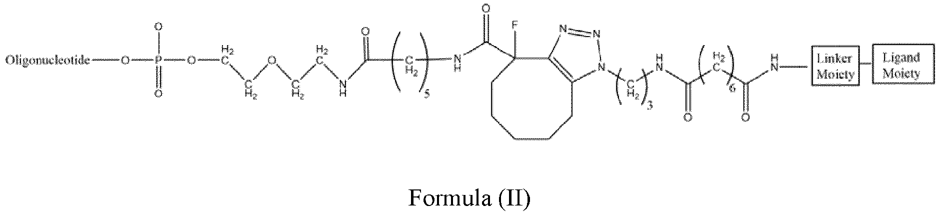
28. A compound of Formula (III): 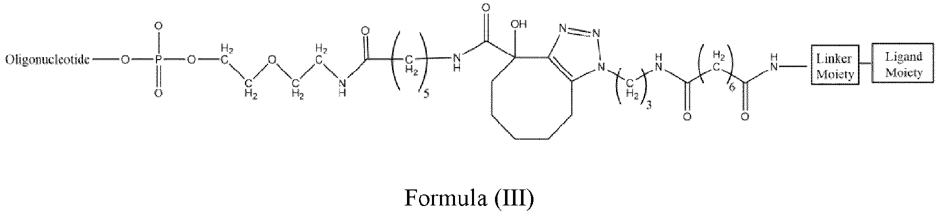
29. A compound according to Sentence 27 or 28, wherein the oligonucleoside comprises an RNA duplex comprising first and second strands, wherein the first strand is at least partially complementary to an RNA sequence of a target gene, and the second strand is at least partially complementary to said first strand, and wherein each of the first and second strands have 5' and 3' ends, and wherein said RNA duplex is attached at the 5' end of its second strand to the adjacent phosphate.
30. A composition comprising a compound of Formula (II) as defined in Sentence 27, and a compound of Formula (III) as defined in Sentence 28, optionally dependent on Sentence 29.
31. A composition according to Sentence 30, wherein said compound of Formula (III) as defined in Sentence 28 is present in an amount in the range of 10 to 15% by weight of said composition.
32. A compound of Formula (IV): 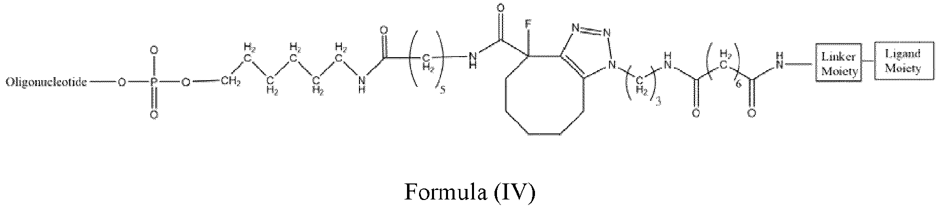
33. A compound of Formula (V): 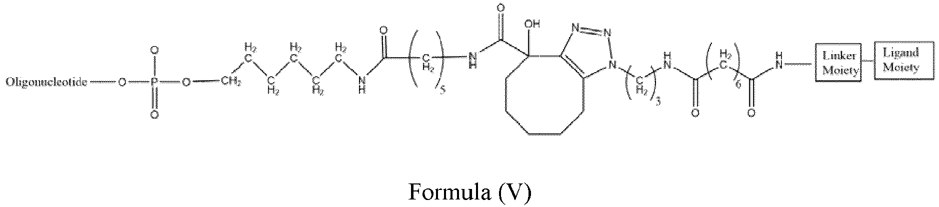
34. A compound according to Sentence 32 or 33, wherein the oligonucleoside comprises an RNA duplex comprising first and second strands, wherein the first strand is at least partially complementary to an RNA sequence of a target gene, and the second strand is at least partially complementary to said first strand, and wherein each of the first and second strands have 5' and 3' ends, and wherein said RNA duplex is attached at the 3' end of its second strand to the adjacent phosphate.
35. A composition comprising a compound of Formula (IV) as defined in Sentence 32, and a compound of Formula (V) as defined in Sentence 33, optionally dependent on Sentence 34.
36. A composition according to Sentence 35, wherein said compound of Formula (V) as defined in Sentence 33 is present in an amount in the range of 10 to 15% by weight of said composition.
37. A compound as defined in any of Sentences 1 to 29, or 32 to 34, wherein the oligonucleoside comprises an RNA duplex which further comprises one or more riboses modified at the 2' position, preferably a plurality of riboses modified at the 2' position.
38. A compound according to Sentence 37, wherein the modifications are chosen from 2'-O-methyl, 2'-deoxy-fluoro, and 2'-deoxy.
39. A compound according to any of Sentences 1 to 29, or 32 to 34, or 37 to 38, wherein the oligonucleoside further comprises one or more degradation protective moieties at one or more ends.
40. A compound according to Sentence 39, wherein said one or more degradation protective moieties are not present at the end of the oligonucleoside strand that carries the ligand moieties, and / or wherein said one or more degradation protective moieties is selected from phosphorothioate internucleoside linkages, phosphorodithioate internucleoside linkages and inverted abasic nucleosides, wherein said inverted abasic nucleosides are present at the distal end of the strand that carries the ligand moieties.
41. A compound according to any of Sentences 1 to 29, or 32 to 34, or 37 to 40, wherein said ligand moiety as depicted in Formula (I) in Sentence 1 comprises one or more ligands.
42. A compound according to Sentence 41, wherein said ligand moiety as depicted in Formula (I) in Sentence 1 comprises one or more carbohydrate ligands.
43. A compound according to Sentence 42, wherein said one or more carbohydrates can be a monosaccharide, disaccharide, trisaccharide, tetrasaccharide, oligosaccharide or polysaccharide.
44. A compound according to Sentence 43, wherein said one or more carbohydrates comprise one or more galactose moieties, one or more lactose moieties, one or more N-AcetylGalactosamine moieties, and / or one or more mannose moieties.
45. A compound according to Sentence 44, wherein said one or more carbohydrates comprise one or more N-Acetyl-Galactosamine moieties.
46. A compound according to Sentence 45, which comprises two or three N-AcetylGalactosamine moieties.
47. A compound according to any of Sentences 41 to 46, wherein said one or more ligands are attached in a linear configuration, or in a branched configuration.
48. A compound according to Sentence 47, wherein said one or more ligands are attached as a biantennary or triantennary branched configuration.
49. A compound according to Sentences 46 to 48, wherein said moiety: 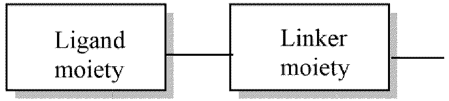 as depicted in Formula (I) in Sentence 1 is any of formulae (VIa), (VIb) or (vIc), preferably formula (VIa): 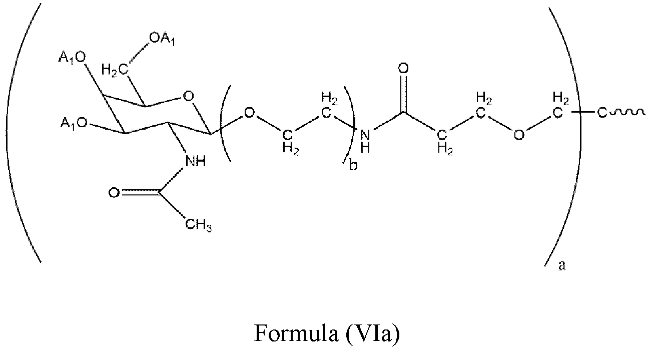 wherein:
   A_{I} is hydrogen, or a suitable hydroxy protecting group;
   a is an integer of 2 or 3; and
   b is an integer of 2 to 5; or 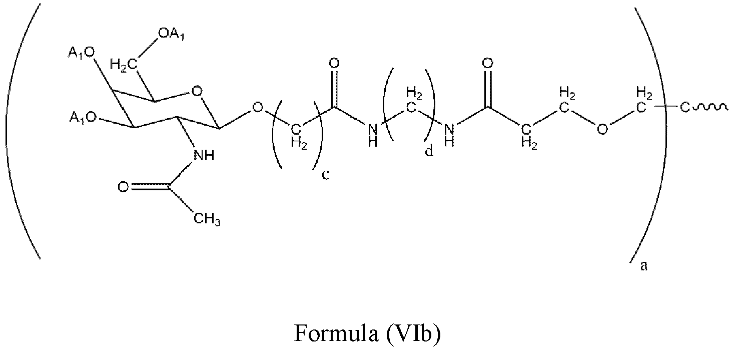
   wherein:
      A_{I} is hydrogen, or a suitable hydroxy protecting group;
      a is an integer of 2 or 3; and
      c and d are independently integers of 1 to 6; or 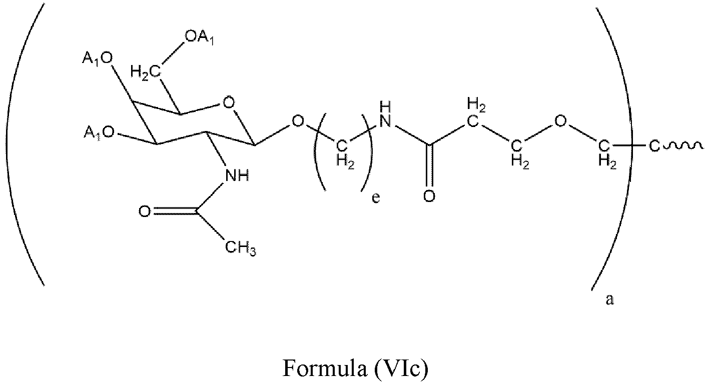 wherein:
         A_{I} is hydrogen, or a suitable hydroxy protecting group;
         a is an integer of 2 or 3; and
         e is an integer of 2 to 10.
50. A compound according to Sentences 46 to 48, wherein said moiety: 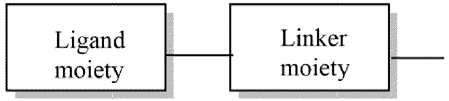 as depicted in Formula (I) in Sentence 1 is Formula (VII): 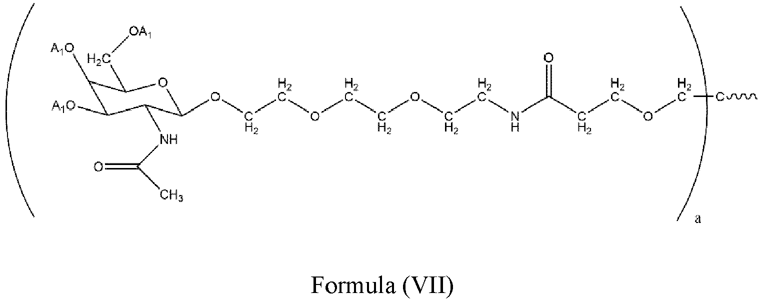 wherein:
   A_{I} is hydrogen;
   a is an integer of 2 or 3.
51. A compound according to Sentence 49 or 50, wherein a = 2.
52. A compound according to Sentence 49 or 50, wherein a = 3.
53. A compound according to Sentence 49, wherein b = 3.
54. A compound of Formula (VIII): 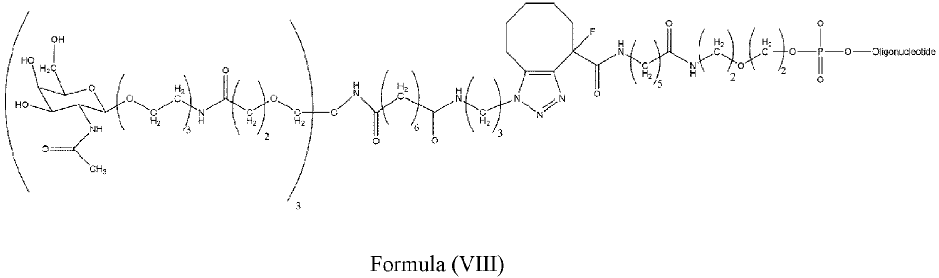
55. A compound of Formula (IX): 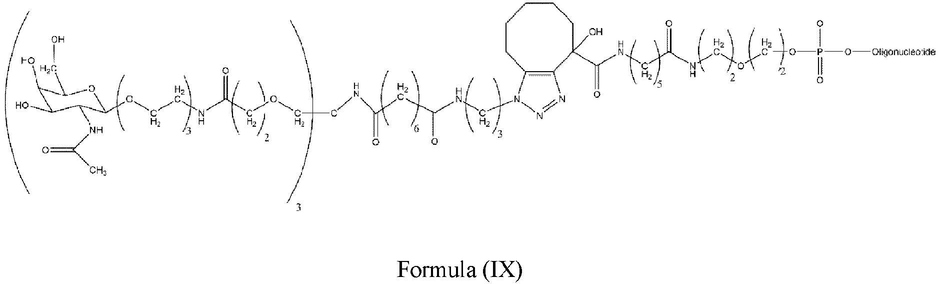
56. A compound according to Sentence 54 or 55, wherein the oligonucleoside comprises an RNA duplex comprising first and second strands, wherein the first strand is at least partially complementary to an RNA sequence of a target gene, and the second strand is at least partially complementary to said first strand, and wherein each of the first and second strands have 5' and 3' ends, and wherein said RNA duplex is attached at the 5' end of its second strand to the adjacent phosphate.
57. A composition comprising a compound of Formula (VIII) as defined in Sentence 54, and a compound of Formula (IX) as defined in Sentence 55, optionally dependent on Sentence 56.
58. A composition according to Sentence 57, wherein said compound of Formula (IX) as defined in Sentence 55 is present in an amount in the range of 10 to 15% by weight of said composition.
59. A compound of Formula (X): 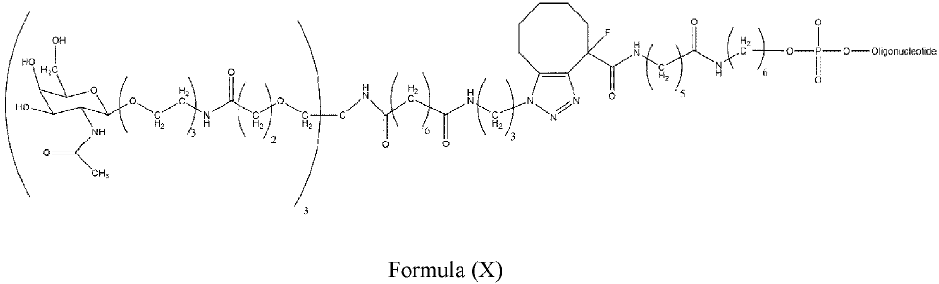
60. A compound of Formula (XI): 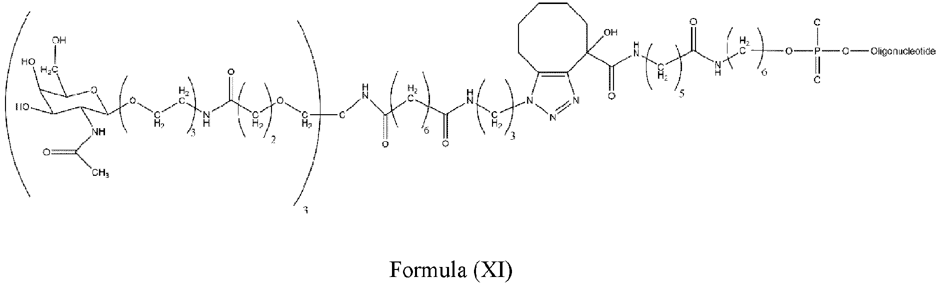
61. A compound according to Sentence 59 or 60, wherein the oligonucleoside comprises an RNA duplex comprising first and second strands, wherein the first strand is at least partially complementary to an RNA sequence of a target gene, and the second strand is at least partially complementary to said first strand, and wherein each of the first and second strands have 5' and 3' ends, and wherein said RNA duplex is attached at the 3' end of its second strand to the adjacent phosphate.
62. A composition comprising a compound of Formula (X) as defined in Sentence 59, and a compound of Formula (XI) as defined in Sentence 60, optionally dependent on Sentence 61.
63. A composition according to Sentence 62, wherein said compound of Formula (XI) as defined in Sentence 60 is present in an amount in the range of 10 to 15% by weight of said composition.
64. A compound as defined in any of Sentences 54 to 63, wherein the oligonucleoside comprises an RNA duplex which further comprises one or more riboses modified at the 2' position, preferably a plurality of riboses modified at the 2' position.
65. A compound according to Sentence 64, wherein the modifications are chosen from 2'-O-methyl, 2'-deoxy-fluoro, and 2'-deoxy.
66. A compound according to any of Sentences 54 to 65, wherein the oligonucleoside further comprises one or more degradation protective moieties at one or more ends.
67. A compound according to Sentence 66, wherein said one or more degradation protective moieties are not present at the end of the oligonucleoside strand that carries the ligand moieties, and / or wherein said one or more degradation protective moieties is selected from phosphorothioate internucleoside linkages, phosphorodithioate internucleoside linkages and inverted abasic nucleosides, wherein said inverted abasic nucleosides are present at the distal end of the strand that carries the ligand moieties, as shown in any of Formulae (VIII), (IX), (X) or (XI) in any of Sentences 54, 55, 59 or 60.
68. A process of preparing a compound according to any of Sentences 1 to 29, 32 to 34, 37 to 56, 59 to 61, and 64 to 67, and / or a composition according to any of Sentences 30, 31, 35, 36, 57, 58, 62, 63, which comprises reacting compounds of Formulae (XII) and (XIII): 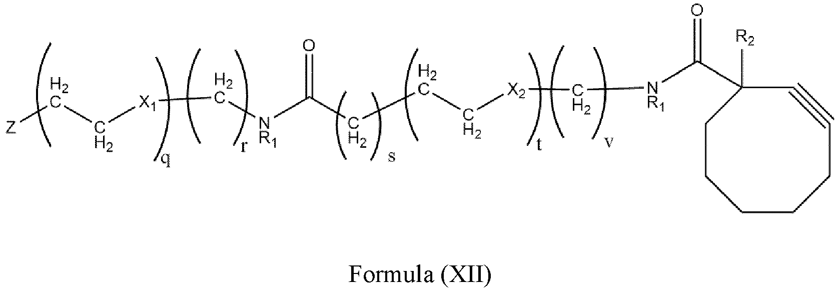 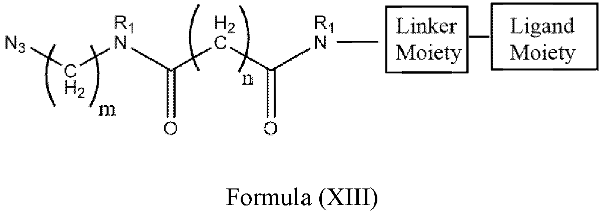 herein:
   R₁ at each occurrence is independently selected from the group consisting of hydrogen, methyl and ethyl;
   R₂ is selected from the group consisting of hydrogen, hydroxy, -OC₁₋₃alkyl, -C(=O)OC₁₋₃alkyl, halo and nitro;
   X₁ and X₂ at each occurrence are independently selected from the group consisting of methylene, oxygen and sulfur;
   m is an integer of from 1 to 6;
   n is an integer of from 1 to 10;
   q, r, s, t, v are independently integers from 0 to 4, with the proviso that:
      (i) q and r cannot both be 0 at the same time; and
      (ii) s, t and v cannot all be 0 at the same time;
   Z is an oligonucleoside moiety;
   and where appropriate carrying out deprotection of the ligand and / or annealing of a second strand for the oligonucleoside moiety.
69. A process according to Sentence 68, wherein a compound of Formula (XII) is prepared by reacting compounds of Formulae (XIV) and (XV): 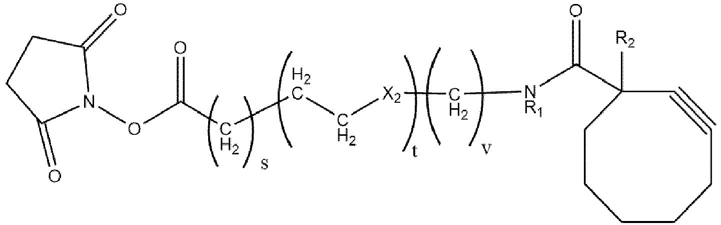 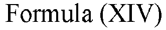 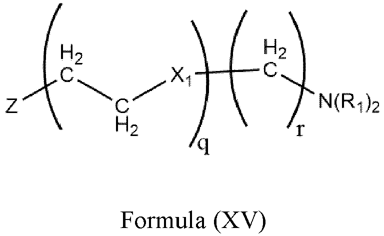
   R₁ at each occurrence is independently selected from the group consisting of hydrogen, methyl and ethyl;
   R₂ is selected from the group consisting of hydrogen, hydroxy, -OC₁₋₃alkyl, -C(=O)OC₁₋₃alkyl, halo and nitro;
   X₁ and X₂ at each occurrence are independently selected from the group consisting of methylene, oxygen and sulfur;
   q, r, s, t, v are independently integers from 0 to 4, with the proviso that:
      (i) q and r cannot both be 0 at the same time; and
      (ii) s, t and v cannot all be 0 at the same time;
   Z is an oligonucleoside moiety.
70. A process according to Sentence 68, to prepare a compound according to any of Sentences 20, 25, 27, 29, 54, 56, and / or a composition according to any of Sentences 30, 31, 57, 58, wherein:
   compound of Formula (XII) is Formula (XIIa): 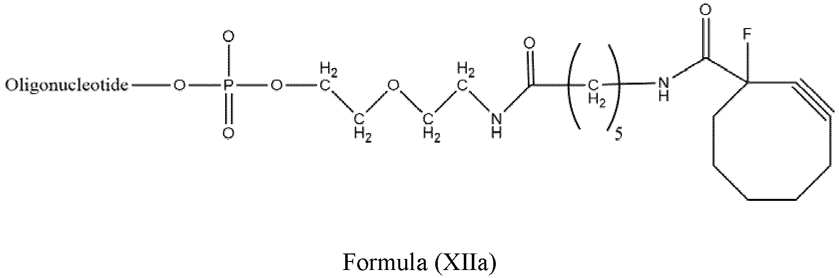
   and compound of Formula (XIII) is Formula (XIIIa): 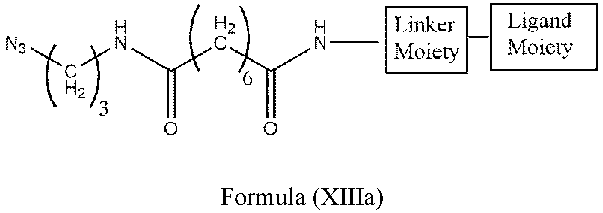
   wherein the oligonucleoside comprises an RNA duplex comprising first and second strands, wherein the first strand is at least partially complementary to an RNA sequence of a target gene, and the second strand is at least partially complementary to said first strand, and wherein each of the first and second strands have 5' and 3' ends, and wherein said RNA duplex is attached at the 5' end of its second strand to the adjacent phosphate.
71. A process according to Sentence 68, to prepare a compound according to any of Sentences 20, 25, 28, 29, 55, 56, and / or a composition according to any of Sentences 30, 31, 57, 58, wherein:
   compound of Formula (XII) is Formula (XIIb): 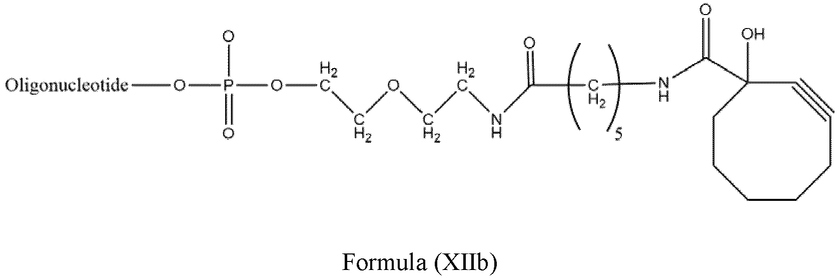
   and compound of Formula (XIII) is Formula (XIIIa): 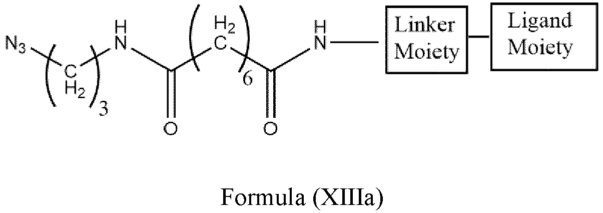
   wherein the oligonucleoside comprises an RNA duplex comprising first and second strands, wherein the first strand is at least partially complementary to an RNA sequence of a target gene, and the second strand is at least partially complementary to said first strand, and wherein each of the first and second strands have 5' and 3' ends, and wherein said RNA duplex is attached at the 5' end of its second strand to the adjacent phosphate.
72. A process according to Sentence 68, to prepare a compound according to any of Sentences 21, 26, 32, 34, 59, 61, and / or a composition according to any of Sentences 35, 36, 62, 63, wherein:
   compound of Formula (XII) is Formula (XIIc): 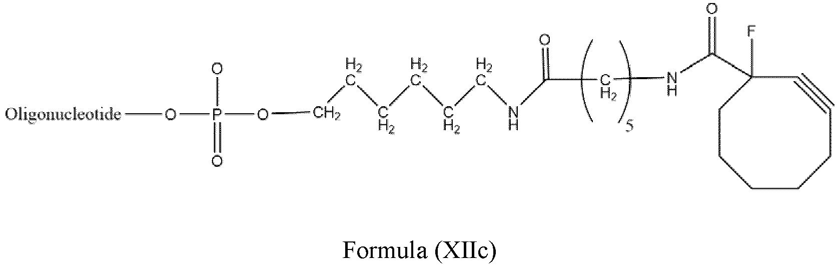
   and compound of Formula (XIII) is Formula (XIIIa): 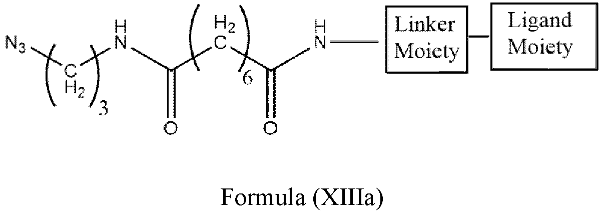
   wherein the oligonucleoside comprises an RNA duplex comprising first and second strands, wherein the first strand is at least partially complementary to an RNA sequence of a target gene, and the second strand is at least partially complementary to said first strand, and wherein each of the first and second strands have 5' and 3' ends, and wherein said RNA duplex is attached at the 3' end of its second strand to the adjacent phosphate.
73. A process according to Sentence 68, to prepare a compound according to any of Sentences 21, 26, 33, 34, 60, 61, and / or a composition according to any of Sentences 35, 36, 62, 63, wherein:
   compound of Formula (XII) is Formula (XIId): 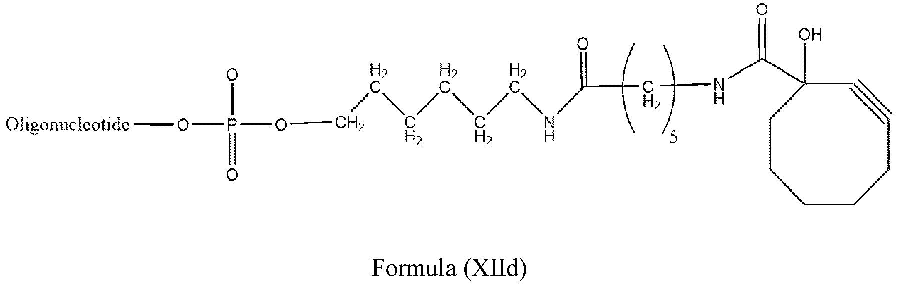
   and compound of Formula (XIII) is Formula (XIIIa): 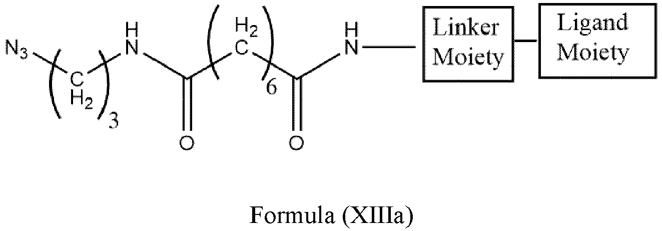
   wherein the oligonucleoside comprises an RNA duplex comprising first and second strands, wherein the first strand is at least partially complementary to an RNA sequence of a target gene, and the second strand is at least partially complementary to said first strand, and wherein each of the first and second strands have 5' and 3' ends, and wherein said RNA duplex is attached at the 3' end of its second strand to the adjacent phosphate.
74. A process according to any of Sentences 70 to 73, wherein:
   compound of Formula (XIIIa) is Formula (XIIIb): 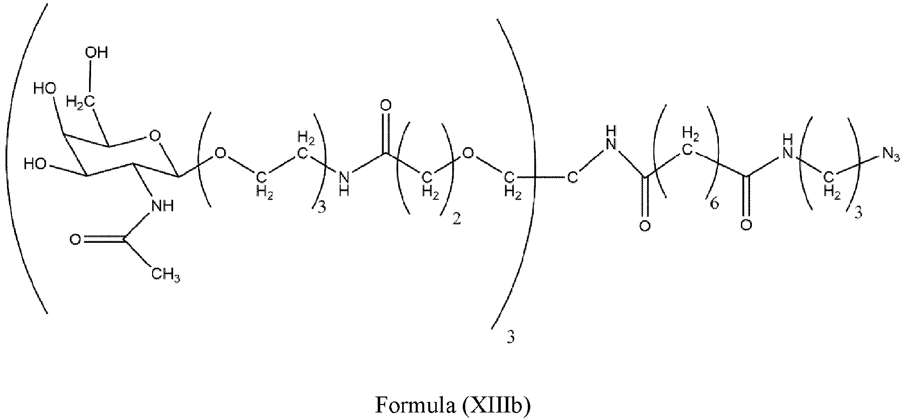
75. A process according to Sentences 69, as dependent on Sentences 70 to 73, wherein:
   compound of Formula (XIV) is either Formula (XIVa) or Formula (XIVb): 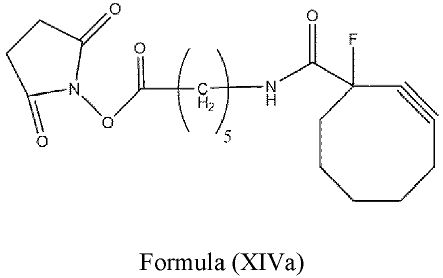 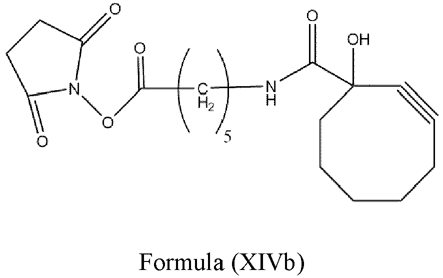
   and compound of Formula (XV) is either Formula (XVa) or Formula (XIVb): 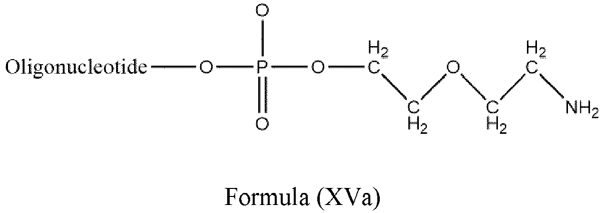 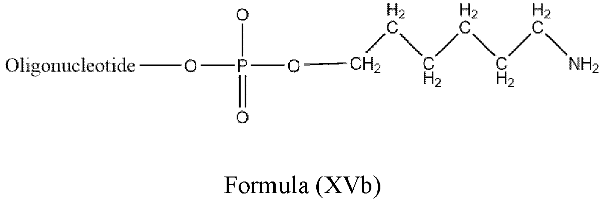
   wherein the oligonucleoside comprises an RNA duplex comprising first and second strands, wherein the first strand is at least partially complementary to an RNA sequence of a target gene, and the second strand is at least partially complementary to said first strand, and wherein each of the first and second strands have 5' and 3' ends, and wherein (i) said RNA duplex is attached at the 5' end of its second strand to the adjacent phosphate in Formula (XVa), or (ii) said RNA duplex is attached at the 3' end of its second strand to the adjacent phosphate in Formula (XVb).
76. A compound of Formula (XII): 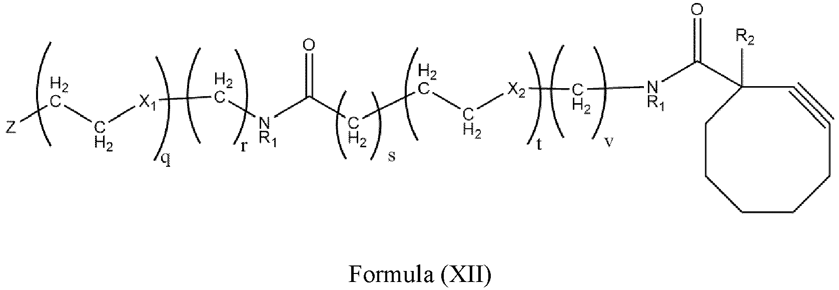 wherein:
   R₁ at each occurrence is independently selected from the group consisting of hydrogen, methyl and ethyl;
   R₂ is selected from the group consisting of hydrogen, hydroxy, -OC₁₋₃alkyl, -C(=O)OC₁₋₃alkyl, halo and nitro;
   X₁ and X₂ at each occurrence are independently selected from the group consisting of methylene, oxygen and sulfur;
   q, r, s, t, v are independently integers from 0 to 4, with the proviso that:
      (i) q and r cannot both be 0 at the same time; and
      (ii) s, t and v cannot all be 0 at the same time;
   Z is an oligonucleoside moiety.
77. A compound of Formula (XIIa): 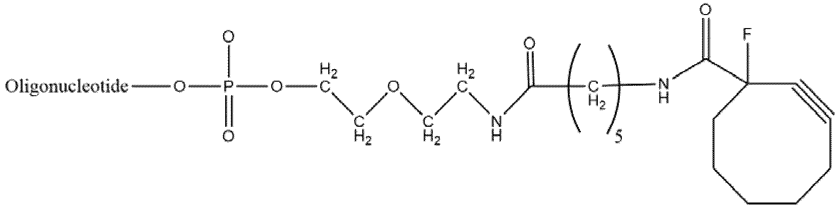 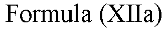
78. A compound of Formula (XIIb): 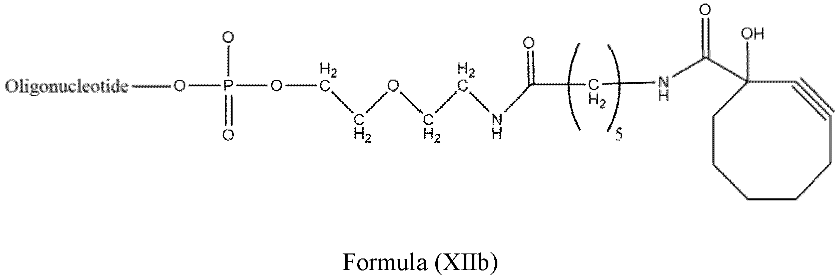
79. A compound of Formula (XIIc): 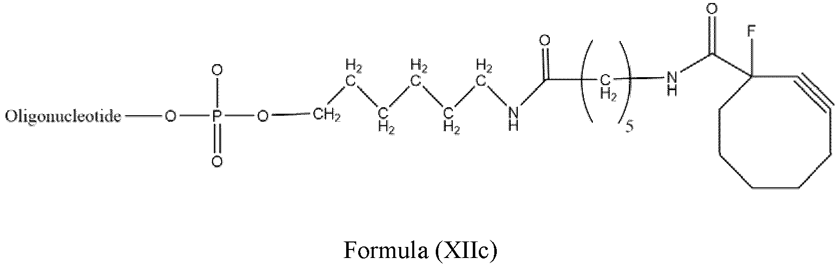
80. A compound of Formula (XIId): 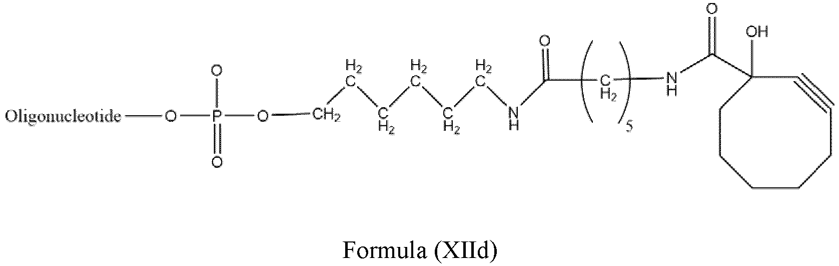
81. A compound of Formula (XIII): 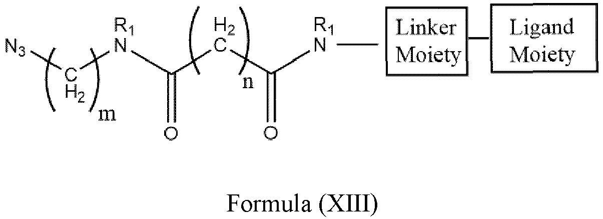 wherein:
   R₁ at each occurrence is independently selected from the group consisting of hydrogen, methyl and ethyl;
   m is an integer of from 1 to 6;
   n is an integer of from 1 to 10.
82. A compound of Formula (XIIIa): 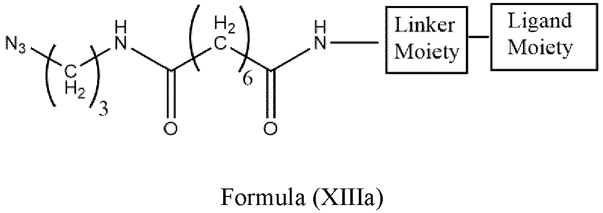
83. A compound of Formula (XIIIb): 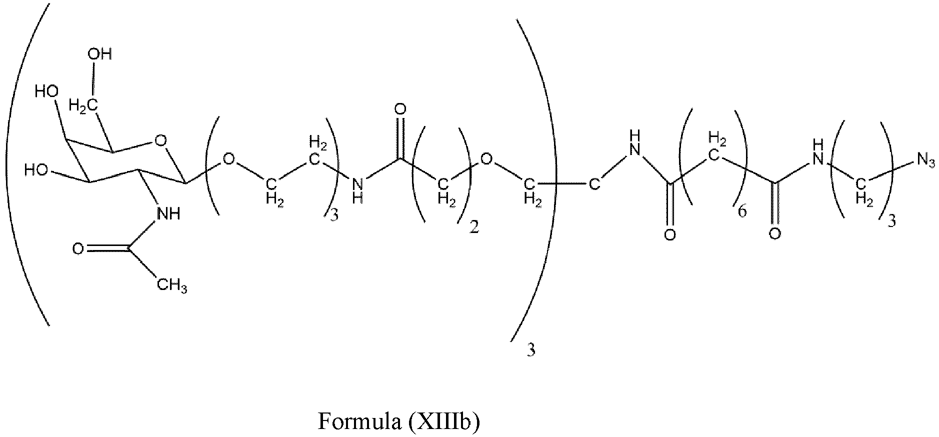
84. A compound of Formula (XIV): 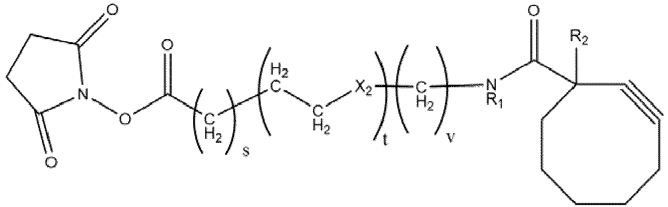 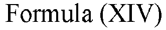 wherein:
   R₁ is selected from the group consisting of hydrogen, methyl and ethyl;
   R₂ is selected from the group consisting of hydrogen, hydroxy, -OC₁₋₃alkyl, -C(=O)OC₁₋₃alkyl, halo and nitro;
   X₂ is selected from the group consisting of methylene, oxygen and sulfur;
   s, t, v are independently integers from 0 to 4, with the proviso that s, t and v cannot all be 0 at the same time.
85. A compound of Formula (XIVa): 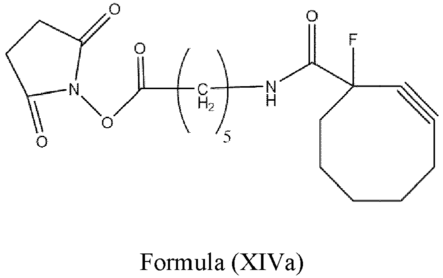
86. A compound of Formula (XIVb): 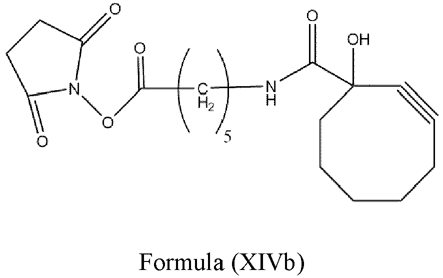
87. A compound of Formula (XV): 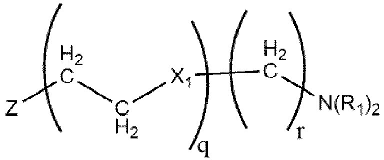 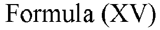 wherein:
   R₁ at each occurrence is independently selected from the group consisting of hydrogen, methyl and ethyl;
   X₁ is selected from the group consisting of methylene, oxygen and sulfur;
   q and r are independently integers from 0 to 4, with the proviso that q and r cannot both be 0 at the same time;
   Z is an oligonucleoside moiety.
88. A compound of formula (XVa): 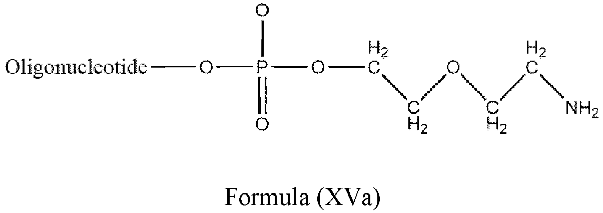
89. A compound of Formula (XVb): 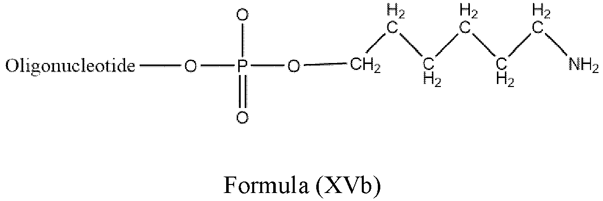
90. Use of a compound according to any of Sentences 76, 81 to 84, 87, for the preparation of a compound according to any of Sentences 1 to 29, 32 to 34, 37 to 56, 59 to 61, and 64 to 67, and / or a composition according to any of Sentences 30, 31, 35, 36, 57, 58, 62 and 63.
91. Use of a compound according to Sentence 85, for the preparation of a compound according to any of Sentences 1 to 29, 32 to 34, 37 to 56, 59 to 61, and 64 to 67, and / or a composition according to any of Sentences 30, 31, 35, 36, 57, 58, 62 and 63, wherein R₂ = F.
92. Use of a compound according to Sentence 86, for the preparation of a compound according to any of Sentences 1 to 29, 32 to 34, 37 to 56, 59 to 61, and 64 to 67, and / or a composition according to any of Sentences 30, 31, 35, 36, 57, 58, 62 and 63, wherein R₂ = OH.
93. Use of a compound according to Sentence 77, for the preparation of a compound according to any of Sentences 20, 25, 27, 29, 54, 56, and / or a composition according to any of Sentences 30, 31, 57, 58.
94. Use of a compound according to Sentence 78, for the preparation of a compound according to any of Sentences 20, 25, 28, 29, 55, 56, and / or a composition according to any of Sentences 30, 31, 57, 58.
95. Use of a compound according to Sentence 79, for the preparation of a compound according to any of Sentences 21, 26, 32, 34, 59, 61, and / or a composition according to any of Sentences 35, 36, 62, 63.
96. Use of a compound according to Sentence 80, for the preparation of a compound according to any of Sentences 21, 26, 33, 34, 60, 61, and / or a composition according to any of Sentences 35, 36, 62, 63.
97. Use of a compound according to Sentence 88, for the preparation of a compound according to any of Sentences 20, 25, 27 to 29, 54 to 56, and / or a composition according to any of Sentences 30, 31, 57, 58.
98. Use of a compound according to Sentence 89, for the preparation of a compound according to any of Sentences 21, 26, 32 to 34, 59 to 61, and / or a composition according to any of Sentences 35, 36, 62, 63.
99. A compound or composition obtained, or obtainable by a process according to any of Sentences 68 to 75.
100. A pharmaceutical composition comprising of a compound according to any of Sentences 1 to 29, 32 to 34, 37 to 56, 59 to 61, and 64 to 67, and / or a composition according to any of Sentences 30, 31, 35, 36, 57, 58, 62 and 63, together with a pharmaceutically acceptable carrier, diluent or excipient.
   A compound according to any of Sentences 1 to 29, 32 to 34, 37 to 56, 59 to 61, and 64 to 67, and / or a composition according to any of Sentences 30, 31, 35, 36, 57, 58, 62 and 63, for use in therapy.

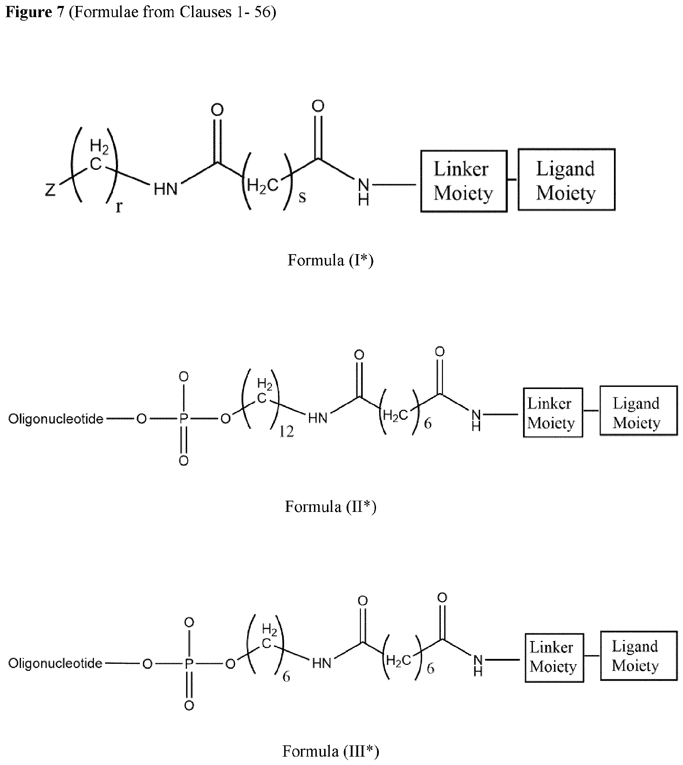

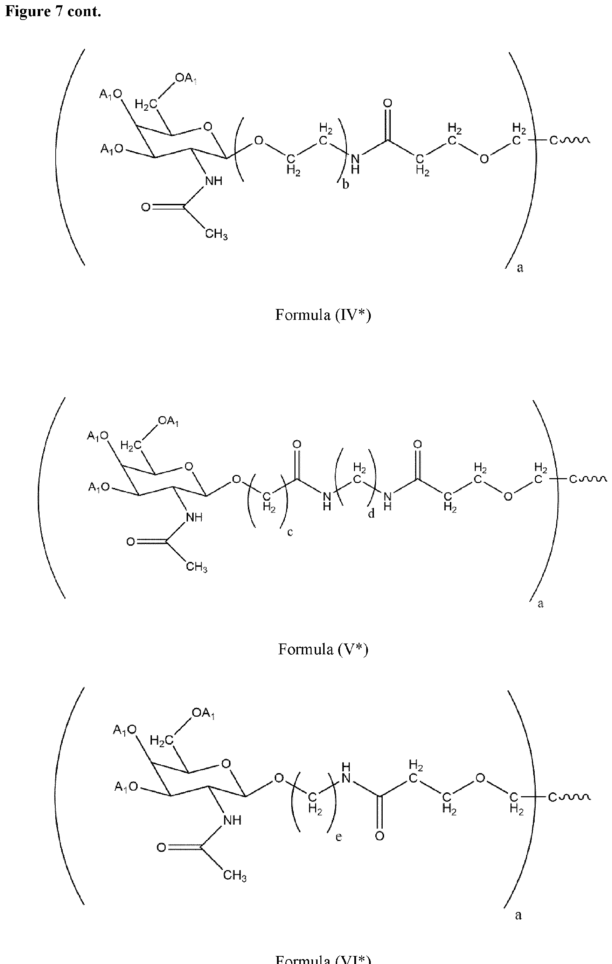

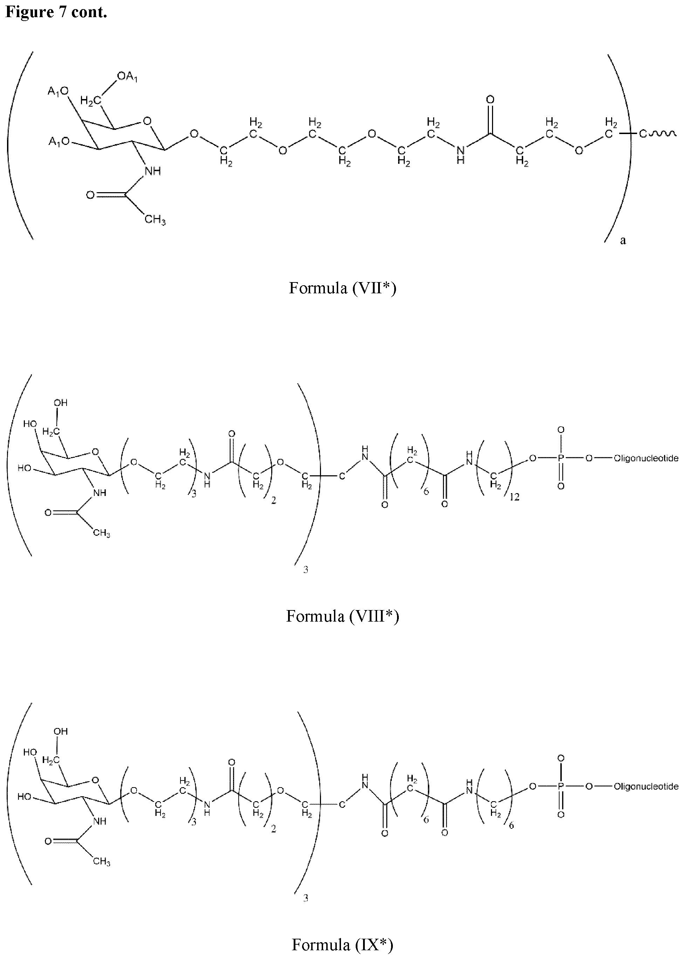

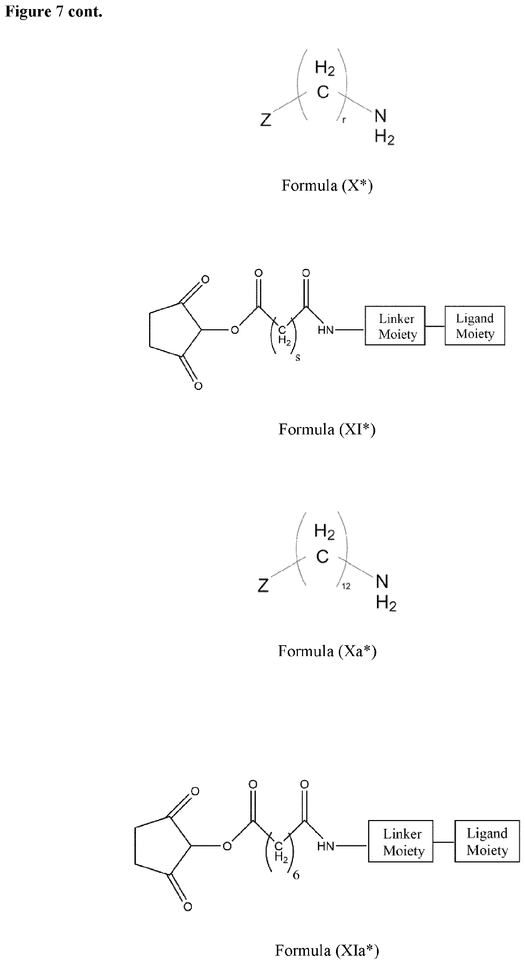

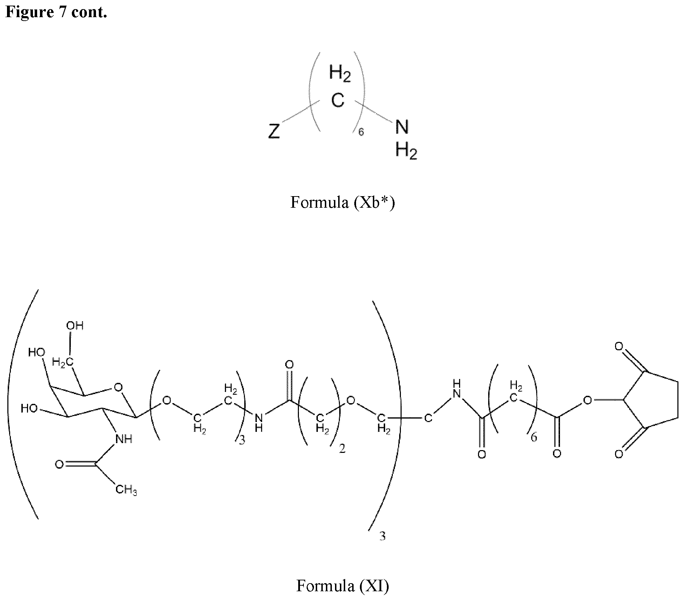

In another aspect the present invention may be applied in the compounds, processes, compositions or uses of the following Clauses numbered 1-56 (wherein reference to any Formula in the Clauses refers only to those Formulas that are defined within Clause 1-56. These formulae are reproduced in Figure 7).
1. A compound comprising the following structure: 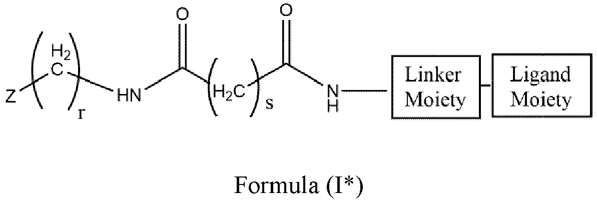 wherein:
   r and s are independently an integer selected from 1 to 16; and
   Z is an oligonucleoside moiety.
2. A compound according to Clause 1, wherein s is an integer selected from 4 to 12.
3. A compound according to Clause 2, wherein s is 6.
4. A compound according to any of Clauses 1 to 3, wherein r is an integer selected from 4 to 14.
5. A compound according to Clause 4, wherein r is 6.
6. A compound according to Clause 4, wherein r is 12.
7. A compound according to Clause 5, which is dependent on Clause 3.
8. A compound according to Clause 6, which is dependent on Clause 3.
9. A compound according to any of Clauses 1 to 8, wherein Z is: 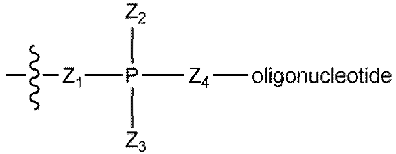 wherein:
   Z₁, Z₂, Z₃, Z₄ are independently at each occurrence oxygen or sulfur; and
   one the bonds between P and Z₂, and P and Z₃ is a single bond and the other bond is a double bond.
10. A compound according to any of Clauses 1 to 9, wherein said oligonucleoside is an RNA compound capable of modulating, preferably inhibiting, expression of a target gene.
11. A compound according to any of Clause 10, wherein said RNA compound comprises an RNA duplex comprising first and second strands, wherein the first strand is at least partially complementary to an RNA sequence of a target gene, and the second strand is at least partially complementary to said first strand, and wherein each of the first and second strands have 5' and 3' ends.
12. A compound according to Clause 11, preferably also dependent on Clauses 3 and 6, wherein the RNA compound is attached at the 5' end of its second strand to the adjacent phosphate.
13. A compound according to Clause 11, preferably also dependent on Clauses 3 and 5, wherein the RNA compound is attached at the 3' end of its second strand to the adjacent phosphate.
14. A compound of Formula (II*), preferably dependent on Clause 12: 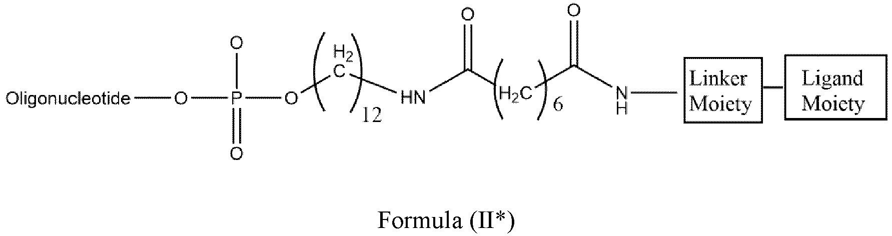
15. A compound of Formula (III*), preferably dependent on Clause 13: 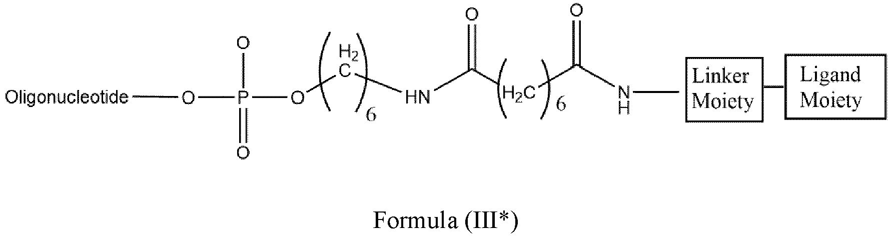
16. A compound as defined in any of Clauses 1 to 15, wherein the oligonucleoside comprises an RNA duplex which further comprises one or more riboses modified at the 2' position, preferably a plurality of riboses modified at the 2' position.
17. A compound according to Clause 16, wherein the modifications are chosen from 2'-O-methyl, 2'-deoxy-fluoro, and 2'-deoxy.
18. A compound according to any of Clauses 1 to 17, wherein the oligonucleoside further comprises one or more degradation protective moieties at one or more ends.
19. A compound according to Clause 18, wherein said one or more degradation protective moieties are not present at the end of the oligonucleoside strand that carries the linker / ligand moieties, and / or wherein said one or more degradation protective moieties is selected from phosphorothioate internucleoside linkages, phosphorodithioate internucleoside linkages and inverted abasic nucleosides, wherein said inverted abasic nucleosides are present at the distal end of the same strand to the end that carries the linker / ligand moieties.
20. A compound according to any of Clauses 1 to 19, wherein said ligand moiety as depicted in Formula (I*) in Clause 1 comprises one or more ligands.
21. A compound according to Clause 20, wherein said ligand moiety as depicted in Formula (I*) in Clause 1 comprises one or more carbohydrate ligands.
22. A compound according to Clause 21, wherein said one or more carbohydrates can be a monosaccharide, disaccharide, trisaccharide, tetrasaccharide, oligosaccharide or polysaccharide.
23. A compound according to Clause 22, wherein said one or more carbohydrates comprise one or more galactose moieties, one or more lactose moieties, one or more N-AcetylGalactosamine moieties, and / or one or more mannose moieties.
24. A compound according to Clause 23, wherein said one or more carbohydrates comprise one or more N-Acetyl-Galactosamine moieties.
25. A compound according to Clause 24, which comprises two or three N-AcetylGalactosamine moieties.
26. A compound according to any of the preceding Clauses, wherein said one or more ligands are attached in a linear configuration, or in a branched configuration.
27. A compound according to Clause 26, wherein said one or more ligands are attached as a biantennary or triantennary branched configuration.
28. A compound according to Clauses 20 to 27, wherein said moiety: 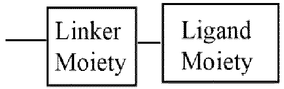
   as depicted in Formula (I*) in Clause 1 is any of Formulae (IV*), (V*) or (VI*), preferably Formula (IV*): 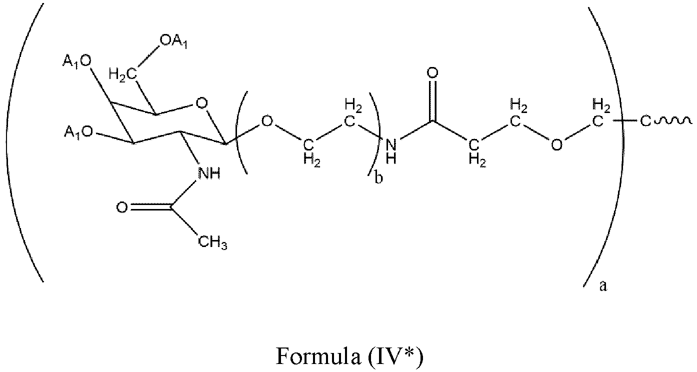
   wherein:
      A_{I} is hydrogen, or a suitable hydroxy protecting group;
      a is an integer of 2 or 3; and
      b is an integer of 2 to 5; or 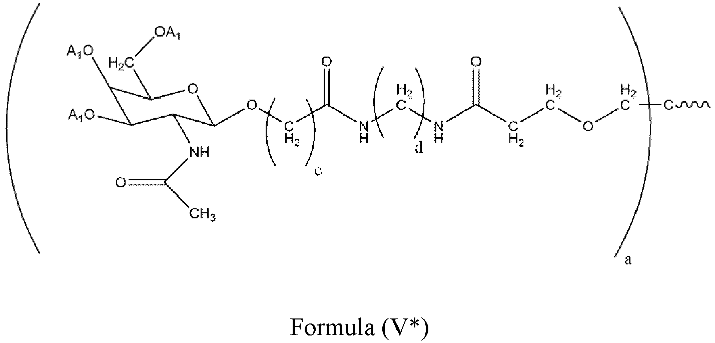
   wherein:
      A_{I} is hydrogen, or a suitable hydroxy protecting group;
      a is an integer of 2 or 3; and
      c and d are independently integers of 1 to 6; or 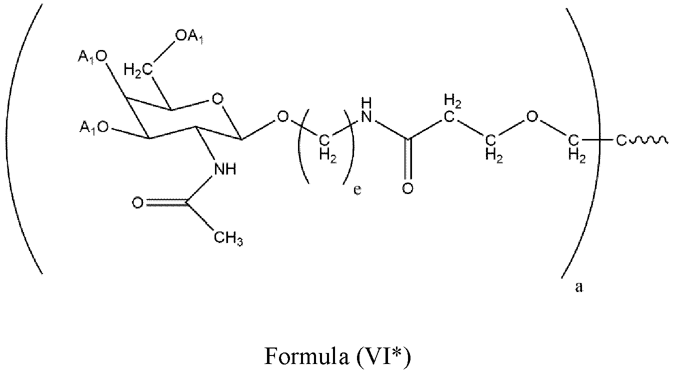
   wherein:
      A_{I} is hydrogen, or a suitable hydroxy protecting group;
      a is an integer of 2 or 3; and
      e is an integer of 2 to 10.
29. A compound according to any of Clauses 1 to 28, wherein said moiety: 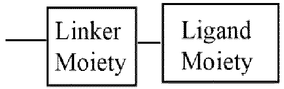
   as depicted in Formula (I*) in Clause 1 is Formula (VII*): 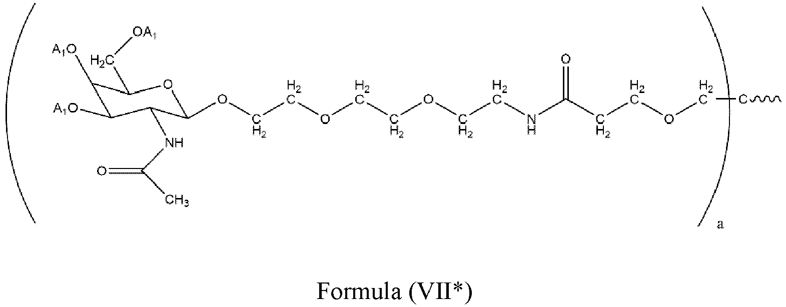
   wherein:
      A_{I} is hydrogen;
      a is an integer of 2 or 3.
30. A compound according to Clause 28 or 29, wherein a = 2.
31. A compound according to Clause 28 or 29, wherein a = 3.
32. A compound according to Clause 28, wherein b = 3.
33. A compound of Formula (VIII*): 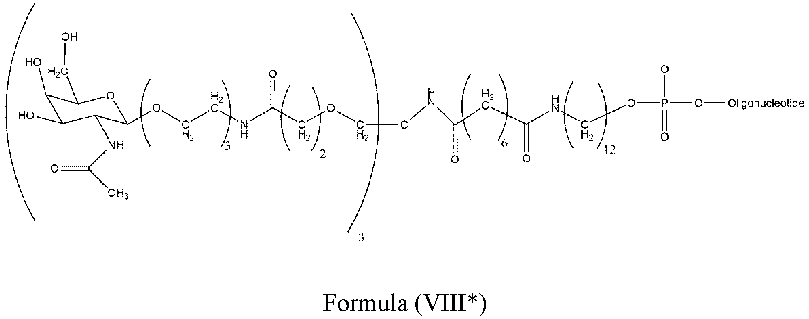
34. A compound of Formula (IX*): 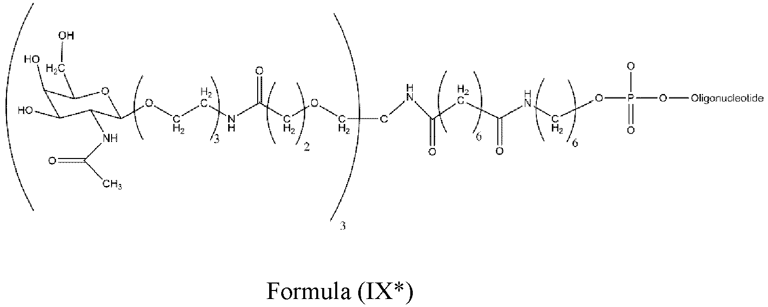
35. A compound according to Clause 33 or 34, wherein the oligonucleoside comprises an RNA duplex which further comprises one or more riboses modified at the 2' position, preferably a plurality of riboses modified at the 2' position.
36. A compound according to Clause 35, wherein the modifications are chosen from 2'-O-methyl, 2'-deoxy-fluoro, and 2'-deoxy.
37. A compound according to any of Clauses 33 to 36, wherein the oligonucleoside further comprises one or more degradation protective moieties at one or more ends.
38. A compound according to Clause 37, wherein said one or more degradation protective moieties are not present at the end of the oligonucleoside strand that carries the linker / ligand moieties, and / or wherein said one or more degradation protective moieties is selected from phosphorothioate internucleoside linkages, phosphorodithioate internucleoside linkages and inverted abasic nucleosides, wherein said inverted abasic nucleosides are present at the distal end of the same strand to the end that carries the linker / ligand moieties.
39. A compound according to Clause 33, wherein the oligonucleoside comprises an RNA duplex comprising first and second strands, wherein the first strand is at least partially complementary to an RNA sequence of a target gene, and the second strand is at least partially complementary to said first strand, and wherein each of the first and second strands have 5' and 3' ends, and wherein said RNA duplex is attached at the 5' end of its second strand to the adjacent phosphate.
40. A compound according to Clause 34, wherein the oligonucleoside comprises an RNA duplex comprising first and second strands, wherein the first strand is at least partially complementary to an RNA sequence of a target gene, and the second strand is at least partially complementary to said first strand, and wherein each of the first and second strands have 5' and 3' ends, and wherein said RNA duplex is attached at the 3' end of its second strand to the adjacent phosphate.
41. A process of preparing a compound according to any of Clauses 1 to 40, which comprises reacting compounds of Formulae (X*) and (XI*): 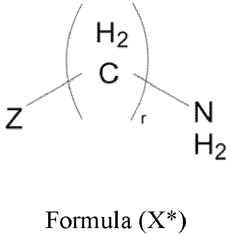 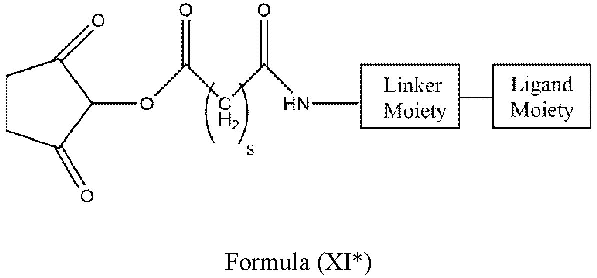 wherein:
   r and s are independently an integer selected from 1 to 16; and
   Z is an oligonucleoside moiety;
   and where appropriate carrying out deprotection of the ligand and / or annealing of a second strand for the oligonucleoside.
42. A process according to Clause 41, to prepare a compound according to any of Clauses 6, 8 to 14, 16 to 33, and 35 to 40, wherein:
   compound of Formula (X*) is Formula (Xa*): 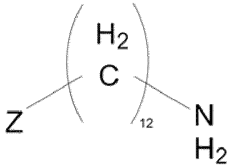 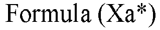
   and compound of formula (XI*) is Formula (XIa*): 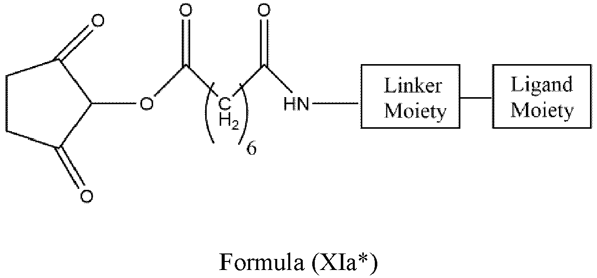
   wherein the oligonucleoside comprises an RNA duplex comprising first and second strands, wherein the first strand is at least partially complementary to an RNA sequence of a target gene, and the second strand is at least partially complementary to said first strand, and wherein each of the first and second strands have 5' and 3' ends, and wherein said RNA duplex is attached at the 5' end of its second strand to the adjacent phosphate.
43. A process according to Clause 41, to prepare a compound according to any of Clauses 5, 7, 9 to 13, 15 to 32, and 34 to 40, wherein:
   compound of Formula (X*) is Formula (Xb*): 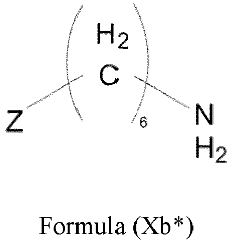
   and compound of formula (XI*) is Formula (XIa*): 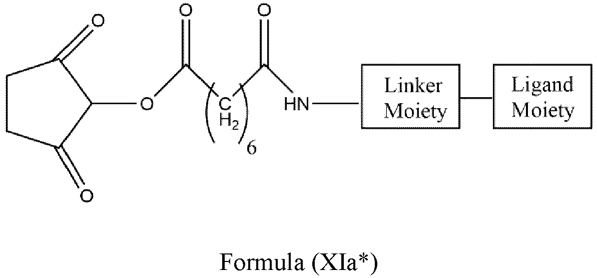
   wherein the oligonucleoside comprises an RNA duplex comprising first and second strands, wherein the first strand is at least partially complementary to an RNA sequence of a target gene, and the second strand is at least partially complementary to said first strand, and wherein each of the first and second strands have 5' and 3' ends, and wherein said RNA duplex is attached at the 3' end of its second strand to the adjacent phosphate.
44. A process according to Clauses 42 or 43, wherein:
   compound of Formula (XIa*) is Formula (XIb*): 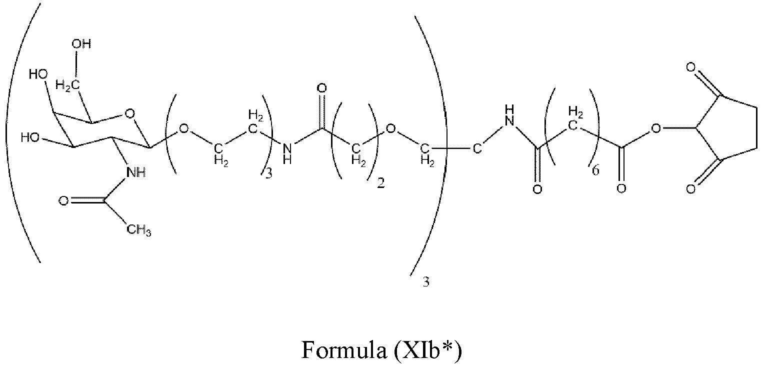
45. A compound of Formula (X*): 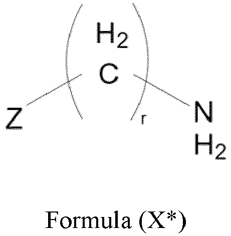 wherein:
   r is independently an integer selected from 1 to 16; and
   Z is an oligonucleoside moiety.
46. A compound of Formula (Xa*): 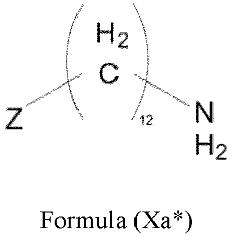
47. A compound of Formula (Xb*): 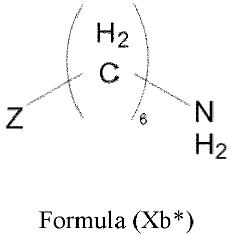
48. A compound of Formula (XI*): 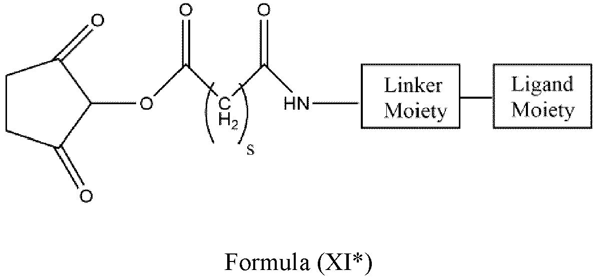 wherein:
   s is independently an integer selected from 1 to 16; and
   Z is an oligonucleoside moiety.
49. A compound of Formula (XIa*): 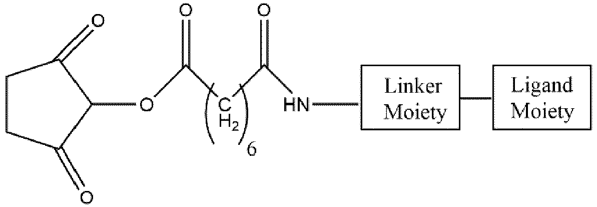 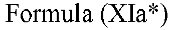
50. A compound of Formula (XIb*): 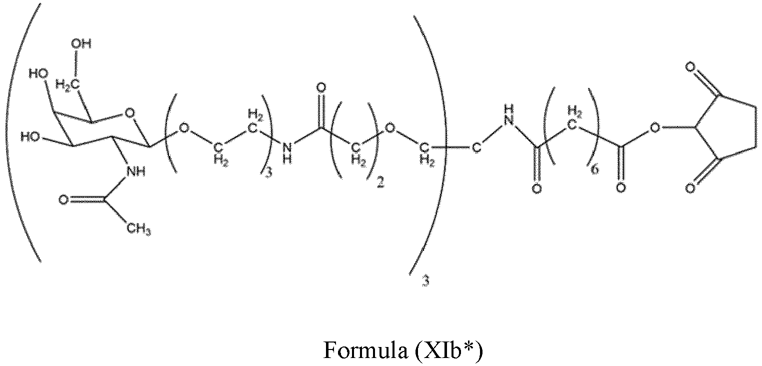
51. Use of a compound according to any of Clauses 45 and 48 to 50, for the preparation of a compound according to any of Clauses 1 to 40.
52. Use of a compound according to Clause 46, for the preparation of a compound according to any of Clauses 6, 8 to 14, 16 to 33, and 35 to 40.
53. Use of a compound according to Clause 47, for the preparation of a compound according to any of Clauses 5, 7, 9 to 13, 15 to 32, and 34 to 40.
54. A compound or composition obtained, or obtainable by a process according to any of Clauses 41 to 44.
55. A pharmaceutical composition comprising of a compound according to any of Clauses 1 to 40, together with a pharmaceutically acceptable carrier, diluent or excipient.

A compound according to any of Clauses 1 to 40, for use in therapy.

### EXAMPLES

The invention will be more fully understood by reference to the following examples. They should not, however, be construed as limiting the scope of the invention. It is understood that the examples and embodiments described herein are for illustrative purposes only and that various modifications or changes in light thereof will be suggested to persons skilled in the art and are to be included within the spirit and purview of this application and scope of the appended claims.

### EXAMPLE 1 - TARGET IDENTIFICATION

### Background

All biological functions arise from the co-ordinated interaction of hundreds of interacting molecules - primarily proteins - and can be thought of as the emergent functional consequence of protein-protein interaction networks where each node in the network is a protein and each edge linking the proteins can represent a range of possible interaction types from complex formation to catalytic activation etc.

Historically such functions have been represented as simplistic linear pathways. As knowledge has grown however, it has become clear that pathways are more complex and that the minimum level of complexity that adequately represents the functional properties of a biological process, and can capture the characteristics of resilience to perturbation and robustness to random damage of individual components, is a network.

Networks subserving biological function may consist of several interacting canonical pathways as well as additional proteins that are primarily engaged when the canonical function is perturbed. It is therefore critical to develop approaches that can model this complexity in a meaningful and tractable way; and generate target hypotheses that take account of the inherent resistance to change that is a consequence of network robustness.

Oversimplification of biology and the failure to make rational drug target choices based on realistic models of biological processes has contributed to the poor success rate of drug discovery. Furthermore, there has been a lack of rigorous objective methods both for making network models of processes and for prioritising protein targets within these models. As a consequence target selection decisions are frequently made on an ad hoc basis based on preference or evidence unrelated to the functional model.

### Process for identifying processes and targets

The starting point for the identification of the target SLC25A5/ANT2 was Genome Wide Association Studies (GWAS) metanalyses for Non-Alcoholic Fatty Liver Disease (NAFLD).

The GWAS metanalyses were QC'ed for sample size, target population, and quality of statistical analysis. More specifically, a sample size of more than 1,000 was used as a cut-off,

SNPs were taken forward if the p-value was reflecting multiple testing corrections. Finally, sex-combined and all-ancestries SNPs were selected (continental European, UK, other); SNPs with gender-specific significance were excluded.

The resulting selected GWAS were used to extract the SNPs with strong association with the trait of interest, in this case NAFLD pathophysiology as well as associated metabolic dysfunction (for example, NAFLD and cardiometabolic diseases or steatosis and risk of NAFLD).

As a subsequent step, the SNPs were mapped to genes using the Variant-to-Gene (V2G) pipeline data from Open Targets Genetics (https://genetics.opentargets.org/). More specifically, for each SNP of interest the gene with the highest score (or genes, in case of a top-score tie) based on the V2G pipeline were selected, along with those genes with a score above 0.2.

The collated gene set was used as input for network construction, based on a proprietary ETX algorithm (algorithm A). Impact analysis (algorithm B) is then applied on the networks, to identify the biological processes that the gene set is involved in. This method takes canonical pathways and evaluates the impact they have on the network; they are then clustered. The resulting clustered processes represented in the networks were assessed by an internal team.

The most relevant processes for NAFLD biology were taken forward; their proteins were used with a proprietary ETX algorithm (algorithm C) to make directed networks. The nodes of each directed network were scored for the degree and type of influence they have on the network based on a set of proprietary ETX metrics. In addition, the nodes were filtered for those genes that are expressed in hepatocytes and scored above a proprietary threshold in the metrics of choice.

As a subsequent step, based on the aforementioned ranking, the top-scoring genes per directed network were assessed by an internal ETX team to identify targets of interest for the treatment of NAFLD metabolic diseases.

ANT2 was identified in the network process (Supercluster) 574, annotated by the ETX algorithm as "Adipogenesis/Non-alcoholic fatty liver disease", having scored above a proprietary threshold in one of the proprietary ETX metrics.

The inventors have thus analysed these network models using proprietary analytical methods. These methods use the directional information to capture key 'target' properties such as whether a protein is an integrator of information, a key conduit of information to other parts of the network, an influencer of key proteins and the extent to which an influencer is influenced or influences other proteins (based on absolute and relative number and direction of inputs and outputs). The directional information also enables hierarchical relationships between proteins to be imputed. Proteins higher in the hierarchy and with certain properties may be preferred over others with otherwise similar properties. The relative specificity and magnitude of each property relative to the others made it possible for the inventors to score and rank proteins in terms of their target suitability.

The ability to characterise the properties of these targets in terms of network relationships enables judgements to be made on the selectivity and magnitude of effect in the chosen context and hence the suitability of each for a given indication.

Proprietary analytical techniques were then applied to the network models to identify pharmacologically viable targets from within the networks whose knockdown will have a significant influence on the network and by extension on the biological function being modelled. The algorithms make extensive use of directional information and hierarchical relationships to identify targets with a range of specific properties that will make them good siRNA targets. Targets were then further filtered by protein class and hepatocyte specific properties according to the therapeutic requirements.

The above workflow leveraging proprietary data resources and network node metrics has identified the provided target for the provided uses.

The outcome of the network approach is shown in Table 6. ANT2 was surprisingly identified as a drug target for NAFLD among various other targets that have been previously associated with metabolic disorders, such NAFLD (APOA5, HMDH, APOC3, NR1H3, MTP, PCSK9, SOAT1 and ABCA1).

**Table 6**

| **Protein name** | **Accession number** | **Prediction score for NAFLD** | **Hepatocellular expression rank** |
|---|---|---|---|
| APOA5 | Q6Q788 | 93.6 | 0.46 |
| HMDH | P04035 | 91.3 | 0.54 |
| APOC3 | P02656 | 91.2 | 0.95 |
| NR1H3 | Q13133 | 91.1 | 0.22 |
| MTP | P55157 | 88.1 | 0.96 |
| **ANT2** | **P05141** | **80** | **0.98** |
| PCSK9 | Q8NBP7 | 79.3 | 0.21 |
| SOAT1 | P35610 | 77.7 | 0.27 |
| ABCA1 | O095477 | 77.0 | 0.46 |

### EXAMPLE 2: SYNTHESIS OF TETHER 1

### General Experimental conditions:

Thin layer chromatography (TLC) was performed on silica-coated aluminium plates with fluorescence indicator 254 nm from Macherey-Nagel. Compounds were visualized under UV light (254 nm), or after spraying with the 5% H₂SO₄ in methanol (MeOH) or ninhydrin reagent according to Stahl (from Sigma-Aldrich), followed by heating. Flash chromatography was performed with a Biotage Isolera One flash chromatography instrument equipped with a dual variable UV wavelength detector (200-400 nm) using Biotage Star Silica 10, 25, 50 or 100 g columns (Uppsala, Sweden).

All moisture-sensitive reactions were carried out under anhydrous conditions using dry glassware, anhydrous solvents, and argon atmosphere. All commercially available reagents were purchased from Sigma-Aldrich and solvents from Carl Roth GmbH + Co. KG. D-Galactosamine pentaacetate was purchased from AK scientific.

HPLC/ESI-MS was performed on a Dionex UltiMate 3000 RS UHPLC system and Thermo Scientific MSQ Plus Mass spectrometer using an Acquity UPLC Protein BEH C4 column from Waters (300Å, 1.7 µm, 2.1 × 100 mm) at 60 °C. The solvent system consisted of solvent A with H₂O containing 0.1% formic acid and solvent B with acetonitrile (ACN) containing 0.1% formic acid. A gradient from 5-100% of B over 15 min with a flow rate of 0.4 mL/min was employed. Detector and conditions: Corona ultra-charged aerosol detection (from esa). Nebulizer Temp.: 25 °C. N₂ pressure: 35.1 psi. Filter: Corona.

₁H and ₁₃C NMR spectra were recorded at room temperature on a Varian spectrometer at 500 MHz (₁H NMR) and 125 MHz (₁₃C NMR). Chemical shifts are given in ppm referenced to the solvent residual peak (CDCl₃ - ₁H NMR: δ at 7.26 ppm and ₁₃C NMR δ at 77.2 ppm; DMSO-d6 - ₁H NMR: δ at 2.50 ppm and ₁₃C NMR δ at 39.5 ppm). Coupling constants are given in Hertz. Signal splitting patterns are described as singlet (s), doublet (d), triplet (t) or multiplet (m).

### Synthesis route for the conjugate building block TriGalNAc Tether!:

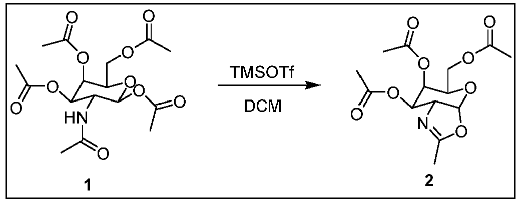

Preparation of compound 2: D-Galactosamine pentaacetate (3.00 g, 7.71 mmol, 1.0 eq.) was dissolved in anhydrous dichloromethane (DCM) (30 mL) under argon and trimethylsilyl trifluoromethanesulfonate (TMSOTf, 4.28 g, 19.27 mmol, 2.5 eq.) was added. The reaction was stirred at room temperature for 3 h. The reaction mixture was diluted with DCM (50 mL) and washed with cold saturated aq. NaHCO₃ (100 mL) and water (100 mL). The organic layer was separated, dried over Na₂SO₄ and concentrated to afford the title compound as yellow oil, which was purified by flash chromatography (gradient elution: 0-10% MeOH in DCM in 10 CV). The product was obtained as colourless oil (2.5 g, 98%, rf= 0.45 (2% MeOH in DCM)). 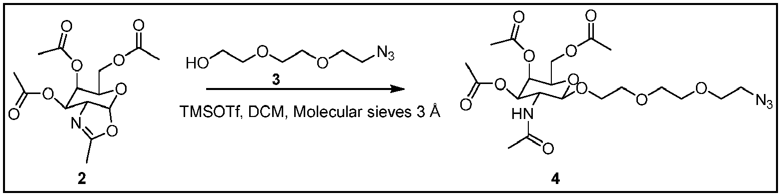 Preparation of compound 4: Compound 2 (2.30 g, 6.98 mmol, 1.0 eq.) and azido-PEG3-OH (1.83 g, 10.5 mmol, 1.5 eq.) were dissolved in anhydrous DCM (40 mL) under argon and molecular sieves 3 Å (5 g) were added to the solution. The mixture was stirred at room temperature for 1 h. TMSOTf (0.77 g, 3.49 mmol, 0.5 eq.) was then added to the mixture and the reaction was stirred overnight. The molecular sieves were filtered, the filtrate was diluted with DCM (100 mL) and washed with cold saturated aq. NaHCO₃ (100 mL) and water (100 mL). The organic layer was separated, dried over Na₂SO₄ and the solvent was removed under reduced pressure. The crude material was purified by flash chromatography (gradient elution: 0-3% MeOH in DCM in 10 CV) to afford the title product as light yellow oil (3.10 g, 88%, rf = 0.25 (2% MeOH in DCM)). MS: calculated for C₂₀H₃₂N₄O₁₁, 504.21. Found 505.4. 1H NMR (500 MHz, CDCl3) δ 6.21-6.14 (m, 1H), 5.30 (dd, J = 3.4, 1.1 Hz, 1H), 5.04 (dd, J = 11.2, 3.4 Hz,1H), 4.76 (d, J = 8.6 Hz, 1H), 4.23-4.08 (m, 3H), 3.91-3.80 (m, 3H), 3.74-3.59 (m, 9H), 3.49-3.41 (m, 2H), 2.14 (s, 3H), 2.02 (s, 3H), 1.97 (d, J = 4.2 Hz, 6H). 13C NMR (125 MHz, CDCl3) δ 170.6 (C), 170.5 (C), 170.4 (C), 170.3 (C), 102.1 (CH), 71.6 (CH), 70.8 (CH), 70.6 (CH), 70.5 (CH), 70.3 (CH2), 69.7 (CH2), 68.5 (CH2), 66.6 (CH2), 61.5 (CH2), 23.1 (CH3), 20.7 (3xCH3).

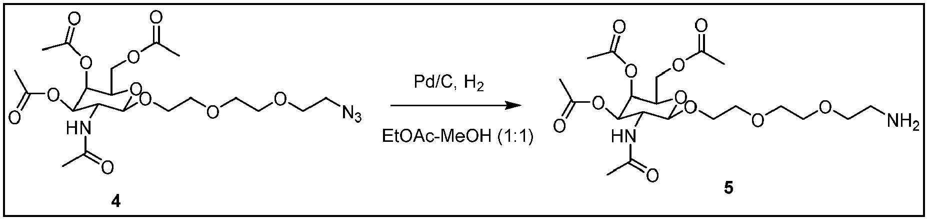

Preparation of compound 5: Compound 4 (1.00 g, 1.98 mmol, 1.0 eq.) was dissolved in a mixture of ethyl acetate (EtOAc) and MeOH (30 mL 1: 1 v/v) and Pd/C (100 mg) was added. The reaction mixture was degassed using vacuum/argon cycles (3x) and hydrogenated under balloon pressure overnight. The reaction mixture was filtered through celite and washed with EtOAc (30 mL). The solvent was removed under reduced pressure to afford the title compound as colourless oil (0.95 g, quantitative yield, rf = 0.25 (10% MeOH in DCM)). The compound was used without further purification. MS: calculated for C₂₀H₃₄N₂O₁₁, 478.2. Found 479.4.

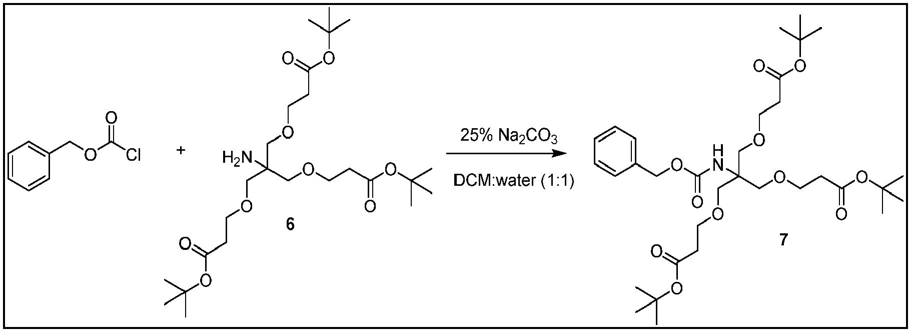

Preparation of compound 7: Tris{[2-(tert-butoxycarbonyl)ethoxy]methyl}-methylamine 6 (3.37 g, 6.67 mmol, 1.0 eq.) was dissolved in a mixture of DCM/water (40 mL 1:1 v/v) and Na₂CO₃ (0.18 g, 1.7 mmol, 0.25 eq.) was added while stirring vigorously. Benzyl chloroformate (2.94 mL, 20.7 mmol, 3.10 eq.) was added dropwise to the previous mixture and the reaction was stirred at room temperature for 24 h. The reaction mixture was diluted with CH₂Cl₂ (100 mL) and washed with water (100 mL). The organic layer was separated and dried over Na2SO4. The solvent was removed under reduced pressure and the resulting crude material was purified by flash chromatography (gradient elution: 0-10% EtOAc in cyclohexane in 12 CV) to afford the title compound as pale yellowish oil (3.9 g, 91%, rf = 0.56 (10% EtOAc in cyclohexane)). MS: calculated for C₃₃H₅₃NO₁₁, 639.3. Found 640.9. 1H NMR (500 MHz, DMSO-d6) δ 7.38-7.26 (m, 5H), 4.97 (s, 2H), 3.54 (t, 6H), 3.50 (s, 6H), 2.38 (t, 6H), 1.39 (s, 27H). 13C NMR (125 MHz, DMSO-d6) δ 170.3 (3xC), 154.5 (C), 137.1 (C), 128.2 (2xCH), 127.7 (CH), 127.6 (2xCH), 79.7 (3xC), 68.4 (3xCH2), 66.8 (3xCH2), 64.9 (C), 58.7 (CH2), 35.8 (3xCH2), 27.7 (9xCH3).

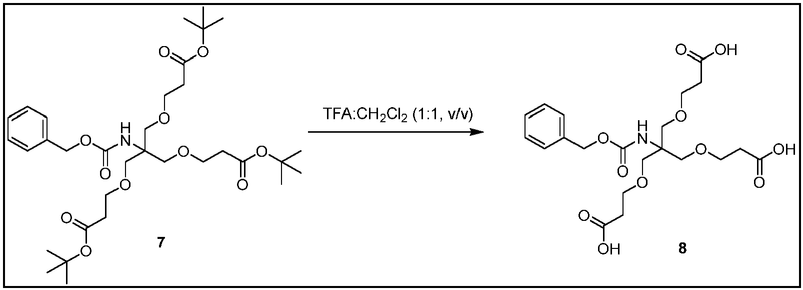

Preparation of compound 8: Cbz-NH-tris-Boc-ester 7 (0.20 g, 0.39 mmol, 1.0 eq.) was dissolved in CH₂Cl₂ (1 mL) under argon, trifluoroacetic acid (TFA, 1 mL) was added and the reaction was stirred at room temperature for 1 h. The solvent was removed under reduced pressure, the residue was co-evaporated 3 times with toluene (5 mL) and dried under high vacuum to get the compound as its TFA salt (0.183 g, 98%). The compound was used without further purification. MS: calculated for C₂₁H₂₉NO₁₁, 471.6. Found 472.4.

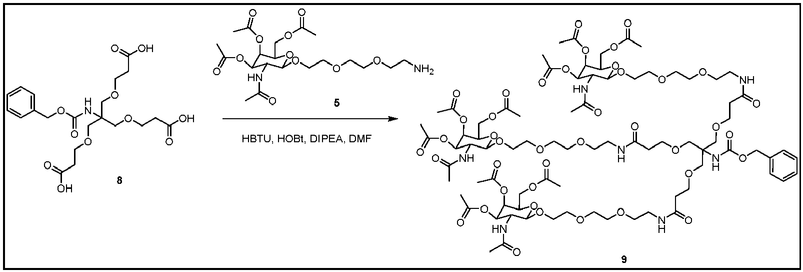

Preparation of compound 9: CbzNH-tris-COOH 8 (0.72 g, 1.49 mmol, 1.0 eq.) and GalNAc-PEG3-NH₂ 5 (3.56 g, 7.44 mmol, 5.0 eq.) were dissolved in N,N-dimethylformamide (DMF) (25 mL). Then N,N,N',N'-tetramethyl-O-(1H-benzotriazol-1-yl)uronium hexafluorophosphate (HBTU) (2.78 g, 7.44 mmol, 5.0 eq.), 1-hydroxybenzotriazole hydrate (HOBt) (1.05 g, 7.44 mmol, 5.0 eq.) and N,N-diisopropylethylamine (DIPEA) (2.07 mL, 11.9 mmol, 8.0 eq.) were added to the solution and the reaction was stirred for 72 h. The solvent was removed under reduced pressure, the residue was dissolved in DCM (100 mL) and washed with saturated aq. NaHCO₃ (100 mL). The organic layer was dried over Na₂SO₄, the solvent evaporated and the crude material was purified by flash chromatography (gradient elution: 0-5% MeOH in DCM in 14 CV). The product was obtained as pale yellowish oil (1.2 g, 43%, rf = 0.20 (5% MeOH in DCM)). MS: calculated for C₈₁H₁₂₅N₇O₄₁, 1852.9. Found 1854.7. 1H NMR (500 MHz, DMSO-d6) δ 7.90-7.80 (m, 10H), 7.65-7.62 (m, 4H), 7.47-7.43 (m, 3H), 7.38-7.32 (m, 8H), 5.24-5.22 (m, 3H), 5.02-4.97 (m, 4H), 4.60-4.57 (m, 3 H), 4.07-3.90 (m 10H), 3.67-3.36 (m, 70H), 3.23-3.07 (m, 25H), 2.18 (s, 10H), 2.00 (s, 13H), 1.89 (s, 11H), 1.80-1.78 (m, 17H). 13C NMR (125 MHz, DMSO-d6) δ 170.1 (C), 169.8 (C), 169.7 (C), 169.4 (C), 169.2 (C), 169.1 (C), 142.7 (C), 126.3 (CH), 123.9 (CH), 118.7 (CH), 109.7 (CH), 100.8 (CH), 70.5 (CH), 69.8 (CH), 69.6 (CH), 69.5 (CH), 69.3 (CH2), 69.0 (CH2), 68.2 (CH2), 67.2 (CH2), 66.7 (CH2), 61.4 (CH2), 22.6 (CH2), 22.4 (3xCH3), 20.7 (9xCH3).

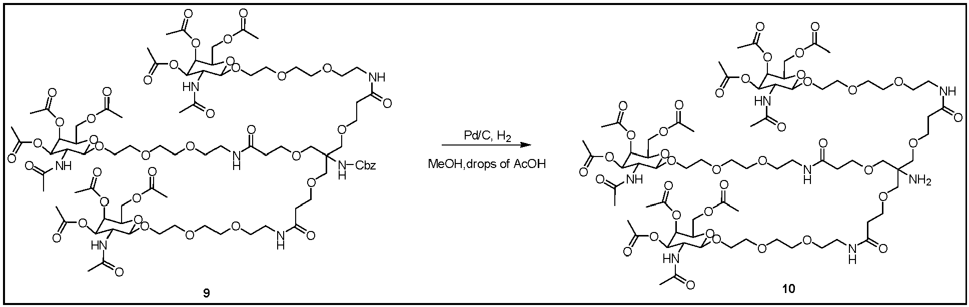

Preparation of compound 10: Triantennary GalNAc compound 9 (0.27 g, 0.14 mmol, 1.0 eq.) was dissolved in MeOH (15 mL), 3 drops of acetic acid (AcOH) and Pd/C (30 mg) was added. The reaction mixture was degassed using vacuum/argon cycles (3x) and hydrogenated under balloon pressure overnight. The completion of the reaction was followed by mass spectrometry and the resulting mixture was filtered through a thin pad of celite. The solvent was evaporated and the residue obtained was dried under high vacuum and used for the next step without further purification. The product was obtained as pale yellowish oil (0.24 g, quantitative yield). MS: calculated for C₇₃H₁₁₉N₇O₃₉, 1718.8. Found 1719.3.

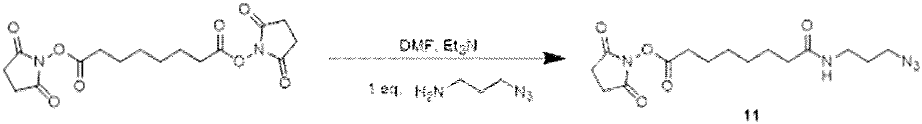

Preparation of compound 11: Commercially available suberic acid bis(N-hydroxysuccinimide ester) (3.67 g, 9.9 mmol, 1.0 eq.) was dissolved in DMF (5 mL) and triethylamine (1.2 mL) was added. To this solution was added dropwise a solution of 3-azido-1-propylamine (1.0 g, 9.9 mmol, 1.0 eq.) in DMF (5 mL). The reaction was stirred at room temperature for 3 h. The reaction mixture was diluted with EtOAc (100 mL) and washed with water (50 mL). The organic layer was separated, dried over Na₂SO₄ and the solvent was removed under reduced pressure. The crude material was purified by flash chromatography (gradient elution: 0-5% MeOH in DCM in 16 CV). The product was obtained as white solid (1.54 g, 43%, rf = 0.71 (5% MeOH in DCM)). MS: calculated for C₁₅H₂₃N₅O₅, 353.4. Found 354.3.

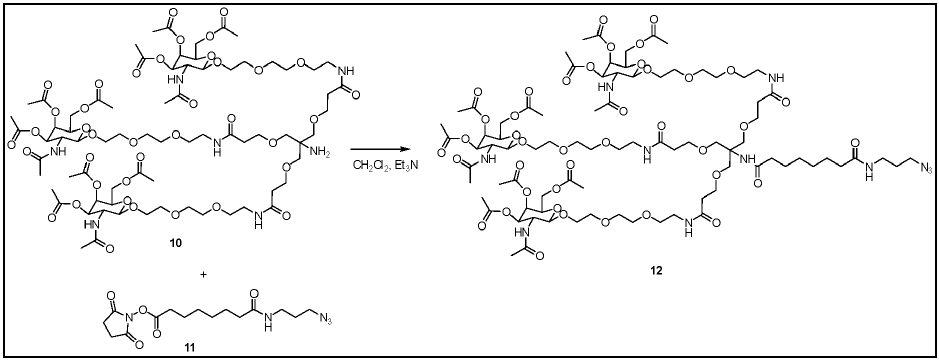

Preparation of TriGalNAc (12): Triantennary GalNAc compound 10 (0.35 g, 0.24 mmol, 1.0 eq.) and compound 11 (0.11 g, 0.31 mmol, 1.5 eq.) were dissolved in DCM (5 mL) under argon and triethylamine (0.1 mL, 0.61 mmol, 3.0 eq.) was added. The reaction was stirred at room temperature overnight. The solvent was removed under reduced pressure, the residue was dissolved in EtOAc (100 mL) and washed with water (100 mL). The organic layer was separated and dried over Na₂SO₄. The solvent was evaporated and the resulting crude material was purified by flash chromatography (elution gradient: 0-10% MeOH in DCM in 20 CV) to afford the title compound as white fluffy solid (0.27 g, 67%, rf = 0.5 (10% MeOH in DCM)). MS: calculated for C₈₄H₁₃₇N₁₁O₄₁, 1957.1. Found 1959.6.

### Conjugation of Tether 1 to a siRNA strand: Monofluoro cyclooctyne (MFCO) conjugation at 5'- or 3'-end

### 5'-end MFCO conjugation

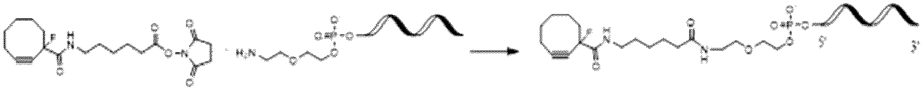

### 3`-end MFCO conjugation

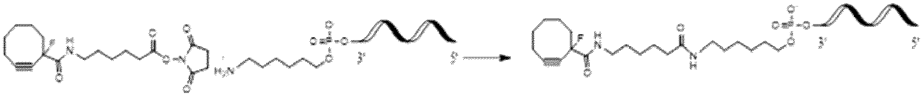

General conditions for MFCO conjugation: Amine-modified single strand was dissolved at 700 OD/mL in 50 mM carbonate/bicarbonate buffer pH 9.6/dimethyl sulfoxide (DMSO) 4:6 (v/v) and to this solution was added one molar equivalent of a 35 mM solution of MFCO-C6-NHS ester (Berry&Associates, Cat. # LK 4300) in DMF. The reaction was carried out at room temperature and after 1 h another molar equivalent of the MFCO solution was added. The reaction was allowed to proceed for an additional hour and was monitored by LC/MS. At least two molar equivalent excess of the MFCO NHS ester reagent relative to the amino modified oligonucleotide were needed to achieve quantitative consumption of the starting material. The reaction mixture was diluted 15-fold with water, filtered through a 1.2 µm filter from Sartorius and then purified by reserve phase (RP HPLC) on an Äkta Pure instrument (GE Healthcare).

Purification was performed using a XBridge C18 Prep 19 x 50 mm column from Waters. Buffer A was 100 mM TEAAc pH 7 and buffer B contained 95% acetonitrile in buffer A. A flow rate of 10 mL/min and a temperature of 60°C were employed. UV traces at 280 nm were recorded. A gradient of 0-100% B within 60 column volumes was employed.

Fractions containing full length conjugated oligonucleotide were pooled, precipitated in the freezer with 3 MNaOAc, pH 5.2 and 85% ethanol and the collected pellet was dissolved in water. Samples were desalted by size exclusion chromatography and concentrated using a speed-vac concentrator to yield the conjugated oligonucleotide in an isolated yield of 40-80%.

### 5'-GalNAc-T1 conjugates

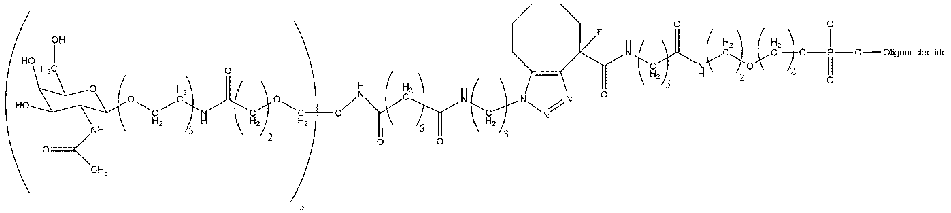

### 3'-GalNAc-T1 conjugates

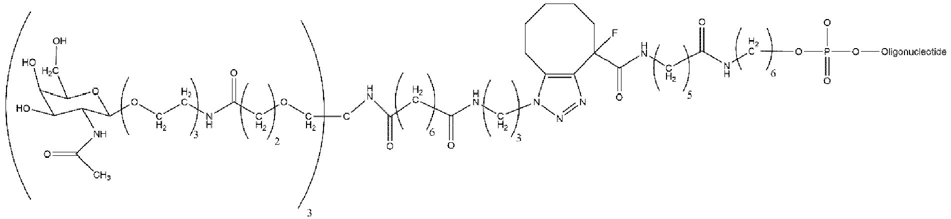

General procedure for TriGalNAc conjugation: MFCO-modified single strand was dissolved at 2000 OD/mL in water and to this solution was added one equivalent solution of compound 12 (10 mM) in DMF. The reaction was carried out at room temperature and after 3 h 0.7 molar equivalent of the compound 12 solution was added. The reaction was allowed to proceed overnight and completion was monitored by LCMS. The conjugate was diluted 15-fold in water, filtered through a 1.2 µm filter from Sartorius and then purified by RP HPLC on an Äkta Pure instrument (GE Healthcare).

RP HPLC purification was performed using a XBridge C18 Prep 19 x 50 mm column from Waters. Buffer A was 100 mM triethylammonium acetate pH 7 and buffer B contained 95% acetonitrile in buffer A. A flow rate of 10 mL/min and a temperature of 60°C were employed. UV traces at 280 nm were recorded. A gradient of 0-100% B within 60 column volumes was employed.

Fractions containing full-length conjugated oligonucleotide were pooled, precipitated in the freezer with 3 M NaOAc, pH 5.2 and 85% ethanol and the collected pellet was dissolved in water to give an oligonucleotide solution of about 1000 OD/mL. The O-acetates were removed by adding 20% aqueous ammonia. Quantitative removal of these protecting groups was verified by LC-MS.

The conjugates were desalted by size exclusion chromatography using Sephadex G25 Fine resin (GE Healthcare) on an Äkta Pure (GE Healthcare) instrument to yield the conjugated oligonucleotides in an isolated yield of 50-70%.

The following schemes further set out the routes of synthesis:

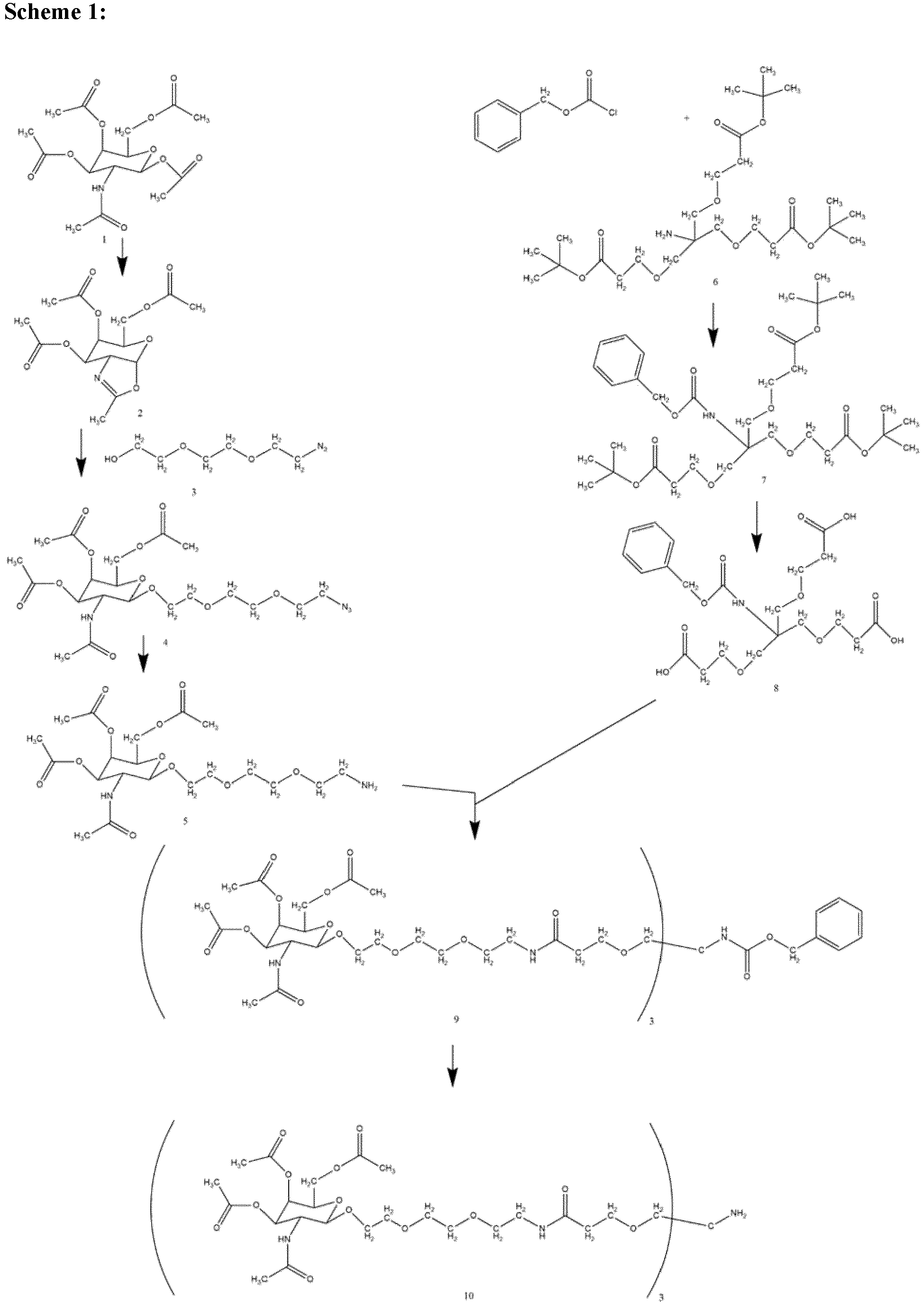

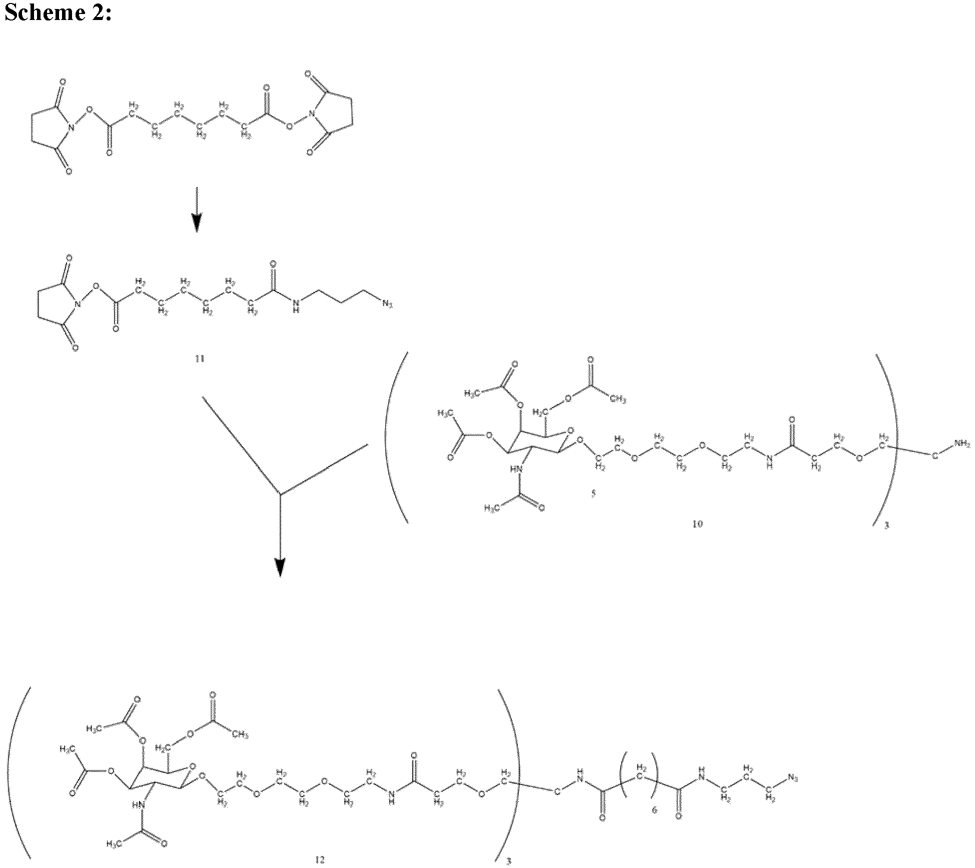

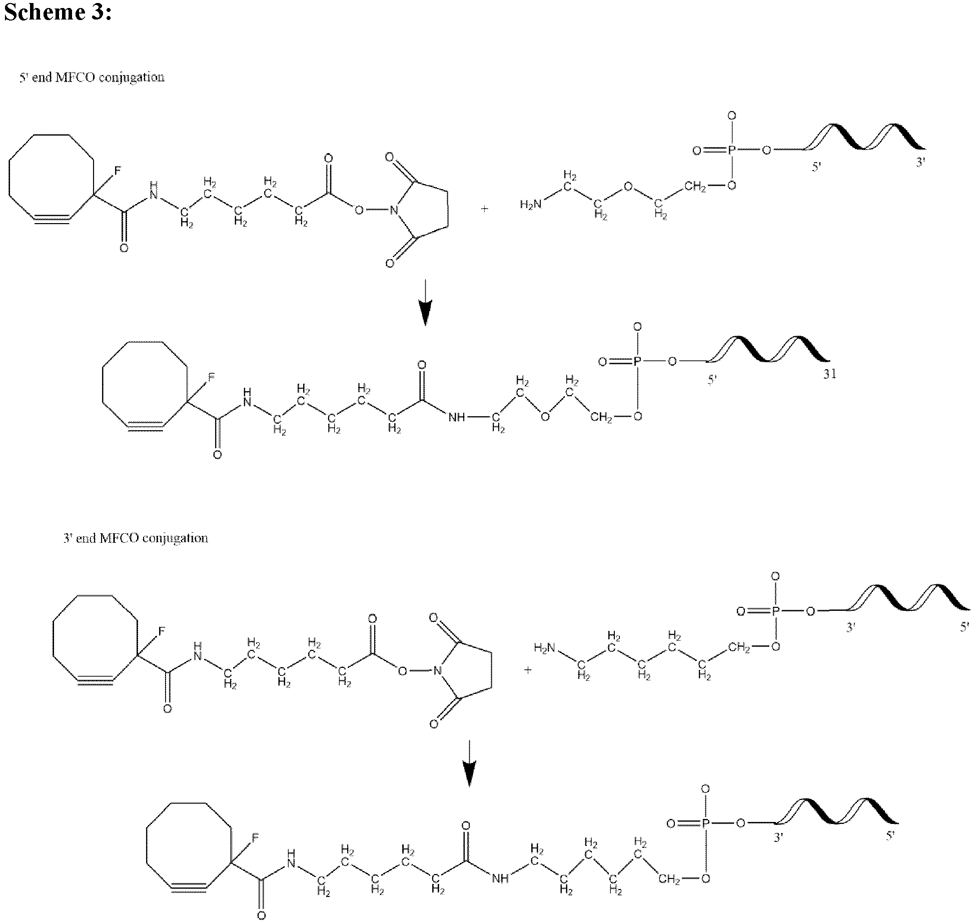

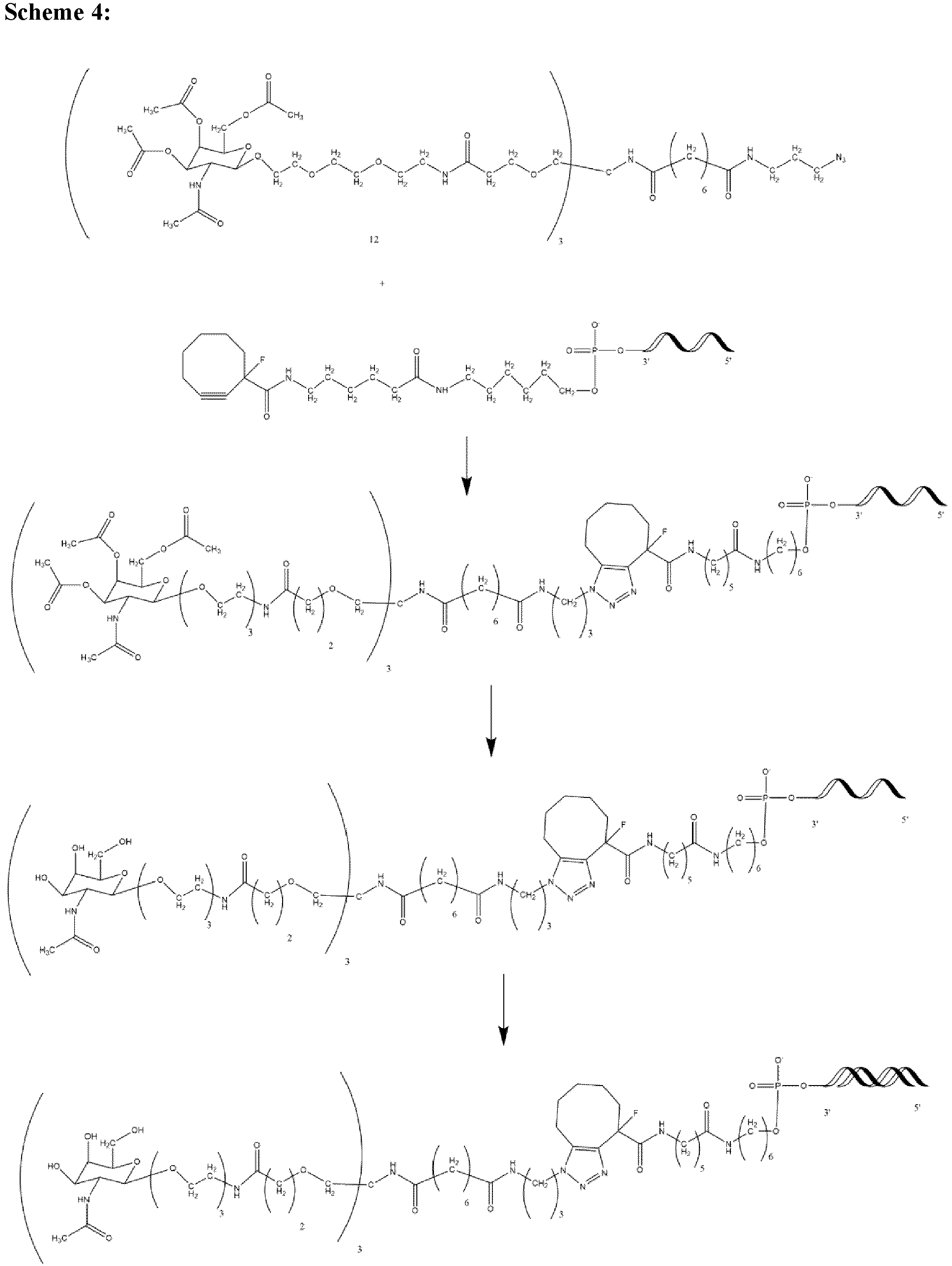

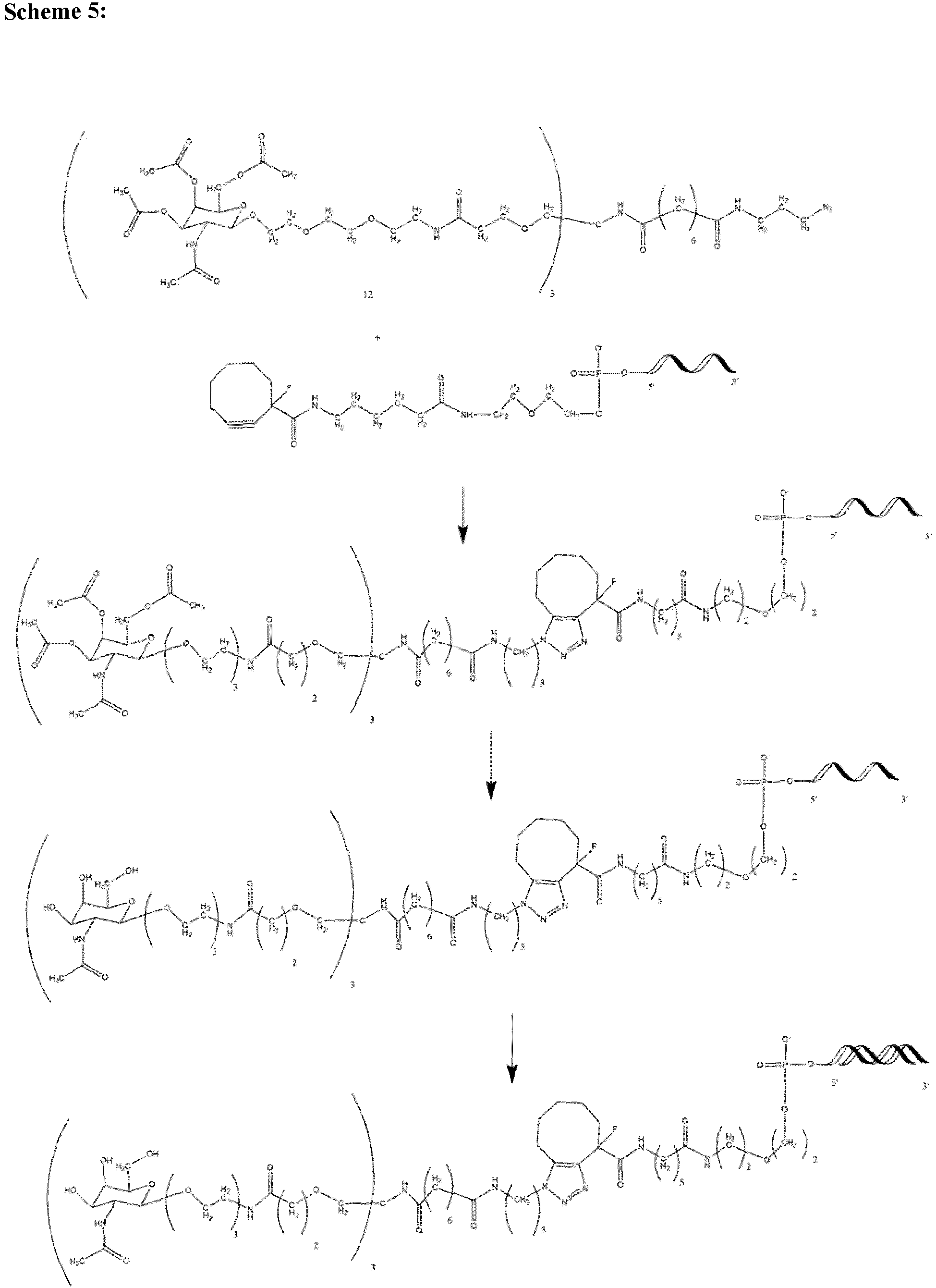

### EXAMPLE 3: DUPLEX ANNEALING

To generate the desired siRNA duplex, the two complementary strands were annealed by combining equimolar aqueous solutions of both strands. The mixtures were placed into a water bath at 70°C for 5 minutes and subsequently allowed to cool to ambient temperature within 2 h. The duplexes were lyophilized for 2 days and stored at -20°C.

The duplexes were analyzed by analytical SEC HPLC on Superdex^{™} 75 Increase 5/150 GL column 5 x 153-158 mm (Cytiva) on a Dionex Ultimate 3000 (Thermo Fisher Scientific) HPLC system. Mobile phase consisted of 1x PBS containing 10% acetonitrile. An isocratic gradient was run in 10 min at a flow rate of 1.5 mL/min at room temperature. UV traces at 260 and 280 nm were recorded. Water (LC-MS grade) was purchased from Sigma-Aldrich and Phosphate-buffered saline (PBS; 10x, pH 7.4) was purchased from GIBCO (Thermo Fisher Scientific).

### EXAMPLE 4: SYNTHESIS OF TETHER 2

### General Experimental conditions:

Thin layer chromatography (TLC) was performed on silica-coated aluminium plates with fluorescence indicator 254 nm from Macherey-Nagel. Compounds were visualized under UV light (254 nm), or after spraying with the 5% H₂SO₄ in methanol (MeOH) or ninhydrin reagent according to Stahl (from Sigma-Aldrich), followed by heating. Flash chromatography was performed with a Biotage Isolera One flash chromatography instrument equipped with a dual variable UV wavelength detector (200-400 nm) using Biotage Star Silica 10, 25, 50 or 100 g columns (Uppsala, Sweden).

All moisture-sensitive reactions were carried out under anhydrous conditions using dry glassware, anhydrous solvents, and argon atmosphere. All commercially available reagents were purchased from Sigma-Aldrich and solvents from Carl Roth GmbH + Co. KG. D-Galactosamine pentaacetate was purchased from AK scientific.

HPLC/ESI-MS was performed on a Dionex UltiMate 3000 RS UHPLC system and Thermo Scientific MSQ Plus Mass spectrometer using an Acquity UPLC Protein BEH C4 column from Waters (300Å, 1.7 µm, 2.1 × 100 mm) at 60 °C. The solvent system consisted of solvent A with H2O containing 0.1% formic acid and solvent B with acetonitrile (ACN) containing 0.1% formic acid. A gradient from 5-100% of B over 15 min with a flow rate of 0.4 mL/min was employed. Detector and conditions: Corona ultra-charged aerosol detection (from esa). Nebulizer Temp.: 25 °C. N₂ pressure: 35.1 psi. Filter: Corona.

₁H and ₁₃C NMR spectra were recorded at room temperature on a Varian spectrometer at 500 MHz (₁H NMR) and 125 MHz (₁₃C NMR). Chemical shifts are given in ppm referenced to the solvent residual peak (CDCl₃ - ₁H NMR: δ at 7.26 ppm and ₁₃C NMR δ at 77.2 ppm; DMSO-d6 - ₁H NMR: δ at 2.50 ppm and ₁₃C NMR δ at 39.5 ppm). Coupling constants are given in Hertz. Signal splitting patterns are described as singlet (s), doublet (d), triplet (t) or multiplet (m).

### Synthesis route for the conjugate building block TriGalNAc _Tether2:

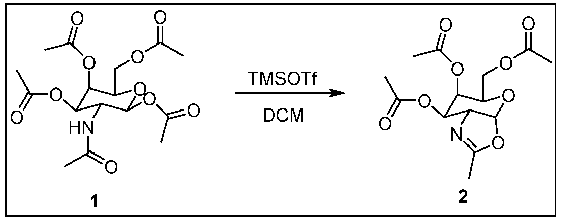

Preparation of compound 2: D-Galactosamine pentaacetate (3.00 g, 7.71 mmol, 1.0 eq.) was dissolved in anhydrous dichloromethane (DCM) (30 mL) under argon and trimethylsilyl trifluoromethanesulfonate (TMSOTf, 4.28 g, 19.27 mmol, 2.5 eq.) was added. The reaction was stirred at room temperature for 3 h. The reaction mixture was diluted with DCM (50 mL) and washed with cold saturated aq. NaHCO₃ (100 mL) and water (100 mL). The organic layer was separated, dried over Na₂SO₄, and concentrated to afford the title compound as yellow oil, which was purified by flash chromatography (gradient elution: 0-10% MeOH in DCM in 10 CV). The product was obtained as colourless oil (2.5 g, 98%, rf= 0.45 (2% MeOH in DCM)).

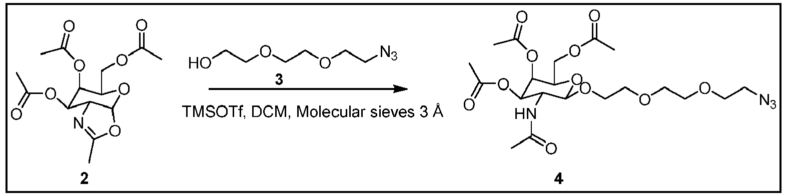

Preparation of compound 4: Compound 2 (2.30 g, 6.98 mmol, 1.0 eq.) and azido-PEG3-OH (1.83 g, 10.5 mmol, 1.5 eq.) were dissolved in anhydrous DCM (40 mL) under argon and molecular sieves 3 Å (5 g) were added to the solution. The mixture was stirred at room temperature for 1 h. TMSOTf (0.77 g, 3.49 mmol, 0.5 eq.) was then added to the mixture and the reaction was stirred overnight. The molecular sieves were filtered, the filtrate was diluted with DCM (100 mL) and washed with cold saturated aq. NaHCO₃ (100 mL) and water (100 mL). The organic layer was separated, dried over Na2SO4 and the solvent was removed under reduced pressure. The crude material was purified by flash chromatography (gradient elution: 0-3% MeOH in DCM in 10 CV) to afford the title product as light-yellow oil (3.10 g, 88%, rf = 0.25 (2% MeOH in DCM)). MS: calculated for C₂₀H₃₂N₄O₁₁, 504.21. Found 505.4. 1H NMR (500 MHz, CDCl3) δ 6.21-6.14 (m, 1H), 5.30 (dd, J = 3.4, 1.1 Hz, 1H), 5.04 (dd, J = 11.2, 3.4 Hz,1H), 4.76 (d, J = 8.6 Hz, 1H), 4.23-4.08 (m, 3H), 3.91-3.80 (m, 3H), 3.74-3.59 (m, 9H), 3.49-3.41 (m, 2H), 2.14 (s, 3H), 2.02 (s, 3H), 1.97 (d, J = 4.2 Hz, 6H). 13C NMR (125 MHz, CDCl3) δ 170.6 (C), 170.5 (C), 170.4 (C), 170.3 (C), 102.1 (CH), 71.6 (CH), 70.8 (CH), 70.6 (CH), 70.5 (CH), 70.3 (CH2), 69.7 (CH2), 68.5 (CH2), 66.6 (CH2), 61.5 (CH2), 23.1 (CH3), 20.7 (3xCH3).

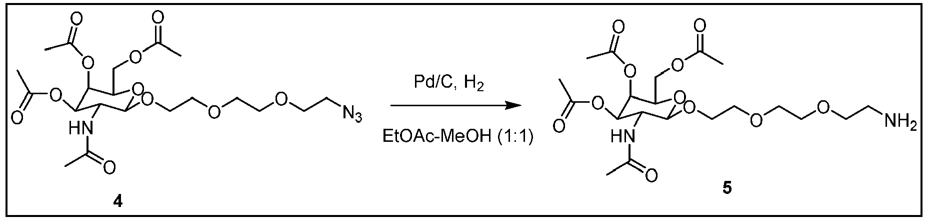

Preparation of compound 5: Compound 4 (1.00 g, 1.98 mmol, 1.0 eq.) was dissolved in a mixture of ethyl acetate (EtOAc) and MeOH (30 mL 1: 1 v/v) and Pd/C (100 mg) was added. The reaction mixture was degassed using vacuum/argon cycles (3x) and hydrogenated under balloon pressure overnight. The reaction mixture was filtered through celite and washed with EtOAc (30 mL). The solvent was removed under reduced pressure to afford the title compound as colourless oil (0.95 g, quantitative yield, rf = 0.25 (10% MeOH in DCM)). The compound was used without further purification. MS: calculated for C₂₀H₃₄N₂O₁₁, 478.2. Found 479.4.

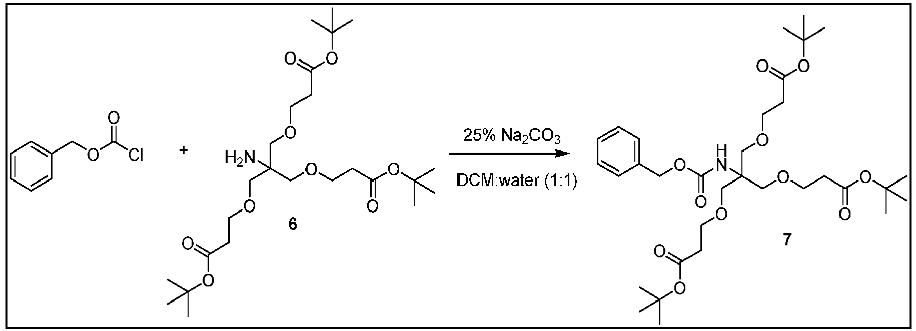

Preparation of compound 7: Tris{[2-(tert-butoxycarbonyl)ethoxy]methyl}-methylamine 6 (3.37 g, 6.67 mmol, 1.0 eq.) was dissolved in a mixture of DCM/water (40 mL 1:1 v/v) and Na₂CO₃ (0.18 g, 1.7 mmol, 0.25 eq.) was added while stirring vigorously. Benzyl chloroformate (2.94 mL, 20.7 mmol, 3.10 eq.) was added dropwise to the previous mixture and the reaction was stirred at room temperature for 24 h. The reaction mixture was diluted with CH₂Cl₂ (100 mL) and washed with water (100 mL). The organic layer was separated and dried over Na₂SO₄. The solvent was removed under reduced pressure and the resulting crude material was purified by flash chromatography (gradient elution: 0-10% EtOAc in cyclohexane in 12 CV) to afford the title compound as pale yellowish oil (3.9 g, 91%, rf = 0.56 (10% EtOAc in cyclohexane)). MS: calculated for C₃₃H₅₃NO₁₁, 639.3. Found 640.9. 1H NMR (500 MHz, DMSO-d6) δ 7.38-7.26 (m, 5H), 4.97 (s, 2H), 3.54 (t, 6H), 3.50 (s, 6H), 2.38 (t, 6H), 1.39 (s, 27H). 13C NMR (125 MHz, DMSO-d6) δ 170.3 (3xC), 154.5 (C), 137.1 (C), 128.2 (2xCH), 127.7 (CH), 127.6 (2xCH), 79.7 (3xC), 68.4 (3xCH2), 66.8 (3xCH2), 64.9 (C), 58.7 (CH2), 35.8 (3xCH2), 27.7 (9xCH3).

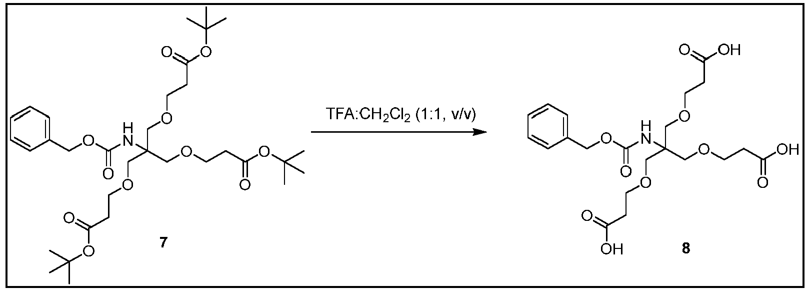

Preparation of compound 8: Cbz-NH-tris-Boc-ester 7 (0.20 g, 0.39 mmol, 1.0 eq.) was dissolved in CH₂Cl₂ (1 mL) under argon, trifluoroacetic acid (TFA, 1 mL) was added and the reaction was stirred at room temperature for 1 h. The solvent was removed under reduced pressure, the residue was co-evaporated 3 times with toluene (5 mL) and dried under high vacuum to get the compound as its TFA salt (0.183 g, 98%). The compound was used without further purification. MS: calculated for C₂₁H₂₉NO₁₁, 471.6. Found 472.4.

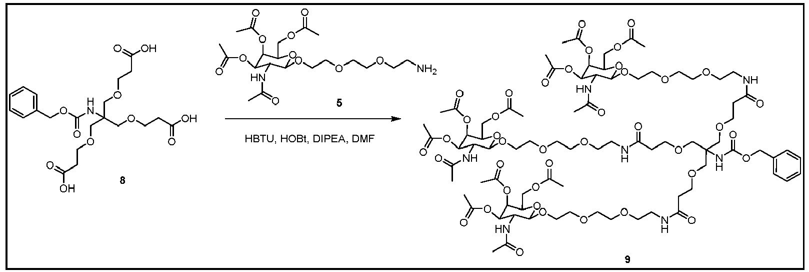

Preparation of compound 9: CbzNH-tris-COOH 8 (0.72 g, 1.49 mmol, 1.0 eq.) and GalNAc-PEG3-NH2 5 (3.56 g, 7.44 mmol, 5.0 eq.) were dissolved in N,N-dimethylformamide (DMF) (25 mL). Then N,N,N',N'-tetramethyl-O-(1H-benzotriazol-1-yl)uronium hexafluorophosphate (HBTU) (2.78 g, 7.44 mmol, 5.0 eq.), 1-hydroxybenzotriazole hydrate (HOBt) (1.05 g, 7.44 mmol, 5.0 eq.) and N,N-diisopropylethylamine (DIPEA) (2.07 mL, 11.9 mmol, 8.0 eq.) were added to the solution and the reaction was stirred for 72 h. The solvent was removed under reduced pressure, the residue was dissolved in DCM (100 mL) and washed with saturated aq. NaHCO3 (100 mL). The organic layer was dried over Na₂SO₄, the solvent evaporated and the crude material was purified by flash chromatography (gradient elution: 0-5% MeOH in DCM in 14 CV). The product was obtained as pale yellowish oil (1.2 g, 43%, rf = 0.20 (5% MeOH in DCM)). MS: calculated for C₈₁H₁₂₅N₇O₄₁, 1852.9. Found 1854.7. 1H NMR (500 MHz, DMSO-d6) δ 7.90-7.80 (m, 10H), 7.65-7.62 (m, 4H), 7.47-7.43 (m, 3H), 7.38-7.32 (m, 8H), 5.24-5.22 (m, 3H), 5.02-4.97 (m, 4H), 4.60-4.57 (m, 3 H), 4.07-3.90 (m 10H), 3.67-3.36 (m, 70H), 3.23-3.07 (m, 25H), 2.18 (s, 10H), 2.00 (s, 13H), 1.89 (s, 11H), 1.80-1.78 (m, 17H). 13C NMR (125 MHz, DMSO-d6) δ 170.1 (C), 169.8 (C), 169.7 (C), 169.4 (C), 169.2 (C), 169.1 (C), 142.7 (C), 126.3 (CH), 123.9 (CH), 118.7 (CH), 109.7 (CH), 100.8 (CH), 70.5 (CH), 69.8 (CH), 69.6 (CH), 69.5 (CH), 69.3 (CH2), 69.0 (CH2), 68.2 (CH2), 67.2 (CH2), 66.7 (CH2), 61.4 (CH2), 22.6 (CH2), 22.4 (3xCH3), 20.7 (9xCH3).

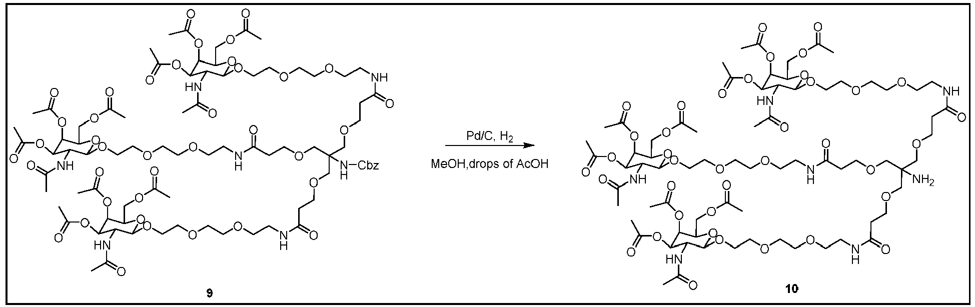

Preparation of compound 10: Triantennary GalNAc compound 9 (0.27 g, 0.14 mmol, 1.0 eq.) was dissolved in MeOH (15 mL), 3 drops of acetic acid (AcOH) and Pd/C (30 mg) was added. The reaction mixture was degassed using vacuum/argon cycles (3x) and hydrogenated under balloon pressure overnight. The completion of the reaction was followed by mass spectrometry and the resulting mixture was filtered through a thin pad of celite. The solvent was evaporated, and the residue obtained was dried under high vacuum and used for the next step without further purification. The product was obtained as pale yellowish oil (0.24 g, quantitative yield). MS: calculated for C₇₃H₁₁₉N₇O₃₉, 1718.8. Found 1719.3.

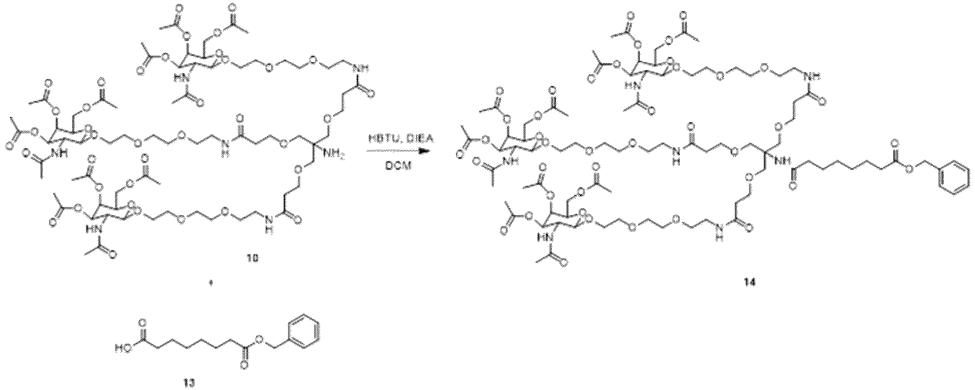

Preparation of compound 14: Triantennary GalNAc compound 10 (0.45 g, 0.26 mmol, 1.0 eq.), HBTU (0.19 g, 0.53 mmol, 2.0 eq.) and DIPEA (0.23 mL, 1.3 mmol, 5.0 eq.) were dissolved in DCM (10 mL) under argon. To this mixture, it was added dropwise a solution of compound 13 (0.14 g, 0.53 mmol, 2.0 eq.) in DCM (5 mL). The reaction was stirred at room temperature overnight. The solvent was removed, and the residue was dissolved in EtOAc (50 mL), washed with water (50 mL) and dried over Na₂SO₄. The solvent was evaporated, and the crude material was purified by flash chromatography (gradient elution: 0-5% MeOH in DCM in 20 CV). The product was obtained as white fluffy solid (0.25 g, 48%, rf = 0.4 (10% MeOH in DCM)). MS: calculated for C₈₈H₁₃₇N₇O₄₂, 1965.1. Found 1965.6.

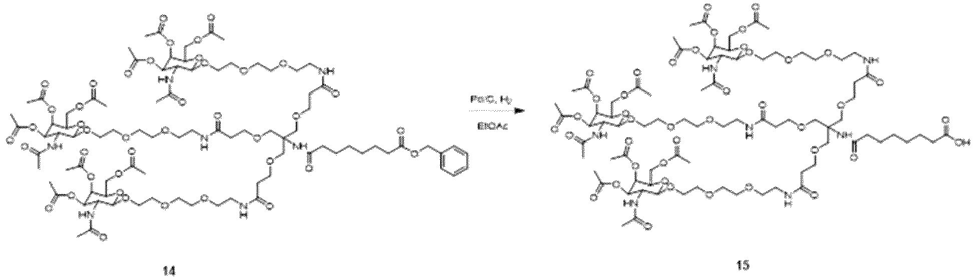

Preparation of TriGalNAc (15): Triantennary GalNAc compound 14 (0.31 g, 0.15 mmol, 1.0 eq.) was dissolved in EtOAc (15 mL) and Pd/C (40 mg) was added. The reaction mixture was degassed by using vacuum/argon cycles (3x) and hydrogenated under balloon pressure overnight. The completion of the reaction was monitored by mass spectrometry and the resulting mixture was filtered through a thin pad of celite. The solvent was removed under reduced pressure and the resulting residue was dried under high vacuum overnight. The residue was used for conjugations to oligonucleosides without further purification (0.28 g, quantitative yield). MS: calculated for C₈₁H₁₃₁N₇O₄₂, 1874.9. Found 1875.3.

Conjugation of Tether 2 to a siRNA strand: TriGalNAc tether 2 (GalNAc-T2) conjugation at 5'- end or 3'-end

### 5'-GaINAc-T2 conjugates

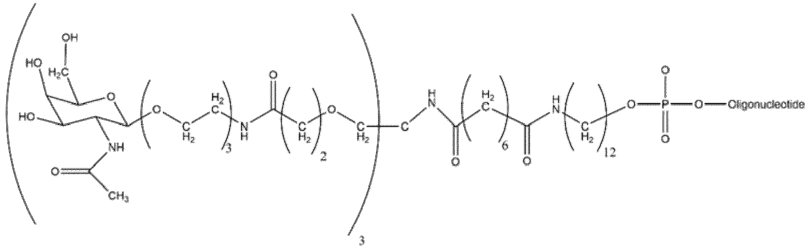

### 3'-GalNAc-T2 conjugates

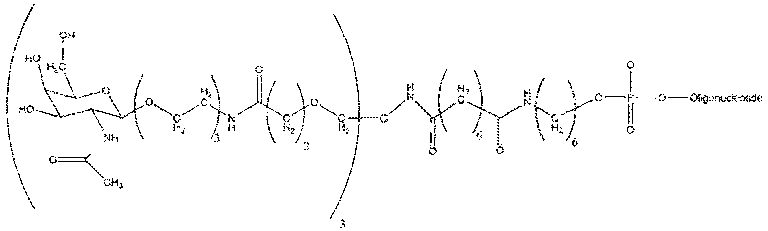

Preparation of TriGalNAc tether 2 NHS ester: To a solution of carboxylic acid tether 2 (compound 15, 227 mg, 121 µmοl) in DMF (2.1 mL), N-hydroxysuccinimide (NHS) (15.3 mg, 133 µmοl) and N,N'-diisopropylcarbodiimide (DIC) (19.7 µL, 127 µmοl) were added. The solution was stirred at room temperature for 18 h and used without purification for the subsequent conjugation reactions.

General procedure for triGalNAc tether 2 conjugation: Amine-modified single strand was dissolved at 700 OD/mL in 50 mM carbonate/bicarbonate buffer pH 9.6/DMSO 4:6 (v/v) and to this solution was added one molar equivalent of Tether 2 NHS ester (57 mM) solution in DMF. The reaction was carried out at room temperature and after 1 h another molar equivalent of the NHS ester solution was added. The reaction was allowed to proceed for one more hour and reaction progress was monitored by LCMS. At least two molar equivalent excess of the NHS ester reagent relative to the amino modified oligonucleoside were needed to achieve quantitative consumption of the starting material. The reaction mixture was diluted 15-fold with water, filtered once through 1.2 µm filter from Sartorius and then purified by reserve phase (RP HPLC) on an Äkta Pure (GE Healthcare) instrument.

The purification was performed using a XBridge C18 Prep 19 x 50 mm column from Waters. Buffer A was 100 mM TEAA pH 7 and buffer B contained 95% acetonitrile in buffer A. A flow rate of 10 mL/min and a temperature of 60°C were employed. UV traces at 280 nm were recorded. A gradient of 0-100% B within 60 column volumes was employed.

Fractions containing full-length conjugated oligonucleosides were pooled together, precipitated in the freezer with 3 M NaOAc, pH 5.2 and 85% ethanol and then dissolved at 1000 OD/mL in water. The O-acetates were removed with 20% ammonium hydroxide in water until completion (monitored by LC-MS).

The conjugates were desalted by size exclusion chromatography using Sephadex G25 Fine resin (GE Healthcare) on an Äkta Pure (GE Healthcare) instrument to yield the conjugated oligonucleotides in an isolated yield of 60-80%.

The conjugates were characterized by HPLC-MS analysis with a 2.1 × 50 mm XBridge C18 column (Waters) on a Dionex Ultimate 3000 (Thermo Fisher Scientific) HPLC system equipped with a Compact ESI-Qq-TOF mass spectrometer (Bruker Daltonics). Buffer A was 16.3 mM triethylamine, 100 mM HFIP in 1% MeOH in H2O and buffer B contained 95% MeOH in buffer A. A flow rate of 250 µL/min and a temperature of 60°C were employed. UV traces at 260 and 280 nm were recorded. A gradient of 1-100% B within 31 min was employed.

The following schemes further set out the routes of synthesis:

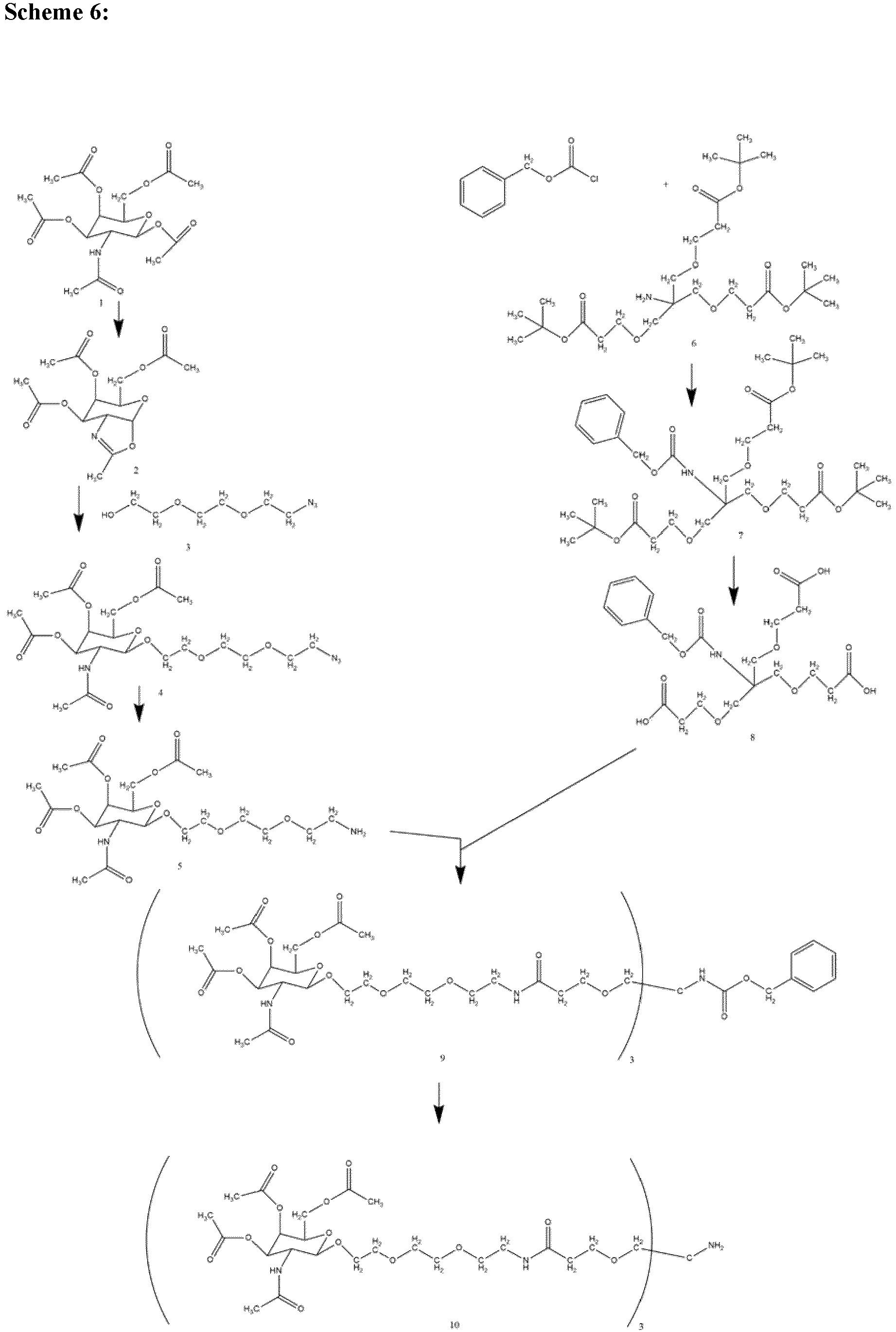

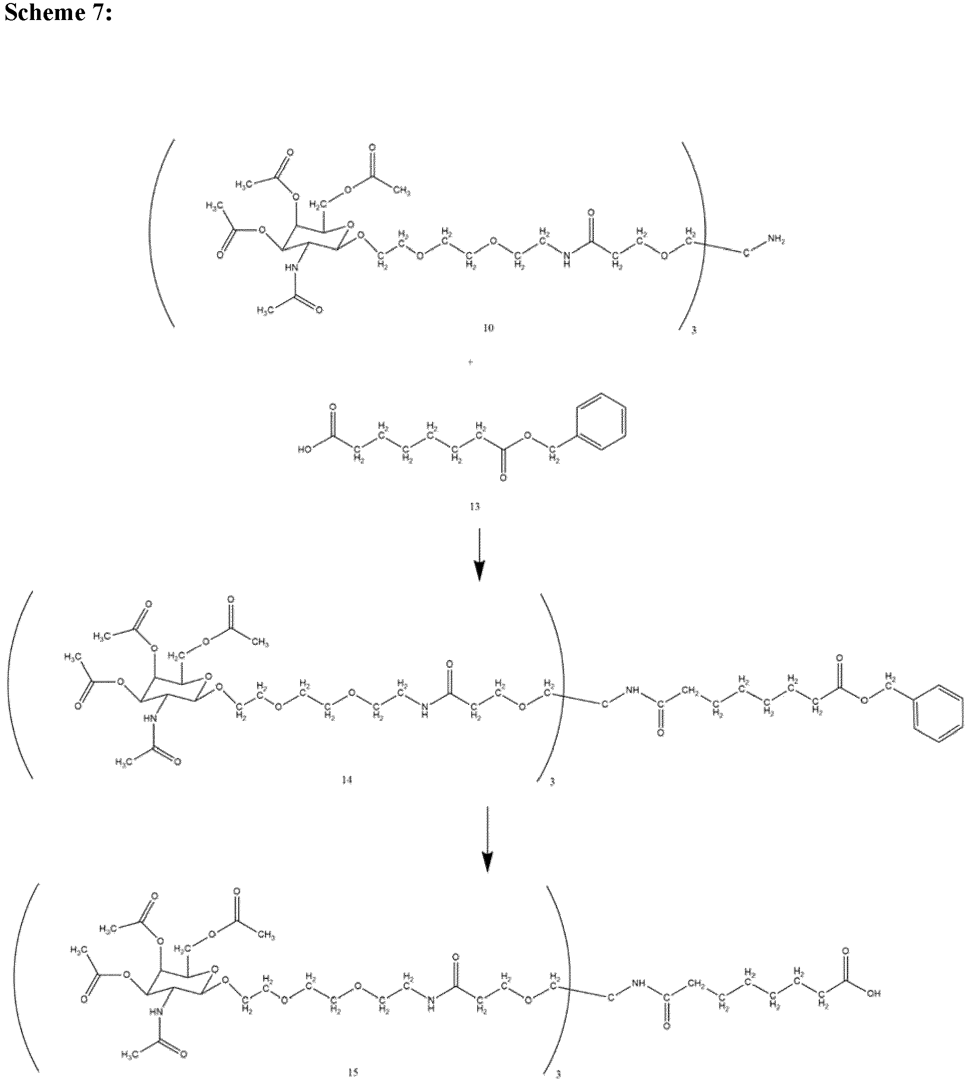

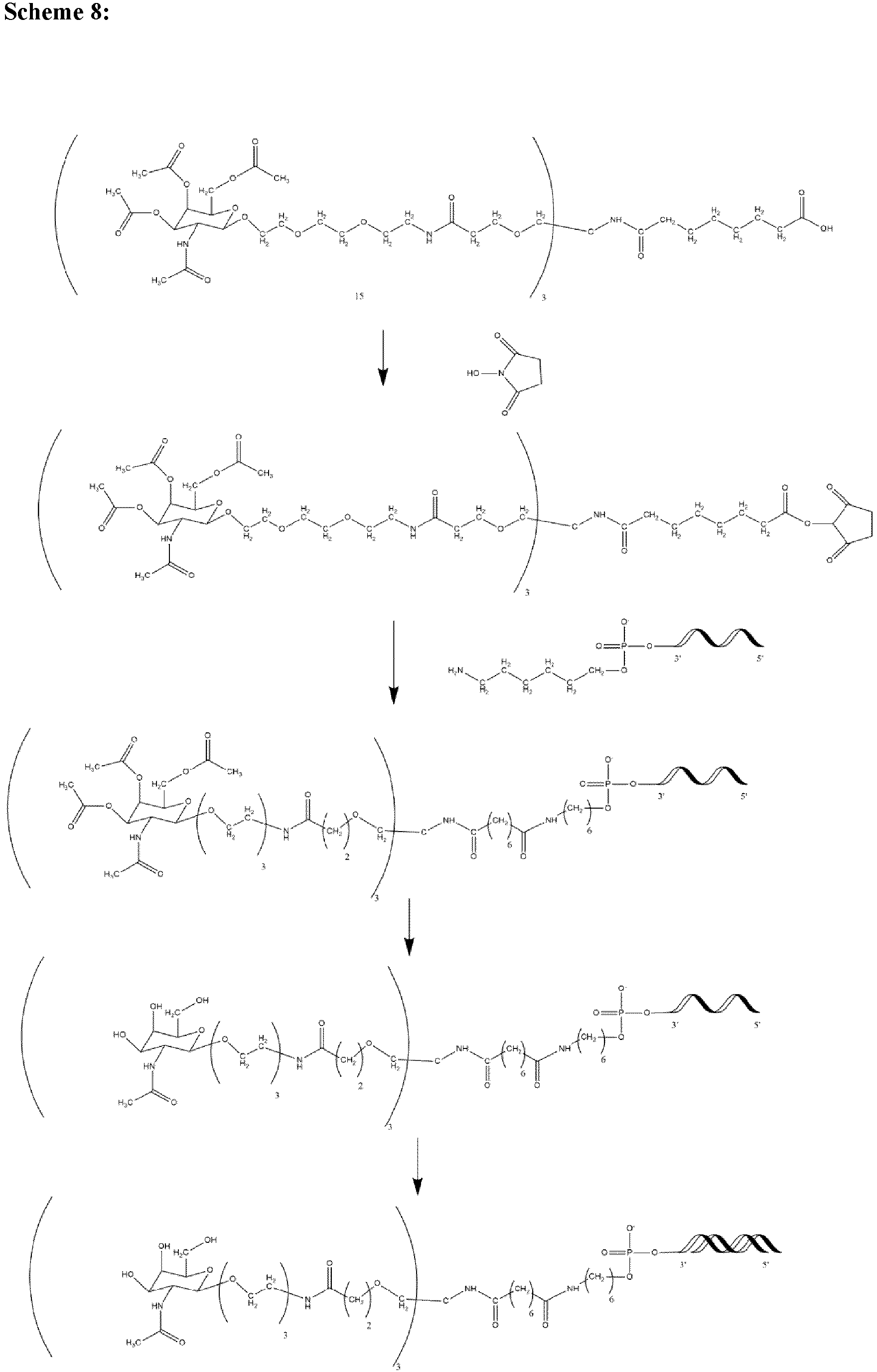

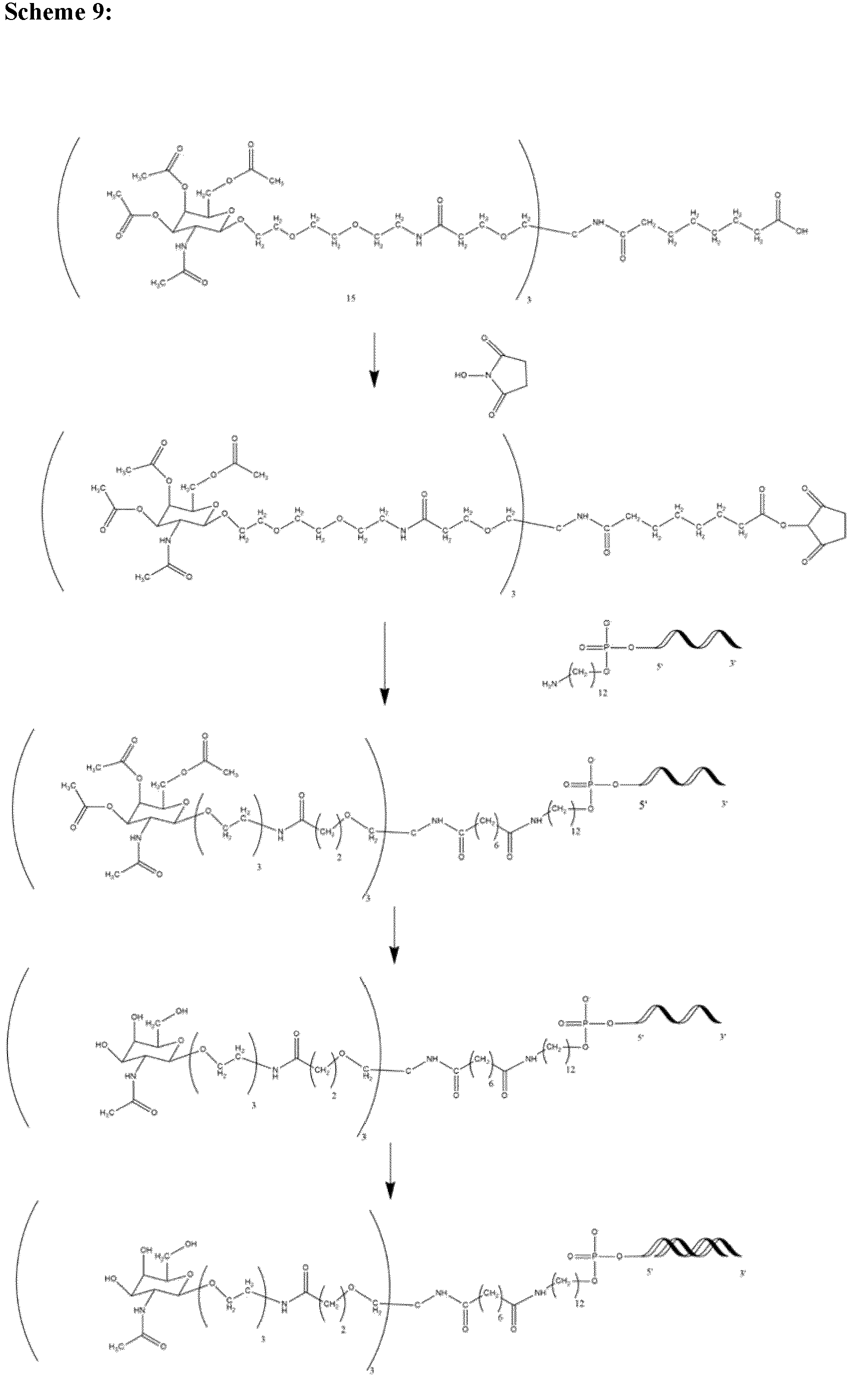

### EXAMPLE 5: DUPLEX ANNEALING

To generate the desired siRNA duplex, the two complementary strands were annealed by combining equimolar aqueous solutions of both strands. The mixtures were placed into a water bath at 70°C for 5 minutes and subsequently allowed to cool to ambient temperature within 2 h. The duplexes were lyophilized for 2 days and stored at -20°C.

The duplexes were analyzed by analytical SEC HPLC on Superdex^{™} 75 Increase 5/150 GL column 5 x 153-158 mm (Cytiva) on a Dionex Ultimate 3000 (Thermo Fisher Scientific) HPLC system. Mobile phase consisted of 1x PBS containing 10% acetonitrile. An isocratic gradient was run in 10 min at a flow rate of 1.5 mL/min at room temperature. UV traces at 260 and 280 nm were recorded. Water (LC-MS grade) was purchased from Sigma-Aldrich and Phosphate-buffered saline (PBS; 10x, pH 7.4) was purchased from GIBCO (Thermo Fisher Scientific).

### EXAMPLE 6: ALTERNATIVE SYNTHESIS ROUTE FOR THE CONJUGATE BUILDING BLOCK TRIGALNAC_TETHER2:

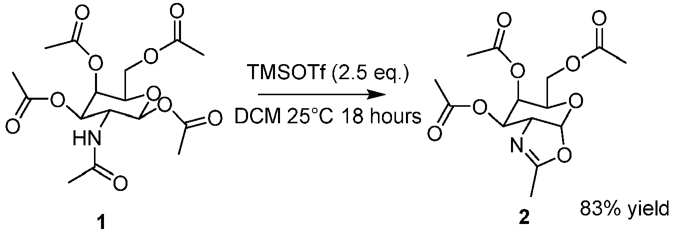

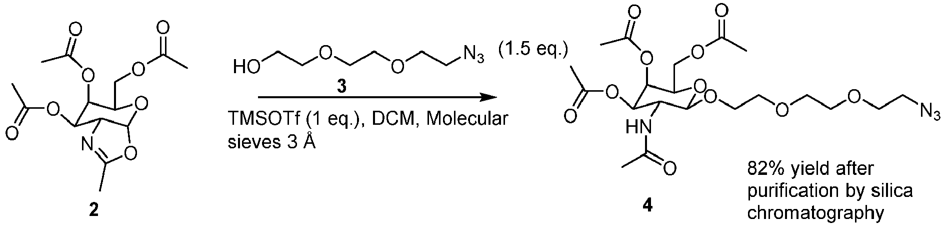

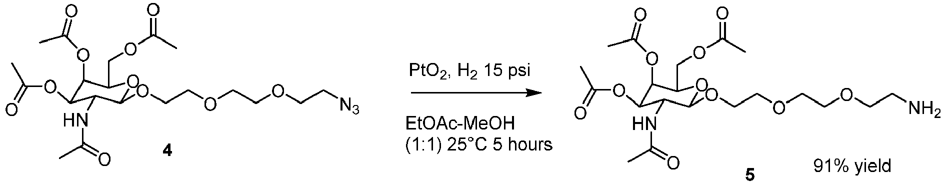

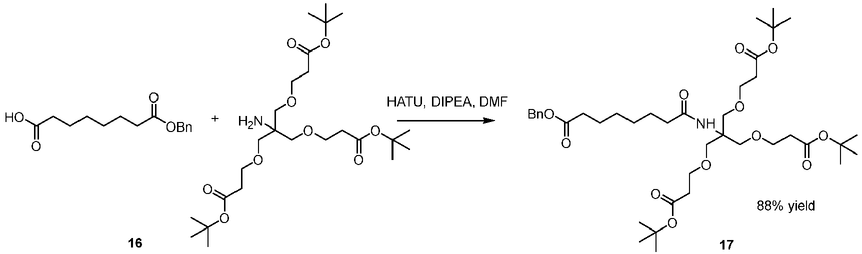

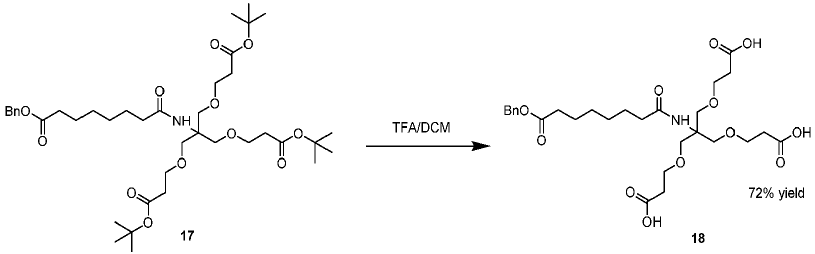

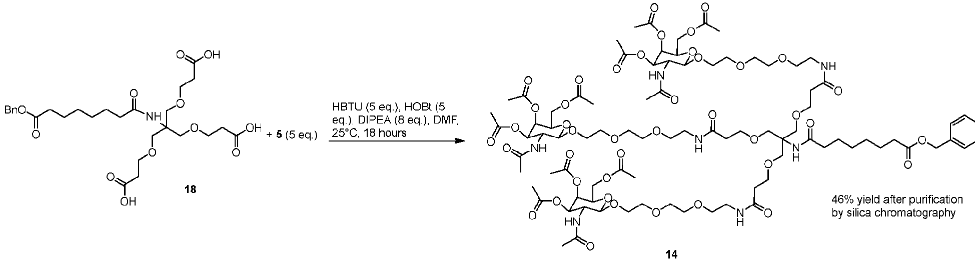

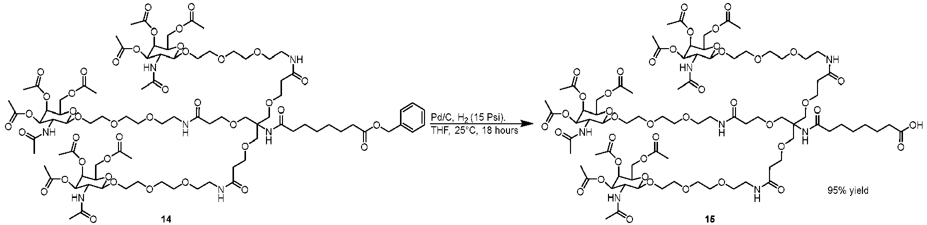

### Conjugation of Tether 2 to a siRNA strand: TriGalNAc tether 2 (GalNAc-T2) conjugation at 5'- end or 3'-end

### Conjugation conditions

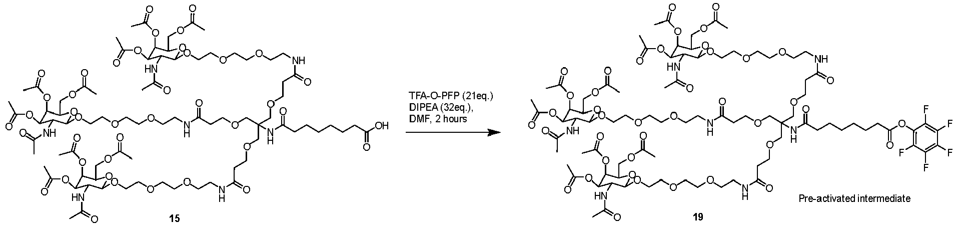

Pre-activation: To a solution of compound 15 (16 umol, 4 eq.) in DMF (160 µL) was added TFA-O-PFP (15 µl, 21 eq.) followed by DIPEA (23 µl, 32 eq.) at 25°C. The tube was shaken for 2 h at 25°C. The reaction was quenched with H2O (10 µL).

Coupling: The resulting mixture was diluted with DMF (400 µl), followed by addition of oligo-amine solution (4.0 µmοl in 10 x PBS, pH 7.4, 500 µL; final oligo concentration in organic and aqueous solution: 4 µmol/ml = 4 mM). The tube was shaken at 25°C for 16 h and the reaction was analysed by LCMS. The resulting mixture was treated with 28% NH4OH (4.5 ml) and shaken for 2 h at 25°C. The mixture was analysed by LCMS, concentrated, and purified by IP-RP HPLC to produce the oligonucleotides conjugated to tether 2 GalNAc.

### 5'-GaINAc-T2 conjugates

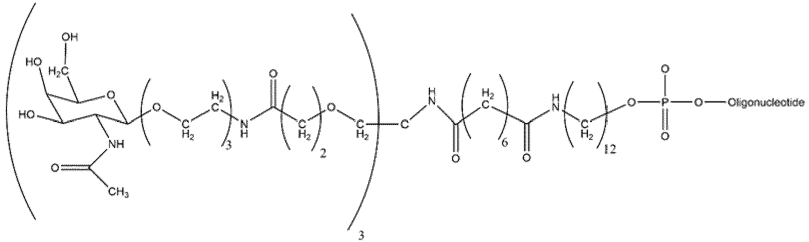

### 3'-GalNAc-T2 conjugates

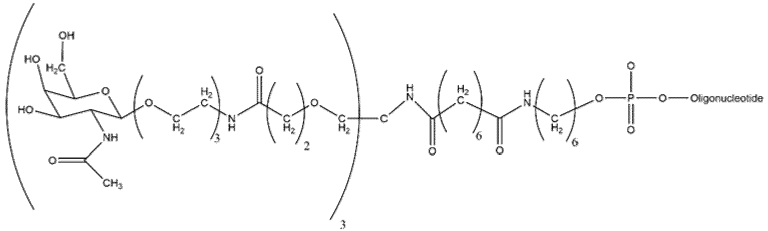

### EXAMPLE 7: SOLID PHASE SYNTHESIS METHOD: SCALE ≤1 µMOL

Syntheses of siRNA sense and antisense strands were performed on a MerMade192X synthesiser with commercially available solid supports made of controlled pore glass with universal linker (Universal CPG, with a loading of 40 µmol/g; LGC Biosearch or Glen Research).

RNA phosphoramidites were purchased from ChemGenes or Hongene.

The 2'-O-Methyl phosphoramidites used were the following: 5'-(4,4'-dimethoxytrityl)-N-benzoyl-adenosine 2'-O-methyl-3'- [(2-cyanoethyl)-(N,N-diisopropyl)]-phosphoramidite, 5'-(4,4'-dimethoxytrityl)-N-acetyl-cytidine 2'-O-methyl-3'- [(2-cyanoethyl)-(N,N-diisopropyl)]-phosphoramidite, 5'-(4,4'-dimethoxytrityl)-N-isobutyryl-guanosine 2'-O-methyl-3'-[(2-cyanoethyl)-(N,N-diisopropyl)]-phosphoramidite, 5'-(4,4'-dimethoxytrityl)-uridine 2'-O-methyl-3'-[(2-cyanoethyl)- (N,N-diisopropyl)]-phosphoramidite.

The 2'-F phosphoramidites used were the following: 5'-dimethoxytrityl-N-benzoyl-deoxyadenosine 2'-fluoro-3'-[(2-cyanoethyl)-(N,N-diisopropyl)]-phosphoramidite, 5'-dimethoxytrityl-N-acetyl-deoxycytidine 2'-fluoro-3'-[(2-cyanoethyl)-(N,N-diisopropyl)]-phosphoramidite, 5'-dimethoxytrityl-N-isobutyryl-deoxyguanosine 2'-fluoro-3'- [(2-cyanoethyl)-(N,N-diisopropyl)]-phosphoramidite and 5'-dimethoxytrityl-deoxyuridine 2'-fluoro-3'-[(2-cyanoethyl)-(N,N-diisopropyl)]-phosphoramidite.

All phosphoramidites were dissolved in anhydrous acetonitrile (Honeywell Research Chemicals) at a concentration of 0.05M, except 2'-O-methyl-uridine phosphoramidite which was dissolved in DMF/MeCN (1:4, v/v). Iodine at 0.02M in acetonitrile/Pyridine/H₂O (DNAchem) was used as oxidizing reagent. Thiolation for phosphorothioate linkages was performed with 0.2 M PADS (TCI) in acetonitrile/pyridine 1: 1 v/v. 5-Ethyl thiotetrazole (ETT), 0.25M mM in acetonitrile was used as activator solution.

Inverted abasic phosphoramidite, 3-O-Dimethoxytrityl-2-deoxyribose-5-[(2-cyanoethyl)-(N, N-diisopropyl)]-phosphoramidite were purchased from Chemgenes (ANP-1422) or Hongene (OP-040).

At each cycle, the DMT was removed by deblock solution, 3% TCA in DCM (DNAchem).

The coupling time was 180 seconds. The oxidizer contact time was set to 80 seconds and thiolation time was 2*100 seconds.

At the end of the synthesis, the oligonucleotides were cleaved from the solid support using a NH₄OH:EtOH solution 4:1 (v/v) for 20 hours at 45°C (TCI). The solid support was then filtered off, the filter was thoroughly washed with H₂O and the volume of the combined solution was reduced by evaporation under reduced pressure.

Oligonucleotide were treated to form the sodium salt by ultracentrifugation using Amicon Ultra-2 Centrifugal Filter Unit; PBS buffer (10x, Teknova, pH 7.4, Sterile) or by EtOH precipitation from 1M sodium acetate.

The single strands identity were assessed by MS ESI- and then, were annealed in water to form the final duplex siRNA and duplex purity were assessed by size exclusion chromatography.

### EXAMPLE 8: SOLID PHASE SYNTHESIS METHOD: SCALE ≥5 µMOL

Syntheses of siRNA sense and antisense strands were performed on a MerMade12 synthesiser with commercially available solid supports made of controlled pore glass with universal linker (Universal CPG, with a loading of 40 µmol/g; LGC Biosearch or Glen Research) at 5 µmοl scale. Sense strand destined to 3' conjugation were sytnthesised at 12 µmοl on 3'-PT-Amino-Modifier C6 CPG 500 Å solid support with a loading of 86 µmol/g (LGC).

RNA phosphoramidites were purchased from ChemGenes or Hongene.

The 2'-O-Methyl phosphoramidites used were the following: 5'-(4,4'-dimethoxytrityl)-N-benzoyl-adenosine 2'-O-methyl-3'- [(2-cyanoethyl)-(N,N-diisopropyl)]-phosphoramidite, 5'-(4,4'-dimethoxytrityl)-N-acetyl-cytidine 2'-O-methyl-3'- [(2-cyanoethyl)-(N,N-diisopropyl)]-phosphoramidite, 5'-(4,4'-dimethoxytrityl)-N-isobutyryl-guanosine 2'-O-methyl-3'-[(2-cyanoethyl)-(N,N-diisopropyl)]-phosphoramidite, 5'-(4,4'-dimethoxytrityl)-uridine 2'-O-methyl-3'-[(2-cyanoethyl)- (N,N-diisopropyl)]-phosphoramidite.

The 2'-F phosphoramidites used were the following: 5'-dimethoxytrityl-N-benzoyl-deoxyadenosine 2'-fluoro-3'-[(2-cyanoethyl)-(N,N-diisopropyl)]-phosphoramidite, 5'-dimethoxytrityl-N-acetyl-deoxycytidine 2'-fluoro-3'-[(2-cyanoethyl)-(N,N-diisopropyl)]-phosphoramidite, 5'-dimethoxytrityl-N-isobutyryl-deoxyguanosine 2'-fluoro-3'- [(2-cyanoethyl)-(N,N-diisopropyl)]-phosphoramidite and 5'-dimethoxytrityl-deoxyuridine 2'-fluoro-3'-[(2-cyanoethyl)-(N,N-diisopropyl)]-phosphoramidite.

Inverted abasic phosphoramidite, 3-O-Dimethoxytrityl-2-deoxyribose-5-[(2-cyanoethyl)-(N, N-diisopropyl)]-phosphoramidite were purchased from Chemgenes (ANP-1422) or Hongene (OP-040).

All phosphoramidites were dissolved in anhydrous acetonitrile (Honeywell Research Chemicals) at a concentration of 0.05M, except 2'-O-methyl-uridine phosphoramidite which was dissolved in DMF/MeCN (1:4, v/v). Iodine at 0.02M in acetonitrile/Pyridine/H₂O (DNAchem) was used as oxidizing reagent. Thiolation for phosphorothioate linkages was performed with 0.2 M PADS (TCI) in acetonitrile/pyridine 1:1 v/v. 5-Ethyl thiotetrazole (ETT), 0.25M mM in acetonitrile was used as activator solution.

At each cycle, the DMT was removed by deblock solution, 3% TCA in DCM (DNAchem).

For strands synthesised on universal CPG the coupling was performed with 8 eq. of amidite for 130 seconds. The oxidation time was 47 seconds, the thiolation time was 210 seconds.

For strands synthesised on 3'-PT-Amino-Modifier C6 CPG the coupling was performed with 8 eq. of amidite for 2*150 seconds. The oxidation time was 47 seconds, the thiolation time was 250 seconds

At the end of the synthesis, the oligonucleotides were cleaved from the solid support using a NH4OH:EtOH solution 4:1 (v/v) for 20 hours at 45°C (TCI). The solid support was then filtered off, the filter was thoroughly washed with H₂O and the volume of the combined solution was reduced by evaporation under reduced pressure.

Oligonucleotide were treated to form the sodium salt by EtOH precipitation from 1M sodium acetate.

The single strand oligonucleotides were purified by IP-RP HPLC on Xbridge BEH C18 5 µm, 130 Å, 19x150 mm (Waters) column with an increasing gradient of B in A. Mobile phase A: 240 mM HFIP, 7 mM TEA and 5% methanol in water; mobile phase B: 240 mM HFIP, 7 mM TEA in methanol.

The single strands purity and identity were assessed by UPLC/MS ESI- on Xbridge BEH C18 2.5 µm, 3x50 mm (Waters) column with an increasing gradient of B in A. Mobile phase A: 100 mM HFIP, 5 mM TEA in water; mobile phase B: 20% mobile phase A: 80% Acetonitrile (v/v).

Sense strands were conjugated as per protocol provided in any of Examples 2, 4, 6.

Sense and Antisense strands were then annealed in water to form the final duplex siRNA and duplex purity were assessed by size exclusion chromatography.

### EXAMPLE 9: PHARMACOLOGICAL STUDY IN MICE

In this Example, the activity of ETX-312 (ETX-M00001378 (ETX-M00001751)), an inhibitor of ANT2/SLC25A5, specifically an inhibitor of expression of ANT2/SLC25A5 on metabolic parameters, hepatic pathology, and NAFLD Activity Score (NAS) including fibrosis stage in male biopsy-confirmed GAN DIO-NASH mice was tested. A total of 9 DIO-NASH groups (n=16) and 1 LEAN-CHOW group (n=10) of male C57BL/6JRj mice were fed 40% HFD, 40% carbohydrate (22% fructose), 2% Cholesterol (GAN) diet or normal chow (Altromin 1324) for 34 weeks prior to study start.

All mice were pre-biopsied at week -4 and stratified based on liver biopsy (only animals with fibrosis stage =F2-F3, steatosis score =3 and inflammation score ≥2 were included). Mice were anesthetized by inhalation anesthesia using isoflurane (2-3%). A small abdominal incision was made in the midline and the left lateral lobe of the liver exposed. A cone shaped wedge of liver tissue (approximately 50 mg) was excised from the distal portion of the lobe and fixated in 10% neutral buffered formalin (10% NBF) for histology. The cut surface of the liver was instantly electrocoagulated using bipolar coagulation (ERBE VIO 100 electrosurgical unit). The liver was returned to the abdominal cavity, the abdominal wall sutured and the skin closed with staplers. For post-operative recovery, mice received carprofen (5mg/kg) administered subcutaneously on the day of the procedure and on post-operation days 1 and 2.

Animals were randomized into groups based on fibrosis stage and picrosirius red (PSR) staining (% fractional area). Mice received a total of 12 weeks of dosing and were divided into the following groups: 1) LEAN-CHOW control (saline) (subcutanoues (SC), once weekly (QW)), 2) DIO-NASH control (saline) (SC, QW), 3) SiCtrl, 5 mg/kg (SC, QW), 4) ETX-312, 5 mg/kg (SC, QW), 5) Semaglutide, 30 nmol/kg (SC, once daily (QD)), 6) Semaglutide, 30 nmol/kg (SC, QD) + ETX-312, 5 mg/kg (SC, QW), 7) Vehicle (oral (PO), QD), 8) HU6, 5 mg/kg (PO, QD), 9) Resmetirom, 3 mg/kg (PO, QD), 10) Resmetirom, 3 mg/kg (PO, QD) + ETX-312, 5 mg/kg (SC, QW). Semaglutide (Ozempic^{®}) was diluted to achieve the required concentration in PBS supplemented with 0.1% BSA and let stand for 1 hour to obtain a homogeneous solution. Semaglutide was dose titrated during week 1 as follows to reach a final dose level of 30 nmol/kg for all remaining doses: 0.6 nmol/kg day 1; 1.2 nmol/kg day 2; 2.4 nmol/kg day 3: 4.8 nmol/kg day 4; 12 nmol/kg day 5, 30 nmol/kg day 6. HU6 stock solution was prepared in DMSO and diluted in 0.5% MC/0.2% Tween 80 resulting in 5% DMSO/0.5% MC/0.2% Tween 80. Resmetirom (MGL-3196) was mixed with a small volume of 0.5% CMC + 0.1% Tween80 (500µL) and crushed with a spatula. Vehicle PO (0.5 % CMC/ 0.1% Tween80) was added to obtain the required concentration of Resmetirom. The suspension was stirred for 2 hours to obtain orange/beige microsuspension.

Body weight was recorded daily and food intake recorded once weekly for 24 hours during the study period.

At the end of the study period, preterminal tail vein blood collection was conducted for non-fasting plasma Alanine transaminase (ALT), Aspartate transaminase (AST), Triglycerides (TG) and Total Cholesterol (TC) analysis. Tail-vein, tongue or cheek blood was collected in heparinized tubes and mixed by inversion 5 times. Blood was placed at 4°C until it was centrifuged at 3000 g for 10 minutes. The plasma supernatants were transferred to new tubes and immediately frozen on dry ice. The samples were stored at -70°. Terminal plasma samples were prepared during anesthesia with isoflurane with the abdominal cavity opened and cardiac blood drawn with a syringe into a Microvette/Vacuette of appropriate dimensions with anticoagulant and mixed by inversion 5 times. Blood was placed at 4°C until it was centrifuged at 3000 g for 10 minutes. The plasma supernatants were transferred to new tubes and immediately frozen on dry ice. The samples were stored at - 70°C.

ALT, AST, TG, and TC assays were measured using commercial kits (Roche Diagnostics), on the Cobas c 501 autoanalyzer. TIMP-1 was measured in plasma collected in EDTA tubes using a commercial ELISA kit (R&D Systems). PIIINP was measured in plasma collected in EDTA tubes using a commercial ELISA kit (Cusabio).

After study termination, the liver was collected and weighed. Specific liver samples and biopsies were dissected and prepared as follows. The liver was divided into a left lateral lobe, medial lobe, right lateral lobe, and caudate lobe. The Post-biopsy piece (~200 mg, less than 0.7 x 0.5 cm) was cut from the left lateral lobe, 4 mm from the prebiopsy site with an edge. The tissue was collected in 10% neutral buffered formalin. Two liver samples collected for future analysis of protein expression (~ 50 mg per piece) were dissected from the left medial lobe, collected in tubes and frozen in liquid nitrogen. A third piece of liver was dissected from the center of the left lateral lobe (~ 50 mg), put in a tube, stored in RNAlater for 24h at 4oC and stored at -70°C.

Data were analysed via fitting of a simple linear model with fitted coefficients for the mean effect of the treatment on the biomarker, compared to a baseline comparator. p-values correspond to one-tailed t-tests on the regression coefficients, testing the alternate hypothesis that the coefficient in question is less than 0 (one-tailed) for non-control groups (equivalent to testing there being a reductive effect versus the comparator) and not equal to 0 (two-tailed) for the SiCtrl group (equivalent to testing there being no difference versus the comparator). Where significant heteroskedasticity was observed between treatment groups, p-values were calculated using robust estimation to the variance-covariance matrix, p-values were subsequently corrected for multiple comparisons via the FDR method. The following biomarkers were tested for increases or decreases (i.e. two-tailed) in the treatment groups: mRNA, Protein.

FFPE biopsies were placed in 10% neutral buffered formalin (10% NBF) for approximately 24 hours and then transferred to 70% ethanol and stored at 4°C. The FFPE biopsies were then placed in the Histokinette to infiltrate prior to embedding in blocks. Biopsy tissues were cut at 3µm on a microtome and the sections were mounted on Superfrost Plus slides and stored at 4 ⁰C.

Glass slides with paraffin embedded sections were deparaffinated in xylene and rehydrated in series of graded ethanol. For Hematoxylin & Eosin (H&E) staining, slides are incubated in Mayer's Hematoxylin (Dako), washed in tap water, stained in Eosin Y solution (Sigma-Aldrich), dehydrated in graded ethanol and cover slipped. For Picrosirius red (PSR) staining, slides were incubated in Weigert's iron hematoxylin (Sigma-Aldrich), washed in tap water, stained in Picrosirius red (Sigma-Aldrich) and washed twice in acidified water. Excess water was removed by shaking the slides and the slides were then dehydrated in three changes of 100% ethanol, cleared in xylene and cover slipped. Immunohistochemistry (IHC) was performed using standard procedures. Briefly, after antigen retrieval and blocking of endogenous peroxidase activity, slides were incubated with primary antibody. The primary antibody was detected using a polymeric HRP-linker antibody conjugate. Next, the primary antibody was visualized with DAB as chromogen. Finally, sections were counterstained in hematoxylin and cover slipped. Slides were scanned under a 20x objective in a ScanScope AT slide scanner (Aperio).

### Antibodies Used:

| **Antigen** | **Primary Antibody** | **Vendor** |
|---|---|---|
| Col1a1 | Goat anti-type I Collagen | Southern Biotech, Cat. 1310-01 |
| Galectin-3 (mouse) | Rat anti-Galectin 3 | Biolegend, Cat. 125402 |
| α-SMA | Rabbit anti-alpha smooth muscle actin [EPR5368] | Abcam, Cat. ab124964 |
| CD45 | Rabbit anti-CD45 | AbCam, Cat. Ab10558 |

Liver samples stained with H&E or PSR were given a score for NAFLD Activity Score (NAS) and fibrosis stage, respectively, using the clinical criteria outlined by Kleiner et al. (Design and validation of a histological scoring system for nonalcoholic fatty liver disease, Hepatology, 2005) shown in the table below. Total NAS represents the sum of scores for steatosis, inflammation, and ballooning, and ranges from 0-8. NAS and fibrosis stage was determined by Gubra Histopathological Objective Scoring Technology (GHOST) deep learning app developed by Gubra using the VIS software (Visiopharm, Denmark) for a more accurate and objective method for staging disease in DIO-NASH mouse models. The app scores were reviewed by a histopathologist blinded to the results. Terminal liver biopsy histomorphometric analysis was also conducted by immunohistochemistry (IHC) for inflammation (Gal-3 and CD45), Collagen (Col1a1), and activated stellate cells (a-SMA).

### NAS Scoring:

| **Feature** | **Degree** | **Score** |
|---|---|---|
| Steatosis (percentage of hepatocytes with lipid droplets) | <5% | 0 |
| | 5-33% | 1 |
| | >33-66% | 2 |
| | >66% | 3 |
| Lobular inflammation | No foci | 0 |
| | <2 foci/200x | 1 |
| | 2-4 foci/200x | 2 |
| | >4 foci/200x | 3 |
| Ballooning degeneration | None | 0 |
| | Few | 1 |
| | Many cells/prominent | 2 |
| | ballooning | |
| Fibrosis stage | None | 0 |
| | Perisinusoidal or periportal | 1 |
| | Perisinusoidal and | 2 |
| | portal/periportal | 3 |
| | Bridging fibrosis | 4 |
| | Cirrhosis | |

For mRNA extraction, 50 mg of mouse liver per animal was homogenized in lysis buffer and DTT (MagMAXTM mirVanaTM Total RNA Isolation Kit, Thermo Fisher Scientific; Tissue Lyser II, QIAGEN). mRNA were isolated using an automated system (KingFisher Apex, Thermo Fisher Scientific), and cDNA synthesis was performed after genomic DNA removal (HiScript^{®} III RT SuperMix for qPCR with gDNA wiper, Vazyme). qPCR was performed in technical triplicates, with TaqMan gene expression assays for the target and the house keeping genes (Mm00846873_g1, Mm99999915_g1, Thermo Fisher Scientific) and a PCR kit (Universal PCR Master Mix, Roche), using the Applied Biosystems QuantStudio Flex by Thermo Fisher Scientific. Relative gene expression for each sample was calculated with the ddCt method, normalized to vehicle treated samples.

For protein extraction, 50 mg of mouse liver per animal was homogenized in RIPA buffer supplemented with protease inhibitor, phosphatase inhibitor and PMSF (Tissue Lyzer, QIAGEN). Protein digestion was performed with 100 ug of liver lysate per animal, using DTT and IAA as reductant and alkylation reagent. (S-Trap 96- well MS sample prep kit, ProtiFi). 500 ng of peptide per liver sample was run on a 60 min LC method in Easy Nano 1200 (mobile phase A: 0.1% formic acid in H2O, mobile phase B: 0.1% formic acid in 80% acetonitrile). The separation gradient was: 7-18% B from 0 to 16 minutes, 18-40% B from 16 to 49 minutes, 40-100% B from 49-52 minutes and maintain at 100 % B to the end. A 25 cm of C18 column was used for peptide separation. The eluted peptide was analyzed with PRM-MS/MS method (Orbitrap Eclipse, Thermo Fisher Scientific). Candidate peptides were identified based on the following criteria: 1. Peptide must be unique for targeted protein; 2. Trypsin digested peptide with no missed cleavage; 3. Peptide with no potential modification sites; 4. M/Z range between 400-1000. The raw data was processed in Skyline, and the peptide quality was manually checked. The protein database from UniProt was used for the library search. Peptides were selected based on: 1. Elution peak without tailing; 2. Well-matched product ions; 3. Quantified peak area greater than 1e6. Consequently, the following two peptides: QIFLGGVDK and EQGVLSFWR were quantified. Relative abundance for each peptide in each liver sample was quantified by averaging the peak area of top three most intense product ions, normalized to the vehicle control, and relative abundances for the two peptides were averaged for final relative protein abundance.

As shown in Figures 10 and 11, the inhibitor ETX-312 resulted in a significant improvement of the NAFLD activity score (NAS). Of note, the effect obtained with ETX-312 was greater than the effects obtained with both positive controls, i.e., the GLP-1 agonist semaglutide and the THR-beta agonist resmetirom. Surprisingly, the most significant improvement was obtained when ETX-312 was combined with semaglutide or resmetirom.

ETX-312 significantly reduced levels of the blood markers ALT, AST, TIMP-1, and PIIINP, whether administered alone or in combination with semaglutide or resmetirom. The combination therapies demonstrated a significantly enhanced effect compared to monotherapies with semaglutide or resmetirom. Additionally, ETX-312, either alone or in combination with semaglutide or resmetirom, significantly improved hepatomegaly and plasma total cholesterol levels. The impact of the inhibitor ETX-312 on the blood markers ALT, AST, TIMP-1 and PIIINP is shown in Figures 12 and 13. The terminal liver weight to body weight ratio in DIO-NASH mice that received different treatments is shown in Figure 14.

### EXAMPLE 10: FURTHER PHARMACOLOGICAL STUDY IN MICE

In this Example, the activity of ETX-312 (ETX-M00001378 also known as ETX-M00001751), an inhibitor of ANT2/SLC25A5, specifically expression of ANT2/SLC25A5 on metabolic parameters, hepatic pathology, NAFLD Activity Score (NAS) and fibrosis stage in male biopsy-confirmed GAN DIO-NASH mice was tested. A total of 6 DIO-NASH groups (n=18) and 1 LEAN-CHOW group (n=10) of male C57BL/6JRj mice were fed 40% HFD, 40% carbohydrate (22% fructose), 2% Cholesterol (GAN) diet (D09100310, Ssniff) or normal chow (Altromin 1324, Bro-garden) for 32 weeks prior to study start.

All mice were pre-biopsied at week -4 as per example 9 and stratified based on liver biopsy (only animals with fibrosis stage =F2-F3, steatosis score =3 and inflammation score ≥2 were included).

Animals were randomized into groups based on fibrosis stage and picrosirius red (PSR) staining (% fractional area). Mice received a total of 16 weeks of dosing and were divided into the following groups: 1) LEAN-CHOW control (saline) (subcutanoues (SC), once weekly (QW)), 2) DIO-NASH Vehicle (saline) (SC, QW), 3) ETX-312, 5 mg/kg (SC, QW), 4) Efruxifermin, 1 mg/kg (SC, QW), 5) Efruxifermin, 1 mg/kg (SC, QW) + ETX-312, 5 mg/kg (SC, QW), 6) Tirzepatide, 10 nmol/kg (SC, QD), 7) Tirzepatide, 10 nmol/kg (SC, QD) + ETX-312, 5 mg/kg (SC, QW). Efruxifermin was supplied as a stock solution of 4.8 mg/ml in 20 mM HEPES, 150 mMNaCl, 240 mM Sucrose, pH 7.2. It was diluted with 10 mM TRIS-base containing 2.32% I-lysine and 0.008% polysorbate 20 to desired concentration. Tirzepatide (hydrochloride) stock solution was prepared and then diluted to the required dosing solution in 50 mM phosphate buffer with 3.5% mannitol, pH 7.4. Efruxifermin was administered at 3 mg/kg for groups 4 and 5 on Day 1 and Day 7 then reduced to 1 mg/kg for all remaining doses. Tirzepatide was dose titrated during week 1 as follows to reach a final dose level of 10 nmol/kg for all remaining doses: 0.6 nmol/kg on Day 1, 1.2 nmol/kg on Day 2, 2.4 nmol/kg on Day 3, 4.8 nmol/kg on Day 4, and 10 nmol/kg on Day 5.

Body weight was recorded daily and food intake recorded once weekly for 24 hours during the study period.

At the end of the study period, plasma Alanine transaminase (ALT), Aspartate transaminase (AST), Triglycerides (TG) and Total Cholesterol (TC), TIMP-1 and PIIINP levels were measured as per example 9. One animal was excluded from TIMP-1 analysis due to high percent CV. PIIINP could be measured in 83 out of 117 samples due to small sample volume or readings below the limit of quantification.

After study termination, the liver was collected and weighed as per example 9. FFPE biopsies were prepared and stained, as per example 9.

Statistical analyses were performed as per example 9 except for ALT and AST endpoints which were log-transformed for statistical analysis due to observed positive skew. Transformations were supported by significant (p<0.05) improvements in fitted model log-likelihoods.

As shown in Figures 19 and 20, the inhibitor ETX-312 resulted in a significant improvement of the blood markers ALT, AST, TIMP-1 and PIIINP alone or in combination with Efruxifermin or Tirzepatide. Figure 21 depicts the terminal liver weight to body weight ratio in DIO-NASH mice that received different treatments showing that ETX-312 alone or in combination with Efruxifermin or Tirzepatide significantly reduced hepatomegaly. ETX-312 alone or in combination with either Efruxifermin or Tirzepatide reduced plasma total cholesterol levels as shown in Figure 22.

The present invention is not intended to be limited in scope to the particular disclosed embodiments, which are provided, for example, to illustrate various aspects of the invention. Various modifications to the compositions and methods described will become apparent from the description and teachings herein. Such variations may be practiced without departing from the true scope and spirit of the disclosure and are intended to fall within the scope of the present disclosure.

The invention also relates to the following numbered clauses:
1. An inhibitor of expression and / or function of SLC25A5/ANT2, wherein said inhibitor is conjugated to one or more ligand moieties.
2. An inhibitor according to clause 1, wherein said inhibitor is an siRNA oligomer.
3. An inhibitor of expression and / or function of SLC25A5/ANT2, wherein said inhibitor is an siRNA oligomer.
4. An inhibitor according to clause 3, wherein said inhibitor comprises an siRNA oligomer conjugated to one or more ligand moieties.
5. An inhibitor of expression and / or function of SLC25A5/ANT2, such as the inhibitor of clauses 1-4, for use in prevention and/or treatment of metabolic disease or disorder, such as a metabolic disease or disorder associated with non-alcoholic fatty liver disease (NAFLD) and/or obesity and/or a disease or disorder associated with adipogenesis and/or for use in reducing adipogenesis.
6. An inhibitor for use according to clause 5, wherein the inhibitor is to be used in combination with a GLP-1 agonist and/or a THR-beta agonist.
7. An inhibitor for use according to clause 5, wherein the inhibitor is to be used in combination with a GLP-1 agonist and/or an FGF-21 analogue.
8. An inhibitor for use according to clause 6 or clause 7, wherein the GLP-1 agonist is a GLP-1/GIP dual agonist, a GLP-1/FGF21 dual agonist, a GLP-1/GCGR dual agonist, or a GLP-1/GIP/GCGR triple agonist.
9. An inhibitor for use according to any one of clauses 6 to 8, wherein the GLP-1 agonist is semaglutide.
10. An inhibitor for use according to any one of clauses 6 to 8, wherein the GLP-1/GIP dual agonist is tirzepatide.
11. An inhibitor for use according to any one of clauses 6 and 8 to 10, wherein the THR-beta agonist is resmetirom.
12. An inhibitor for use according to any one of clauses 7 to 10, wherein the FGF-21 analogue is efruxifermin.
13. An inhibitor for use according to any one of clauses 5 to 12, wherein the inhibitor is to be used in combination with one or more of: an amylin receptor agonist (such as pramlintide), and/or a dual amylin + calcitonin receptor agonist, and/or a glucagon receptor agonist, and/or an FXR receptor agonist (such as cilofexor or obeticholic acid), and/or an FGF-21 analogue or FGF-21 receptor agonist (such as efruxifermin), and/or an FGF-19 analogue or FGF-19 receptor agonist (such as aldafermin), and/or a galectin 3 inhibitor (such as belapectin), and/or a PPARα agonist (such as elafibrinor), and/or a PPARγ agonist (such as pioglitazone or rosiglitazone), and/or a mixed PPARα and/or δ and/or γ agonist, and/or a pan PPAPαγδ agonist (such as lanafibranor), and/or an acetyl CoA desaturase activator, and/or an ASK1 inhibitor (such as selonsertib), and/or an LOXL2 inhibitor (such as simtuzumab), and/or a dual CCR2/5 inhibitor (such as cenicriviroc), and/or an inhibitor of an enzyme in the de novo lipogenesis (DNL) pathway including citrate/isocitrate carrier (CIC), ATP-citrate lyase (ACLY), acetyl-CoA carboxylase (ACC) and fatty acid synthase (FAS), and/or an inhibitor of an enzyme in the cholesterol biosynthesis pathway (such as an HMGCoA reductase inhibitor, such as atorvastatin).
14. An inhibitor according to clause 1, 2 or 4, wherein said one or more ligand moieties comprise one or more GalNAc ligands or comprise one more GalNAc ligand derivatives.
15. An inhibitor for use according to clause 1, 2 or 4 wherein said one or more ligand moieties comprise one or more GalNAc ligand derivatives.
16. An inhibitor or an inhibitor for use according to one or more preceding clauses, wherein the target of the inhibitor is SLC25A5/ANT2.
17. An inhibitor, or inhibitor for use, according to one or more preceding clauses, which is an siRNA oligomer having a first and a second strand wherein:
   i) the first strand of the siRNA has a length in the range of 15 to 30 nucleosides, preferably 19 to 25 nucleosides, more preferably 23 or 25; even more preferably 23; and / or
   ii) the second strand of the siRNA has a length in the range of 15 to 30 nucleosides, preferably 19 to 25 nucleosides, more preferably 21 nucleosides.
18. An inhibitor, or inhibitor for use, according to clause 17, wherein the second sense strand further comprises one or more abasic nucleosides in a terminal region of the second strand, and wherein said abasic nucleoside(s) is / are connected to an adjacent nucleoside through a reversed internucleoside linkage.
19. An inhibitor, or inhibitor for use, according to clause 18, wherein the second strand comprises:
   i 2, or more than 2, abasic nucleosides in a terminal region of the second strand; and / or
   ii 2, or more than 2, abasic nucleosides in either the 5' or 3' terminal region of the second strand; and / or
   iii 2, or more than 2, abasic nucleosides in either the 5' or 3' terminal region of the second strand, wherein the abasic nucleosides are present in an overhang as herein described; and / or
   iv 2, or more than 2, consecutive abasic nucleosides in a terminal region of the second strand, wherein preferably one such abasic nucleoside is a terminal nucleoside; and / or
   v 2, or more than 2, consecutive abasic nucleosides in either the 5' or 3' terminal region of the second strand, wherein preferably one such abasic nucleoside is a terminal nucleoside in either the 5' or 3' terminal region of the second strand; and / or
   vi a reversed internucleoside linkage connects at least one abasic nucleoside to an adjacent basic nucleoside in a terminal region of the second strand; and / or
   vii a reversed internucleoside linkage connects at least one abasic nucleoside to an adjacent basic nucleoside in either the 5' or 3' terminal region of the second strand; and / or
   viii an abasic nucleoside as the penultimate nucleoside which is connected via the reversed linkage to the nucleoside which is not the terminal nucleoside (called the antepenultimate nucleoside herein); and / or
   ix abasic nucleosides as the 2 terminal nucleosides connected via a 5'-3' linkage when reading the strand in the direction towards that terminus;
   x abasic nucleosides as the 2 terminal nucleosides connected via a 3'-5' linkage when reading the strand in the direction towards the terminus comprising the terminal nucleosides;
   xi abasic nucleosides as the terminal 2 positions, wherein the penultimate nucleoside is connected via the reversed linkage to the antepenultimate nucleoside, and wherein the reversed linkage is a 5-5' reversed linkage or a 3'-3' reversed linkage;
   xii abasic nucleosides as the terminal 2 positions, wherein the penultimate nucleoside is connected via the reversed linkage to the antepenultimate nucleoside, and wherein either
      (1) the reversed linkage is a 5-5' reversed linkage and the linkage between the terminal and penultimate abasic nucleosides is 3'5' when reading towards the terminus comprising the terminal and penultimate abasic nucleosides; or
      (2) the reversed linkage is a 3-3' reversed linkage and the linkage between the terminal and penultimate abasic nucleosides is 5'3' when reading towards the terminus comprising the terminal and penultimate abasic nucleosides.
20. An inhibitor, or inhibitor for use, according clause 18 or 19, wherein the reversed internucleoside linkage is at a terminal region which is distal to the 5' terminal region of the second strand, or at a terminal region which is distal to the 3' terminal region of the second strand.
21. An inhibitor, or inhibitor for use, according to any one of clauses 18 to 20, wherein the reversed internucleoside linkage is a 3'3 reversed linkage.
22. An inhibitor, or inhibitor for use, according to any one of clauses 18 to 20, wherein the reversed internucleoside linkage is a 5'5 reversed linkage.
23. An inhibitor, or inhibitor for use, according to any one of clauses 17 to 22, wherein one or more nucleosides on the first strand and / or the second strand is / are modified, to form modified nucleosides.
24. An inhibitor, or inhibitor for use, according to clause 23, wherein the modification is a modification at the 2'-OH group of the ribose sugar, optionally selected from 2'-Me or 2'-F modifications.
25. An inhibitor, or inhibitor for use, according to clause 23 or 24, wherein the first strand comprises a 2'-F at any of position 14, position 2, position 6, or any combination thereof, counting from position 1 of said first strand.
26. An inhibitor, or inhibitor for use, according to any one of clauses 23 to 25, wherein the second strand comprises a 2'-F modification at position 7 and / or 9, and / or 11 and / or 13, counting from position 1 of said second strand.
27. An inhibitor, or inhibitor for use, according to any one of clauses 23 to 26, wherein the first and second strand each comprise 2'-Me and 2'-F modifications.
28. An inhibitor, or inhibitor for use, according to any one of clauses 23 to 27, which is an siRNA, wherein the siRNA comprises at least one thermally destabilizing modification, suitably at one or more of positions 1 to 9 of the first strand counting from position 1 of the first strand, and / or at one or more of positions on the second strand aligned with positions 1 to 9 of the first strand, wherein the destabilizing modification is selected from a modified unlocked nucleic acid (UNA) and a glycol nucleic acid (GNA), preferably a glycol nucleic acid.
29. An inhibitor, or inhibitor for use, according to clause 28, wherein the siRNA comprises at least one thermally destabilizing modification at position 7 of the first strand, counting from position 1 of the first strand.
30. An inhibitor, or inhibitor for use, according to any one of clauses 23 to 29, which is an siRNA, wherein the siRNA comprises 3 or more 2'-F modifications at positions 7 to 13 of the second strand, such as 4, 5, 6 or 7 2'-F modifications at positions 7 to 13 of the second strand, counting from position 1 of said second strand
31. An inhibitor, or inhibitor for use, according to any one of clauses 23 to 30, which is an siRNA, wherein said second strand comprises at least 3, such as 4, 5 or 6, 2'-Me modifications at positions 1 to 6 of the second strand, counting from position 1 of said second strand.
32. An inhibitor, or inhibitor for use, according to any one of clauses 23 to 31, which is an siRNA, wherein said first strand comprises at least 5 2'-Me consecutive modifications at the 3' terminal region, preferably including the terminal nucleoside at the 3' terminal region, or at least within 1 or 2 nucleosides from the terminal nucleoside at the 3' terminal region.
33. An inhibitor, or inhibitor for use, according to any one of clauses 23 to 32, which is an siRNA wherein said first strand comprises 7 2'-Me consecutive modifications at the 3' terminal region, preferably including the terminal nucleoside at the 3' terminal region.
34. An inhibitor, or inhibitor for use, according to any one of clauses 23 to 29, which is an siRNA, wherein said modified nucleosides of said second strand comprise a modification pattern according to any one of the following (5'-3'):
   (Me)₈ - (F)₃ - (Me)₁₀
35. An inhibitor, or inhibitor for use, according to any one of clauses 23 to 29 or 34, which is an siRNA, wherein nucleosides of said first strand comprise a 2' sugar modification pattern wherein said modifications are selected at least from 2'Me and 2'F sugar modifications, provided that the overall number of 2'F sugar modifications in the first strand does not consist of four, or six, 2'F modifications.
36. An inhibitor, or inhibitor for use, according to any one of clauses 23 to 29 or 34 to 35, which is an siRNA, wherein said modifications are selected at least from 2'Me and 2'F sugar modifications, wherein the overall number of 2'F sugar modifications in the first strand consists of three, five or seven 2'F modifications.
37. An inhibitor, or inhibitor for use, according to any one of clauses 23 to 36, wherein the siRNA oligomer further comprises one or more phosphorothioate internucleoside linkages.
38. An inhibitor, or inhibitor for use, according to clause 37, wherein said one or more phosphorothioate internucleoside linkages are respectively between at least three consecutive positions in a 5' or 3' near terminal region of the second strand, whereby said near terminal region is preferably adjacent said terminal region wherein said one or more abasic nucleosides of said second strand is / are located according to at least clause 18.
39. An inhibitor, or inhibitor for use, according to claim 37 or 38, wherein said one or more phosphorothioate internucleoside linkages are respectively between at least three consecutive positions in a 5' and / or 3' terminal region of the first strand, whereby preferably a terminal position at the 5' and / or 3' terminal region of said first strand is attached to its adjacent position by a phosphorothioate internucleoside linkage.
40. An inhibitor, or inhibitor for use according to any one of clauses 17 to 39, wherein the oligomer is an siRNA and the second strand of the siRNA is conjugated directly or indirectly to one or more ligand moiety(s), wherein said ligand moiety is typically present at a terminal region of the second strand, preferably at the 3' terminal region thereof.
41. An inhibitor, or inhibitor for use according to clause 40, wherein the ligand moiety comprises
   i) one or more GalNAc ligands; and / or
   ii) one or more GalNAc ligand derivatives; and / or
   iii) one or more GalNAc ligands and / or GalNAc ligand derivatives conjugated to said SiRNA through a linker.
42. An inhibitor, or inhibitor for use according to clause 41, wherein said one or more GalNAc ligands and / or GalNAc ligand derivatives are conjugated directly or indirectly to the 5' or 3' terminal region of the second strand of the siRNA oligomer, preferably at the 3' terminal region thereof.
43. An inhibitor, or inhibitor for use according to clause 41 or 42, wherein the ligand moiety comprises 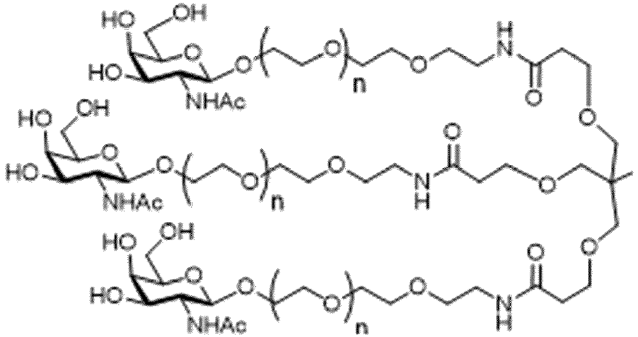
44. An inhibitor, or inhibitor for use according to clause 41 or 42, having the structure: 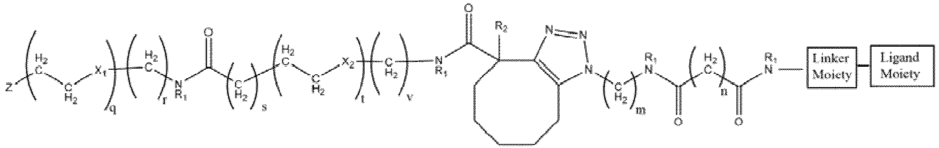 wherein:
   R₁ at each occurrence is independently selected from the group consisting of hydrogen, methyl and ethyl;
   R₂ is selected from the group consisting of hydrogen, hydroxy, -OC₁₋₃alkyl, -C(=O)OC₁₋₃alkyl, halo and nitro;
   X₁ and X₂ at each occurrence are independently selected from the group consisting of methylene, oxygen and sulfur;
   m is an integer of from 1 to 6;
   n is an integer of from 1 to 10;
   q, r, s, t, v are independently integers from 0 to 4, with the proviso that:
      (i) q and r cannot both be 0 at the same time; and
      (ii) s, t and v cannot all be 0 at the same time;
   Z is an oligomer
45. An inhibitor, or inhibitor for use according to clause 41 or 42, having the structure 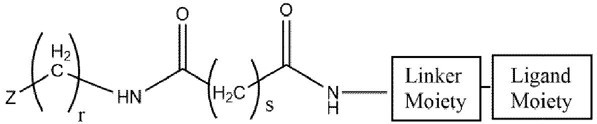 wherein:
   r and s are independently an integer selected from 1 to 16; and
   Z is an oligomer.
46. An inhibitor, or inhibitor for use according to one or more preceding clauses, formulated as a pharmaceutical composition with an excipient and / or carrier.
47. A pharmaceutical composition comprising an inhibitor according to one or more preceding clauses, in combination with a pharmaceutically acceptable excipient or carrier.
48. A pharmaceutical composition comprising an inhibitor according to one or more preceding clauses, in combination with a pharmaceutically acceptable excipient or carrier, for use in prevention and/or treatment of metabolic disease or disorder, such as a metabolic disease or disorder associated with non-alcoholic fatty liver disease (NAFLD) and/or obesity and/or a disease or disorder associated with adipogenesis and/or for use in reducing adipogenesis.
49. A pharmaceutical composition for use according to clause 48, wherein the pharmaceutical composition is to be used in combination with a GLP-1 agonist and/or a THR-beta agonist.
50. A pharmaceutical composition for use according to clause 48, wherein the pharmaceutical composition is to be used in combination with a GLP-1 agonist and/or an FGF-21 analogue.
51. A pharmaceutical composition for use according to clause 49 or clause 50, wherein the GLP-1 agonist is a GLP-1/GIP dual agonist, a GLP-1/FGF21 dual agonist, a GLP-1/GCGR dual agonist, or a GLP-1/GIP/GCGR triple agonist.
52. A pharmaceutical composition for use according to any one of clauses 49 to 51, wherein the GLP-1 agonist is semaglutide.
53. A pharmaceutical composition for use according to any one of clauses 49 to 51, wherein the GLP-1/GIP dual agonist is tirzepatide.
54. A pharmaceutical composition for use according to any one of clauses 49 and 51-53, wherein the THR-beta agonist is resmetirom.
55. A pharmaceutical composition for use according to any one of clauses 50 to 53, wherein the FGF-21 analogue is efruxifermin.
56. A pharmaceutical composition for use according to any one of clauses 48 to 55, wherein the composition is to be used in combination with one or more of: an amylin receptor agonist (such as pramlintide), and/or a dual amylin + calcitonin receptor agonist, and/or a glucagon receptor agonist, and/or an FXR receptor agonist (such as cilofexor or obeticholic acid), and/or an FGF-21 analogue or FGF-21 receptor agonist (such as efruxifermin), and/or an FGF-19 analogue or FGF-19 receptor agonist (such as aldafermin), and/or a galectin 3 inhibitor (such as belapectin), and/or a PPARα agonist (such as elafibrinor), and/or a PPARγ agonist (such as pioglitazone or rosiglitazone), and/or a mixed PPARα and/or δ and/or γ agonist, and/or a pan PPARαγδ agonist (such as lanafibranor), and/or an acetyl CoA desaturase activator, and/or an ASK1 inhibitor (such as selonsertib), and/or an LOXL2 inhibitor (such as simtuzumab), and/or a dual CCR2/5 inhibitor (such as cenicriviroc), and/or an inhibitor of an enzyme in the de novo lipogenesis (DNL) pathway including citrate/isocitrate carrier (CIC), ATP-citrate lyase (ACLY), acetyl-CoA carboxylase (ACC) and fatty acid synthase (FAS), and/or an inhibitor of an enzyme in the cholesterol biosynthesis pathway (such as an HMGCoA reductase inhibitor, such as atorvastatin).
57. Use of SLC25A5/ANT2 as a target for identifying one or more therapeutic agents for the treatment of a metabolic disease or disorder, such as a metabolic disease or disorder associated with non-alcoholic fatty liver disease (NAFLD) and/or obesity and/or a disease or disorder associated with adipogenesis and/or for use in reducing adipogenesis.
58. A method of treating or preventing a metabolic disease or disorder, such as a metabolic disease or disorder associated with non-alcoholic fatty liver disease (NAFLD) and/or obesity and/or a disease or disorder associated with adipogenesis and/or for use in reducing adipogenesis, the method comprising administering an inhibitor of SLC25A5/ANT2.
59. A method according to clause 58, wherein the inhibitor of SLC25A5/ANT2 is administered together with a GLP-1 agonist and/or a THR-beta agonist.
60. A method according to clause 58, wherein the inhibitor of SLC25A5/ANT2 is administered together with a GLP-1 agonist and/or an FGF-21 analogue.
61. A method according to clause 59 or clause 60, wherein the GLP-1 agonist is a GLP-1/GIP dual agonist, a GLP-1/FGF21 dual agonist, a GLP-1/GCGR dual agonist, or a GLP-1/GIP/GCGR triple agonist.
62. A method according to any one of clauses 59 to 61, wherein the GLP-1 agonist is semaglutide.
63. A method according to any one of clauses 59 to 61, wherein the GLP-1/GIP dual agonist is tirzepatide.
64. A method according to any one of clauses 59 and 61 to 63, wherein the THR-beta agonist is resmetirom.
65. A method according to any one of clauses 60 to 63, wherein the FGF-21 analogue is efruxifermin.
66. A method according to any one of clauses 58 to 65, wherein the inhibitor is to be used in combination with one or more of: an amylin receptor agonist (such as pramlintide), and/or a dual amylin + calcitonin receptor agonist, and/or a glucagon receptor agonist, and/or an FXR receptor agonist (such as cilofexor or obeticholic acid), and/or an FGF-21 analogue or FGF-21 receptor agonist (such as efruxifermin), and/or an FGF-19 analogue or FGF-19 receptor agonist (such as aldafermin), and/or a galectin 3 inhibitor (such as belapectin), and/or a PPARα agonist (such as elafibrinor), and/or a PPARγ agonist (such as pioglitazone or rosiglitazone), and/or a mixed PPARα and/or δ and/or γ agonist, and/or a pan PPAPαγδ agonist (such as lanafibranor), and/or an acetyl CoA desaturase activator, and/or an ASK1 inhibitor (such as selonsertib), and/or an LOXL2 inhibitor (such as simtuzumab), and/or a dual CCR2/5 inhibitor (such as cenicriviroc), and/or an inhibitor of an enzyme in the de novo lipogenesis (DNL) pathway including citrate/isocitrate carrier (CIC), ATP-citrate lyase (ACLY), acetyl-CoA carboxylase (ACC) and fatty acid synthase (FAS), and/or an inhibitor of an enzyme in the cholesterol biosynthesis pathway (such as an HMGCoA reductase inhibitor, such as atorvastatin).
67. SLC25A5/ANT2 for use as a biomarker of a metabolic disease or disorder, such as a metabolic disease or disorder associated with non-alcoholic fatty liver disease (NAFLD) and/or obesity and/or a disease or disorder associated with adipogenesis and/or for reducing adipogenesis.
68. SLC25A5/ANT2 for use in an *in vivo* method of predicting susceptibility to a metabolic disease related to a disorder, such as a metabolic disease or disorder associated with non-alcoholic fatty liver disease (NAFLD) and/or obesity and/or a disease or disorder associated with adipogenesis and/or for use in reducing adipogenesis, typically by monitoring the sequence and/ or level of expression and / or function of SLC25A5/ANT2 in a sample obtained from a patient.
69. A method of predicting susceptibility to a metabolic disease or disorder, such as a metabolic disease or disorder associated with non-alcoholic fatty liver disease (NAFLD) and/or obesity and/or a disease or disorder associated with adipogenesis and/or in reducing adipogenesis in a patient, said method comprising:
   (a) obtaining a sample from the patient,
   (b) detecting the sequence and / or expression and / or function of SLC25A5/ANT2 in said sample obtained from the patient,
   (c) predicting susceptibility to a disease or disorder, such as a metabolic disease or disorder associated with non-alcoholic fatty liver disease (NAFLD) and/or obesity and/or a disease or disorder associated with adipogenesis, based on the sequence and / or expression and / or function of SLC25A5/ANT2 in said sample obtained from the patient,
   (d) preferably administering to the diagnosed patient an effective amount of an inhibitor of SLC25A5/ANT2.
70. An inhibitor or composition according to any preceding clause, in the preparation of a medicament for use in the treatment of a metabolic disease or disorder, such as a metabolic disease or disorder associated with non-alcoholic fatty liver disease (NAFLD) and/or obesity and/or a disease or disorder associated with adipogenesis.

## Claims

1. An inhibitor of expression and / or function of SLC25A5/ANT2, wherein said inhibitor is conjugated to one or more ligand moieties, optionally wherein said inhibitor is an siRNA oligomer.

2. An inhibitor of expression and / or function of SLC25A5/ANT2, wherein said inhibitor is an siRNA oligomer, optionally wherein said inhibitor comprises an siRNA oligomer conjugated to one or more ligand moieties.

3. An inhibitor of expression and / or function of SLC25A5/ANT2, such as the inhibitor of claim 1 or claim 2, for use in prevention and/or treatment of metabolic disease or disorder, such as a metabolic disease or disorder associated with non-alcoholic fatty liver disease (NAFLD) and/or obesity and/or a disease or disorder associated with adipogenesis and/or for use in reducing adipogenesis, optionally wherein the inhibitor is to be used in combination with a GLP-1 agonist and/or a THR-beta agonist, further optionally wherein the GLP-1 agonist is a GLP-1/GIP dual agonist, a GLP-1/FGF21 dual agonist, a GLP-1/GCGR dual agonist, or a GLP-1/GIP/GCGR triple agonist, for example wherein the GLP-1 agonist is semaglutide, or wherein the GLP-1/GIP dual agonist is tirzepatide, or, wherein the THR-beta agonist is resmetirom.

4. An inhibitor for use according to claim 3, wherein the inhibitor is to be used in combination with one or more of: an amylin receptor agonist (such as pramlintide), and/or a dual amylin + calcitonin receptor agonist, and/or a glucagon receptor agonist, and/or an FXR receptor agonist (such as cilofexor or obeticholic acid), and/or an FGF-21 analogue or FGF-21 receptor agonist (such as efruxifermin), and/or an FGF-19 analogue or FGF-19 receptor agonist (such as aldafermin), and/or a galectin 3 inhibitor (such as belapectin), and/or a PPARα agonist (such as elafibrinor), and/or a PPARγ agonist (such as pioglitazone or rosiglitazone), and/or a mixed PPARα and/or δ and/or γ agonist, and/or a pan PPAPαγδ agonist (such as lanafibranor), and/or an acetyl CoA desaturase activator, and/or an ASK1 inhibitor (such as selonsertib), and/or an LOXL2 inhibitor (such as simtuzumab), and/or a dual CCR2/5 inhibitor (such as cenicriviroc), and/or an inhibitor of an enzyme in the de novo lipogenesis (DNL) pathway including citrate/isocitrate carrier (CIC), ATP-citrate lyase (ACLY), acetyl-CoA carboxylase (ACC) and fatty acid synthase (FAS), and/or an inhibitor of an enzyme in the cholesterol biosynthesis pathway (such as an HMGCoA reductase inhibitor, such as atorvastatin).

5. An inhibitor according to claim 1 or claim 2, wherein said one or more ligand moieties comprise one or more GalNAc ligands or comprise one more GalNAc ligand derivatives.

6. An inhibitor or an inhibitor for use according to one or more preceding claims, wherein the target of the inhibitor is SLC25A5/ANT2.

7. An inhibitor, or inhibitor for use, according to one or more preceding claims, which is an siRNA oligomer having a first and a second strand wherein:
i) the first strand of the siRNA has a length in the range of 15 to 30 nucleosides, preferably 19 to 25 nucleosides, more preferably 23 or 25; even more preferably 23; and / or
ii) the second strand of the siRNA has a length in the range of 15 to 30 nucleosides, preferably 19 to 25 nucleosides, more preferably 21 nucleosides.

8. An inhibitor, or inhibitor for use according to one or more preceding claims, formulated as a pharmaceutical composition with an excipient and / or carrier.

9. A pharmaceutical composition comprising an inhibitor according to one or more preceding claims, in combination with a pharmaceutically acceptable excipient or carrier.

10. A pharmaceutical composition comprising an inhibitor according to one or more preceding claims, in combination with a pharmaceutically acceptable excipient or carrier, for use in prevention and/or treatment of metabolic disease or disorder, such as a metabolic disease or disorder associated with non-alcoholic fatty liver disease (NAFLD) and/or obesity and/or a disease or disorder associated with adipogenesis and/or for use in reducing adipogenesis.

11. Use of SLC25A5/ANT2 as a target for identifying one or more therapeutic agents for the treatment of a metabolic disease or disorder, such as a metabolic disease or disorder associated with non-alcoholic fatty liver disease (NAFLD) and/or obesity and/or a disease or disorder associated with adipogenesis and/or for use in reducing adipogenesis.

12. A method of treating or preventing a metabolic disease or disorder, such as a metabolic disease or disorder associated with non-alcoholic fatty liver disease (NAFLD) and/or obesity and/or a disease or disorder associated with adipogenesis and/or for use in reducing adipogenesis, the method comprising administering an inhibitor of SLC25A5/ANT2.

13. SLC25A5/ANT2 for use as a biomarker of a metabolic disease or disorder, such as a metabolic disease or disorder associated with non-alcoholic fatty liver disease (NAFLD) and/or obesity and/or a disease or disorder associated with adipogenesis and/or for reducing adipogenesis.

14. SLC25A5/ANT2 for use in an *in vivo* method of predicting susceptibility to a metabolic disease related to a disorder, such as a metabolic disease or disorder associated with non-alcoholic fatty liver disease (NAFLD) and/or obesity and/or a disease or disorder associated with adipogenesis and/or for use in reducing adipogenesis, typically by monitoring the sequence and/ or level of expression and / or function of SLC25A5/ANT2 in a sample obtained from a patient.

15. A method of predicting susceptibility to a metabolic disease or disorder, such as a metabolic disease or disorder associated with non-alcoholic fatty liver disease (NAFLD) and/or obesity and/or a disease or disorder associated with adipogenesis and/or in reducing adipogenesis in a patient, said method comprising:
(a) obtaining a sample from the patient,
(b) detecting the sequence and / or expression and / or function of SLC25A5/ANT2 in said sample obtained from the patient,
(c) predicting susceptibility to a disease or disorder, such as a metabolic disease or disorder associated with non-alcoholic fatty liver disease (NAFLD) and/or obesity and/or a disease or disorder associated with adipogenesis, based on the sequence and / or expression and / or function of SLC25A5/ANT2 in said sample obtained from the patient,
(d) preferably administering to the diagnosed patient an effective amount of an inhibitor of SLC25A5/ANT2.
